(19) Europäisches Patentamt
European Patent Office
Office européen des brevets

(11) EP 4 653 437 A2

(12) **EUROPEAN PATENT APPLICATION**
published in accordance with Art. 153(4) EPC

(43) Date of publication:
**26.11.2025 Bulletin 2025/48**

(21) Application number: **24744761.8**

(22) Date of filing: **02.01.2024**

(51) International Patent Classification (IPC):
***C07D 471/04*** *(2006.01)* ***A61K 31/4545*** *(2006.01)*
***A61K 31/4709*** *(2006.01)* ***A61K 31/4725*** *(2006.01)*
***A61K 31/5377*** *(2006.01)* ***A61K 31/496*** *(2006.01)*

(52) Cooperative Patent Classification (CPC):
**A61K 31/4545; A61K 31/4709; A61K 31/4725; A61K 31/496; A61K 31/5377; C07D 471/04**

(86) International application number:
**PCT/KR2024/000042**

(87) International publication number:
**WO 2024/154979 (25.07.2024 Gazette 2024/30)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **20.01.2023 KR 20230009009**

(71) Applicant: **Innovo Therapeutics Inc.**
**Seoul 04174 (KR)**

(72) Inventors:
- **KIM, Hak Joong**
  **Sejong 30150 (KR)**
- **KO, Seungyun**
  **Daejeon 34009 (KR)**
- **JEONG, Eunju**
  **Daejeon 35019 (KR)**
- **KIM, Tae Hun**
  **Daejeon 34130 (KR)**
- **RYU, Jaeyoon**
  **Daejeon 34002 (KR)**

(74) Representative: **Graf von Stosch Patentanwaltsgesellschaft mbH**
**Triftstraße 5**
**80538 München (DE)**

# (54) NOVEL HETEROCYCLIC COMPOUNDS

(57) The present invention provides novel heterocyclic compounds as 15-PGDH inhibitors, or hydrates, solvates or pharmaceutically acceptable salts thereof. The novel heterocyclic compounds of the present invention were confirmed to exhibit excellent 15-PGDH inhibitory activity, and thus, it has been found that these can be used for the prevention or treatment of 15-PGDH-related diseases.

Therefore, the novel heterocyclic compounds of the present invention can be effectively used for the prevention or treatment of 15-PGDH-related diseases in the medical and pharmaceutical fields.

EP 4 653 437 A2

## Description

## FIELD

This application claims priority to Korean Patent Application No. 10-2023-0009009, filed on January 20, 2023. The entire disclosure in the specification of which is incorporated herein by reference. The present invention relates to novel heterocyclic compounds as 15-PGDH inhibitors.

## BACKGROUND

Prostaglandins are physiologically active substances synthesized in the body, and are widely distributed in organs and body fluids, and perform physiological functions in extremely small amounts. Prostaglandins are fatty acid derivatives consisting of 20 carbons, and have a basic skeleton of prostanic acid with a 5-membered ring in the center of the molecular structure. They are classified into groups A to J according to the formula difference of the 5-membered ring, and into series 1 to 3 according to the number of double bonds in the two side chains. They are widely present in animal tissues, and are known to be rapidly metabolized after being synthesized from polyunsaturated fatty acids. These prostaglandins can stimulate smooth muscle contraction depending on their form, and in some animals, they lower or raise blood pressure, and reduce or increase blood coagulation, and they are also known to promote ion transport on the membrane, stimulate inflammation, and suppress cardiovascular disease and viral infection.

15-PGDH (15-hydroxyprostaglandin dehydrogenase) uses active prostaglandins such as PGD2, PGE1, PGE2, PGF2$\alpha$, and PGI2 as substrates, and is involved in the inactivation of these, for example, the conversion to 15-keto-PGE2, which catalyzes the oxidation reaction of the hydroxyl group at position 15 of PGE2.

The receptors corresponding to the substrates of 15-PGDH are widely distributed with different expression sites in the body, and are known to be involved in various physiological functions in the body. For example, the substrates of 15-PGDH include those having antifibrotic action, anti-inflammatory action, blood circulation improvement action, proliferation promotion action, stem cell increase promotion action, smooth muscle contraction/relaxation action, immunosuppressive action, and bone metabolism action. Therefore, 15-PGDH inhibitors can be effectively used in the treatment or prevention of diseases such as fibrosis [pulmonary fibrosis (such as idiopathic pulmonary fibrosis), liver fibrosis, renal fibrosis, myocardial fibrosis, scleroderma, myelofibrosis, etc.], inflammatory diseases [chronic obstructive pulmonary disease (COPD), acute lung injury, sepsis, exacerbation of asthma and lung disease, inflammatory bowel disease (ulcerative colitis, Crohn's disease, etc.), peptic ulcer (such as NSAID-induced ulcer), autoinflammatory diseases (such as Behcet disease), vasculitis syndrome, acute liver injury, acute kidney injury, nonalcoholic fatty liver disease (NASH), atopic dermatitis, psoriasis, interstitial cystitis, prostatitis syndrome (such as chronic prostatitis/chronic pelvic pain syndrome)], circulatory diseases [pulmonary hypertension, angina pectoris, myocardial infarction, heart failure, ischemic cardiac damage, chronic kidney disease, renal failure, stroke, peripheral circulatory disorder, etc.], wounds [diabetic ulcer, laceration, bedsore, acute mucosal injury including Stevens-Johnson syndrome, anticancer chemotherapy, immunotherapy or radiation, mucosal injury (mucositis or stomatitis) related to graft-versus-host disease, etc.], autoimmune diseases [multiple sclerosis, rheumatoid arthritis, etc.], graft-versus-host disease (GVHD), hair loss, osteoporosis, ear diseases [hearing loss, tinnitus, vertigo, balance disorder, etc.], ophthalmic diseases [glaucoma, dry eye, etc.], diabetes, underactive bladder, neutropenia, promotion of engraftment in stem cell and bone marrow transplantation or organ transplantation, neurogenesis and neuronal cell death [psychiatric disorders, nerve injury, neurotoxic disorder, neuropathic pain, neurodegeneration], muscle regeneration [muscular dystrophy, muscle damage], and cervical ripening.

## DETAILED DESCRIPTION OF THE INVENTION

## OBJECT OF THE INVENTION

The problem to be addressed by the present invention is to provide a novel 15-PGDH inhibitory compound having a structure that exhibits excellent 15-PGDH inhibitory activity.

Further, the problem to be addressed by the present invention is to provide a pharmaceutical composition for preventing or treating a 15-PGDH related disease, which comprises a 15-PGDH inhibitory compound having the novel structure.

Further, the problem to be addressed by the present invention is to provide a method for preventing or treating a 15-PGDH-related disease, which comprises administering a therapeutically effective amount of a 15-PGDH inhibitory compound having the novel structure to a subject in need thereof.

Further, the problem to be addressed by the present invention is to provide a use of the novel 15-PGDH inhibitory compound having the novel structure as an effective ingredient for the manufacture of a medicine for preventing or treating a 15-PGDH related disease.

The problems to be addressed by the present invention are not limited to the problems mentioned above, and other technical problems not mentioned can be clearly understood by a person having ordinary skill in the technical field to which the present invention belongs from the description below.

## SOLUTIONS TO THE PROBLEM

In order to address the above problem, according to one aspect of the present invention, there is provided a heterocyclic compound represented by the following Formula 1, a hydrate thereof, a solvate thereof or a pharmaceutically acceptable salt thereof:

<Formula 1>

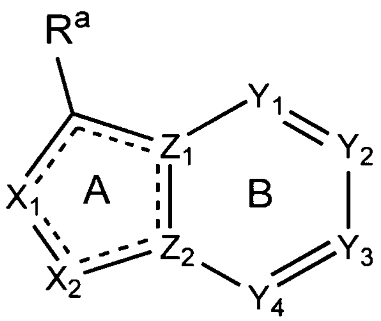

wherein,

a fused bicyclic ring of A ring and B ring is formed,
$X_1$, $Y_1$, $Y_2$, $Y_3$ and $Y_4$ are independently CH or N,
$X_2$ is CH, N or NH,
$Z_1$ and $Z_2$ are C or N,
at least one of $X_1$, $X_2$, $Y_1$, $Y_2$, $Y_3$, $Y_4$, $Z_1$ and $Z_2$ is N or NH,
$R^a$ is H, $C_{1-6}$ straight or branched chain alkyl substituted with $R^{a-1}$, or $C(O)R^{a-1}$,
$R^{a-1}$ is $C_{1-6}$ straight or branched chain alkyl, $C_{3-8}$ cycloalkyl, hydroxy, $R^s$, or $R^u$, and $R^{a-1}$ is optionally substituted with one or more independent $R^{a-2}$,
$R^{a-2}$ is $C_{1-6}$ straight or branched chain alkyl, halogen, $C_{1-4}$ haloalkyl,
$X_1$ is optionally substituted with $R^b$, wherein $R^b$ is amino, or $C(O)R^s$,
$Y_1$ is optionally substituted with $R^1$, $R^1$ is $R^f$,
$Y_2$ is optionally substituted with $R^2$,
$R^2$ is $C_{1-6}$ straight or branched chain alkyl, halogen, $C_{5-12}$ aryl with 1 to 2 ring(s), $R^u$, or $R^f$, and $R^2$ is optionally substituted with one or more independent $R^{2-1}$,
$R^{2-1}$ is $C_{1-4}$ alkoxy, halogen or hydroxy,
$Y_3$ is optionally substituted with $R^3$,
$R^3$ is $C_{5-7}$ aryl, $R^u$, or Rf, and $R^3$ is optionally substituted with one or more independent $R^{3-1}$,
$R^{3-1}$ is $C_{1-4}$ alkoxy, halogen, hydroxy or cyano,
$Y_4$ is optionally substituted with $R^4$ or $-L-R^4$,
L is $-NH(CH_2)_m-$, $-NHC(O)-$, $-NHSO_2-$ or -S-, m is an integer from 0 to 3,
$R^4$ is $C_{5-12}$ aryl with 1 to 2 ring(s), $R^u$, $R^s$, or $R^f$, and $R^4$ is optionally substituted with one or more independent Q,
Q is $C_{1-6}$ straight or branched chain alkyl, halogen, hydroxy, $C_{1-4}$ haloalkyl, $C_{1-4}$ alkoxy, nitro, cyano, amino, carboxylate, carboxylic acid, $CHR^5$, $CH_2R^5$, $NR^5R^6$, $C(O)R^5$, $C(O)OR^5$, $C(O)NR^5R^6$, $NHC(O)(CH_2)_pR^5$, $NHC(O)CH_2OR^5$, $NHC(O)CH_2NR^5R^6$, $NHCH_2CH_2R^5$, $NR^5C(O)R^6$, $R^u$, or $R^s$, p is an integer from 0 to 3, and Q is optionally substituted with one or more independent $R^7$,
$R^5$ and $R^6$ are independently H, $C_{1-6}$ straight or branched chain alkyl, $C_{3-8}$ cycloalkyl, $C_{1-4}$ alkoxy, amino, sulfonyl, $C_{5-7}$ aryl, arylalkyl, $R^u$, or $R^s$,
$R^7$ is $C_{1-6}$ straight or branched chain alkyl, $C_{3-8}$ cycloalkyl, halogen, $C_{1-4}$ haloalkyl, hydroxy, $C_{1-4}$ alkoxy, hydroxyalkyl, cyano, amino, alkylamino, $C_{1-6}$ alkylcarboxylate, nitro, $R^u$, $C_{1-4}$ alkyl substituted with $R^u$, or $R^s$, and the bond between Q and $R^7$ is a single bond or a double bond,
$R^s$ is a 4- to 10-membered saturated heterocycloalkyl ring with 1 to 2 ring(s) having 1 to 3 heteroatoms selected from N, O and S,

$R^u$ is a 5- to 7-membered unsaturated heterocycloalkyl ring having 1 to 4 heteroatoms selected from N, O and S,
$R^f$ is a fused bicyclic ring in which 5- to 7-membered saturated or unsaturated heterocycloalkyl ring having 1 to 2 heteroatoms selected from N, O and S is fused with an aryl ring or a 5- to 7-membered heteroaryl ring having 1 to 2 N,
$R^s$, $R^u$ and $R^f$ are independently optionally substituted with one to two oxo (O) or thioxo (S).

According to another aspect of the present invention, there is provided a pharmaceutical composition for preventing or treating a 15-PGDH-related disease, comprising a heterocyclic compound represented by the Formula 1, a hydrate thereof, a solvate thereof or a pharmaceutically acceptable salt thereof, as an active ingredient.

According to another aspect of the present invention, there is provided a method for preventing or treating a 15-PGDH-related disease, comprising administering a therapeutically effective amount of a heterocyclic compound represented by the Formula 1, a hydrate thereof, a solvate thereof or a pharmaceutically acceptable salt thereof, to a subject in need thereof.

According to another aspect of the present invention, there is provided a use of a heterocyclic compound represented by the Formula 1, a hydrate thereof, a solvate thereof or a pharmaceutically acceptable salt thereof, as an effective ingredient for the manufacture of a medicine for preventing or treating a 15-PGDH related disease.

According to another aspect of the present invention, there is provided a pharmaceutical composition comprising a heterocyclic compound represented by the Formula 1, a hydrate thereof, a solvate thereof or a pharmaceutically acceptable salt thereof, and a pharmaceutically acceptable additive.

## EFFECTS OF THE INVENTION

The novel heterocyclic compound of the present invention was confirmed to exhibit excellent 15-PGDH inhibitory activity, and thus, it has been found that these can be used for the prevention or treatment of 15-PGDH-related diseases.

Therefore, the novel heterocyclic compounds of the present invention can be effectively used for the prevention or treatment of 15-PGDH-related diseases in the medical and pharmaceutical fields.

The effects of the present invention are not limited to the effects described above, and should be understood to include all effects that can be inferred from the composition of the invention described in the description or claims of the present invention.

## BEST MODE OF THE INVENTION

The present invention provides a heterocyclic compound represented by the Formula 1, a hydrate thereof, a solvate thereof or a pharmaceutically acceptable salt thereof:

<Formula 1>

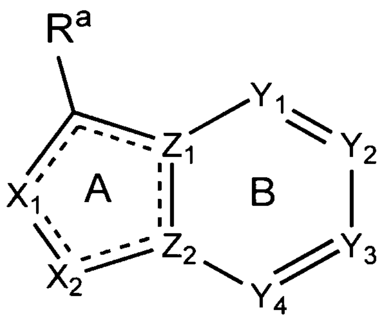

wherein,

a fused bicyclic ring of A ring and B ring is formed,
$X_1$, $Y_1$, $Y_2$, $Y_3$ and $Y_4$ are independently CH or N,
$X_2$ is CH, N or NH,
$Z_1$ and $Z_2$ are C or N,
at least one of $X_1$, $X_2$, $Y_1$, $Y_2$, $Y_3$, $Y_4$, $Z_1$ and $Z_2$ is N or NH,
$R^a$ is H, $C_{1-6}$ straight or branched chain alkyl substituted with $R^{a-1}$, or $C(O)R^{a-1}$,
$R^{a-1}$ is $C_{1-6}$ straight or branched chain alkyl, $C_{3-8}$ cycloalkyl, hydroxy, $R^s$, or $R^u$, and $R^{a-1}$ is optionally substituted with one or more independent $R^{a-2}$,
$R^{a-2}$ is $C_{1-6}$ straight or branched chain alkyl, halogen, $C_{1-4}$ haloalkyl,

$X_1$ is optionally substituted with $R^b$, wherein $R^b$ is amino, or C(O)$R^s$,
$Y_1$ is optionally substituted with $R^1$, $R^1$ is $R^f$,
$Y_2$ is optionally substituted with $R^2$,
$R^2$ is $C_{1-6}$ straight or branched chain alkyl, halogen, $C_{5-12}$ aryl with 1 to 2 ring(s), $R^u$, or $R^f$, and $R^2$ is optionally substituted with one or more independent $R^{2-1}$,
$R^{2-1}$ is $C_{1-4}$ alkoxy, halogen or hydroxy,
$Y_3$ is optionally substituted with $R^3$,
$R^3$ is $C_{5-7}$ aryl, $R^u$, or $R^f$, and $R^3$ is optionally substituted with one or more independent $R^{3-1}$,
$R^{3-1}$, is $C_{1-4}$ alkoxy, halogen, hydroxy or cyano,
$Y_4$ is optionally substituted with $R^4$ or -L-$R^4$,
L is $-NH(CH_2)_m-$, -NHC(O)-, $-NHSO_2-$ or -S-, m is an integer from 0 to 3,
$R^4$ is $C_{5-12}$ aryl with 1 to 2 ring(s), $R^u$, $R^s$, or $R^f$, and $R^4$ is optionally substituted with one or more independent Q,
Q is $C_{1-6}$ straight or branched chain alkyl, halogen, hydroxy, $C_{1-4}$ haloalkyl, $C_{1-4}$ alkoxy, nitro, cyano, amino, carboxylate, carboxylic acid, $CHR^5$, $CH_2R^5$, $NR^5R^6$, C(O)$R^5$, C(O)O$R^5$, C(O)N$R^5R^6$, NHC(O)$(CH_2)_pR^5$, NHC(O)$CH_2OR^5$, NHC(O)$CH_2NR^5R^6$, $NHCH_2CH_2R^5$, $NR^5C(O)R^6$, $R^u$, or $R^s$, p is an integer from 0 to 3, and Q is optionally substituted with one or more independent $R^7$,
$R^5$ and $R^6$ are independently H, $C_{1-6}$ straight or branched chain alkyl, $C_{3-8}$ cycloalkyl, $C_{1-4}$ alkoxy, amino, sulfonyl, $C_{5-7}$ aryl, arylalkyl, $R^u$, or $R^s$,
$R^7$ is $C_{1-6}$ straight or branched chain alkyl, $C_{3-8}$ cycloalkyl, halogen, $C_{1-4}$ haloalkyl, hydroxy, $C_{1-4}$ alkoxy, hydroxyalkyl, cyano, amino, alkylamino, $C_{1-6}$ alkylcarboxylate, nitro, $R^u$, $C_{1-4}$ alkyl substituted with $R^u$, or $R^s$, and the bond between Q and $R^7$ is a single bond or a double bond,
$R^s$ is a 4- to 10-membered saturated heterocycloalkyl ring with 1 to 2 ring(s) having 1 to 3 heteroatoms selected from N, O and S,
$R^u$ is a 5- to 7-membered unsaturated heterocycloalkyl ring having 1 to 4 heteroatoms selected from N, O and S,
$R^f$ is a fused bicyclic ring in which 5- to 7-membered saturated or unsaturated heterocycloalkyl ring having 1 to 2 heteroatoms selected from N, O and S is fused with an aryl ring or a 5- to 7-membered heteroaryl ring having 1 to 2 N,
$R^s$, $R^u$ and $R^f$ are independently optionally substituted with one to two oxo (O) or thioxo (S).

In one embodiment, the A ring may be pyrrole, pyrazole, imidazole, or triazole, and the B ring may be benzene, pyridine, pyrimidine, or triazine.

In one embodiment, the fused bicyclic ring of A ring and B ring may be indole, indazole, pyrrolopyridine, pyrrolopyrimidine, pyrrolotriazine, pyrazolopyridine, imidazopyridine, or triazolopyridine. more specifically, the fused bicyclic ring of A ring and B ring may be one selected from the group consisting of the following:

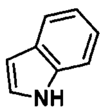 , 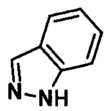 , 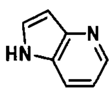 , 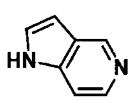 , 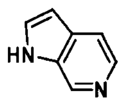 , 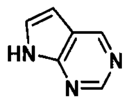 , 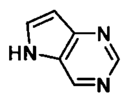 , 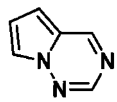 ,

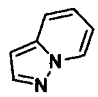 , 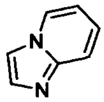 and 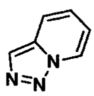 .

In one embodiment, $R^a$ may be H, propyl, C(O)$R^{a-1}$, $R^{a-1}$ may be methyl, butyl, cyclohexyl, hydroxy, piperidinyl, azaspiroheptanyl, azaspirooctanyl, azaspirononanyl, azabicycloheptanyl, azabicyclooctanyl, azabicyclononanyl or tetrahydropyridinyl, and $R^{a-2}$ may be methyl, ethyl, propyl, isopropyl, fluoro, or trifluoromethyl.

In one embodiment, $R^b$ may be amino or piperidinyl-carbonyl.

In one embodiment, $R^1$ may be benzodioxolyl.

In one embodiment, $R^2$ may be methyl, fluoro, phenyl, naphthyl (naphthalenyl), furanyl, pyridinyl, isoindolinone, benzodioxolyl, dihydrobenzodioxinyl, benzofuranyl or indazolyl, and $R^{2-1}$ may be methoxy, chloro, fluoro or hydroxy.

In one embodiment, $R^3$ may be phenyl, pyridinyl or benzodioxolyl, and $R^{3-1}$, may be methoxy, chloro, hydroxy or cyano.

In one embodiment, L may be -NH-, $-NHCH_2-$, $-NH(CH_2)_2-$, -NHC(O)-, $-NHSO_2-$ or -S-.

In one embodiment, $R^4$ may be phenyl, naphthyl (naphthalenyl), furanyl, pyrazolyl, dihydropyridinyl, pyridinyl, pyrimidinyl, pyridinone, pyrimidinone, isoxazolyl, piperidinyl, benzodioxolyl, isoindolinone, dihydroisoquinolinone, benzofuranyl, benzothiophenyl, indolyl, indazolyl, quinolinyl, quinolinone, isoquinolinone, dihydropyrrolopyridinone, benzoisoxazolone, dihydronaphthyridinone, benzoxazolyl, benzoisoxazolyl, triazolopyridinone, phthalazinone, quinazolinone,

or pyridopyrimidinone.

In one embodiment, Q may be methyl, propyl, isopropyl, fluoro, chloro, bromo, hydroxy, trifluoromethyl, methoxy, nitro, cyano, amino, carboxylate, carboxylic acid, $CHR^5$, $CH_2R^5$, $NR^5R^6$, $C(O)R^5$, $C(O)OR^5$, $C(O)NR^5R^6$, $NHC(O)R^5$, $NHC(O)CH_2R^5$, $NHC(O)(CH_2)_2R^5$, $NHC(O)CH_2OR^5$, $NHC(O)CH_2NR^5R^6$, $NHCH_2CH_2R^5$, $NR^5C(O)R^6$, tetrazolyl, oxadiazolyl, oxadiazolone, furyl, thienyl, imidazolyl, pyrazolyl, isoxazolyl, thiazolyl, oxo-thiadiazolyl, thioxo-oxadiazolyl, pyridinyl, pyrimidinyl, azetidinyl, oxazolidinone, piperidinyl, piperazinyl, morpholino, azaspiroheptanyl, azaspirooctanyl, azaspirononanyl, azabicycloheptanyl, or azabicyclooctanyl, $R^5$ may be H, methyl, ethyl, butyl, cyclopropyl, cyclohexane, methoxy, amino, phenyl, benzyl, imidazolyl, oxadiazolyl, oxadiazolone, pyridinyl, pyrimidinyl, azetidinyl, piperidinyl, piperidinone, piperazinyl, morpholino, thiazolidinedione, piperazinone, piperazine-dione, azaspiroheptanyl, or azaspirononanyl, and $R^6$ may be H, methyl, ethyl, propyl, isopropyl, sulfonyl, pyrazolyl, pyridinyl, pyridazinyl, azetidinyl or piperidinyl.

In one embodiment, $R^7$ may be methyl, propyl, isopropyl, cyclopropyl, fluoro, chloro, difluoromethyl, trifluoromethyl, hydroxy, methoxy, hydroxymethyl, cyano, amino, dimethylamino, methylcarboxylate, *tert*-butylcarboxylate, nitro, isoxazolyl, thiazolyl, pyridinyl, or oxadiazolylmethyl.

This specification uses the following definitions when defining the compounds of Formula 1 unless specifically defined.

The term "alkyl" refers to a straight or branched chain hydrocarbonyl group, preferably $C_1$-$C_{10}$ alkyl. Examples of alkyl include, but are not limited to, methyl, ethyl, *n*-propyl, *iso*-propyl, *n*-butyl, *iso*-butyl, *tert*-butyl, *n*-pentyl, *iso*-pentyl, *n*-hexyl, 3-methylhexyl, 2,2-dimethylpentyl, 2,3-dimethylpentyl, *n*-heptyl, *n*-octyl, *n*-nonyl, and *n*-decyl, etc..

The term "cycloalkyl" refers to a partially or fully saturated single or fused ring hydrocarbon, preferably $C_3$-$C_{10}$-cycloalkyl. Examples of cycloalkyl include, but are not limited to, cyclopropyl, cyclobutyl, cyclopentyl, cyclohexyl, and cyclohexenyl.

The term "hydroxy" or "hydroxyl" is defined as -OH, and the term "alkoxy" means alkyloxy, a radical in which the hydrogen atom of the hydroxy group is substituted by 1 to 10 alkyl, unless otherwise defined.

The term "halogen" or "halo" refers to fluorine/fluorine (F), chlorine (Cl), bromine (Br), or iodine (I).

The terms "haloalkyl" and "haloalkoxy" mean alkyl or alkoxy substituted with one or more halogen atoms.

The term "heteroatom" means N, O, or S.

The term "aryl" means an aromatic hydrocarbon, including polycyclic aromatic ring systems in which a carbocyclic aromatic ring or a heteroaryl ring is fused with one or more other rings. Preferably it is $C_{5-12}$ aryl, more preferably $C_{5-10}$ aryl. For example, the aryl includes, but is not limited to, phenyl, naphthyl, tetrahydronaphthyl, and the like. Also included are groups in which a heteroaryl ring is fused to a cycloalkyl or a non-aromatic heterocyclic ring, such as indanyl or tetrahydrobenzopyranyl.

The term "heteroaryl" or "aromatic heterocycle" means a 3 to 12 membered, more preferably 5 to 10 membered aromatic hydrocarbon group having one or more heteroatoms selected from N, O and S as a ring atom, and forming a single or fused ring which may be fused with benzo or $C_{3-8}$ cycloalkyl. For example, the heteroaryl includes, but is not limited to, pyrrolyl, imidazolyl, pyrazolyl, triazolyl, pyridyl, pyrazinyl, pyrimidyl, pyridazinyl, triazinyl, oxadiazolyl, isoxadiazolyl, tetrazolyl, indolyl, indazolyl, isoxazolyl, oxazolyl, thiazolyl, isothiazolyl, furanyl, benzofuranyl, thiophenyl, benzothiazolyl, benzoxazolyl, benzimidazolyl, quinolinyl, isoquinolinyl, and the like.

Representative examples of the heterocyclic compounds according to the present invention are as follows:

[1] 7-phenylpyrazolo[1,5-*a*]pyridin-3-yl)(piperidin-1-yl)methanone,
[2] (7-(3-chlorophenyl)pyrazolo[1,5-*a*]pyridin-3-yl)(piperidin-1-yl)methanone,
[3] methyl 3-(3-(piperidine-1-carbonyl)pyrazolo[1,5-*a*]pyridin-7-yl)benzoate,
[4] (7-(3-methoxyphenyl)pyrazolo[1,5-*a*]pyridin-3-yl)(piperidin-1-yl)methanone,
[5] (7-(3-nitrophenyl)pyrazolo[1,5-*a*]pyridin-3-yl)(piperidin-1-yl)methanone,
[6] 3-(3-(piperidine-1-carbonyl)pyrazolo[1,5-*a*]pyridin-7-yl)benzonitrile,
[7] (7-(3-aminophenyl)pyrazolo[1,5-*a*]pyridin-3-yl)(piperidin-1-yl)methanone,
[8] (7-(2-chloropyridin-4-yl)pyrazolo[1,5-*a*]pyridin-3-yl)(piperidin-1-yl)methanone,
[9] (7-(5-aminopyridin-3-yl)pyrazolo[1,5-*a*]pyridin-3-yl)(piperidin-1-yl)methanone,
[10] 4-(3-(piperidine-1-carbonyl)pyrazolo[1,5-*a*]pyridin-7-yl)benzoic acid,
[11] methyl 4-(3-(piperidine-1-carbonyl)pyrazolo[1,5-*a*]pyridin-7-yl)benzoate,
[12] (7-(4-chlorophenyl)pyrazolo[1,5-*a*]pyridin-3-yl)(piperidin-1-yl)methanone,
[13] (7-(4-nitrophenyl)pyrazolo[1,5-*a*]pyridin-3-yl)(piperidin-1-yl)methanone,
[14] (7-(4-aminophenyl)pyrazolo[1,5-*a*]pyridin-3-yl)(piperidin-1-yl)methanone,
[15] 4-(3-(piperidine-1-carbonyl)pyrazolo[1,5-*a*]pyridin-7-yl)benzonitrile,
[16] (7-(6-nitropyridin-3-yl)pyrazolo[1,5-*a*]pyridin-3-yl)(piperidin-1-yl)methanone,
[17] (7-(6-aminopyridin-3-yl)pyrazolo[1,5-*a*]pyridin-3-yl)(piperidin-1-yl)methanone,
[18] 5-(3-(piperidine-1-carbonyl)pyrazolo[1,5-*a*]pyridin-7-yl)picolinonitrile,

[19] (7-(6-fluoropyridin-3-yl)pyrazolo[1,5-*a*]pyridin-3-yl)(piperidin-1-yl)methanone,
[20] (7-(4-methoxy-3-(trifluoromethyl)phenyl)pyrazolo[1,5-*a*]pyridin-3-yl)(piperidin-1-yl)methanone,
[21] (7-(3-chloro-4-hydroxyphenyl)pyrazolo[1,5-*a*]pyridin-3-yl)(piperidin-1-yl)methanone],
[22] 2-fluoro-4-(3-(piperidine-1-carbonyl)pyrazolo[1,5-*a*]pyridin-7-yl)benzonitrile,
[23] 2-(dimethylamino)-4-(3-(piperidine-1-carbonyl)pyrazolo[1,5-*a*]pyridin-7-yl)benzonitrile,
[24] (7-(3,4-dimethoxyphenyl)pyrazolo[1,5-*a*]pyridin-3-yl)(piperidin-1-yl)methanone,
[25] (7-(1-methyl-1*H*-pyrazol-5-yl)pyrazolo[1,5-*a*]pyridin-3-yl)(piperidin-1-yl)methanone,
[26] (7-(3-(morpholine-4-carbonyl)phenyl)pyrazolo[1,5-*a*]pyridin-3-yl)(piperidin-1-yl)methanone,
[27] *N*-methyl-3-(3-(piperidine-1-carbonyl)pyrazolo[1,5-*a*]pyridin-7-yl)benzamide,
[28] *N*-isopropyl-3-(3-(piperidine-1-carbonyl)pyrazolo[1,5-*a*]pyridin-7-yl)benzamide,
[29] azetidin-1-yl(3-(3-(piperidine-1-carbonyl)pyrazolo[1,5-*a*]pyridin-7-yl)phenyl)methanone,
[30] *N*-(2-methoxyethyl)-3-(3-(piperidine-1-carbonyl)pyrazolo[1,5-*a*]pyridin-7-yl)benzamide,
[31] *N*-(2-(dimethylamino)ethyl)-3-(3-(piperidine-1-carbonyl)pyrazolo[1,5-*a*]pyridin-7-yl)benzamide,
[32] *N*-(cyclopropylmethyl)-3-(3-(piperidine-1-carbonyl)pyrazolo[1,5-*a*]pyridin-7-yl)benzamide,
[33] *N*-(1-methylpiperidin-4-yl)-3-(3-(piperidine-1-carbonyl)pyrazolo[1,5-*a*]pyridin-7-yl)benzamide,
[34] piperazin-1-yl(3-(3-(piperidine-1-carbonyl)pyrazolo[1,5-*a*]pyridin-7-yl)phenyl)methanone,
[35] *tert*-butyl 4-(3-(3-(piperidine-1-carbonyl)pyrazolo[1,5-*a*]pyridin-7-yl)benzoyl)piperazine-1-carboxylate,
[36] *N*-(2-cyanoethyl)-3-(3-(piperidine-1-carbonyl)pyrazolo[1,5-*a*]pyridin-7-yl)benzamide,
[37] 3-(3-(piperidine-1-carbonyl)pyrazolo[1,5-*a*]pyridin-7-yl)-*N*-(pyridin-3-yl)benzamide,
[38] 3-(3-(piperidine-1-carbonyl)pyrazolo[1,5-*a*]pyridin-7-yl)-*N*-(pyridin-4-yl)benzamide,
[39] (7-(5-(morpholine-4-carbonyl)pyridin-3-yl)pyrazolo[1,5-*a*]pyridin-3-yl)(piperidin-1-yl)methanone,
[40] *N*-methyl-5-(3-(piperidine-1-carbonyl)pyrazolo[1,5-*a*]pyridin-7-yl)nicotinamide,
[41] azetidin-1-yl(5-(3-(piperidine-1-carbonyl)pyrazolo[1,5-*a*]pyridin-7-yl)pyridin-3-yl)methanone,
[42] (3-hydroxyazetidin-1-yl)(5-(3-(piperidine-1-carbonyl)pyrazolo[1,5-*a*]pyridin-7-yl)pyridin-3-yl)methanone,
[43] (7-(5-(2-azaspiro[3.3]heptane-2-carbonyl)pyridin-3-yl)pyrazolo[1,5-*a*]pyridin-3-yl)(piperidin-1-yl)methanone,
[44] (3,3-difluoroazetidin-1-yl)(5-(3-(piperidine-1-carbonyl)pyrazolo[1,5-*a*]pyridin-7-yl)pyridin-3-yl)methanone,
[45] *N*-(2-methoxyethyl)-5-(3-(piperidine-1-carbonyl)pyrazolo[1,5-*a*]pyridin-7-yl)nicotinamide,
[46] *N*-(cyclopropylmethyl)-5-(3-(piperidine-1-carbonyl)pyrazolo[1,5-*a*]pyridin-7-yl)nicotinamide,
[47] *N*-(1-methylpiperidin-4-yl)-5-(3-(piperidine-1-carbonyl)pyrazolo[1,5-*a*]pyridin-7-yl)nicotinamide,
[48] piperazin-1-yl(5-(3-(piperidine-1-carbonyl)pyrazolo[1,5-*a*]pyridin-7-yl)pyridin-3-yl)methanone,
[49] (4-methylpiperazin-1-yl)(5-(3-(piperidine-1-carbonyl)pyrazolo[1,5-*a*]pyridin-7-yl)pyridin-3-yl)methanone,
[50] (4-isopropylpiperazin-1-yl)(5-(3-(piperidine-1-carbonyl)pyrazolo[1,5-*a*]pyridin-7-yl)pyridin-3-yl)methanone,
[51] *N*-(2-cyanoethyl)-5-(3-(piperidine-1-carbonyl)pyrazolo[1,5-*a*]pyridin-7-yl)nicotinamide,
[52] *N*-(cyanomethyl)-5-(3-(piperidine-1-carbonyl)pyrazolo[1,5-*a*]pyridin-7-yl)nicotinamide,
[53] 5-(3-(piperidine-1-carbonyl)pyrazolo[1,5-*a*]pyridin-7-yl)-*N*-(pyridin-3-yl)nicotinamide,
[54] 5-(3-(piperidine-1-carbonyl)pyrazolo[1,5-*a*]pyridin-7-yl)-*N*-(pyridin-4-yl)nicotinamide,
[55] *N*-(isoxazol-3-ylmethyl)-5-(3-(piperidine-1-carbonyl)pyrazolo[1,5-*a*]pyridin-7-yl)nicotinamide,
[56] 5-(3-(piperidine-1-carbonyl)pyrazolo[1,5-*a*]pyridin-7-yl)-*N*-(pyridazin-4-yl)nicotinamide,
[57] 5-(3-(piperidine-1-carbonyl)pyrazolo[1,5-a]pyridin-7-yl)-*N*-(thiazol-5-ylmethyl)nicotinamide,
[58] *N*-methyl-4-(3-(piperidine-1-carbonyl)pyrazolo[1,5-*a*]pyridin-7-yl)benzamide,
[59] *N,N*-dimethyl-4-(3-(piperidine-1-carbonyl)pyrazolo[1,5-*a*]pyridin-7-yl)benzamide,
[60] *N*-isopropyl-4-(3-(piperidine-1-carbonyl)pyrazolo[1,5-*a*]pyridin-7-yl)benzamide,
[61] azetidin-1-yl(4-(3-(piperidine-1-carbonyl)pyrazolo[1,5-*a*]pyridin-7-yl)phenyl)methanone,
[62] *N*-(2-methoxyethyl)-4-(3-(piperidine-1-carbonyl)pyrazolo[1,5-*a*]pyridin-7-yl)benzamide,
[63] *N*-(2-(dimethylamino)ethyl)-4-(3-(piperidine-1-carbonyl)pyrazolo[1,5-a]pyridin-7-yl)benzamide,
[64] *N*-(cyclopropylmethyl)-4-(3-(piperidine-1-carbonyl)pyrazolo[1,5-*a*]pyridin-7-yl)benzamide,
[65] *N*-(1-methylpiperidin-4-yl)-4-(3-(piperidine-1-carbonyl)pyrazolo[1,5-*a*]pyridin-7-yl)benzamide,
[66] piperazin-1-yl(4-(3-(piperidine-1-carbonyl)pyrazolo[1,5-*a*]pyridin-7-yl)phenyl)methanone,
[67] (4-methylpiperazin-1-yl)(4-(3-(piperidine-1-carbonyl)pyrazolo[1,5-*a*]pyridin-7-yl)phenyl)methanone,
[68] (4-isopropylpiperazin-1-yl)(4-(3-(piperidine-1-carbonyl)pyrazolo[1,5-*a*]pyridin-7-yl)phenyl)methanone,
[69] *N*-(2-cyanoethyl)-4-(3-(piperidine-1-carbonyl)pyrazolo[1,5-*a*]pyridin-7-yl)benzamide,
[70] *N*-(cyanomethyl)-4-(3-(piperidine-1-carbonyl)pyrazolo[1,5-*a*]pyridin-7-yl)benzamide,
[71] 4-(3-(piperidine-1-carbonyl)pyrazolo[1,5-a]pyridin-7-yl)-*N*-(pyridin-3-yl)benzamide,
[72] 4-(3-(piperidine-1-carbonyl)pyrazolo[1,5-a]pyridin-7-yl)-*N*-(pyridin-4-yl)benzamide,
[73] *N*-(isoxazol-3-ylmethyl)-4-(3-(piperidine-1-carbonyl)pyrazolo[1,5-*a*]pyridin-7-yl)benzamide,
[74] (7-(6-(morpholine-4-carbonyl)pyridin-3-yl)pyrazolo[1,5-*a*]pyridin-3-yl)(piperidin-1-yl)methanone,
[75] *N*-methyl-5-(3-(piperidine-1-carbonyl)pyrazolo[1,5-*a*]pyridin-7-yl)picolinamide,
[76] *N*-isopropyl-5-(3-(piperidine-1-carbonyl)pyrazolo[1,5-*a*]pyridin-7-yl)picolinamide,

[77] azetidin-1-yl(5-(3-(piperidine-1-carbonyl)pyrazolo[1,5-*a*]pyridin-7-yl)pyridin-2-yl)methanone,
[78] *N*-(2-methoxyethyl)-5-(3-(piperidine-1-carbonyl)pyrazolo[1,5-*a*]pyridin-7-yl)picolinamide,
[79] *N*-(2-(dimethylamino)ethyl)-5-(3-(piperidine-1-carbonyl)pyrazolo[1,5-*a*]pyridin-7-yl)picolinamide,
[80] *N*-(cyclopropylmethyl)-5-(3-(piperidine-1-carbonyl)pyrazolo[1,5-*a*]pyridin-7-yl)picolinamide,
[81] *N*-(1-methylpiperidin-4-yl)-5-(3-(piperidine-1-carbonyl)pyrazolo[1,5-*a*]pyridin-7-yl)picolinamide,
[82] piperazin-1-yl(5-(3-(piperidine-1-carbonyl)pyrazolo[1,5-*a*]pyridin-7-yl)pyridin-2-yl)methanone,
[83] (4-methylpiperazin-1-yl)(5-(3-(piperidine-1-carbonyl)pyrazolo[1,5-*a*]pyridin-7-yl)pyridin-2-yl)methanone,
[84] (4-isopropylpiperazin-1-yl)(5-(3-(piperidine-1-carbonyl)pyrazolo[1,5-*a*]pyridin-7-yl)pyridin-2-yl)methanone,
[85] *N*-(2-cyanoethyl)-5-(3-(piperidine-1-carbonyl)pyrazolo[1,5-*a*]pyridin-7-yl)picolinamide,
[86] *N*-(cyanomethyl)-5-(3-(piperidine-1-carbonyl)pyrazolo[1,5-*a*]pyridin-7-yl)picolinamide,
[87] 5-(3-(piperidine-1-carbonyl)pyrazolo[1,5-*a*]pyridin-7-yl)-*N*-(pyridin-3-yl)picolinamide,
[88] 5-(3-(piperidine-1-carbonyl)pyrazolo[1,5-*a*]pyridin-7-yl)-*N*-(pyridin-4-yl)picolinamide,
[89] *N*-(isoxazol-3-ylmethyl)-5-(3-(piperidine-1-carbonyl)pyrazolo[1,5-*a*]pyridin-7-yl)picolinamide,
[90] *N*-(2-cyanoethyl)-5-(3-(piperidine-1-carbonyl)pyrazolo[1,5-*a*]pyridin-7-yl)furan-2-carboxamide,
[91] 3-methoxy-*N*-(3-(3-(piperidine-1-carbonyl)pyrazolo[1,5-*a*]pyridin-7-yl)phenyl)propanamide,
[92] 1-(3-(3-(piperidine-1-carbonyl)pyrazolo[1,5-*a*]pyridin-7-yl)phenyl)urea,
[93] 1-methyl-*N*-(3-(3-(piperidine-1-carbonyl)pyrazolo[1,5-*a*]pyridin-7-yl)phenyl)piperidine-4-carboxamide,
[94] *N*-(3-(3-(piperidine-1-carbonyl)pyrazolo[1,5-*a*]pyridin-7-yl)phenyl)cyclopropanecarboxamide,
[95] *N*-(3-(3-(piperidine-1-carbonyl)pyrazolo[1,5-*a*]pyridin-7-yl)phenyl)nicotinamide,
[96] *N*-(3-(3-(piperidine-1-carbonyl)pyrazolo[1,5-*a*]pyridin-7-yl)phenyl)cyclohexanecarboxamide,
[97] *N*-(4-(3-(piperidine-1-carbonyl)pyrazolo[1,5-*a*]pyridin-7-yl)phenyl)acetamide,
[98] 3-methoxy-*N*-(4-(3-(piperidine-1-carbonyl)pyrazolo[1,5-*a*]pyridin-7-yl)phenyl)propanamide,
[99] 1-methyl-*N*-(4-(3-(piperidine-1-carbonyl)pyrazolo[1,5-*a*]pyridin-7-yl)phenyl)piperidine-4-carboxamide,
[100] *N*-(4-(3-(piperidine-1-carbonyl)pyrazolo[1,5-*a*]pyridin-7-yl)phenyl)cyclopropanecarboxamide,
[101] *N*-(4-(3-(piperidine-1-carbonyl)pyrazolo[1,5-*a*]pyridin-7-yl)phenyl)nicotinamide,
[102] *N*-(4-(3-(piperidine-1-carbonyl)pyrazolo[1,5-*a*]pyridin-7-yl)phenyl)cyclohexanecarboxamide,
[103] 3-methoxy-*N*-(5-(3-(piperidine-1-carbonyl)pyrazolo[1,5-*a*]pyridin-7-yl)pyridin-2-yl)propanamide,
[104] 2-(dimethylamino)-*N*-(5-(3-(piperidine-1-carbonyl)pyrazolo[1,5-*a*]pyridin-7-yl)pyridin-2-yl)acetamide,
[105] 2-oxo-*N*-(S-(3-(piperidine-1-carbonyl)pyrazolo[1,5-*a*]pyridin-7-yl)pyridin-2-yl)piperidine-4-carboxamide,
[106] *N*-(5-(3-(piperidine-1-carbonyl)pyrazolo[1,5-*a*]pyridin-7-yl)pyridin-2-yl)-2-(pyridin-3-yl)acetamide,
[107] (7-(6-((3-methoxypropyl)amino)pyridin-3-yl)pyrazolo[1,5-*a*]pyridin-3-yl)(piperidin-1-yl)methanone,
[108] (7-(6-morpholinopyridin-3-yl)pyrazolo[1,5-*a*]pyridin-3-yl)(piperidin-1-yl)methanone,
[109] (7-(6-(isopropylamino)pyridin-3-yl)pyrazolo[1,5-*a*]pyridin-3-yl)(piperidin-1-yl)methanone,
[110] piperidin-1-yl(7-(6-(piperidin-1-yl)pyridin-3-yl)pyrazolo[1,5-*a*]pyridin-3-yl)methanone,
[111] (7-(6-(4-methylpiperazin-1-yl)pyridin-3-yl)pyrazolo[1,5-*a*]pyridin-3-yl)(piperidin-1-yl)methanone,
[112] *N*-(5-(3-(piperidine-1-carbonyl)pyrazolo[1,5-*a*]pyridin-7-yl)pyridin-2-yl)methanesulfonamide,
[113] (7-(3-(1*H*-tetrazol-5-yl)phenyl)pyrazolo[1,5-*a*]pyridin-3-yl)(piperidin-1-yl)methanone,
[114] (7-(4-(1*H*-tetrazol-5-yl)phenyl)pyrazolo[1,5-*a*]pyridin-3-yl)(piperidin-1-yl)methanone,
[115] 3-(3-(3-(piperidine-1-carbonyl)pyrazolo[1,5-*a*]pyridin-7-yl)phenyl)-1,2,4-oxadiazol-5(4*H*)-one,
[116] 3-(4-(3-(piperidine-1-carbonyl)pyrazolo[1,5-*a*]pyridin-7-yl)phenyl)-1,2,4-oxadiazol-5(4H)-one,
[117] 3-(5-(3-(piperidine-1-carbonyl)pyrazolo[1,5-*a*]pyridin-7-yl)pyridin-2-yl)-1,2,4-oxadiazol-5(4*H*)-one,
[118] (7-(4-(5-methyl-1,2,4-oxadiazol-3-yl)phenyl)pyrazolo[1,5-*a*]pyridin-3-yl)(piperidin-1-yl)methanone,
[119] (*Z*)-5-(3-(3-(piperidine-1-carbonyl)pyrazolo[1,5-*a*]pyridin-7-yl)benzylidene)thiazolidine-2,4-dione,
[120] *tert*-butyl 4-(3-(piperidine-1-carbonyl)pyrazolo[1,5-*a*]pyridin-7-yl)-3,6-dihydropyridine-1(2*H*)-carboxylate,
[121] (7-(benzo[*d*][1,3]dioxol-5-yl)pyrazolo[1,5-*a*]pyridin-3-yl)(piperidin-1-yl)methanone,
[122] (7-(benzofuran-3-yl)pyrazolo[1,5-*a*]pyridin-3-yl)(piperidin-1-yl)methanone,
[123] (7-(benzofuran-2-yl)pyrazolo[1,5-*a*]pyridin-3-yl)(piperidin-1-yl)methanone,
[124] (7-(benzo[*b*]thiophen-3-yl)pyrazolo[1,5-*a*]pyridin-3-yl)(piperidin-1-yl)methanone,
[125] (7-(1*H*-indazol-6-yl)pyrazolo[1,5-*a*]pyridin-3-yl)(piperidin-1-yl)methanone,
[126] (7-(1*H*-indol-5-yl)pyrazolo[1,5-*a*]pyridin-3-yl)(piperidin-1-yl)methanone,
[127] (7-(benzofuran-5-yl)pyrazolo[1,5-*a*]pyridin-3-yl)(piperidin-1-yl)methanone,
[128] (7-(naphthalen-1-yl)pyrazolo[1,5-*a*]pyridin-3-yl)(piperidin-1-yl)methanone,
[129] (7-(naphthalen-2-yl)pyrazolo[1,5-*a*]pyridin-3-yl)(piperidin-1-yl)methanone,
[130] piperidin-1-yl(7-(quinolin-3-yl)pyrazolo[1,5-*a*]pyridin-3-yl)methanone,
[131] 7-(3-(piperidine-1-carbonyl)pyrazolo[1,5-*a*]pyridin-7-yl)-3,4-dihydroisoquinolin-1(2*H*)-one,
[132] 2-methyl-7-(3-(piperidine-1-carbonyl)pyrazolo[1,5-*a*]pyridin-7-yl)-3,4-dihydroisoquinolin-1(2*H*)-one,
[133] 2-isopropyl-7-(3-(piperidine-1-carbonyl)pyrazolo[1,5-*a*]pyridin-7-yl)-3,4-dihydroisoquinolin-1(2*H*)-one,
[134] 7-(3-(piperidine-1-carbonyl)pyrazolo[1,5-*a*]pyridin-7-yl)isoquinolin-1(2*H*)-one,

[135] 2-methyl-7-(3-(piperidine-1-carbonyl)pyrazolo[1,5-*a*]pyridin-7-yl)isoquinolin-1(2*H*)-one,
[136] 6-(3-(piperidine-1-carbonyl)pyrazolo[1,5-*a*]pyridin-7-yl)-3,4-dihydroisoquinolin-1(2*H*)-one,
[137] 2-methyl-6-(3-(piperidine-1-carbonyl)pyrazolo[1,5-*a*]pyridin-7-yl)-3,4-dihydroisoquinolin-1(2*H*)-one,
[138] 6-(3-(piperidine-1-carbonyl)pyrazolo[1,5-*a*]pyridin-7-yl)isoquinolin-1(2*H*)-one,
[139] 2-methyl-6-(3-(piperidine-1-carbonyl)pyrazolo[1,5-*a*]pyridin-7-yl)isoquinolin-1(2*H*)-one,
[140] 6-(3-(piperidine-1-carbonyl)pyrazolo[1,5-*a*]pyridin-7-yl)isoindolin-1-one,
[141] 2-methyl-6-(3-(piperidine-1-carbonyl)pyrazolo[1,5-*a*]pyridin-7-yl)isoindolin-1-one,
[142] 2-isopropyl-6-(3-(piperidine-1-carbonyl)pyrazolo[1,5-*a*]pyridin-7-yl)isoindolin-1-one,
[143] 5-(3-(piperidine-1-carbonyl)pyrazolo[1,5-*a*]pyridin-7-yl)isoindolin-1-one,
[144] 2-methyl-5-(3-(piperidine-1-carbonyl)pyrazolo[1,5-*a*]pyridin-7-yl)isoindolin-1-one,
[145] 6-(3-(piperidine-1-carbonyl)pyrazolo[1,5-*a*]pyridin-7-yl)quinolin-2(1*H*)-one,
[146] 4-((3-(piperidine-1-carbonyl)pyrazolo[1,5-*a*]pyridin-7-yl)amino)benzonitrile,
[147] 3-((3-(piperidine-1-carbonyl)pyrazolo[1,5-*a*]pyridin-7-yl)amino)pyridin-2(1*H*)-one,
[148] piperidin-1-yl(7-((pyrimidin-2-ylmethyl)amino)pyrazolo[1,5-*a*]pyridin-3-yl)methanone,
[149] piperidin-1-yl(7-((2-(pyridin-4-yl)ethyl)amino)pyrazolo[1,5-*a*]pyridin-3-yl)methanone,
[150] (7-((1-methylpiperidin-4-yl)amino)pyrazolo[1,5-*a*]pyridin-3-yl)(piperidin-1-yl)methanone,
[151] 1-(7-(quinolin-3-yl)pyrazolo[1,5-*a*]pyridin-3-yl)pentan-1-one,
[152] 4-(3-acetylpyrazolo[1,5-*a*]pyridin-7-yl)benzonitrile,
[153] 4-(3-(2-hydroxypropan-2-yl)pyrazolo[1,5-*a*]pyridin-7-yl)benzonitrile,
[154] 7-(3-(4-fluoropiperidine-1-carbonyl)pyrazolo[1,5-*a*]pyridin-7-yl)-3,4-dihydroisoquinolin-1(2*H*)-one,
[155] 6-(3-(4-fluoropiperidine-1-carbonyl)pyrazolo[1,5-*a*]pyridin-7-yl)isoindolin-1-one,
[156] 4-(3-(4-fluoropiperidine-1-carbonyl)pyrazolo[1,5-*a*]pyridin-7-yl)benzonitrile,
[157] (4-fluoropiperidin-1-yl)(7-(4-nitrophenyl)pyrazolo[1,5-*a*]pyridin-3-yl)methanone,
[158] cyclohexyl(7-phenylpyrazolo[1,5-*a*]pyridin-3-yl)methanone,
[159] 7-(2-amino-3-(piperidine-1-carbonyl)pyrazolo[1,5-*a*]pyridin-7-yl)-3,4-dihydroisoquinolin-1(2*H*)-one,
[160] 7-(2-amino-3-(piperidine-1-carbonyl)pyrazolo[1,5-*a*]pyridin-7-yl)-2-methyl-3,4-dihydroisoquinolin-1(2*H*)-one,
[161] (7-(3-chlorophenyl)-1*H*-indazol-3-yl)(piperidin-1-yl)methanone,
[162] (8-(3-chlorophenyl)imidazo[1,2-*a*]pyridin-3-yl)(piperidin-1-yl)methanone,
[163] (8-(4-nitrophenyl)imidazo[1,2-*a*]pyridin-3-yl)(piperidin-1-yl)methanone,
[164] (8-(4-aminophenyl)imidazo[1,2-*a*]pyridin-3-yl)(piperidin-1-yl)methanone,
[165] (8-(3-chlorophenyl)-6-methylimidazo[1,2-*a*]pyridin-3-yl)(piperidin-1-yl)methanone,
[166] (8-(2-chloropyridin-4-yl)-6-methylimidazo[1,2-*a*]pyridin-3-yl)(piperidin-1-yl)methanone,
[167] (8-(3-chlorophenyl)-6-fluoroimidazo[1,2-*a*]pyridin-3-yl)(piperidin-1-yl)methanone,
[168] (7-(4-methoxyphenyl)-[1,2,3]triazolo[1,5-*a*]pyridin-3-yl)(piperidin-1-yl)methanone,
[169] (7-(3-chlorophenyl)-1*H*-indol-3-yl)(piperidin-1-yl)methanone,
[170] (6-(3-chlorophenyl)pyrazolo[1,5-*a*]pyridin-3-yl)(piperidin-1-yl)methanone,
[171] (6-(4-methoxyphenyl)pyrazolo[1,5-*a*]pyridin-3-yl)(piperidin-1-yl)methanone,
[172] (6-(benzo[d][1,3]dioxol-5-yl)-1*H*-pyrrolo[3,2-*b*]pyridin-2-yl)(piperidin-1-yl)methanone,
[173] (5-(benzo[d][1,3]dioxol-5-yl)-1*H*-pyrrolo[3,2-*b*]pyridin-2-yl)(piperidin-1-yl)methanone,
[174] (4-(benzo[d][1,3]dioxol-5-yl)-1*H*-pyrrolo[3,2-*c*]pyridin-2-yl)(piperidin-1-yl)methanone,
[175] (4-(benzo[d][1,3]dioxol-5-yl)-1*H*-pyrrolo[2,3-*c*]pyridin-2-yl)(piperidin-1-yl)methanone,
[176] (4-(benzo[d][1,3]dioxol-5-yl)pyrrolo[2,1-*f*][1,2,4]triazin-6-yl)(piperidin-1-yl)methanone,
[177] (4-(benzo[d][1,3]dioxol-5-yl)-7*H*-pyrrolo[2,3-*d*]pyrimidin-6-yl)(piperidin-1-yl)methanone,
[178] (5-(2,3-dihydrobenzo[b][1,4]dioxin-6-yl)-1*H*-pyrrolo[3,2-*b*]pyridin-2-yl)(piperidin-1-yl)methanone,
[179] (5-(4-methoxyphenyl)-1*H*-pyrrolo[3,2-*b*]pyridin-2-yl)(piperidin-1-yl)methanone,
[180] (5-(4-chlorophenyl)-1*H*-pyrrolo[3,2-*b*]pyridin-2-yl)(piperidin-1-yl)methanone,
[181] (5-(3,4-dimethoxyphenyl)-1*H*-pyrrolo[3,2-*b*]pyridin-2-yl)(piperidin-1-yl)methanone,
[182] (5-(4-fluorophenyl)-1*H*-pyrrolo[3,2-*b*]pyridin-2-yl)(piperidin-1-yl)methanone,
[183] (5-(furan-3-yl)-1*H*-pyrrolo[3,2-*b*]pyridin-2-yl)(piperidin-1-yl)methanone,
[184] piperidin-1-yl(5-(pyridin-4-yl)-1*H*-pyrrolo[3,2-*b*]pyridin-2-yl)methanone,
[185] (5-(3-chlorophenyl)-1*H*-pyrrolo[3,2-*b*]pyridin-2-yl)(piperidin-1-yl)methanone,
[186] (5-(3-chloro-4-hydroxyphenyl)-1*H*-pyrrolo[3,2-*b*]pyridin-2-yl)(piperidin-1-yl)methanone,
[187] (2-(benzo[*d*][1,3]dioxol-5-yl)-5*H*-pyrrolo[3,2-*d*]pyrimidin-6-yl)(piperidin-1-yl)methanone,
[188] (5-(benzofuran-3-yl)-1*H*-pyrrolo[3,2-*b*]pyridin-2-yl)(piperidin-1-yl)methanone,
[189] (5-(naphthalen-2-yl)-1*H*-pyrrolo[3,2-*b*]pyridin-2-yl)(piperidin-1-yl)methanone,
[190] (5-(1*H*-indazol-6-yl)-1*H*-pyrrolo[3,2-*b*]pyridin-2-yl)(piperidin-1-yl)methanone,
[191] 2-(2-(piperidine-1-carbonyl)-1*H*-pyrrolo[3,2-*b*]pyridin-5-yl)isoindolin-1-one,
[192] (3-fluoroazetidin-1-yl)(5-(3-(piperidine-1-carbonyl)pyrazolo[1,5-*a*]pyridin-7-yl)pyridin-3-yl)methanone,

[193] *N*-(1-methyl-1H-pyrazol-3-yl)-5-(3-(piperidine-1-carbonyl)pyrazolo[1,5-*a*]pyridin-7-yl)nicotinamide,
[194] (7-((3-methoxyphenyl)thio)pyrazolo[1,5-*a*]pyridin-3-yl)(piperidin-1-yl)methanone,
[195] 3-(3-(piperidine-1-carbonyl)pyrazolo[1,5-*a*]pyridin-7-yl)-7,8-dihydro-1,6-naphthyridin-5(6*H*)-one,
[196] 2-(dimethylamino)-*N*-(5-(3-(piperidine-1-carbonyl)pyrazolo[1,5-*a*]pyridin-7-yl)pyridin-3-yl)acetamide,
[197] 3-methoxy-*N*-(5-(3-(piperidine-1-carbonyl)pyrazolo[1,5-*a*]pyridin-7-yl)pyridin-3-yl)propenamide,
[198] 2-oxo-*N*-(5-(3-(piperidine-1-carbonyl)pyrazolo[1,5-*a*]pyridin-7-yl)pyridin-3-yl)piperidine-4-carboxamide,
[199] 6-methyl-3-(3-(piperidine-1-carbonyl)pyrazolo[1,5-*a*]pyridin-7-yl)-7,8-dihydro-1,6-naphthyridin-5(6*H*)-one,
[200] *N*-(azetidin-3-yl)-5-(3-(piperidine-1-carbonyl)pyrazolo[1,5-a]pyridin-7-yl)nicotinamide,
[201] *N*-methylsulfonyl-5-[3-(piperidine-1-carbonyl)pyrazolo[1,5-a]pyridin-7-yl]pyridine-3-carboxamide,
[202] *N*-(1-methylazetidin-3-yl)-5-[3-(piperidine-1-carbonyl)pyrazolo[1,5-*a*]pyridin-7-yl]pyridine-3-carboxamide,
[203] 4-[5-[3-(piperidine-1-carbonyl)pyrazolo[1,5-*a*]pyridin-7-yl]pyridine-3-carbonyl]piperazin-2-one,
[204] 4-[5-[3-(piperidine-1-carbonyl)pyrazolo[1,5-*a*]pyridin-7-yl]pyridine-3-carbonyl]piperazine-2,6-dione,
[205] 3-fluoro-5-[3-(piperidine-1-carbonyl)pyrazolo[1,5-*a*]pyridin-7-yl]pyridine-2-carbonitrile,
[206] (7-(6-(azetidin-1-yl)pyridin-3-yl)pyrazolo[1,5-*a*]pyridin-3-yl)(piperidin-1-yl)methanone,
[207] 3-methyl-6-(3-(piperidine-1-carbonyl)pyrazolo[1,5-*a*]pyridin-7-yl)pyrimidin-4(3H)-one,
[208] (7-(6-(3-fluoroazetidin-1-yl)pyridin-3-yl)pyrazolo[1,5-*a*]pyridin-3-yl)(piperidin-1-yl)methanone,
[209] (7-(6-(3,3-difluoroazetidin-1-yl)pyridin-3-yl)pyrazolo[1,5-*a*]pyridin-3-yl)(piperidin-1-yl)methanone,
[210] (7-(6-(3-hydroxyazetidin-1-yl)pyridin-3-yl)pyrazolo[1,5-*a*]pyridin-3-yl)(piperidin-1-yl)methanone,
[211] 7-[[3-(piperidine-1-carbonyl)pyrazolo[1,5-*a*]pyridin-7-yl]amino]-3,4-dihydro-2H-isoquinolin-1-one,
[212] 6-[[3-(piperidine-1-carbonyl)pyrazolo[1,5-*a*]pyridin-7-yl]amino]isoindolin-1-one,
[213] (3-fluoroazetidin-1-yl)-[5-[3-(piperidine-1-carbonyl)pyrazolo[1,5-*a*]pyridin-7-yl]-2-pyridyl]methanone,
[214] 2-azaspiro[3.3]heptan-2-yl-[5-[3-(piperidine-1-carbonyl)pyrazolo[1,5-*a*]pyridin-7-yl]-2-pyridyl]methanone,
[215] (7-(6-(2-azabicyclo[2.2.1]heptan-2-yl)pyridin-3-yl)pyrazolo[1,5-*a*]pyridin-3-yl)(piperidin-1-yl)methanone,
[216] (7-(6-(2-azabicyclo[2.2.2]octan-2-yl)pyridin-3-yl)pyrazolo[1,5-*a*]pyridin-3-yl)(piperidin-1-yl)methanone,
[217] -(6-(7-azabicyclo[2.2.1]heptan-7-yl)pyridin-3-yl)pyrazolo[1,5-*a*]pyridin-3-yl)(piperidin-1-yl)methanone,
[218] (7-(6-(3-(dimethylamino)azetidin-1-yl)pyridin-3-yl)pyrazolo[1,5-*a*]pyridin-3-yl)(piperidin-1-yl)methanone,
[219] methyl 1-(5-(3-(piperidine-1-carbonyl)pyrazolo[1,5-*a*]pyridin-7-yl)pyridin-2-yl)azetidine-3-carboxylate,
[220] (7-(6-(2-azaspiro[3.3]heptan-2-yl)pyridin-3-yl)pyrazolo[1,5-*a*]pyridin-3-yl)(piperidin-1-yl)methanone,
[221] (7-(6-(3-(hydroxymethyl)azetidin-1-yl)pyridin-3-yl)pyrazolo[1,5-*a*]pyridin-3-yl)(piperidin-1-yl)methanone,
[222] (7-(6-(7-azaspiro[3.5]nonan-7-yl)pyridin-3-yl)pyrazolo[1,5-*a*]pyridin-3-yl)(piperidin-1-yl)methanone,
[223] (7-(6-(6-azaspiro[3.4]octan-6-yl)pyridin-3-yl)pyrazolo[1,5-*a*]pyridin-3-yl)(piperidin-1-yl)methanone,
[224] 7-(3-(piperidine-1-carbonyl)pyrazolo[1,5-*a*]pyridin-7-yl)phthalazin-1(2*H*)-one,
[225] (6-hydroxy-2-azaspiro[3.3]heptan-2-yl)-[5-[3-(piperidine-1-carbonyl)pyrazolo[1,5-*a*]pyridin-7-yl]-3-pyridyl] methanone,
[226] 2-oxa-7-azaspiro[3.5]nonan-7-yl-[5-[3-(piperidine-1-carbonyl)pyrazolo[1,5-*a*]pyridin-7-yl]-3-pyridyl]methanone,
[227] (2-azabicyclo[2.2.1]heptan-2-yl)(7-phenylpyrazolo[1,5-*a*]pyridin-3-yl)methanone,
[228] (2-azabicyclo[2.2.2]octan-2-yl)(7-phenylpyrazolo[1,5-*a*]pyridin-3-yl)methanone,
[229] (7-azabicyclo[2.2.1]heptan-7-yl)(7-phenylpyrazolo[1,5-*a*]pyridin-3-yl)methanone,
[230] (3-hydroxyiminoazetidin-1-yl)-[5-[3-(piperidine-1-carbonyl)pyrazolo[1,5-*a*]pyridin-7-yl]-3-pyridyl]methanone,
[231] 6-(3-(4-fluoropiperidine-1-carbonyl)pyrazolo[1,5-*a*]pyridin-7-yl)-2-methylisoindolin-1-one,
[232] 3-(3-(piperidine-1-carbonyl)pyrazolo[1,5-a]pyridin-7-yl)-6,7-dihydro-5*H*-pyrrolo[3,4-*b*]pyridin-5-one,
[233] 6-(3-(piperidine-1-carbonyl)pyrazolo[1,5-*a*]pyridin-7-yl)phthalazin-1(2*H*)-one,
[234] 6-(3-(4,4-difluoropiperidine-1-carbonyl)pyrazolo[1,5-*a*]pyridin-7-yl)isoindolin-1-one,
[235] 4-[[3-(piperidine-1-carbonyl)pyrazolo[1,5-*a*]pyridin-7-yl]amino]-1*H*-pyrimidin-6-one,
[236] 3-methyl-6-[[3-(piperidine-1-carbonyl)pyrazolo[1,5-*a*]pyridin-7-yl]amino]pyrimidin-4-one,
[237] (3,3-difluoroazetidin-1-yl)-[5-[3-(piperidine-1-carbonyl)pyrazolo[1,5-*a*]pyridin-7-yl]-2-pyridyl]methanone,
[238] [7-[(4-chlorophenyl)methylamino]pyrazolo[1,5-*a*]pyridin-3-yl]-(1-piperidyl)methanone,
[239] 4-chloro-N[3-(piperidine-1-carbonyl)pyrazolo[1,5-*a*]pyridin-7-yl]benzamide,
[240] 1,3,5-trimethyl-*N*-[3-(piperidine-1-carbonyl)pyrazolo[1,5-*a*]pyridin-7-yl]pyrazole-4-sulfonamide,
[241] 5-fluoro-*N*-[3-(piperidine-1-carbonyl)pyrazolo[1,5-*a*]pyridin-7-yl]pyridine-3-carboxamide,
[242] [7-[2-(4-chlorophenyl)ethylamino]pyrazolo[1,5-*a*]pyridin-3-yl]-(1-piperidyl)methanone,
[243] 3-fluoro-5-[3-(piperidine-1-carbonyl)pyrazolo[1,5-*a*]pyridin-7-yl]pyridine-2-carboxylic acid,
[244] methyl 3-fluoro-5-[3-(piperidine-1-carbonyl)pyrazolo[1,5-*a*]pyridin-7-yl]pyridine-2-carboxylate,
[245] 3-methyl-6-(3-(piperidine-l-carbonyl)pyrazolo[1,5-*a*]pyridin-7-yl)quinazolin-4(3*H*)-one,
[246] 2-methyl-6-(3-(piperidine-1-carbonyl)pyrazolo[1,5-*a*]pyridin-7-yl)-[1,2,4]triazolo[4,3-a]pyridin-3(2*H*)-one,
[247] 3-[3-fluoro-5-[3-(piperidine-1-carbonyl)pyrazolo[1,5-a]pyridin-7-yl]-2-pyridyl]-4*H*-1,2,4-oxadiazol-5-one,
[248] [7-[6-(azetidine-1-carbonyl)-5-fluoro-3-pyridyl]pyrazolo[1,5-*a*]pyridin-3-yl]-(1-piperidyl)methanone,

[249] [7-[5-fluoro-6-(morpholine-4-carbonyl)-3-pyridyl]pyrazolo[1,5-*a*]pyridin-3-yl]-(1-piperidyl)methanone,
[250] 3-fluoro-*N*-methyl-5-[3-(piperidine-1-carbonyl)pyrazolo[1,5-*a*]pyridin-7-yl]pyridine-2-carboxamide,
[251] 3-methyl-7-(3-(piperidine-1-carbonyl)pyrazolo[1,5-*a*]pyridin-7-yl)quinazolin-4(3*H*)-one,
[252] 2-methyl-7-(3-(piperidine-1-carbonyl)pyrazolo[1,5-*a*]pyridin-7-yl)-[1,2,4]triazolo[4,3-a]pyridin-3(2*H*)-one,
[253] 3-(3-(piperidine-1-carbonyl)pyrazolo[1,5-*a*]pyridin-7-yl)benzaldehyde oxime,
[254] 2-fluoro-5-[3-(piperidine-1-carbonyl)pyrazolo[1,5-*a*]pyridin-7-yl]benzonitrile,
[255] 3-methyl-6-(3-(piperidine-1-carbonyl)pyrazolo[1,5-*a*]pyridin-7-yl)pyrido[2,3-*d*]pyrimidin-4(3*H*)-one,
[256] 3-methyl-7-(3-(piperidine-1-carbonyl)pyrazolo[1,5-*a*]pyridin-7-yl)pyrido[3,2-*d*]pyrimidin-4(3*H*)-one,
[257] (7-(2-(3-fluoroazetidin-1-yl)pyridin-4-yl)pyrazolo[1,5-*a*]pyridin-3-yl)(piperidin-1-yl)methanone,
[258] 3-chloro-5-[3-(piperidine-1-carbonyl)pyrazolo[1,5-*a*]pyridin-7-yl]pyridine-2-carboxylic acid,
[259] [7-(3-amino-1,2-benzoxazol-5-yl)pyrazolo[1,5-*a*]pyridin-3-yl]-(1-piperidyl)methanone,
[260] [7-(3-amino-1H-indazol-5-yl)pyrazolo[1,5-*a*]pyridin-3-yl]-(1-piperidyl)methanone,
[261] [7-(3-amino-1-methyl-indazol-5-yl)pyrazolo[1,5-*a*]pyridin-3-yl]-(1-piperidyl)methanone,
[262] 3-chloro-*N*-methyl-5-[3-(piperidine-1-carbonyl)pyrazolo[1,5-*a*]pyridin-7-yl]pyridine-2-carboxamide,
[263] [7-[5-fluoro-6-(5-methyl-1,2,4-oxadiazol-3-yl)-3-pyridyl]pyrazolo[1,5-*a*]pyridin-3-yl]-(1-piperidyl)methanone,
[264] (7-(2-chlorophenyl)pyrazolo[1,5-*a*]pyridin-3-yl)(piperidin-1-yl)methanone,
[265] (7-(5-bromo-2-fluoropyridin-3-yl)pyrazolo[1,5-*a*]pyridin-3-yl)(piperidin-1-yl)methanone,
[266] (7-(2,6-difluoropyridin-3-yl)pyrazolo[1,5-*a*]pyridin-3-yl)(piperidin-1-yl)methanone,
[267] (7-(2-chloropyrimidin-5-yl)pyrazolo[1,5-*a*]pyridin-3-yl)(piperidin-1-yl)methanone,
[268] 2-methyl-5-(3-(piperidine-1-carbonyl)pyrazolo[1,5-*a*]pyridin-7-yl)benzo[*d*]isoxazol-3(2*H*)-one,
[269] (7-(3-methoxybenzo[*d*]isoxazol-5-yl)pyrazolo[1,5-*a*]pyridin-3-yl)(piperidin-1-yl)methanone,
[270] 2-(dimethylamino)-5-(3-(piperidine-1-carbonyl)pyrazolo[1,5-*a*]pyridin-7-yl)nicotinonitrile,
[271] (7-(6-fluoropyridin-2-yl)pyrazolo[1,5-*a*]pyridin-3-yl)(piperidin-1-yl)methanone,
[272] (7-(2-chloro-6-(trifluoromethyl)pyridin-3-yl)pyrazolo[1,5-*a*]pyridin-3-yl)(piperidin-1-yl)methanone,
[273] (7-(2,6-difluoropyridin-4-yl)pyrazolo[1,5-*a*]pyridin-3-yl)(piperidin-1-yl)methanone,
[274] *N*-(4-(3-(piperidine-1-carbonyl)pyrazolo[1,5-*a*]pyridin-7-yl)phenyl)-2-(pyridin-3-yl)acetamide,
[275] 3-[2-fluoro-5-[3-(piperidine-1-carbonyl)pyrazolo[1,5-*a*]pyridin-7-yl]phenyl]-4*H*-1,2,4-oxadiazol-5-one,
[276] [7-[4-fluoro-3-(5-methyl-1,2,4-oxadiazol-3-yl)phenyl]pyrazolo[1,5-*a*]pyridin-3-yl]-(1-piperidyl)methanone,
[277] [7-[2-(azetidine-1-carbonyl)pyrimidin-5-yl]pyrazolo[1,5-*a*]pyridin-3-yl]-(1-piperidyl)methanone,
[278] *N*-(2,6-difluoro-4-(3-(piperidine-1-carbonyl)pyrazolo[1,5-*a*]pyridin-7-yl)phenyl)-2-(pyridin-3-yl)acetamide,
[279] (7-(3-chloro-5-fluorophenyl)pyrazolo[1,5-*a*]pyridin-3-yl)(piperidin-1-yl)methanone,
[280] 5,6-dimethyl-3-(3-(piperidine-1-carbonyl)pyrazolo[1,5-*a*]pyridin-7-yl)-5,6-dihydro-7*H*-pyrrolo[3,4-*b*]pyridin-7-one,
[281] (7-(6-chloro-5-fluoropyridin-3-yl)pyrazolo[1,5-*a*]pyridin-3-yl)(piperidin-1-yl)methanone,
[282] (7-(2,6-dichloropyridin-4-yl)pyrazolo[1,5-*a*]pyridin-3-yl)(piperidin-1-yl)methanone,
[283] (7-(5-chloro-2-fluoropyridin-3-yl)pyrazolo[1,5-*a*]pyridin-3-yl)(piperidin-1-yl)methanone,
[284] (7-(5-fluoropyridin-3-yl)pyrazolo[1,5-*a*]pyridin-3-yl)(piperidin-1-yl)methanone,
[285] 3-fluoro-4-[3-(piperidine-1-carbonyl)pyrazolo[1,5-*a*]pyridin-7-yl]benzonitrile,
[286] [7-[6-(azetidine-1-carbonyl)-5-chloro-3-pyridyl]pyrazolo[1,5-*a*]pyridin-3-yl]-(1-piperidyl)methanone,
[287] *N*-(5-(3-(piperidine-1-carbonyl)pyrazolo[1,5-*a*]pyridin-7-yl)pyridin-2-yl)nicotinamide,
[288] (7-(5,6-difluoropyridin-3-yl)pyrazolo[1,5-*a*]pyridin-3-yl)(piperidin-1-yl)methanone,
[289] (7-(5-chloro-6-fluoropyridin-3-yl)pyrazolo[1,5-*a*]pyridin-3-yl)(piperidin-1-yl)methanone,
[290] 2,6-difluoro-4-(3-(piperidine-1-carbonyl)pyrazolo[1,5-*a*]pyridin-7-yl)benzonitrile,
[291] 3-fluoro-5-(3-(piperidine-1-carbonyl)pyrazolo[1,5-*a*]pyridin-7-yl)benzonitrile,
[292] (7-(4-bromophenyl)pyrazolo[1,5-*a*]pyridin-3-yl)(piperidin-1-yl)methanone,
[293] 5-[3-(piperidine-1-carbonyl)pyrazolo[1,5-*a*]pyridin-7-yl]pyrimidine-2-carbonitrile,
[294] [7-(2-hydroxypyrimidin-5-yl)pyrazolo[1,5-*a*]pyridin-3-yl]-(1-piperidyl)methanone,
[295] 3-[3-fluoro-4-[3-(piperidine-1-carbonyl)pyrazolo[1,5-*a*]pyridin-7-yl]phenyl]-4H-1,2,4-oxadiazol-5-one,
[296] [7-[2-fluoro-4-(5-methyl-1,2,4-oxadiazol-3-yl)phenyl]pyrazolo[1,5-*a*]pyridin-3-yl]-(1-piperidyl)methanone,
[297] 3-(5-(3-(piperidine-1-carbonyl)pyrazolo[1,5-*a*]pyridin-7-yl)pyridin-3-yl)-1,2,4-oxadiazol-5(4*H*)-one,
[298] (7-(5-chloro-2-fluoropyridin-4-yl)pyrazolo[1,5-*a*]pyridin-3-yl)(piperidin-1-yl)methanone,
[299] 3-(3-fluoro-5-(3-(piperidine-1-carbonyl)pyrazolo[1,5-*a*]pyridin-7-yl)phenyl)-1,2,4-oxadiazol-5(4*H*)-one,
[300] [7-[4-(2-methylpyrazol-3-yl)phenyl]pyrazolo[1,5-*a*]pyridin-3-yl]-(1-piperidyl)methanone,
[301] *N*-(3-chloro-5-(3-(piperidine-1-carbonyl)pyrazolo[1,5-*a*]pyridin-7-yl)pyridin-2-yl)nicotinamide,
[302] [7-[4-(3-furyl)phenyl]pyrazolo[1,5-*a*]pyridin-3-yl]-(1-piperidyl)methanone,
[303] [7-[3-fluoro-4-(5-methyl-1,2,4-oxadiazol-3-yl)phenyl]pyrazolo[1,5-*a*]pyridin-3-yl]-(1-piperidyl)methanone,
[304] 3-[2-fluoro-4-[3-(piperidine-1-carbonyl)pyrazolo[1,5-a]pyridin-7-yl]phenyl]-4*H*-1,2,4-oxadiazol-5-one,
[305] 5-[4-[3-(piperidine-1-carbonyl)pyrazolo[1,5-*a*]pyridin-7-yl]phenyl]pyrimidine-2-carbonitrile,

[306] [7-[4-(2-hydroxypyrimidin-5-yl)phenyl]pyrazolo[1,5-*a*]pyridin-3-yl]-(1-piperidyl)methanone,
[307] 5-[4-[3-(piperidine-1-carbonyl)pyrazolo[1,5-*a*]pyridin-7-yl]phenyl]pyrimidine-2-carboxamide,
[308] 3-(5-(3-(piperidine-1-carbonyl)pyrazolo[1,5-*a*]pyridin-7-yl)pyrimidin-2-yl)oxazolidin-2-one,
[309] [7-(3-amino-1*H*-indazol-6-yl)pyrazolo[1,5-*a*]pyridin-3-yl]-(1-piperidyl)methanone,
[310] [7-(3-amino-1-methyl-indazol-6-yl)pyrazolo[1,5-*a*]pyridin-3-yl]-(1-piperidyl)methanone,
[311] *N*-(3-chloro-5-(3-(piperidine-1-carbonyl)pyrazolo[1,5-*a*]pyridin-7-yl)pyridin-2-yl)-2-(pyridin-3-yl)acetamide,
[312] 3-nitro-5-(3-(piperidine-1-carbonyl)pyrazolo[1,5-*a*]pyridin-7-yl)benzonitrile,
[313] (7-(6-bromopyridin-3-yl)pyrazolo[1,5-*a*]pyridin-3-yl)(piperidin-1-yl)methanone,
[314] (7-(6-chloropyridin-3-yl)pyrazolo[1,5-*a*]pyridin-3-yl)(piperidin-1-yl)methanone,
[315] (7-(6-chloro-2-fluoropyridin-3-yl)pyrazolo[1,5-*a*]pyridin-3-yl)(piperidin-1-yl)methanone,
[316] 3-(5-(3-(piperidine-1-carbonyl)pyrazolo[1,5-*a*]pyridin-7-yl)pyridin-2-yl)oxazolidin-2-one,
[317] [7-[2-(5-methyl-1,2,4-oxadiazol-3-yl)pyrimidin-5-yl]pyrazolo[1,5-*a*]pyridin-3-yl]-(1-piperidyl)methanone,
[318] (7-(5-nitropyridin-3-yl)pyrazolo[1,5-*a*]pyridin-3-yl)(piperidin-1-yl)methanone,
[319] (7-(5-(5-methyl-1,2,4-oxadiazol-3-yl)pyridin-3-yl)pyrazolo[1,5-*a*]pyridin-3-yl)(piperidin-1-yl)methanone,
[320] [7-(3-amino-1,2-benzoxazol-6-yl)pyrazolo[1,5-*a*]pyridin-3-yl]-(1-piperidyl)methanone,
[321] 3-[5-[3-(piperidine-1-carbonyl)pyrazolo[1,5-*a*]pyridin-7-yl]pyrimidin-2-yl]-4*H*-1,2,4-oxadiazol-5-one,
[322] (7-(3-fluoro-5-(5-methyl-1,2,4-oxadiazol-3-yl)phenyl)pyrazolo[1,5-*a*]pyridin-3-yl)(piperidin-1-yl)methanone,
[323] 3-(3-fluoro-5-(3-(piperidine-1-carbonyl)pyrazolo[1,5-*a*]pyridin-7-yl)pyridin-2-yl)oxazolidin-2-one,
[324] [7-[4-(3,5-dimethylisoxazol-4-yl)phenyl]pyrazolo[1,5-*a*]pyridin-3-yl]-(1-piperidyl)methanone,
[325] (7-(2-fluoro-5-(1-methyl-1*H*-pyrazol-5-yl)pyridin-3-yl)pyrazolo[1,5-*a*]pyridin-3-yl)(piperidin-1-yl)methanone,
[326] *N*-(5-(3-(piperidine-1-carbonyl)pyrazolo[1,5-*a*]pyridin-7-yl)pyridin-3-yl)nicotinamide,
[327] *N*-(5-(3-(piperidine-1-carbonyl)pyrazolo[1,5-*a*]pyridin-7-yl)pyridin-3-yl)-2-(pyridin-3-yl)acetamide,
[328] (7-(3,5-difluoro-4-(5-methyl-1,2,4-oxadiazol-3-yl)phenyl)pyrazolo[1,5-*a*]pyridin-3-yl)(piperidin-1-yl)methanone,
[329] 3-(3-nitro-5-(3-(piperidine-1-carbonyl)pyrazolo[1,5-*a*]pyridin-7-yl)phenyl)-1,2,4-oxadiazol-5(4*H*)-one,
[330] (7-(3-(5-methyl-1,2,4-oxadiazol-3-yl)-5-nitrophenyl)pyrazolo[1,5-*a*]pyridin-3-yl)(piperidin-1-yl)methanone,
[331] 3-(2,6-difluoro-4-(3-(piperidine-1-carbonyl)pyrazolo[1,5-*a*]pyridin-7-yl)phenyl)-1,2,4-oxadiazol-5(4H)-one,
[332] piperidin-1-yl(7-(6-((2-(pyridin-3-yl)ethyl)amino)pyridin-3-yl)pyrazolo[1,5-*a*]pyridin-3-yl)methanone,
[333] 1,2-dimethyl-*N*-(5-(3-(piperidine-1-carbonyl)pyrazolo[1,5-*a*]pyridin-7-yl)pyridin-3-yl)-1*H*-imidazole-5-carboxamide,
[334] *N*-(5-(3-(piperidine-1-carbonyl)pyrazolo[1,5-*a*]pyridin-7-yl)pyridin-3-yl)pyrimidine-5-carboxamide,
[335] 5-fluoro-*N*-(5-(3-(piperidine-1-carbonyl)pyrazolo[1,5-*a*]pyridin-7-yl)pyridin-3-yl)nicotinamide,
[336] (7-(6-fluoropyridin-3-yl)pyrazolo[1,5-*a*]pyridin-3-yl)(3-methylpiperidin-1-yl)methanone,
[337] (3-ethylpiperidin-1-yl)(7-(6-fluoropyridin-3-yl)pyrazolo[1,5-*a*]pyridin-3-yl)methanone,
[338] (7-(6-fluoropyridin-3-yl)pyrazolo[1,5-*a*]pyridin-3-yl)(3-(trifluoromethyl)piperidin-1-yl)methanone,
[339] 3-(5-(3-(3-(trifluoromethyl)piperidine-1-carbonyl)pyrazolo[1,5-*a*]pyridin-7-yl)pyridin-2-yl)oxazolidin-2-one,
[340] *N*-(2-nitrobenzyl)-*N*-(4-(3-(piperidine-1-carbonyl)pyrazolo[1,5-*a*]pyridin-7-yl)phenyl)nicotinamide,
[341] (7-(3-fluoro-5-(1,2,4-oxadiazol-3-yl)phenyl)pyrazolo[1,5-*a*]pyridin-3-yl)(piperidin-1-yl)methanone,
[342] (7-(3-nitro-5-(1,2,4-oxadiazol-3-yl)phenyl)pyrazolo[1,5-*a*]pyridin-3-yl)(piperidin-1-yl)methanone,
[343] (7-(3-amino-5-(5-methyl-1,2,4-oxadiazol-3-yl)phenyl)pyrazolo[1,5-*a*]pyridin-3-yl)(piperidin-1-yl)methanone,
[344] (7-(6-(4-amino-1*H*-pyrazol-1-yl)pyridin-3-yl)pyrazolo[1,5-a]pyridin-3-yl)(piperidin-1-yl)methanone,
[345] (7-(3-(3-hydroxyazetidin-1-yl)-5-(isoxazol-4-yl)phenyl)pyrazolo[1,5-*a*]pyridin-3-yl)(piperidin-1-yl)methanone,
[346] 1-piperidyl-[7-[4-(1H-pyrazol-4-yl)phenyl]pyrazolo[1,5-*a*]pyridin-3-yl]methanone,
[347] 1-piperidyl-[7-[4-(3-thienyl)phenyl]pyrazolo[1,5-*a*]pyridin-3-yl]methanone,
[348] [7-[4-(5-methyl-1,3,4-oxadiazol-2-yl)phenyl]pyrazolo[1,5-*a*]pyridin-3-yl]-(1-piperidyl)methanone,
[349] 5-[4-[3-(piperidine-1-carbonyl)pyrazolo[1,5-*a*]pyridin-7-yl]phenyl]-3H-1,3,4-oxadiazol-2-one,
[350] [7-[4-(1,3,4-oxadiazol-2-yl)phenyl]pyrazolo[1,5-*a*]pyridin-3-yl]-(1-piperidyl)methanone,
[351] [7-[4-(5-methylthiazol-2-yl)phenyl]pyrazolo[1,5-*a*]pyridin-3-yl]-(1-piperidyl)methanone,
[352] [7-[4-(1,2,4-oxadiazol-3-yl)phenyl]pyrazolo[1,5-*a*]pyridin-3-yl]-(1-piperidyl)methanone,
[353] 3-(5-(3-(3-methylpiperidine-1-carbonyl)pyrazolo[1,5-*a*]pyridin-7-yl)pyridin-2-yl)oxazolidin-2-one,
[354] 3-(5-(3-(3-ethylpiperidine-1-carbonyl)pyrazolo[1,5-*a*]pyridin-7-yl)pyridin-2-yl)oxazolidin-2-one,
[355] (7-(6-(4-nitro-1*H*-pyrazol-1-yl)pyridin-3-yl)pyrazolo[1,5-*a*]pyridin-3-yl)(piperidin-1-yl)methanone,
[356] (7-(2-(4-amino-1*H*-pyrazol-1-yl)-6-bromopyridin-4-yl)pyrazolo[1,5-*a*]pyridin-3-yl)(piperidin-1-yl)methanone,
[357] (7-(2-(3-hydroxyazetidin-1-yl)-6-(1-methyl-1*H*-pyrazol-5-yl)pyridin-4-yl)pyrazolo[1,5-*a*]pyridin-3-yl)(piperidin-1-yl)methanone,
[358] 7-[4-(1,2-dimethylimidazol-4-yl)phenyl]pyrazolo[1,5-*a*]pyridin-3-yl]-(1-piperidyl)methanone,
[359] [7-[4-(5-amino-1,2,4-oxadiazol-3-yl)phenyl]pyrazolo[1,5-*a*]pyridin-3-yl]-(1-piperidyl)methanone,
[360] 5-[5-[3-(piperidine-1-carbonyl)pyrazolo[1,5-*a*]pyridin-7-yl]-2-pyridyl]-3*H*-1,3,4-oxadiazol-2-one,

[361] 1-piperidyl-[7-[4-[5-(trifluoromethyl)-1,2,4-oxadiazol-3-yl]phenyl]pyrazolo[1,5-*a*]pyridin-3-yl]methanone,
[362] *N*-(3-fluoro-5-(3-(piperidine-1-carbonyl)pyrazolo[1,5-*a*]pyridin-7-yl)pyridin-2-yl)-2-(pyridin-3-yl)acetamide,
[363] (7-(5-bromo-2-(3-(hydroxymethyl)azetidin-1-yl)pyridin-3-yl)pyrazolo[1,5-*a*]pyridin-3-yl)(piperidin-1-yl)methanone,
[364] [7-[6-(5-methyl-1,3,4-oxadiazol-2-yl)-3-pyridyl]pyrazolo[1,5-*a*]pyridin-3-yl]-(1-piperidyl)methanone,
[365] 1-piperidyl-[7-[6-(2-thioxo-3*H*-1,3,4-oxadiazol-5-yl)-3-pyridyl]pyrazolo[1,5-*a*]pyridin-3-yl]methanone,
[366] (7-(2-(isoxazol-4-yl)-6-morpholinopyridin-4-yl)pyrazolo[1,5-*a*]pyridin-3-yl)(piperidin-1-yl)methanone,
[367] 3-(6-(1-methyl-1*H*-pyrazol-5-yl)-4-(3-(piperidine-1-carbonyl)pyrazolo[1,5-*a*]pyridin-7-yl)pyridin-2-yl)oxazolidin-2-one,
[368] 3-(6-(3,5-dimethylisoxazol-4-yl)-4-(3-(piperidine-1-carbonyl)pyrazolo[1,5-*a*]pyridin-7-yl)pyridin-2-yl)oxazolidin-2-one,
[369] (7-(2-(1-methyl-1*H*-pyrazol-5-yl)-6-morpholinopyridin-4-yl)pyrazolo[1,5-*a*]pyridin-3-yl)(piperidin-1-yl)methanone,
[370] 3-(5'-fluoro-4-(3-(piperidine-1-carbonyl)pyrazolo[1,5-*a*]pyridin-7-yl)-[2,3'-bipyridin]-6-yl)oxazolidin-2-one,
[371] (7-(2-(3,5-dimethylisoxazol-4-yl)-6-morpholinopyridin-4-yl)pyrazolo[1,5-*a*]pyridin-3-yl)(piperidin-1-yl)methanone,
[372] 3-(6-(2-methylpyrimidin-5-yl)-4-(3-(piperidine-1-carbonyl)pyrazolo[1,5-*a*]pyridin-7-yl)pyridin-2-yl)oxazolidin-2-one,
[373] 5-[3-fluoro-5-[3-(piperidine-1-carbonyl)pyrazolo[1,5-*a*]pyridin-7-yl]-2-pyridyl]-3*H*-1,3,4-oxadiazol-2-one,
[374] (7-(5-(1,2,4-oxadiazol-3-yl)pyridin-3-yl)pyrazolo[1,5-*a*]pyridin-3-yl)(piperidin-1-yl)methanone,
[375] (7-(6-(3-(dimethylamino)azetidin-1-yl)-5'-fluoro-[2,3'-bipyridin]-4-yl)pyrazolo[1,5-*a*]pyridin-3-yl)(piperidin-1-yl)methanone,
[376] (7-(5'-fluoro-6-morpholino-[2,3'-bipyridin]-4-yl)pyrazolo[1,5-*a*]pyridin-3-yl)(piperidin-1-yl)methanone,
[377] (7-(2-(2-methylpyrimidin-5-yl)-6-morpholinopyridin-4-yl)pyrazolo[1,5-*a*]pyridin-3-yl)(piperidin-1-yl)methanone,
[378] 6-(3-(3-fluoropiperidine-1-carbonyl)pyrazolo[1,5-*a*]pyridin-7-yl)isoindolin-1-one,
[379] [7-[5-fluoro-6-(5-methyl-1,3,4-oxadiazol-2-yl)-3-pyridyl]pyrazolo[1,5-*a*]pyridin-3-yl]-(1-piperidyl)methanone,
[380] [7-[5-fluoro-6-(1,3,4-oxadiazol-2-yl)-3-pyridyl]pyrazolo[1,5-*a*]pyridin-3-yl]-(1-piperidyl)methanone,
[381] [7-[6-(3,5-dimethylisoxazol-4-yl)-5-fluoro-3-pyridyl]pyrazolo[1,5-*a*]pyridin-3-yl]-(1-piperidyl)methanone,
[382] 6-(3-(3,3-difluoropiperidine-1-carbonyl)pyrazolo[1,5-*a*]pyridin-7-yl)isoindolin-1-one,
[383] 3-(5-(3-(3-fluoropiperidine-1-carbonyl)pyrazolo[1,5-*a*]pyridin-7-yl)pyridin-2-yl)oxazolidin-2-one,
[384] 3-(5-(3-(3,3-difluoropiperidine-1-carbonyl)pyrazolo[1,5-*a*]pyridin-7-yl)pyridin-2-yl)oxazolidin-2-one,
[385] (*R*)-6-(3-(3-methylpiperidine-1-carbonyl)pyrazolo[1,5-*a*]pyridin-7-yl)isoindolin-1-one,
[386] (*S*)-6-(3-(3-methylpiperidine-1-carbonyl)pyrazolo[1,5-*a*]pyridin-7-yl)isoindolin-1-one,
[387] 6-(3-(3-methylpiperidine-1-carbonyl)pyrazolo[1,5-*a*]pyridin-7-yl)isoindolin-1-one,
[388] *N*-(5-(3-(piperidine-1-carbonyl)pyrazolo[1,5-*a*]pyridin-7-yl)pyridin-3-yl)-2-(6-(trifluoromethyl)pyridin-3-yl)acetamide,
[389] 2-phenyl-N-(5-(3-(piperidine-1-carbonyl)pyrazolo[1,5-*a*]pyridin-7-yl)pyridin-3-yl)acetamide,
[390] 6-(3-(2-azaspiro[3.3]heptane-2-carbonyl)pyrazolo[1,5-*a*]pyridin-7-yl)isoindolin-1-one,
[391] 6-(3-(6-azaspiro[3.4]octane-6-carbonyl)pyrazolo[1,5-*a*]pyridin-7-yl)isoindolin-1-one,
[392] 6-(3-(7-azaspiro[3.5]nonane-7-carbonyl)pyrazolo[1,5-*a*]pyridin-7-yl)isoindolin-1-one,
[393] 6-(3-(3-ethylpiperidine-1-carbonyl)pyrazolo[1,5-*a*]pyridin-7-yl)isoindolin-1-one,
[394] [7-[5-fluoro-6-[5-(trifluoromethyl)-1,3,4-oxadiazol-2-yl]-3-pyridyl]pyrazolo[1,5-*a*]pyridin-3-yl]-(1-piperidyl)methanone,
[395] (7-(1-((1,2,4-oxadiazol-3-yl)methyl)-1*H*-pyrazol-4-yl)pyrazolo[1,5-*a*]pyridin-3-yl)(piperidin-1-yl)methanone,
[396] 3-((4-(3-(piperidine-1-carbonyl)pyrazolo[1,5-a]pyridin-7-yl)-1*H*-pyrazol-1-yl)methyl)-1,2,4-oxadiazol-5(4*H*)-one,
[397] (7-(2-(1-((1,2,4-oxadiazol-3-yl)methyl)-1*H*-pyrazol-4-yl)pyridin-4-yl)pyrazolo[1,5-*a*]pyridin-3-yl)(piperidin-1-yl)methanone,
[398] 6-(3-(3-(trifluoromethyl)piperidine-1-carbonyl)pyrazolo[1,5-*a*]pyridin-7-yl)isoindolin-1-one,
[399] [7-[2-(3,5-dimethylisoxazol-4-yl)pyrimidin-5-yl]pyrazolo[1,5-*a*]pyridin-3-yl]-(1-piperidyl)methanone,
[400] 6-(3-(3-isopropylpiperidine-1-carbonyl)pyrazolo[1,5-*a*]pyridin-7-yl)isoindolin-1-one,
[401] 6-(3-(1,2,3,6-tetrahydropyridine-1-carbonyl)pyrazolo[1,5-*a*]pyridin-7-yl)isoindolin-1-one,
[402] 2-(5-fluoropyridin-3-yl)-*N*-(5-(3-(piperidine-1-carbonyl)pyrazolo[1,5-*a*]pyridin-7-yl)pyridin-3-yl)acetamide,
[403] *N*-(5-(3-(piperidine-1-carbonyl)pyrazolo[1,5-*a*]pyridin-7-yl)pyridin-3-yl)-2-(pyrimidin-5-yl)acetamide,
[404] [7-[2-[5-(difluoromethyl)-1-oxo-1,3,4-thiadiazol-2-yl]pyrimidin-5-yl]pyrazolo[1,5-a]pyridin-3-yl]-(1-piperidyl)methanone,
[405] 6-(3-(6-azaspiro[3.5]nonane-6-carbonyl)pyrazolo[1,5-*a*]pyridin-7-yl)isoindolin-1-one,

[406] 2-(4-chlorophenoxy)-*N*-(5-(3-(piperidine-1-carbonyl)pyrazolo[1,5-*a*]pyridin-7-yl)pyridin-3-yl)acetamide,
[407] *N*-[5-[3-(piperidine-1-carbonyl)pyrazolo[1,5-*a*]pyridin-7-yl]-2-pyridyl]-2-pyrimidin-5-yl-acetamide,
[408] *N*-[5-[3-(piperidine-1-carbonyl)pyrazolo[1,5-*a*]pyridin-7-yl]-2-pyridyl]-2-[6-(trifluoromethyl)-3-pyridyl]acetamide,
[409] 2-(5-fluoropyridin-3-yl)-*N*-(5-(3-(piperidine-1-carbonyl)pyrazolo[1,5-*a*]pyridin-7-yl)pyridin-2-yl)acetamide,
[410] 2-(4-chlorophenoxy)-*N*-[5-[3-(piperidine-1-carbonyl)pyrazolo[1,5-*a*]pyridin-7-yl]-2-pyridyl]acetamide,
[411] 6-(3-(6-fluoro-2-azaspiro[3.3]heptane-2-carbonyl)pyrazolo[1,5-*a*]pyridin-7-yl)isoindolin-1-one,
[412] 6-(3-(6,6-difluoro-2-azaspiro[3.3]heptane-2-carbonyl)pyrazolo[1,5-*a*]pyridin-7-yl)isoindolin-1-one, and
[413] 3-hydroxy-5-[2-(piperidine-1-carbonyl)pyrazolo[1,5-*a*]pyridin-6-yl]pyridine-2-carbonitrile.

The compound represented by the above Formula 1 according to the present invention can be prepared and used in the form of prodrugs, hydrates, solvates and pharmaceutically acceptable salts to enhance *in vivo* absorption or increase solubility, so the prodrugs, hydrates, solvates and pharmaceutically acceptable salts are also within the scope of the present invention.

The term "prodrug" refers to a substance that is transformed *in vivo* into the parent drug. Prodrugs are often used because, in some cases, they are easier to administer than the parent drug. For example, they may be bioavailable by oral administration, whereas the parent drug may not be. Prodrugs may also have improved solubility in pharmaceutical compositions than the parent drug. For example, prodrugs may be *in vivo* hydrolysable esters of the compound according to the present invention and pharmaceutically acceptable salts thereof. Another example of a prodrug may be a short peptide (polyamino acid) that is linked to an acid group that is metabolized to reveal the active site of the peptide.

The term "hydrate" means a compound of the present invention or a salt thereof containing a stoichiometric or non-stoichiometric amount of water bound by non-covalent intermolecular forces.

The term "solvate" means a compound of the present invention or a salt thereof which contains a stoichiometric or non-stoichiometric amount of a solvent bound by non-covalent intermolecular forces. Preferred solvents therefor include those which are volatile, non-toxic, and/or suitable for administration to humans.

The term "isomer" means a compound of the present invention or a salt thereof having the same chemical formula or molecular formula but structurally or sterically different. Such isomers include structural isomers such as tautomers, and stereoisomers such as R or S isomers having an asymmetric carbon center, and geometric isomers (trans, cis). All of these isomers and mixtures thereof are also included in the scope of the present invention.

The term "pharmaceutically acceptable salt" means a salt form of a compound which does not cause serious irritation to the organism to which the compound is administered and does not impair the biological activity and physical properties of the compound. The pharmaceutical salts include acid addition salts formed by acids which form non-toxic acid addition salts containing pharmaceutically acceptable anions, for example, inorganic acids such as hydrochloric acid, sulfuric acid, nitric acid, phosphoric acid, hydrobromic acid, hydroiodic acid, etc., organic carboxylic acids such as tartaric acid, formic acid, citric acid, acetic acid, trichloroacetic acid, trifluoroacetic acid, gluconic acid, benzoic acid, lactic acid, fumaric acid, maleic acid, salicylic acid, etc., sulfonic acids such as methanesulfonic acid, ethanesulfonic acid, benzenesulfonic acid, p-toluenesulfonic acid, etc. For example, pharmaceutically acceptable carboxylic acid salts include metal salts or alkaline earth metal salts formed by lithium, sodium, potassium, calcium, magnesium, etc.; amino acid salts such as lysine, arginine, guanidine, etc.; organic salts such as dicyclohexylamine, N-methyl-D-glucamine, tris(hydroxymethyl) methylamine, diethanolamine, choline, and triethylamine. The compound of Formula 1 according to the present invention may be converted into its salt by a conventional method.

The present invention also provides a method for preparing the compound of Formula 1.

The methods for preparing compounds of Formula 1 of the present invention are exemplified by Schemes 1 to 55, and the methods for preparing compounds of Formula 1 of the present invention are not intended to limit the methods for preparing compounds of Formula 1 of the present invention. The methods for preparing compounds of Reaction Schemes 1 to 55 are merely examples, and it is obvious that they may be easily modified by those skilled in the art depending on specific substituents.

The present invention also provides a pharmaceutical composition for preventing or treating a 15-PGDH-related disease, comprising a heterocyclic compound represented by the Formula 1, a hydrate thereof, a solvate thereof or a pharmaceutically acceptable salt thereof as an active ingredient.

The present invention also provides a method for preventing or treating a 15-PGDH-related disease, comprising administering a therapeutically effective amount of a heterocyclic compound represented by the Formula 1, a hydrate thereof, a solvate thereof or a pharmaceutically acceptable salt thereof, to a subject in need thereof.

The present invention also provides a use of a heterocyclic compound represented by the Formula 1, a hydrate thereof, a solvate thereof or a pharmaceutically acceptable salt thereof, as an effective ingredient for the manufacture of a medicine for preventing or treating a 15-PGDH related disease.

In one embodiment, the 15-PGDH-related disease may be one or more selected from the group consisting of an inflammatory disease (inflammatory bowel disease (ulcerative colitis, Crohn's disease), chronic obstructive pulmonary

disease (COPD), Behcet's disease, acute liver injury, acute kidney injury, acute lung injury, sepsis, exacerbation of asthma and lung disease, peptic ulcer (ulcer caused by NSAIDs), vasculitis syndrome, non-alcoholic fatty liver disease (NASH), atopic dermatitis, psoriasis, interstitial cystitis, prostatitis syndrome), fibrosis (pulmonary fibrosis (idiopathic pulmonary fibrosis), renal fibrosis (glomerulosclerosis), cardiac fibrosis (myocardial fibrosis), oral fibrosis, deltoid fibrosis, liver fibrosis, myelofibrosis, scleroderma), autoimmune disease (systemic lupus erythematosus, rheumatoid arthritis, autoimmune hepatitis, severe combined immunodeficiency syndrome (SCID)), ophthalmic disease (dry eye, conjunctivitis, keratitis), thrombocytopenia (drug-induced thrombocytopenia, autoimmune thrombocytopenia, idiopathic thrombocytopenia, thrombocytopenia due to viral infection), myopathy (muscular dystrophy, muscle damage), anemia (aplastic anemia, anemia of chronic disease, Fanconi anemia, beta-thalassemia or anemia of unknown cause), circulatory disease (cardiac disease (angina pectoris, heart failure, myocardial infarction), kidney disease (chronic kidney disease, renal failure), stroke, pulmonary hypertension, peripheral circulatory disorder), nerve cell death (psychiatric disorders, neurodegenerative diseases, nerve injuries), cytopenia (immune cytopenia, neutropenia, lymphopenia), osteoporosis, fracture, leukemia (acute myeloid leukemia (AML), acute lymphoblastic leukemia (ALL), chronic myeloid leukemia (CML)), lymphoma (Hodgkin's lymphoma, non-Hodgkin's lymphoma), radiation exposure toxicity, 15-PGDH expressing cancer, multiple myeloma, paroxysmal nocturnal hemoglobinuria (PNH), pure red cell aplasia, megakaryocytosis, Wiskott-Aldrich syndrome, sickle cell disease, Hurler syndrome, adrenoleukodystrophy, metachromatic leukodystrophy, myelodysplasia, Duchenne muscular dystrophy, solid tumors, chronic granulomatous disease, systemic sclerosis, scars, wounds, otologic diseases (vertigo, tinnitus, balance disorders), hair loss, skin aging, periodontal disease, diabetes, stem cell and bone marrow transplantation or organ transplantation, cervical ripening, Alzheimer's disease, and Parkinson's disease.

As a result of measuring the 15-PGDH inhibitory activity of the heterocyclic compound of the present invention, it was confirmed that it exhibited excellent 15-PGDH inhibitory activity.

The present invention also provides a pharmaceutical composition comprising a heterocyclic compound represented by the Formula 1, a hydrate thereof, a solvate thereof or a pharmaceutically acceptable salt thereof, and a pharmaceutically acceptable additive.

The above additives may include pharmaceutically acceptable carriers or diluents, and may be formulated in the form of oral formulations such as powders, granules, tablets, capsules, suspensions, emulsions, syrups, aerosols; external preparations; suppositories; and sterile injectable solutions, respectively, according to conventional methods.

The pharmaceutically acceptable carriers include lactose, dextrose, sucrose, sorbitol, mannitol, xylitol, erythritol, maltitol, starch, acacia gum, alginate, gelatin, calcium phosphate, calcium silicate, cellulose, methylcellulose, microcrystalline cellulose, polyvinyl pyrrolidone, water, methylhydroxybenzoate, propylhydroxybenzoate, talc, magnesium stearate, mineral oil, and the like. In addition, diluents or excipients such as fillers, bulking agents, binders, wetting agents, disintegrating agents, and surfactants are included. Oral solid preparations include tablets, pills, powders, granules, capsules, etc., and these solid preparations may contain at least one excipient, for example, starch, calcium carbonate, sucrose or lactose, gelatin, etc., and may contain a lubricant, such as magnesium stearate, talc, etc. Oral liquid preparations include suspensions, oral solutions, emulsions, syrups, etc., and may contain diluents, such as water or liquid paraffin, wetting agents, sweeteners, flavoring agents, preservatives, etc. Parenteral preparations include sterilized aqueous solutions, non-aqueous solvents, suspensions, emulsions, creams, lyophilized preparations, suppositories, and non-aqueous solvents and suspending agents include propylene glycol, polyethylene glycol, vegetable oils, such as olive oil, and injectable esters, such as ethyl oleate. Suppository bases that may be used include witepsol, macrogol, Tween 61, cocoa butter, laurin butter, and glycerogelatin.

The dosage of the active ingredient contained in the pharmaceutical composition of the present invention varies depending on the patient's condition and weight, the degree of the disease, the form of the active ingredient, the route and period of administration, and may be appropriately adjusted depending on the patient. For example, the active ingredient may be administered at a dosage of 0.0001 to 1000 mg/kg per day, preferably 0.001 to 100 mg/kg, and the dose may be administered once a day or divided into several times. In addition, the pharmaceutical composition of the present invention may contain the active ingredient at a weight percentage of 0.001 to 90% based on the total weight of the composition.

The pharmaceutical composition of the present invention may be administered to mammals such as rats, mice, livestock, and humans by various routes, for example, orally, cutaneously, intraperitoneally, rectally, or by intravenous, intramuscular, subcutaneous, intrauterine, or intracerebroventricular injection.

Hereinafter, the present invention will be described in more detail through Examples and Experimental examples. However, the following Examples and Experimental examples are intended to illustrate the present invention, and the scope of the present invention is not limited thereto.

## Example 1. 7-phenylpyrazolo[1,5-*a*]pyridin-3-yl)(piperidin-1-yl)methanone

<Scheme 1>

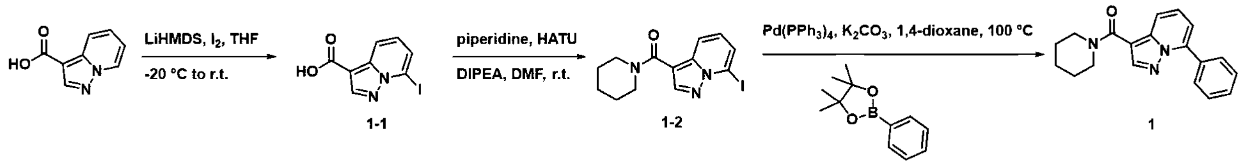


Step 1-1: Pyrazolo[1,5-*a*]pyridine-3-carboxylic acid (1.0 g, 6.18 mmol) was dissolved in tetrahydrofuran (20 mL), and the temperature was lowered to -20 °C. Lithium bis(trimethylsilyl)amide dissolved in 1 M tetrahydrofuran (18.5 mL, 18.5 mmol) was slowly added, and the mixture was stirred for 30 minutes. Iodine (1.878 g, 7.4 mmol) dissolved in tetrahydrofuran (5 mL) was added to the reaction solution, and the mixture was stirred for 30 minutes, then the temperature was slowly raised to room temperature, and the mixture was stirred for 12 hours. After the reaction was complete, a saturated sodium bicarbonate aqueous solution (50 mL) was added, and dichloromethane (50 mL x 3) was used to extract. The combined organic layers were washed with brine, dried over anhydrous magnesium sulfate, filtered, and concentrated. After solidifying with a small amount of dichloromethane, filtering, and air drying, the target compound **1-1** (1.86 g, 94%) was obtained.

Step 1-2: Compound **1-1** (1.7 g, 5.9 mmol) was dissolved in N,Ndimethylformamide (30 mL), HATU (2.69 g, 7.1 mmol) and DIPEA (3.1 mL, 17.7 mmol) were added, and the mixture was stirred at room temperature for 10 minutes. Piperidine (0.7 mL, 7.1 mmol) was added to the reaction solution, and the mixture was stirred for 4 hours. After completion of the reaction, the reaction solution was concentrated, diluted with ethyl acetate (100 mL), and washed sequentially with water (50 mL), saturated ammonium chloride aqueous solution (50 mL), saturated sodium bicarbonate aqueous solution (50 mL), and brine (50 mL). The organic layer was dried over anhydrous magnesium sulfate, filtered, and concentrated. After solidification with diethyl ether/ethyl acetate (10:1) solution (20 mL), filtration and air drying were performed to obtain the target compound **1-2** (1.51 g, 72%). [HATU (1-[Bis(dimethylamino)methylene]-1*H*-1,2,3-triazolo[4,5-*b*]pyridinium 3-oxide hexafluorophosphate, Hexafluorophosphate Azabenzotriazole Tetramethyl Uronium), DIPEA (N,N-Diisopropylethylamine)]

Step 1-3: Compound **1-2** (70 mg, 0.19 mmol) was dissolved in 1,4-dioxane (7 mL), benzeneboronic acid (24 mg, 0.19 mmol), tetrakis(triphenylphosphine)palladium (57 mg, 0.04 mmol), and 2 M potassium carbonate aqueous solution (0.19 mL, 0.39 mmol) were added, and nitrogen was flowed for 10 minutes, and then the mixture was stirred at 100 °C for 12 hours. After completion of the reaction, the reaction solution was concentrated and diluted with dichloromethane (10 mL). The remaining palladium was filtered off using diatomaceous earth (Celite), and the filtrate was washed with water and brine. The organic layer was dried over anhydrous magnesium sulfate, filtered, concentrated, and separated using column chromatography to obtain the target compound **1** (42.3 mg, 70%).

$^1$H NMR (400 MHz, DMSO-$d_6$) δ(ppm): 8.23 (s, 1H), 7.90 (ddd, *J* = 16.6, 8.4, 2.0 Hz, 3H), 7.60-7.45 (m, 4H), 7.16 (dd, *J* = 7.1, 1.4 Hz, 1H), 3.62 (t, *J* = 5.4 Hz, 4H), 1.65 (s, 2H), 1.60-1.43 (m, 4H).

MS (ESI, LR) Calc. for $C_{19}H_{20}N_3O$ $[M+H]^+$: 306.2, found: 306.2

**Example 2. (7-(3-chlorophenyl)pyrazolo[1,5-*a*]pyridin-3-yl)(piperidin-1-yl)methanone**

**Example 3. methyl 3-(3-(piperidine-1-carbonyl)pyrazolo[1,5-*a*]pyridin-7-yl)benzoate**

**Example 4. (7-(3-methoxyphenyl)pyrazolo[1,5-*a*]pyridin-3-yl)(piperidin-1-yl)methanone**

**Example 5. (7-(3-nitrophenyl)pyrazolo[1,5-*a*]pyridin-3-yl)(piperidin-1-yl)methanone**

**Example 6. 3-(3-(piperidine-1-carbonyl)pyrazolo[1,5-*a*]pyridin-7-yl)benzonitrile**

**Example 8. (7-(2-chloropyridin-4-yl)pyrazolo[1,5-*a*]pyridin-3-yl)(piperidin-1-**yl)methanone

**Example 10. 4-(3-(piperidine-1-carbonyl)pyrazolo[1,5-*a*]pyridin-7-yl)benzoic acid**

**Example 11. methyl 4-(3-(piperidine-1-carbonyl)pyrazolo[1,5-*a*]pyridin-7-yl)benzoate**

**Example 12. (7-(4-chlorophenyl)pyrazolo[1,5-*a*]pyridin-3-yl)(piperidin-1-yl)methanone**

**Example 13. (7-(4-nitrophenyl)pyrazolo[1,5-*a*]pyridin-3-yl)(piperidin-1-yl)methanone**

**Example 15.** **4-(3-(piperidine-1-carbonyl)pyrazolo[1,5-*a*]pyridin-7-yl)benzonitrile**

**Example 16.** **(7-(6-nitropyridin-3-yl)pyrazolo[1,5-*a*]pyridin-3-yl)(piperidin-1-yl)methanone**

**Example 18.** **5-(3-(piperidine-1-carbonyl)pyrazolo[1,5-*a*]pyridin-7-yl)picolinonitrile**

**Example 19.** **(7-(6-fluoropyridin-3-yl)pyrazolo[1,5-*a*]pyridin-3-yl)(piperidin-1-yl)methanone**

**Example 20.** **(7-(4-methoxy-3-(trifluoromethyl)phenyl)pyrazolo[1,5-*a*]pyridin-3-yl)(piperidin-1-yl)methanone**

**Example 21.** **(7-(3-chloro-4-hydroxyphenyl)pyrazolo[1,5-*a*]pyridin-3-yl)(piperidin-1-yl)methanone]**

**Example 22.** **2-fluoro-4-(3-(piperidine-1-carbonyl)pyrazolo[1,5-*a*]pyridin-7-yl)benzonitrile**

**Example 23.** **2-(dimethylamino)-4-(3-(piperidine-1-carbonyl)pyrazolo[1,5-*a*]pyridin-7-yl)benzonitrile**

**Example 24.** **(7-(3,4-dimethoxyphenyl)pyrazolo[1,5-*a*]pyridin-3-yl)(piperidin-1-yl)methanone**

**Example 25.** **(7-(1-methyl-1*H*-pyrazol-5-yl)pyrazolo[1,5-*a*]pyridin-3-yl)(piperidin-1-yl)methanone**

**Example 120.** ***tert*-butyl 4-(3-(piperidine-1-carbonyl)pyrazolo[1,5-*a*]pyridin-7-yl)-3,6-dihydropyridine-1(2H)-carboxylate**

**Example 121.** **(7-(benzo[*d*][1,3]dioxol-5-yl)pyrazolo[1,5-*a*]pyridin-3-yl)(piperidin-1-yl)methanone**

**Example 122.** **(7-(benzofuran-3-yl)pyrazolo[1,5-*a*]pyridin-3-yl)(piperidin-1-yl)methanone**

**Example 123.** **(7-(benzofuran-2-yl)pyrazolo[1,5-*a*]pyridin-3-yl)(piperidin-1-yl)methanone**

**Example 124.** **(7-(benzo[*b*]thiophen-3-yl)pyrazolo[1,5-*a*]pyridin-3-yl)(piperidin-1-yl)methanone**

**Example 125.** **(7-(1*H*-indazol-6-yl)pyrazolo[1,5-*a*]pyridin-3-yl)(piperidin-1-yl)methanone**

**Example 126.** **(7-(1*H*-indol-5-yl)pyrazolo[1,5-*a*]pyridin-3-yl)(piperidin-1-yl)methanone**

**Example 127.** **(7-(benzofuran-5-yl)pyrazolo[1,5-*a*]pyridin-3-yl)(piperidin-1-yl)methanone**

**Example 128.** **(7-(naphthalen-1-yl)pyrazolo[1,5-*a*]pyridin-3-yl)(piperidin-1-yl)methanone**

**Example 129.** **(7-(naphthalen-2-yl)pyrazolo[1,5-*a*]pyridin-3-yl)(piperidin-1-yl)methanone**

**Example 130.** **piperidin-1-yl(7-(quinolin-3-yl)pyrazolo[1,5-*a*]pyridin-3-yl)methanone**

**Example 131.** 7-(3-(piperidine-1-carbonyl)pyrazolo[1,5**-*a*]pyridin-7-yl)-3,4-dihydroisoquinolin-1(2*H*)-one**

**Example 134.** **7-(3-(piperidine-1-carbonyl)pyrazolo[1,5-*a*]pyridin-7-yl)isoquinolin-1(2*H*)-one**

**Example 136.** **6-(3-(piperidine-1-carbonyl)pyrazolo[1,5-*a*]pyridin-7-yl)-3,4-dihydroisoquinolin-1(2H)-one**

**Example 138.** **6-(3-(piperidine-1-carbonyl)pyrazolo[1,5-*a*]pyridin-7-yl)isoquinolin-1(2*H*)-one**

**Example 140.** **6-(3-(piperidine-1-carbonyl)pyrazolo[1,5-*a*]pyridin-7-yl)isoindolin-1-one**

**Example 143.** **5-(3-(piperidine-1-carbonyl)pyrazolo[1,5-*a*]pyridin-7-yl)isoindolin-1-one**

**Example 145.** **6-(3-(piperidine-1-carbonyl)pyrazolo[1,5-*a*]pyridin-7-yl)quinolin-2(1*H*)-one**

**Example 195.** **3-(3-(piperidine-1-carbonyl)pyrazolo[1,5-*a*]pyridin-7-yl)-7,8-dihydro-1,6-naphthyridin-5(6*H*)-one**

**Example 205. 3-fluoro-5-[3-(piperidine-1-carbonyl)pyrazolo[1,5-*a*]pyridin-7-yl]pyridine-2-carbonitrile**

**Example 207. 3-methyl-6-(3-(piperidine-1-carbonyl)pyrazolo[1,5-a]pyridin-7-yl)pyrimidin-4(3*H*)-one**

**Example 224. 7-(3-(piperidine-1-carbonyl)pyrazolo[1,5-*a*]pyridin-7-yl)phthalazin-1(2*H*)-one**

**Example 232. 3-(3-(piperidine-1-carbonyl)pyrazolo[1,5-*a*]pyridin-7-yl)-6,7-dihydro-5*H*-pyrrolo[3,4-b]pyridin-5-one**

**Example 233. 6-(3-(piperidine-1-carbonyl)pyrazolo[1,5-*a*]pyridin-7-yl)phthalazin-1(2*H*)-one**

**Example 244. methyl 3-fluoro-5-[3-(piperidine-1-carbonyl)pyrazolo[1,5-*a*]pyridin-7-yl]pyridine-2-carboxylate**

**Example 245. 3-methyl-6-(3-(piperidine-1-carbonyl)pyrazolo[1,5-*a*]pyridin-7-yl)quinazolin-4(3*H*)-one**

**Example 246. 2-methyl-6-(3-(piperidine-1-carbonyl)pyrazolo[1,5-*a*]pyridin-7-yl)-[1,2,4]triazolo[4,3-*a*]pyridin-3(2*H*)-one**

**Example 251. 3-methyl-7-(3-(piperidine-1-carbonyl)pyrazolo[1,5-*a*]pyridin-7-yl)quinazolin-4(3*H*)-one**

**Example 252. 2-methyl-7-(3-(piperidine-1-carbonyl)pyrazolo[1,5-*a*]pyridin-7-yl)-[1,2,4]triazolo[4,3-*a*]pyridin-3(2*H*)-one**

**Example 254. 2-fluoro-5-[3-(piperidine-1-carbonyl)pyrazolo[1,5-*a*]pyridin-7-yl]benzonitrile**

**Example 255. 3-methyl-6-(3-(piperidine-1-carbonyl)pyrazolo[1,5-*a*]pyridin-7-yl)pyrido[2,3-*d*]pyrimidin-4(3*H*)-one**

**Example 256. 3-methyl-7-(3-(piperidine-1-carbonyl)pyrazolo[1,5-*a*]pyridin-7-yl)pyrido[3,2-*d*]pyrimidin-4(3*H*)-one**

**Example 264. (7-(2-chlorophenyl)pyrazolo[1,5-*a*]pyridin-3-yl)(piperidin-1-yl)methanone**

**Example 265. (7-(5-bromo-2-fluoropyridin-3-yl)pyrazolo[1,5-*a*]pyridin-3-yl)(piperidin-1-yl)methanone**

**Example 266. (7-(2,6-difluoropyridin-3-yl)pyrazolo[1,5-*a*]pyridin-3-yl)(piperidin-1-yl)methanone**

**Example 267. (7-(2-chloropyrimidin-5-yl)pyrazolo[1,5-*a*]pyridin-3-yl)(piperidin-1-yl)methanone**

Example 268. **2-methyl-5-(3-(piperidine-1-carbonyl)pyrazolo[1,5-*a*]pyridin-7-**yl)benzo[d]isoxazol-3(2H)-one

Example 269. (7-(3-methoxybenzo[d]isoxazol-5-yl)pyrazolo[1,5-a]pyridin-3-yl)(piperidin-1-yl)methanone

**Example 270. 2-(dimethylamino)-5-(3-(piperidine-1-carbonyl)pyrazolo[1,5-a]pyridin-7-yl)nicotinonitrile**

**Example 271. (7-(6-fluoropyridin-2-yl)pyrazolo[1,5-a]pyridin-3-yl)(piperidin-1-yl)methanone**

**Example 272. (7-(2-chloro-6-(trifluoromethyl)pyridin-3-yl)pyrazolo[1,5-a]pyridin-3-yl)(piperidin-1-yl)methanone**

**Example 273. (7-(2,6-difluoropyridin-4-yl)pyrazolo[1,5-a]pyridin-3-yl)(piperidin-1-yl)methanone**

**Example 279. (7-(3-chloro-5-fluorophenyl)pyrazolo[1,5-a]pyridin-3-yl)(piperidin-1-yl)methanone**

**Example 280. 5,6-dimethyl-3-(3-(piperidine-1-carbonyl)pyrazolo[1,5-a]pyridin-7-yl)-5,6-dihydro-7*H*-pyrrolo[3,4-*b*]pyridin-7-one**

**Example 281. (7-(6-chloro-5-fluoropyridin-3-yl)pyrazolo[1,5-a]pyridin-3-yl)(piperidin-1-yl)methanone**

**Example 282. (7-(2,6-dichloropyridin-4-yl)pyrazolo[1,5-a]pyridin-3-yl)(piperidin-1-yl)methanone**

**Example 283. (7-(5-chloro-2-fluoropyridin-3-yl)pyrazolo[1,5-a]pyridin-3-yl)(piperidin-1-yl)methanone**

**Example 284. (7-(5-fluoropyridin-3-yl)pyrazolo[1,5-*a*]pyridin-3-yl)(piperidin-1-yl)methanone**

**Example 285. 3-fluoro-4-[3-(piperidine-1-carbonyl)pyrazolo[1,5-*a*]pyridin-7-yl]benzonitrile**

**Example 288. (7-(5,6-difluoropyridin-3-yl)pyrazolo[1,5-*a*]pyridin-3-yl)(piperidin-1-yl)methanone**

**Example 289. (7-(5-chloro-6-fluoropyridin-3-yl)pyrazolo[1,5-*a*]pyridin-3-yl)(piperidin-1-yl)methanone**

**Example 290. 2,6-difluoro-4-(3-(piperidine-1-carbonyl)pyrazolo[1,5-*a*]pyridin-7-yl)benzonitrile**

**Example 291. 3-fluoro-5-(3-(piperidine-1-carbonyl)pyrazolo[1,5-*a*]pyridin-7-yl)benzonitrile**

**Example 292. (7-(4-bromophenyl)pyrazolo[1,5-*a*]pyridin-3-yl)(piperidin-1-yl)methanone**

**Example 293. 5-[3-(piperidine-1-carbonyl)pyrazolo[1,5-*a*]pyridin-7-yl]pyrimidine-2-carbonitrile**

**Example 294. [7-(2-hydroxypyrimidin-5-yl)pyrazolo[1,5-*a*]pyridin-3-yl]-(1-piperidyl)methanone**

**Example 298. (7-(5-chloro-2-fluoropyridin-4-yl)pyrazolo[1,5-*a*]pyridin-3-yl)(piperidin-1-yl)methanone**

**Example 312. 3-nitro-5-(3-(piperidine-1-carbonyl)pyrazolo[1,5-*a*]pyridin-7-yl)benzonitrile**

**Example 313. (7-(6-bromopyridin-3-yl)pyrazolo[1,5-*a*]pyridin-3-yl)(piperidin-1-yl)methanone**

**Example 314. (7-(6-chloropyridin-3-yl)pyrazolo[1,5-*a*]pyridin-3-yl)(piperidin-1-yl)methanone**

**Example 315. (7-(6-chloro-2-fluoropyridin-3-yl)pyrazolo[1,5-*a*]pyridin-3-yl)(piperidin-1-yl)methanone**

**Example 318. (7-(5-nitropyridin-3-yl)pyrazolo[1,5-*a*]pyridin-3-yl)(piperidin-1-yl)methanone**

For Examples 2, 3, 4, 5, 6, 8, 10, 11, 12, 13, 15, 16, 18, 19, 20, 21, 22, 23, 24, 25, 120, 121, 122, 123, 124, 125, 126, 127, 128, 129, 130, 131, 134, 136, 138, 140, 143, 145, 195, 205, 207, 224, 232, 233, 244, 245, 246, 251, 252, 254, 255, 256, 264, 265, 266, 267, 268, 269, 270, 271, 272, 273, 279, 280, 281, 282, 283, 284, 285, 288, 289, 290, 291, 292, 293, 294, 298, 312, 313, 314, 315, 318, the compounds were obtained by the same synthetic method as step 1-3 in Example **1** using the corresponding compounds and compound **1-2.** The structural and spectral data are shown in Table 1 below.

[Table 1]

| Example | Structure | $^1$H NMR | MS |
|---|---|---|---|
| **2** | 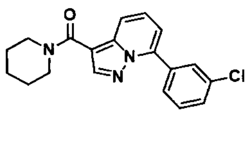 | $^1$H NMR (400 MHz, DMSO-$d_6$) δ(ppm): 8.26 (s, 1H), 8.05 (d, *J* = 2.1 Hz, 1H), 7.93-7.84 (m, 2H), 7.64-7.56 (m, 2H), 7.52 (dd, *J* = 8.9, 7.0 Hz, 1H), 7.24 (dd, *J* = 7.1, 1.3 Hz, 1H), 3.62 (t, *J* = 5.4 Hz, 4H), 1.65 (d, *J* = 6.5 Hz, 2H), 1.57 (d, *J* = *8.4* Hz, 4H). | MS (ESI, LR) Calc. for $C_{19}H_{19}ClN_3O$ $[M+H]^+$: 340.1, found: 340.1. |
| **3** | 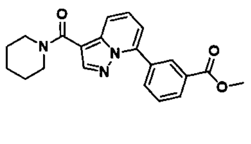 | $^1$H NMR (400 MHz, DMSO-$d_6$) δ(ppm): 8.53 (t, *J* = 1.8 Hz, 1H), 8.27 (s, 1H), 8.18 (dt, *J* = *7.8,* 1.5 Hz, 1H), 8.12 (dt, *J* = 7.8, 1.4 Hz, 1H), 7.92 (dd, *J* = 8.9, 1.4 Hz, 1H), 7.73 (t, *J* = 7.8 Hz, 1H), 7.53 (dd, *J* = 8.9, 7.1 Hz, 1H), 7.25 (dd, *J* = 7.0, 1.4 Hz, 1H), 3.90 (s, 3H), 3.63 (t, *J* = 5.4 Hz, 4H), 1.65 (d, *J* = 5.6 Hz, 2H), 1.57 (d, *J* = 7.2 Hz, 4H). | MS (ESI, LR) Calc. for $C_{21}H_{22}N_3O_3$ $[M+H]^+$: 364.2, found: 364.2. |

(continued)

| Example | Structure | $^1$H NMR | MS |
|---|---|---|---|
| **4** | 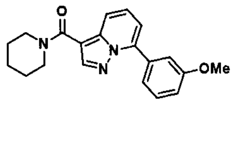 | $^1$H NMR (400 MHz, DMSO-$d_6$) δ(ppm): 8.23 (s, 1H), 7.88 (dd, $J$ = 8.9, 1.4 Hz, 1H), 7.53-7.45 (m, 4H), 7.18 (dd, $J$ = 7.0, 1.4 Hz, 1H), 7.14-7.08 (m, 1H), 3.83 (s, 3H), 3.62 (t, $J$ = 5.4 Hz, 4H), 1.69-1.61 (m, 2H), 1.61-1.51 (m, 4H). | MS (ESI, LR) Calc. for $C_{20}H_{22}N_3O_2$ $[M+H]^+$: 336.2, found: 336.1. |
| **5** | 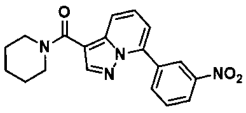 | $^1$H NMR (400 MHz, DMSO-$d_6$) δ(ppm): 8.87 (t, $J$ = 2.0 Hz, 1H), 8.42-8.35 (m, 2H), 8.29 (s, 1H), 7.95 (dd, $J$ = 8.9, 1.3 Hz, 1H), 7.87 (t, $J$ = 8.0 Hz, 1H), 7.56 (dd, $J$ = 8.9, 7.0 Hz, 1H), 7.36 (dd, $J$ = 7.1, 1.3 Hz, 1H), 3.63 (t, $J$ = 5.4 Hz, 4H), 1.66 (d, $J$ = 5.7 Hz, 2H), 1.57 (d, $J$ = 7.6 Hz, 4H). | MS (ESI, LR) Calc. for $C_{19}H_{19}N_4O_3$ $[M+H]^+$: 351.1, found: 351.1. |
| **6** | 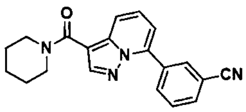 | $^1$H NMR (400 MHz, DMSO-$d_6$) δ(ppm): 8.21 (s, 1H), 7.83 (dd, $J$= 8.9, 1.4 Hz, 1H), 7.47 (dd, $J$ = 8.9, 7.0 Hz, 1H), 7.17 (d, $J$ = 7.8 Hz, 1H), 7.09 (t, $J$ = 2.0 Hz, 1H), 7.05 (dd, $J$ = 7.1, 1.4 Hz, 1H), 6.99-6.94 (m, 1H), 6.75-6.68 (m, 1H), 5.27 (s, 2H), 3.62 (t, $J$ = 5.4 Hz, 4H), 1.65 (d, $J$ = 5.2 Hz, 2H), 1.57 (s, 4H). | MS (ESI, LR) Calc. for $C_{20}H_{19}N_4O$ $[M+H]^+$: 331.2, found: 331.2. |
| **8** | 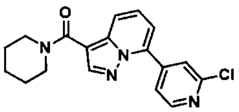 | $^1$H NMR (400 MHz, DMSO-$d_6$) δ(ppm): 8.62 (d, $J$ = 5.2 Hz, 1H), 8.30 (s, 1H), 8.17 (d, $J$ = 1.5 Hz, 1H), 8.03-7.94 (m, 2H), 7.55 (dd, $J$ = 8.9, 7.0 Hz, 1H), 7.43 (dd, $J$ = 7.1, 1.3 Hz, 1H), 3.62 (t, $J$ = 5.4 Hz, 4H), 1.65 (d, $J$ = 5.6 Hz, 2H), 1.62-1.52 (m, 4H). | MS (ESI, LR) Calc. for $C_{18}H_{18}ClN_{40}$ $[M+H]^+$: 341.1, found: 341.0. |
| **10** | 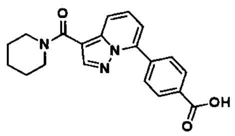 | $^1$H NMR (400 MHz, DMSO-$d_6$) δ(ppm): 8.25 (s, 1H), 8.08 (q, $J$ = 8.5 Hz, 4H), 7.91 (dd, $J$ = 8.9, 1.4 Hz, 1H), 7.53 (dd, $J$ = 8.9, 7.1 Hz, 1H), 7.25 (dd, $J$ = 7.0, 1.4 Hz, 1H), 3.62 (t, $J$ = 5.4 Hz, 4H), 1.72-1.61 (m, 2H), 1.58-1.54 (m, 4H). | MS (ESI, LR) Calc. for $C_{20}H_{20}N_3O_3$ $[M+H]^+$: 350.1, found: 350.2. |
| **11** | 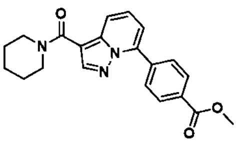 | $^1$H NMR (400 MHz, DMSO-$d_6$) δ(ppm): 8.26 (s, 1H), 8.18 - 8.06 (m, 4H), 7.92 (dd, $J$ = 8.9, 1.3 Hz, 1H), 7.54 (dd, $J$ = 8.9, 7.0 Hz, 1H), 7.27 (dd, $J$ = 7.0, 1.4 Hz, 1H), 3.92 (s, 3H), 3.62 (t, J = 5.4 Hz, 4H), 1.65 (d, $J$ = 5.6 Hz, 2H), 1.57 (s, 4H). | MS (ESI, LR) Calc. for $C_{21}H_{22}N_3O_3$ $[M+H]^+$: 364.2, found: 364.2. |
| **12** | 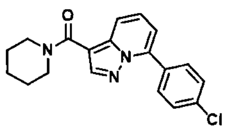 | $^1$H NMR (400 MHz, DMSO-$d_6$) δ(ppm): 8.24 (s, 1H), 8.02-7.93 (m, 2H), 7.89 (dd, $J$ = 8.9, 1.3 Hz, 1H), 7.68-7.58 (m, 2H), 7.51 (dd, $J$ = 8.9, 7.1 Hz, 1H), 7.20 (dd, $J$ = 7.1, 1.4 Hz, 1H), 3.62 (t, $J$ = 5.4 Hz, 4H), 1.65 (d, $J$ = 8.2 Hz, 2H), 1.57 (d, $J$ = 7.7 Hz, 4H). | MS (ESI, LR) Calc. for $C_{19}H_{19}ClN_3O$ $[M+H]^+$: 340.1, found: 340.0. |
| **13** | 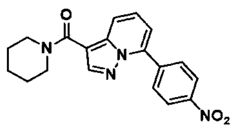 | $^1$H NMR (400 MHz, DMSO-$d_6$) δ(ppm): 8.46-8.36 (m, 2H), 8.32-8.20 (m, 3H), 7.96 (dd, $J$ = 8.9, 1.3 Hz, 1H), 7.56 (dd, $J$ = 8.9, 7.1 Hz, 1H), 7.33 (dd, $J$ = 7.1, 1.4 Hz, 1H), 3.63 (t, $J$ = 5.4 Hz, 4H), 1.65 (d, $J$ = 5.2 Hz, 2H), 1.62-1.45 (m, 4H). | MS (ESI, LR) Calc. for $C_{19}H_{19}N_4O_3$ $[M+H]^+$: 351.1, found: 351.1. |
| **15** | 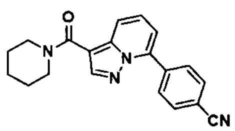 | $^1$H NMR (400 MHz, DMSO-$d_6$) δ(ppm): 8.26 (s, 1H), 8.20-8.11 (m, 2H), 8.09-7.99 (m, 2H), 7.94 (dd, $J$ = 8.9, 1.3 Hz, 1H), 7.54 (dd, $J$ = 8.9, 7.0 Hz, 1H), 7.28 (dd, $J$ = 7.0, 1.3 Hz, 1H), 3.62 (t, $J$ = 5.5 Hz, 4H), 1.71-1.62 (m, 2H), 1.57 (s, 4H). | MS (ESI, LR) Calc. for $C_{20}H_{19}N_4O$ $[M+H]^+$: 331.2, found: 331.2. |

(continued)

| Example | Structure | $^1$H NMR | MS |
|---|---|---|---|
| **16** | 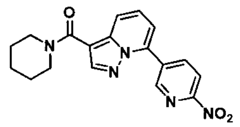 | $^1$H NMR (400 MHz, DMSO-$d_6$) δ(ppm): 9.21 (d, $J$ = 2.2 Hz, 1H), 8.87 (dd, $J$ = 8.5, 2.3 Hz, 1H), 8.51 (d, $J$ = 8.4 Hz, 1H), 8.29 (s, 1H), 8.00 (dd, $J$ = 8.9, 1.3 Hz, 1H), 7.67-7.64 (m, 1H), 7.46 (dd, $J$ = 7.0, 1.4 Hz, 1H), 3.63 (t, $J$ = 5.4 Hz, 4H), 1.66 (d, $J$ = 5.4 Hz, 2H), 1.57 (d, $J$ = 7.1 Hz, 4H). | MS (ESI, LR) Calc. for $C_{18}H_{18}N_3O_3$ $[M+H]^+$: 352.1, found: 352.1. |
| **18** | 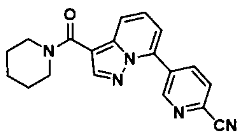 | $^1$H NMR (400 MHz, DMSO-$d_6$) δ(ppm): 9.30 (d, $J$ = 2.2 Hz, 1H), 8.68 (dd, $J$ = 8.2, 2.2 Hz, 1H), 8.30-8.24 (m, 2H), 7.98 (dd, $J$ = 8.9, 1.3 Hz, 1H), 7.65 (t, $J$ = 1.3 Hz, 1H), 7.44-7.39 (m, 1H), 3.62 (t, $J$ = 5.5 Hz, 4H), 1.65 (d, $J$ = 7.9 Hz, 2H), 1.56 (s, 4H). | MS (ESI, LR) Calc. for $C_{19}H_{18}N_5O$ $[M+H]^+$: 332.1, found: 332.1. |
| **19** | 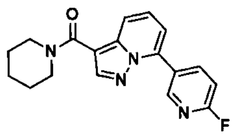 | $^1$H NMR (400 MHz, DMSO-$d_6$) δ(ppm): 8.77 (d, $J$ = 2.5 Hz, 1H), 8.60 (td, $J$ = 8.3, 2.5 Hz, 1H), 8.25 (d, $J$ = 5.6 Hz, 1H), 7.93 (d, $J$ = 8.9 Hz, 1H), 7.65 (s, 1H), 7.41 (dd, $J$ = 8.6, 2.8 Hz, 1H), 7.30 (d, $J$ = 7.1 Hz, 1H), 3.62 (t, $J$ = 5.5 Hz, 4H), 1.65 (d, $J$ = 6.0 Hz, 2H), 1.56 (s, 4H). | MS (ESI, LR) Calc. for $C_{18}H_{18}FN_4O$ $[M+H]^+$: 325.1, found: 325.1. |
| **20** | 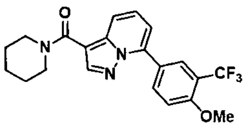 | $^1$H NMR (400 MHz, DMSO-$d_6$) δ(ppm): 8.27 (d, $J$ = 2.7 Hz, 2H), 8.20 (dd, $J$ = 8.8, 2.3 Hz, 1H), 7.88 (dd, $J$ = 8.8, 1.3 Hz, 1H), 7.55-7.43 (m, 2H), 7.24 (dd, $J$ = 7.1, 1.4 Hz, 1H), 4.00 (s, 3H), 3.62 (t, $J$ = 5.4 Hz, 4H), 1.65 (d, $J$ = 5.4 Hz, 2H), 1.61-1.49 (m, 4H). | MS (ESI, LR) Calc. for $C_{21}H_{21}F_3N_3O_2$ $[M+H]^+$: 404.2, found: 404.1. |
| **21** | 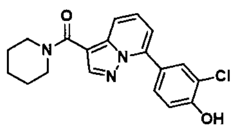 | $^1$H NMR (400 MHz, DMSO-$d_6$) δ(ppm): 8.25 (s, 1H), 8.04 (d, $J$ = 2.2 Hz, 1H), 7.83 (dd, $J$ = 8.9, 1.3 Hz, 1H), 7.74 (dd, $J$ = 8.5, 2.3 Hz, 1H), 7.48 (dd, $J$ = 8.9, 7.1 Hz, 1H), 7.15 (dd, $J$ = 7.1, 1.4 Hz, 1H), 7.12 (d, $J$ = 8.6 Hz, 1H), 3.62 (t, $J$= 5.4 Hz, 4H), 1.65 (d, $J$ = 5.0 Hz, 2H), 1.57 (d, $J$ = 7.0 Hz, 4H). | MS (ESI, LR) Calc. for $C_{19}H_{19}ClN_3O_2$ $[M+H]^+$: 356.1, found: 356.1. |
| **22** | 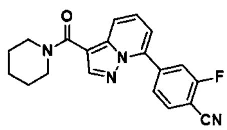 | $^1$H NMR (400 MHz, DMSO-$d_6$) δ(ppm): 8.28 (s, 1H), 8.20 (dd, $J$ = 10.8, 1.5 Hz, 1H), 8.13 (dd, $J$ = 8.2, 6.9 Hz, 1H), 8.02 (dd, $J$ = 8.2, 1.6 Hz, 1H), 7.96 (dd, $J$ = 8.9, 1.4 Hz, 1H), 7.55 (dd, $J$ = 8.9, 7.1 Hz, 1H), 7.36 (dd, $J$ = 7.1, 1.3Hz, 1H), 3.62 (t, $J$ = 5.4 Hz, 4H), 1.65 (d, $J$ = 8.2 Hz, 2H), 1.57 (s, 4H). | MS (ESI, LR) Calc. for $C_{20}H_{18}FN_4O$ $[M+H]^+$: 349.1, found: 349.1. |
| **23** | 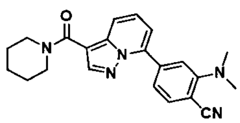 | $^1$H NMR (400 MHz, DMSO-$d_6$) δ(ppm): 8.24 (s, 1H), 7.92 (dd, J=9.0, 1.4 Hz, 1H), 7.76 (d, $J$ = 8.1 Hz, 1H), 7.55-7.49 (m, 2H), 7.43 (dd, $J$ = 8.1, 1.5 Hz, 1H), 7.27 (dd, $J$ = 7.1, 1.3 Hz, 1H), 3.62 (t, $J$ = 5.4 Hz, 4H), 3.06 (s, 6H), 1.65 (d, $J$ = 7.4 Hz, 2H), 1.62-1.53 (m, 4H). | MS (ESI, LR) Calc. for $C_{22}H_{24}N_5O$ $[M+H]^+$: 374.2, found: 374.2. |
| **24** | 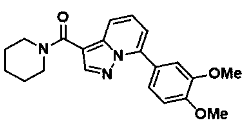 | $^1$H NMR (400 MHz, DMSO-$d_6$) δ(ppm): 8.24 (s, 1H), 7.84 (dd, $J$ = 8.9, 1.3 Hz, 1H), 7.64-7.61 (m, 2H), 7.49 (dd,$J$ = 8.9, 7.0 Hz, 1H), 7.17 (dd, $J$ = 7.0, 1.4 Hz, 1H), 7.13 (d,$J$ = 8.3 Hz, 1H), 3.85 (s, 3H), 3.82 (s, 3H), 3.62 (t, $J$ = 5.5 Hz, 4H), 1.65 (d, $J$ = 6.9 Hz, 2H), 1.57 (d, $J$ = 8.4 Hz, 4H). | MS (ESI, LR) Calc. for $C_{21}H_{24}N_3O_3$ $[M+H]^+$: 366.2, found: 366.1. |
| **25** | 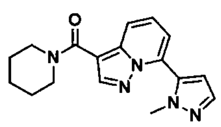 | $^1$H NMR (400 MHz, DMSO-$d_6$) δ(ppm): 8.26 (s, 1H), 7.97 (dd, $J$ = 9.0, 1.4 Hz, 1H), 7.61 (d, $J$ = 1.9 Hz, 1H), 7.52 (dd, $J$ = 8.9, 7.0 Hz, 1H), 7.22 (dd, $J$ = 7.0, 1.3 Hz, 1H), 6.69 (d, $J$ = 1.9 Hz, 1H), 3.74 (s, 3H), 3.62 (t, $J$ = 5.5 Hz, 4H), 1.70-1.61 (m, 2H), 1.57 (s, 4H). | MS (ESI, LR) Calc. for $C_{17}H_{20}N_5O$ $[M+H]^+$: 310.2, found: 310.1. |

(continued)

| Example | Structure | $^1$H NMR | MS |
|---|---|---|---|
| **120** | 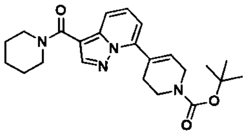 | $^1$H NMR (400 MHz, DMSO-$d_6$) δ(ppm): 8.24 (d, $J$ = 5.2 Hz, 1H), 7.82 (dd, $J$ = 15.5, 8.7 Hz, 1H), 7.43 (dt, $J$ = 8.9, 6.3 Hz, 1H), 7.02 (dd, $J$ = 23.5, 7.1 Hz, 1H), 6.51 (d, $J$ = 27.0 Hz, 1H), 4.10 (s, 2H), 3.60 (t, $J$ = 5.3 Hz, 6H), 2.68 (s, 2H), 1.64 (s, 2H), 1.56 (s, 4H), 1.46 (d, $J$ = 4.8 Hz, 9H). | MS (ESI, LR) Calc. for $C_{23}H_{31}N_4O_3$ $[M+H]^+$: 411.2, found: 411.2. |
| **121** | 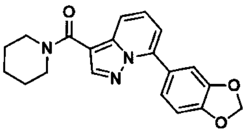 | $^1$H NMR (400 MHz, DMSO-$d_6$) δ(ppm): 8.23 (s, 1H), 7.84 (dd, $J$ = 8.9, 1.3 Hz, 1H), 7.53 (d, $J$ = 1.7 Hz, 1H), 7.48 (dd, $J$ = 8.9, 7.1 Hz, 1H), 7.43 (dd, $J$ = 8.1, 1.8 Hz, 1H), 7.13 (dd, $J$ = 7.1, 1.4 Hz, 1H), 7.10 (d, $J$ = 8.1 Hz, 1H), 6.14 (s, 2H), 3.62 (t, $J$ = 5.3 Hz, 4H), 1.65 (s, 2H), 1.57 (s, 4H). | MS (ESI, LR) Calc. for $C_{20}H_{20}N_3O_3$ $[M+H]^+$: 350.1, found: 350.1. |
| **122** | 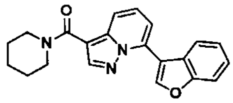 | $^1$H NMR (400 MHz, DMSO-$d_6$) δ(ppm): 9.16 (s, 1H), 8.36 (s, 1H), 8.03 (d, $J$ = 7.8 Hz, 1H), 7.93 (dd, $J$ = 8.8, 1.4 Hz, 1H), 7.79 (d, $J$ = 8.1 Hz, 1H), 7.68 (dd, $J$ = 7.2, 1.4 Hz, 1H), 7.60 (dd, $J$ = 8.8, 7.1 Hz, 1H), 7.53-7.46 (m, 1H), 7.46-7.40 (m, 1H), 3.64 (t, $J$ = 5.4 Hz, 4H), 1.66 (d, $J$ = 5.5 Hz, 2H), 1.58 (d, $J$ = 7.6 Hz, 4H). | MS (ESI, LR) Calc. for $C_{21}H_{20}N_3O_2$ $[M+H]^+$: 346.2, found: 346.1. |
| **123** | 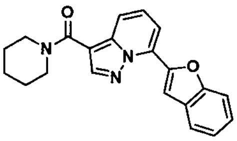 | $^1$H NMR (400 MHz, DMSO-$d_6$) δ(ppm): 8.47 (d, $J$ = 1.6 Hz, 2H), 7.97 (dd, $J$ = 8.8, 1.3 Hz, 1H), 7.91-7.82 (m, 2H), 7.74 (d, $J$ = 8.3 Hz, 1H), 7.63 (dd, $J$ = 8.8, 7.3 Hz, 1H), 7.48 (td, $J$ = 7.8, 1.3 Hz, 1H), 7.37 (t, $J$ = 7.5 Hz, 1H), 3.65 (t, $J$ = 5.4 Hz, 4H), 1.67 (d, $J$ = 6.2 Hz, 2H), 1.59 (s, 4H). | MS (ESI, LR) Calc. for $C_{21}H_{20}N_3O_2$ $[M+H]^+$: 346.2, found: 346.1. |
| **124** | 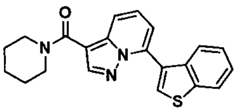 | $^1$H NMR (400 MHz, DMSO-$d_6$) δ(ppm): 8.33 (s, 1H), 8.19 (s, 1H), 8.13 (d, $J$ = 8.0 Hz, 1H), 7.96 (dd, $J$ = 8.9, 1.3 Hz, 1H), 7.60-7.34 (m, 4H), 7.26 (dd, $J$ = 7.0, 1.4 Hz, 1H), 3.63 (t, $J$ = 5.4 Hz, 4H), 1.64 (dd, $J$ = 7.8, 3.6 Hz, 2H), 1.57 (td, $J$ = 6.6, 3.4 Hz, 4H). | MS (ESI, LR) Calc. for $C_{21}H_{20}N_3OS$ $[M+H]^+$: 362.1, found: 362.1. |
| **125** | 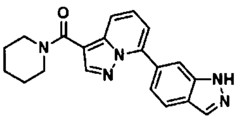 | $^1$H NMR (400 MHz, DMSO-$d_6$) δ(ppm): 13.32 (s, 1H), 8.25 (s, 1H), 8.18 (d, $J$ = 3.9 Hz, 2H), 7.97-7.83 (m, 2H), 7.62-7.49 (m, 2H), 7.26 (dd, $J$ = 7.1, 1.4 Hz, 1H), 3.63 (t, $J$ = 5.4 Hz, 4H), 1.65 (d, $J$ = 7.2 Hz, 2H), 1.57 (d, $J$ = 6.9 Hz, 4H). | MS (ESI, LR) Calc. for $C_{20}H_{20}N_5O$ $[M+H]^+$: 346.2, found: 346.1. |
| **126** | 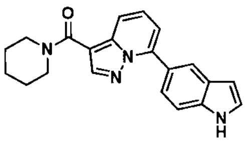 | $^1$H NMR (400 MHz, DMSO-$d_6$) δ(ppm): 11.34 (s, 1H), 8.23 (s, 1H), 8.19-8.12 (m, 1H), 7.83 (dd, $J$ = 8.8, 1.3 Hz, 1H), 7.64 (dd, $J$ = 8.5, 1.7 Hz, 1H), 7.57-7.42 (m, 3H), 7.14 (dd, $J$ = 7.1, 1.4 Hz, 1H), 6.56 (t, $J$ = 2.6 Hz, 1H), 3.63 (t, $J$ = 5.4 Hz, 4H), 1.65 (d, $J$ = 7.6 Hz, 2H), 1.57 (d, J = 6.9 Hz, 4H). | MS (ESI, LR) Calc. for $C_{21}H_{21}N_4O$ $[M+H]^+$: 345.2, found: 345.1. |
| **127** | 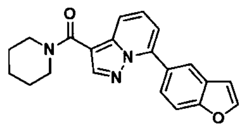 | $^1$H NMR (400 MHz, DMSO-$d_6$) δ(ppm): 8.24 (d, $J$ = 2.1 Hz, 2H), 8.12 (d, $J$ = 2.2 Hz, 1H), 7.86 (ddd, $J$ = 15.3, 8.8, 1.6 Hz, 2H), 7.77 (d, $J$ = 8.6 Hz, 1H), 7.52 (dd, $J$ = 8.9, 7.0 Hz, 1H), 7.19 (dd, $J$ = 7.0, 1.4 Hz, 1H), 7.09 (d, $J$ = 2.2 Hz, 1H), 3.63 (t, $J$ = 5.4 Hz, 4H), 1.69-1.62 (m, 2H), 1.57 (q, $J$ = 5.8 Hz, 4H). | MS (ESI, LR) Calc. for $C_{21}H_{20}N_3O_2$ $[M+H]^+$: 346.2, found: 346.1. |
| **128** | 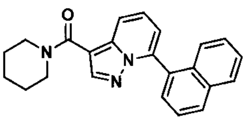 | $^1$HNMR(400 MHz, DMSO-$d_6$) δ(ppm): 8.17-8.11 (m, 1H), 8.06 (d, $J$ = 6.4 Hz, 2H), 8.00 (dd, $J$ = 9.0, 1.4 Hz, 1H), 7.71 - 7.66 (m, 2H), 7.62-7.53 (m, 2H), 7.42 (ddd, $J$ = 8.3, 6.8, 1.3 Hz, 1H), 7.22-7.14 (m, 2H), 3.63 (t, $J$ = 5.4 Hz, 4H), 1.65 (d, $J$ = 5.3 Hz, 2H), 1.57 (d, $J$ = 7.6 Hz, 4H). | MS (ESI, LR) Calc. for $C_{23}H_{22}N_3O$ $[M+H]^+$: 356.2, found: 356.1. |

(continued)

| Example | Structure | $^1$H NMR | MS |
|---|---|---|---|
| **129** | 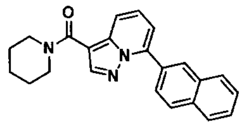 | $^1$H NMR (400 MHz, DMSO-$d_6$) δ(ppm): 8.51 (d, *J* = 1.6 Hz, 1H), 8.27 (s, 1H), 8.12-7.99 (m, 4H), 7.93 (dd, *J* = 8.8, 1.4 Hz, 1H), 7.69-7.49 (m, 3H), 7.31 (dd, *J* = 7.1, 1.4 Hz, 1H), 3.64 (t, *J* = 5.4 Hz, 4H), 1.66 (d, *J* = 5.6 Hz, 2H), 1.58 (d, *J* = 7.1 Hz, 4H). | MS (ESI, LR) Calc. for $C_{23}H_{22}N_3O$ $[M+H]^+$: 356.2, found: 356.1. |
| **130** | 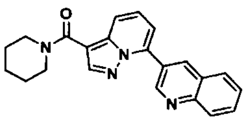 | $^1$H NMR (400 MHz, DMSO-$d_6$) δ(ppm): 9.39 (d, *J* = 2.2 Hz, 1H), 8.98 (d, *J* = 2.2 Hz, 1H), 8.30 (s, 1H), 8.13 (d, *J* = 8.3 Hz, 2H), 7.97 (dd, *J* = 8.9, 1.3 Hz, 1H), 7.94-7.84 (m, 1H), 7.78-7.67 (m, 1H), 7.60 (dd, *J* = 8.9, 7.0 Hz, 1H), 7.43 (dd, *J* = 7.0, 1.3 Hz, 1H), 3.64 (t, *J* = 5.4 Hz, 4H), 1.66 (d, *J* = *6.2* Hz, 2H), 1.58 (d, *J*= 7.7 Hz, 4H). | MS (ESI, LR) Calc. for $C_{22}H_{21}N_4O$ $[M+H]^+$: 357.2, found: 357.1. |
| **131** | 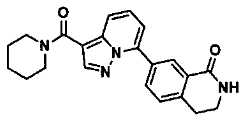 | $^1$H NMR (400 MHz, DMSO-$d_6$) δ(ppm): 8.40 (d, *J* = 2.0 Hz, 1H), 8.24 (s, 1H), 8.07 (s, 1H), 8.00 (dd, *J* = 7.9, 2.0 Hz, 1H), 7.89 (dd, *J* = 8.9, 1.4 Hz, 1H), 7.51 (dd, *J* = 9.3, 7.3 Hz, 2H), 7.19 (dd, *J* = 7.0, 1.4 Hz, 1H), 3.63 (t, *J* = 5.4 Hz, 4H), 3.45 (td, *J* = 6.5, 2.7 Hz, 2H), 3.01 (t, *J* = 6.5 Hz, 2H), 1.68-1.62 (m, 2H), 1.57 (s, 4H). | MS (ESI, LR) Calc. for $C_{22}H_{23}N_4O_2$ $[M+H]^+$: 375.2, found: 375.1. |
| **134** | 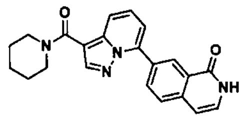 | $^1$H NMR (400 MHz, DMSO-$d_6$) δ(ppm): 11.42 (d, *J* = 5.6 Hz, 1H), 8.78 (d, *J* = 2.0 Hz, 1H), 8.32-8.18 (m, 2H), 7.95-7.77 (m, 2H), 7.54 (dd, *J* = 8.9, 7.0 Hz, 1H), 7.35-7.24 (m, 2H), 6.66 (d, *J* = 7.1 Hz, 1H), 3.64 (t, *J* = 5.5 Hz, 4H), 1.66 (s, 2H), 1.57 (s, 4H). | MS (ESI, LR) Calc. for $C_{22}H_{21}N_4O_2$ $[M+H]^+$: 373.2, found: 373.1. |
| **136** | 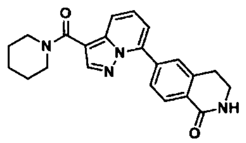 | $^1$H NMR (400 MHz, DMSO-$d_6$) δ(ppm): 8.26 (s, 1H), 8.06 (s, 1H), 7.99 (d, *J* = 8.6 Hz, 1H), 7.95-7.84 (m, 3H), 7.53 (dd, *J* = 8.9, 7.0 Hz, 1H), 7.22 (dd, *J* = 7.1, 1.3 Hz, 1H), 3.62 (t, *J* = 5.6 Hz, 4H), 3.49-3.41 (m, 2H), 3.00 (t, *J* = 6.6 Hz, 2H), 1.65 (d, *J* = 5.4 Hz, 2H), 1.57 (s, 4H). | MS (ESI, LR) Calc. for $C_{22}H_{23}N_4O_2$ $[M+H]^+$: 375.2, found: 375.2. |
| **138** | 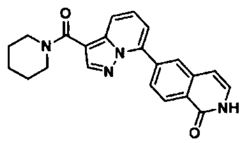 | $^1$H NMR (400 MHz, DMSO-$d_6$) δ(ppm): 11.38 (s, 1H), 8.31 (d, *J* = 8.4 Hz, 1H), 8.27 (s, 1H), 8.22 (d, *J* = 1.8 Hz, 1H), 7.99 (dd, *J* = 8.3, 1.7 Hz, 1H), 7.94 (dd, *J* = 8.9, 1.3 Hz, 1H), 7.56 (dd, *J* = 8.8, 7.0 Hz, 1H), 7.34-7.19 (m, 2H), 6.65 (d, *J* = 7.1 Hz, 1H), 3.63 (t, *J* = 5.4 Hz, 4H), 1.65 (s, 2H), 1.57 (s, 4H). | MS (ESI, LR) Calc. for $C_{22}H_{21}N_4O_2$ $[M+H]^+$: 373.2, found: 373.2. |
| **140** | 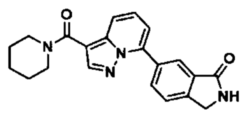 | $^1$H NMR (400 MHz, DMSO-$d_6$) δ(ppm): 8.71 (s, 1H), 8.25 (d, *J* = 3.2 Hz, 2H), 8.12 (dd, *J* = 7.9, 1.7 Hz, 1H), 7.91 (dd, *J* = 8.9, 1.4 Hz, 1H), 7.76 (d, *J* = 7.8 Hz, 1H), 7.53 (dd, *J* = 8.9, 7.0 Hz, 1H), 7.26 (dd, *J* = 7.1, 1.4 Hz, 1H), 4.50 (s, 2H), 3.63 (t, *J* = 5.4 Hz, 4H), 1.73-1.50 (m, 6H). | MS (ESI, LR) Calc. for $C_{21}H_{21}N_4O_2$ $[M+H]^+$: 361.2, found: 361.1. |
| **143** | 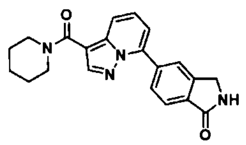 | $^1$H NMR (400 MHz, DMSO-$d_6$) δ(ppm): 8.72 (s, 1H), 8.25 (s, 1H), 8.14 (s, 1H), 8.00 (d, *J* = 8.0 Hz, 1H), 7.94-7.89 (m, 1H), 7.83 (d, *J* = 7.9 Hz, 1H), 7.54 (dd, *J* = 8.9, 7.0 Hz, 1H), 7.23 (d, *J* = 7.0 Hz, 1H), 4.48 (s, 2H), 3.63 (t, *J* = 5.5 Hz, 4H), 1.65 (s, 2H), 1.57 (s, 4H). | MS (ESI, LR) Calc. for $C_{21}H_{21}N_4O_2$ $[M+H]^+$: 361.2, found: 361.2. |
| **145** | 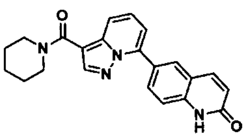 | $^1$H NMR (400 MHz, DMSO-$d_6$) δ(ppm): 11.97 (s, 1H), 8.33-8.20 (m, 2H), 8.06 (dd, *J* = 8.6, 2.0 Hz, 1H), 8.01 (d, *J* = 9.6 Hz, 1H), 7.88 (dd, *J* = 8.9, 1.3 Hz, 1H), 7.53 (dd, *J* = 8.9, 7.0 Hz, 1H), 7.45 (d, *J* = 8.6 Hz, 1H), 7.21 (dd, *J* = 7.0, 1.4 Hz, 1H), 6.58 (d, *J* = 9.5 Hz, 1H), 3.63 (t, *J* = 5.4 Hz, 4H), 1.65 (d, *J* = 6.0 Hz, 2H), 1.57 (s, 4H). | MS (ESI, LR) Calc. for $C_{22}H_{21}N_4O_2$ $[M+H]^+$: 373.2, found: 373.1. |

(continued)

| Example | Structure | $^1$H NMR | MS |
|---|---|---|---|
| **195** | 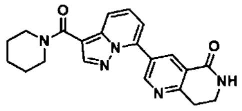 | $^1$H NMR (400 MHz, DMSO-$d_6$) δ(ppm): 9.10 (d, *J* = 2.3 Hz, 1H), 8.77 (d, *J* = 2.3 Hz, 1H), 8.27 (s, 2H), 7.93 (dd, *J* = 8.9, 1.4 Hz, 1H), 7.54 (dd, *J* = 8.9, 7.1 Hz, 1H), 7.34 (dd, *J* = 7.0, 1.4 Hz, 1H), 3.63 (t, *J* = 5.4 Hz, 4H), 3.55 (td, *J* = 6.7, 2.8 Hz, 2H), 3.16 (t, *J* = 6.7 Hz, 2H), 1.65 (d, *J* = 5.3 Hz, 2H), 1.57 (d, *J* = 7.1 Hz, 4H). | MS (ESI, LR) Calc. for $C_{21}H_{22}N_5O_2$ $[M+H]^+$: 376.1, found: 376.1. |
| **205** | 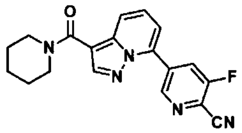 | $^1$H NMR (400 MHz, DMSO-$d_6$) δ(ppm): 9.23 (t, J = 1.6 Hz, 1H), 8.80 (dd, J = 10.3, 1.7 Hz, 1H), 8.31 (s, 1H), 8.01 (dd, J = 8.8, 1.4 Hz, 1H), 7.58 (dd, J = 8.9, 7.1 Hz, 1H), 7.49 (dd, J = 7.2, 1.4 Hz, 1H), 3.62 (t, J = 5.5 Hz, 4H), 1.65 (d, J = 6.8 Hz, 2H), 1.57 (s, 4H). | MS (ESI, LR) Calc. for $C_{19}H_{17}FN_5O$ $[M+H]^+$: 350.1, found: 350.1. |
| **207** | 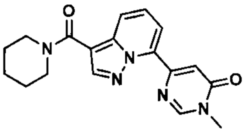 | $^1$H NMR (400 MHz, DMSO-$d_6$) δ(ppm): 8.64 (s, 1H), 8.40 (s, 1H), 8.25 (s, 1H), 8.04 (d, *J* = 8.0 Hz, 2H), 7.59 (t, *J* = 8.0 Hz, 1H), 3.62 (dt, *J* = 10.9, 5.2 Hz, 4H), 3.49 (s, 3H), 1.65 (d, *J* = 5.9 Hz, 2H), 1.56 (p, *J* = 5.6 Hz, 4H). | MS (ESI, LR) Calc. for $C_{18}H_{20}N_5O_2$ $[M+H]^+$: 338.2, found: 338.1. |
| **224** | 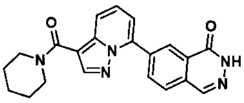 | $^1$H NMR (400 MHz, DMSO-$d_6$) δ(ppm): 12.80 (s, 1H), 8.83 (d, *J* = 1.8 Hz, 1H), 8.46 (dd, *J* = 8.2, 1.4 Hz, 2H), 8.29 (s, 1H), 8.11 (d, *J* = 8.3 Hz, 1H), 7.96 (dd, *J* = 8.9, 1.4 Hz, 1H), 7.57 (dd, *J* = 8.9, 7.0 Hz, 1H), 7.37 (dd, *J* = 7.1, 1.3 Hz, 1H), 3.64 (t, *J* = 5.4 Hz, 4H), 1.66 (d, *J* = 6.2 Hz, 2H), 1.58 (s, 4H). | MS (ESI, LR) Calc. for $C_{21}H_{20}N_5O_2$ $[M+H]^+$: 374.2, found: 374.1. |
| **232** | 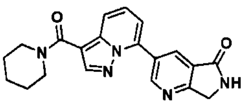 | $^1$H NMR (400 MHz, DMSO-$d_6$) δ(ppm): 9.24 (d, *J* = 2.1 Hz, 1H), 8.94 (s, 1H), 8.67 (d, *J* = 2.1 Hz, 1H), 8.27 (s, 1H), 7.95 (dd, *J* = 8.9, 1.3 Hz, 1H), 7.56 (dd, *J* = 8.9, 7.0 Hz, 1H), 7.38 (dd, *J* = 7.1, 1.4 Hz, 1H), 4.56 (s, 2H), 3.62 (dt, *J* = 10.9, 5.4 Hz, 6H), 1.71-1.53 (m, 8H). | MS (ESI, LR) Calc. for $C_{20}H_{20}N_5O_2$ $[M+H]^+$: 362.2, found: 362.1. |
| **233** | 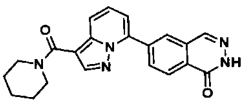 | $^1$H NMR (400 MHz, DMSO-$d_6$) δ(ppm): 12.78 (s, 1H), 8.52 (t, *J* = 1.2 Hz, 1H), 8.46 (s, 1H), 8.37 (d, *J* = 1.3 Hz, 2H), 8.29 (s, 1H), 7.97 (dd, *J* = 8.9, 1.4 Hz, 1H), 7.58 (dd, *J* = 8.9, 7.0 Hz, 1H), 7.35 (dd, J = 7.1, 1.4 Hz, 1H), 3.63 (t, *J* = 5.4 Hz, 4H), 1.66 (d, *J* = 5.7 Hz, 2H), 1.58 (d, *J* = 6.3 Hz, 4H). | MS (ESI, LR) Calc. for $C_{21}H_{20}N_5O_2$ $[M+H]^+$: 374.2, found: 374.1. |
| **244** | 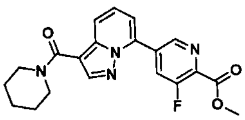 | $^1$H NMR (400 MHz, DMSO-$d_6$) δ(ppm): 9.12 (t,*J* =1.5 Hz, 1H), 8.63 (dd, *J* = 11.6, 1.7 Hz, 1H), 8.31 (s, 1H), 7.99 (dd, *J* = 8.9, 1.4 Hz, 1H), 7.58 (dd, *J* = 8.9, 7.1 Hz, 1H), 7.48 (dd, *J* = 7.1, 1.4 Hz, 1H), 3.96 (s, 3H), 3.63 (t, *J* = 5.4 Hz, 4H), 1.65 (d, *J* = 5.2 Hz, 2H), 1.57 (d, *J* = 7.3 Hz, 4H). | MS (ESI, LR) Calc. for $C_{20}H_{20}FN_4O_3$ $[M+H]^+$: 383.1, found: 383.1. |
| **245** | 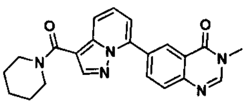 | $^1$H NMR (400 MHz, DMSO-$d_6$) δ(ppm): 8.76 (d, *J* = 2.1 Hz, 1H), 8.48 (s, 1H), 8.36 (dd, *J* = 8.6, 2.2 Hz, 1H), 8.28 (s, 1H), 7.92 (dd, *J* = 8.9, 1.3 Hz, 1H), 7.83 (d, *J* = 8.5 Hz, 1H), 7.55 (dd, *J* = 8.9, 7.1 Hz, 1H), 7.32 (dd, *J* = 7.0, 1.3 Hz, 1H), 3.64 (t, *J* = 5.4 Hz, 4H), 3.55 (s, 3H), 1.66 (d, *J* = 5.5 Hz, 2H), 1.57 (d, *J* = 7.5 Hz, 4H). | MS (ESI, LR) Calc. for $C_{22}H_{22}N_5O_2$ $[M+H]^+$: 388.2, found: 388.1. |
| **246** | 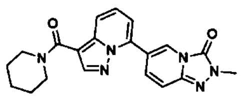 | $^1$H NMR (400 MHz, DMSO-$d_6$) δ(ppm): 8.82 (t, *J* = 1.4 Hz, 1H), 8.33 (s, 1H), 7.92 (dd, *J* = 8.8, 1.4 Hz, 1H), 7.77 (dd, *J* = 9.8, 1.8 Hz, 1H), 7.53 (dd, *J* = 8.8, 7.1 Hz, 1H), 7.44-7.35 (m, 2H), 3.61 (d, *J* = 13.8 Hz, 7H), 1.66 (q, *J* = 5.9 Hz, 2H), 1.58 (q, *J* = 5.6 Hz, 4H). | MS (ESI, LR) Calc. for $C_{20}H_{21}N_6O_2$ $[M+H]^+$: 377.2, found: 377.1. |

(continued)

| Example | Structure | $^1$H NMR | MS |
|---|---|---|---|
| **251** | 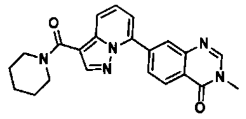 | $^1$H NMR (400 MHz, DMSO-$d_6$) δ(ppm): 8.46 (s, 1H), 8.32-8.24 (m, 3H), 8.07 (dd, $J$ = 8.3, 1.8 Hz, 1H), 7.95 (dd, $J$ = 8.9, 1.3 Hz, 1H), 7.55 (dd, J= 8.9, 7.1 Hz, 1H), 7.34 (dd, $J$ = 7.0, 1.4 Hz, 1H), 3.63 (t, $J$ = 5.4 Hz, 4H), 3.55 (s, 3H), 1.66 (d, $J$ = 5.4 Hz, 2H), 1.57 (d, $J$ = 7.5 Hz, 4H). | MS (ESI, LR) Calc. for $C_{22}H_{22}N_5O_2$ $[M+H]^+$: 388.2, found: 388.1. |
| **252** | 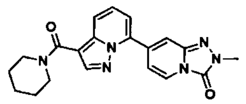 | $^1$H NMR (400 MHz, *D*MSO-$d_6$) δ(ppm): 8.30 (s, 1H), 8.00-7.92 (m, 3H), 7.54 (dd, $J$ = 8.9, 7.1 Hz, 1H), 7.39 (dd, $J$ = 7.1, 1.4 Hz, 1H), 7.15-7.10 (m, 1H), 3.62 (d, $J$ = 8.7 Hz, 7H), 1.69-1.63 (m, 2H), 1.57 (d, $J$ = 5.8 Hz, 4H). | MS (ESI, LR) Calc. for $C_{20}H_{21}N_6O_2$ $[M+H]^+$: 377.2, found: 377.1. |
| **254** | 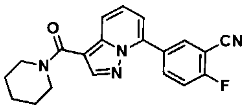 | $^1$H NMR (400 MHz, DMSO-$d_6$) δ(ppm): 8.54 (dd, $J$ = 6.3, 2.4 Hz, 1H), 8.38 (ddd, $J$ = 8.9, 5.3, 2.4 Hz, 1H), 8.27 (s, 1H), 7.93 (dd, $J$ = 8.9, 1.4 Hz, 1H), 7.74 (t, $J$ = 9.1 Hz, 1H), 7.54 (dd, $J$ = 8.9, 7.1 Hz, 1H), 7.29 (dd, $J$ = 7.1, 1.3 Hz, 1H), 3.62 (t, $J$ = 5.4 Hz, 4H), 1.65 (d, $J$ = 5.2 Hz, 2H), 1.57 (d, $J$ = 7.4 Hz, 4H). | MS (ESI, LR) Calc. for $C_{20}H_{18}FN_4O$ $[M+H]^+$: 349.1, found: 349.1. |
| **255** | 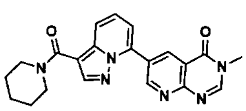 | $^1$H NMR (400 MHz, DMSO-$d_6$) δ(ppm): 9.46 (d, $J$ = 2.5 Hz, 1H), 9.21 (d, $J$ = 2.5 Hz, 1H), 8.69 (s, 1H), 8.31 (s, 1H), 7.96 (dd, $J$ = 8.9, 1.4 Hz, 1H), 7.60-7.55 (m, 1H), 7.47 (dd, $J$ = 7.1, 1.4 Hz, 1H), 3.64 (t, $J$ = 5.5 Hz, 4H), 3.56 (s, 3H), 1.66 (d, $J$ = 5.9 Hz, 2H), 1.57 (s, 4H). | MS (ESI, LR) Calc. for $C_{21}H_{21}N_6O_2$ $[M+H]^+$: 389.2, found: 389.2. |
| **256** | 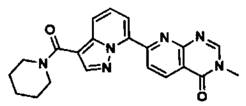 | $^1$H NMR (400 MHz, DMSO-$d_6$) δ(ppm): 9.31 (d, $J$ = 2.1 Hz, 1H), 8.73 (d, $J$ = 2.1 Hz, 1H), 8.55 (s, 1H), 8.31 (s, 1H), 8.00 (dd, $J$ = 8.8, 1.4 Hz, 1H), 7.59 (dd, $J$ = 8.9, 7.0 Hz, 1H), 7.49 (dd, $J$ = 7.1, 1.4 Hz, 1H), 3.64 (t, $J$ = 5.5 Hz, 4H), 3.59 (s, 3H), 1.66 (d, $J$ = 5.7 Hz, 2H), 1.58 (d, $J$ = 7.0 Hz, 4H). | MS (ESI, LR) Calc. for $C_{21}H_{21}N_6O_2$ $[M+H]^+$: 389.2, found: 389.1. |
| **264** | 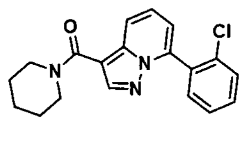 | $^1$H NMR (400 MHz, DMSO-$d_6$) δ(ppm): 8.16 (s, 1H), 7.95 (dd, $J$ = 9.0, 1.4 Hz, 1H), 7.66 (dd, $J$ = 8.4, 1.5 Hz, 1H), 7.63-7.56 (m, 2H), 7.53 (ddd, $J$ = 9.1, 6.6, 2.5 Hz, 2H), 7.08 (dd, $J$ = 6.9, 1.4 Hz, 1H), 3.62 (t, $J$ = 5.4 Hz, 4H), 1.74 1.60 (m, 2H), 1.57 (q, $J$ = 5.8 Hz, 4H). | MS (ESI, LR) Calc. for $C_{19}H_{19}ClN_3O$ $[M+H]^+$: 340.1, found: 340.1. |
| **265** | 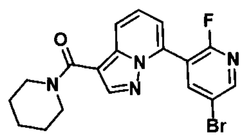 | $^1$H NMR (400 MHz, DMSO-$d_6$) δ(ppm): 8.63 (d, $J$ = 7.3 Hz, 2H), 8.24 (s, 1H), 7.99 (dd, $J$ = 9.0, 1.3 Hz, 1H), 7.55 (dd, $J$ = 9.0, 7.0 Hz, 1H), 7.34-7.29 (m, 1H), 3.62 (t, $J$ = 5.5 Hz, 4H), 1.65 (d, $J$ = 6.0 Hz, 2H), 1.57 (d, $J$ = 6.6 Hz, 4H). | MS (ESI, LR) Calc. for $C_{18}H_{17}BrFN_4O$ $[M+H]^+$: 403, found: 403. |
| **266** | 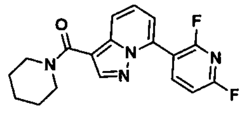 | $^1$H NMR (400 MHz, DMSO-$d_6$) δ(ppm): 8.54 (dt, $J$ = 9.3, 7.9 Hz, 1H), 8.23 (s, 1H), 7.98 (dd, $J$ = 9.0, 1.4 Hz, 1H), 7.55 (dd, $J$ = 9.0, 7.0 Hz, 1H), 7.43 (dd, $J$ = 8.1, 2.4 Hz, 1H), 7.27 (dd, $J$ = 7.1, 1.3 Hz, 1H), 3.62 (t, $J$ = 5.5 Hz, 4H), 1.65 (q, $J$ = 4.8 Hz, 2H), 1.56 (h, $J$ = 4.4 Hz, 4H). | MS (ESI, LR) Calc. for $C_{18}H_{17}F_2N_4O$ $[M+H]^+$: 343.1, found: 343.1. |
| **267** | 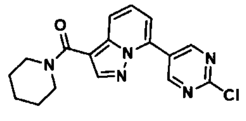 | $^1$H NMR (400 MHz, DMSO-$d_6$) δ(ppm): 9.39 (s, 2H), 8.31 (s, 1H), 7.98 (dd, $J$ = 8.9, 1.4 Hz, 1H), 7.57 (dd, $J$ = 8.9, 7.0 Hz, 1H), 7.45 (dd, $J$ = 7.1, 1.4 Hz, 1H), 3.63 (t, $J$ = 5.4 Hz, 4H), 1.65 (d, $J$ = 8.2 Hz, 2H), 1.57 (d, $J$ = 5.7 Hz, 4H). | MS (ESI, LR) Calc. for $C_{17}H_{17}C1N_5O$ $[M+H]^+$: 342.1, found: 342.1. |

(continued)

| Example | Structure | $^1$H NMR | MS |
|---|---|---|---|
| **268** | 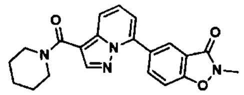 | $^1$H NMR (400 MHz, DMSO-$d_6$) δ(ppm): 8.39 (d, $J$ = 1.9 Hz, 1H), 8.30-8.24 (m, 2H), 7.91 (dd, $J$ = 8.9, 1.3 Hz, 1H), 7.69 (d, $J$ = 8.8 Hz, 1H), 7.53 (dd, $J$ = 8.9, 7.0 Hz, 1H), 7.28 (dd, $J$ = 7.1, 1.4 Hz, 1H), 3.64 (d, $J$ = 9.0 Hz, 7H), 1.66 (d, $J$ = 5.6 Hz, 2H), 1.57 (d, $J$ = 7.6 Hz, 4H). | MS (ESI, LR) Calc. for $C_{21}H_{21}N_4O_3$ $[M+H]^+$: 377.2, found: 377.1. |
| **269** | 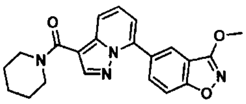 | $^1$H NMR (400 MHz, DMSO-$d_6$) δ(ppm): 8.35 (d, $J$ = 1.8 Hz, 1H), 8.26 (s, 1H), 8.20 (dd, $J$ = 8.8, 1.8 Hz, 1H), 7.91 (dd, $J$ = 8.9, 1.4 Hz, 1H), 7.82 (d, $J$ = 8.8 Hz, 1H), 7.53 (dd, $J$ = 8.9, 7.0 Hz, 1H), 7.27 (dd, $J$ = 7.1, 1.4 Hz, 1H), 4.17 (s, 3H), 3.63 (t, $J$= 5.4 Hz, 4H), 1.66 (d, $J$ = 5.7 Hz, 2H), 1.57 (d, $J$ = 7.0 Hz, 4H). | MS (ESI, LR) Calc. for $C_{21}H_{21}N_4O_3$ $[M+H]^+$: 377.2, found: 377.1. |
| **270** | 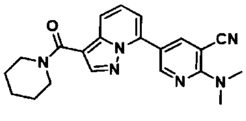 | $^1$H NMR (400 MHz, DMSO-$d_6$) δ(ppm): 8.91 (d, $J$ = 2.4 Hz, 1H), 8.67 (d, $J$ = 2.5 Hz, 1H), 8.27 (s, 1H), 7.85 (dd, $J$ = 8.9, 1.3 Hz, 1H), 7.50 (dd, $J$ = 8.9, 7.1 Hz, 1H), 7.29 (dd, $J$ = 7.1, 1.4 Hz, 1H), 3.62 (t, $J$ = 5.4 Hz, 4H), 3.34 (s, 6H), 1.65 (d, $J$ = 5.5 Hz, 2H), 1.57 (d, $J$ = 7.5 Hz, 4H). | MS (ESI, LR) Calc. for $C_{21}H_{23}N_6O$ $[M+H]^+$: 375.2, found: 375.1. |
| **271** | 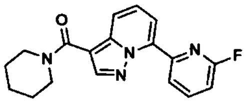 | $^1$H NMR (400 MHz, DMSO-$d_6$) δ(ppm): 8.77 (dd, $J$ = 7.6, 2.7 Hz, 1H), 8.35 (s, 1H), 8.25 (q, $J$ = 8.1 Hz, 1H), 8.00 (dd, $J$ = 8.8, 1.5 Hz, 1H), 7.72 (dd, $J$ = 7.2, 1.5 Hz, 1H), 7.59 (dd, $J$ = 8.8, 7.1 Hz, 1H), 7.37 (dd, $J$ = 8.2, 2.7 Hz, 1H), 3.63 (t, $J$ = 5.4 Hz, 4H), 1.71-1.63 (m, 2H), 1.63-1.54 (m, 4H). | MS (ESI, LR) Calc. for $C_{18}H_{18}FN_4O$ $[M+H]^+$: 325.1, found: 325.1. |
| **272** | 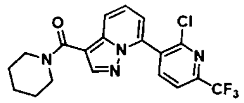 | $^1$H NMR (400 MHz, DMSO-$d_6$) δ(ppm): 8.47 (d, $J$ = 7.8 Hz, 1H), 8.21 (d, $J$ = 7.7 Hz, 2H), 8.02 (dd, $J$ = 9.0, 1.4 Hz, 1H), 7.59 (dd, $J$ = 9.0, 7.0 Hz, 1H), 7.28 (dd, $J$ = 7.0, 1.3 Hz, 1H), 3.62 (t, $J$ = 5.5 Hz, 4H), 1.65 (d, $J$ = 7.0 Hz, 2H), 1.57 (d, $J$ = 7.6 Hz, 4H). | MS (ESI, LR) Calc. for $C_{19}H_{17}C1F_3N_4O$ $[M+H]^+$: 409.1, found: 409.1. |
| **273** | 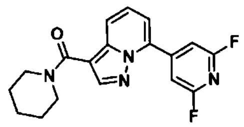 | $^1$H NMR (400 MHz, DMSO-$d_6$) δ(ppm): 8.33 (s, 1H), 8.02 (dd, $J$ = 8.8, 1.4 Hz, 1H), 7.88 (s, 2H), 7.57 (dd, $J$ = 8.8, 7.1 Hz, 1H), 7.51 (dd, $J$ = 7.1, 1.5 Hz, 1H), 3.63 (t, $J$ = 5.4 Hz, 4H), 1.66 (d, $J$ = 5.0 Hz, 2H), 1.57 (d, $J$ = 7.8 Hz, 4H). | MS (ESI, LR) Calc. for $C_{18}H_{17}F_2N_4O$ $[M+H]^+$: 343.1, found: 343.1. |
| **279** | 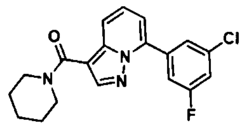 | $^1$H NMR (400 MHz, DMSO-$d_6$) δ(ppm): 8.28 (s, 1H), 7.96-7.90 (m, 2H), 7.88-7.82 (m, 1H), 7.64 (dt, $J$ = 8.7, 2.2 Hz, 1H), 7.52 (dd, $J$ = 8.9, 7.1 Hz, 1H), 7.31 (dd, $J$ = 7.1, 1.4 Hz, 1H), 3.62 (t, $J$ = 5.4 Hz, 4H), 1.69-1.61 (m, 2H), 1.57 (q, $J$ = 5.9 Hz, 4H). | MS (ESI, LR) Calc. for $C_{19}H_{18}ClFN_3O$ $[M+H]^+$: 358.1, found: 358.1. |
| **280** | 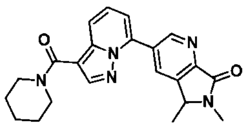 | $^1$H NMR (400 MHz, DMSO-$d_6$) δ(ppm): 9.19 (d, $J$ = 2.0 Hz, 1H), 8.69 (d, $J$ = 1.9 Hz, 1H), 8.29 (s, 1H), 7.97 (dd, $J$ = 8.8, 1.3 Hz, 1H), 7.57 (dd, $J$ = 8.9, 7.0 Hz, 1H), 7.39-7.34 (m, 1H), 4.74 (q, $J$ = 6.8 Hz, 1H), 3.63 (t, $J$ = 5.4 Hz, 4H), 3.11 (s, 3H), 1.66 (d, $J$ = 5.5 Hz, 2H), 1.57 (s, 4H), 1.52 (d, $J$ = 6.7 Hz, 2H). | MS (ESI, LR) Calc. for $C_{22}H_{24}N_5O_2$ $[M+H]^+$: 390.2, found: 390.1. |
| **281** | 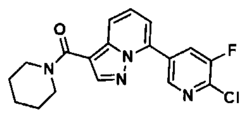 | $^1$H NMR (400 MHz, DMSO-$d_6$) δ(ppm): 8.88 (d, $J$ = 1.9 Hz, 1H), 8.64 (dd, $J$ = 9.8, 2.0 Hz, 1H), 8.30 (s, 1H), 7.97 (dd, $J$ = 8.9, 1.4 Hz, 1H), 7.56 (dd, $J$ = 8.9, 7.0 Hz, 1H), 7.41 (dd, $J$ = 7.1, 1.4 Hz, 1H), 3.62 (t, $J$ = 5.4 Hz, 4H), 1.65 (d, $J$ = 4.5 Hz, 2H), 1.57 (d, $J$ = 6.1 Hz, 4H). | MS (ESI, LR) Calc. for $C_{18}H_{17}ClFN_4O$ $[M+H]^+$: 359.1, found: 359.1. |

(continued)

| Example | Structure | $^1H$ NMR | MS |
|---|---|---|---|
| **282** | 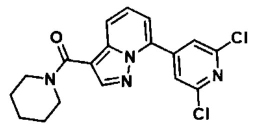 | $^1H$ NMR (400 MHz, DMSO-$d_6$) δ(ppm): 8.33 (s, 1H), 8.23 (s, 2H), 8.00 (dd, $J$ = 8.7, 1.6 Hz, 1H), 7.58-7.49 (m, 2H), 3.62 (t, $J$ = 5.5 Hz, 4H), 1.65 (d, $J$ = 7.6 Hz, 2H), 1.57 (s, 4H). | MS (ESI, LR) Calc. for $C_{18}H_{17}Cl_2N_4O$ $[M+H]^+$: 375.1, found: 375. |
| **283** | 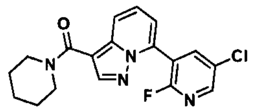 | $^1H$ NMR(400 MHz, DMSO-$d_6$) δ(ppm): 8.57-8.52 (m, 2H), 8.24 (s, 1H), 7.99 (dd, $J$ = 9.0, 1.4 Hz, 1H), 7.56 (dd, $J$ = 8.9, 7.0 Hz, 1H), 7.32 (dd, $J$ = 7.1, 1.4 Hz, 1H), 3.62 (t, $J$ = 5.4 Hz, 4H), 1.65 (d, $J$ = 5.2 Hz, 2H), 1.57 (d, $J$ = 7.2 Hz, 4H). | MS (ESI, LR) Calc. for $C_{18}H_{17}ClFN_4O$ $[M+H]^+$: 359.1, found: 359.1. |
| **284** | 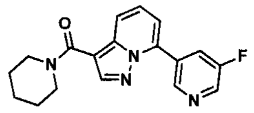 | $^1H$ NMR (400 MHz, DMSO-$d_6$) δ(ppm): 8.99 (t, $J$ = 1.8 Hz, 1H), 8.76 (d, $J$ = 2.8 Hz, 1H), 8.40 (ddd, $J$ = 10.1, 2.8, 1.7 Hz, 1H), 8.28 (s, 1H), 7.96 (dd, $J$ = 8.9, 1.3 Hz, 1H), 7.55 (dd, $J$ = 8.9, 7.0 Hz, 1H), 7.37 (dd, $J$ = 7.1, 1.4 Hz, 1H), 3.63 (t, $J$ = 5.4 Hz, 4H), 1.66 (q, $J$ = 5.8 Hz, 2H), 1.57 (h, $J$ = 5.9 Hz, 4H). | MS (ESI, LR) Calc. for $C_{18}H_{18}FN_4O$ $[M+H]^+$: 325.1, found: 325.1. |
| **285** | 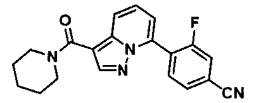 | $^1H$ NMR (400 MHz, DMSO-$d_6$) δ(ppm): 8.21 (s, 1H), 8.14-8.06 (m, 1H), 7.99 (dd, $J$ = 8.9, 1.3 Hz, 1H), 7.94 (d, $J$ = 5.8 Hz, 2H), 7.55 (dd, $J$ = 9.0, 7.0 Hz, 1H), 7.24 (dd, $J$ = 7.0, 1.3 Hz, 1H), 3.62 (t, $J$ = 5.4 Hz, 4H), 1.65 (d, $J$ = 5.4 Hz, 2H), 1.61-1.46 (m, 4H). | MS (ESI, LR) Calc. for $C_{20}H_{18}FN_4O$ $[M+H]^+$: 349.1, found: 349.1. |
| **288** | 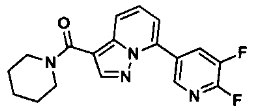 | $^1H$ NMR (400 MHz, DMSO-$d_6$) δ(ppm): 8.72 (ddd, $J$ = 11.1, 9.3, 2.0 Hz, 1H), 8.63 (t, $J$ = 1.8 Hz, 1H), 8.28 (s, 1H), 7.96 (dd, $J$ = 8.9, 1.4 Hz, 1H), 7.55 (dd, $J$ = 8.9, 7.0 Hz, 1H), 7.36 (dd, $J$ = 7.0, 1.4 Hz, 1H), 3.63 (t, $J$ = 5.5 Hz, 4H), 1.72-1.62 (m, 2H), 1.57 (d, $J$ = 4.4 Hz, 4H). | MS (ESI, LR) Calc. for $C_{18}H_{17}F_2N_4O$ $[M+H]^+$: 343.1, found: 343.1. |
| **289** | 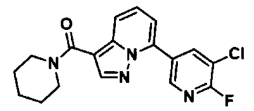 | $^1H$ NMR (400 MHz, DMSO-$d_6$) δ(ppm): 8.86 (dd, $J$ = 8.9, 2.2 Hz, 1H), 8.76-8.72 (m, 1H), 8.29 (s, 1H), 7.95 (dd, $J$ = 8.9, 1.3 Hz, 1H), 7.55 (dd, $J$ = 8.9, 7.0 Hz, 1H), 7.37 (dd, $J$ = 7.1, 1.4 Hz, 1H), 3.63 (t, $J$ = 5.4 Hz, 4H), 1.69-1.63 (m, 2H), 1.57 (s, 4H). | MS (ESI, LR) Calc. for $C_{18}H_{17}ClFN_4O$ $[M+H]^+$: 359.1, found: 359.1. |
| **290** | 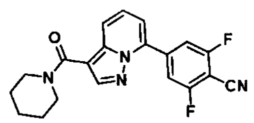 | $^1H$ NMR (400 MHz, DMSO-$d_6$) δ(ppm): 8.31 (s, 1H), 8.14 (d, $J$ = 9.9 Hz, 2H), 7.99 (dd, $J$ = 8.9, 1.4 Hz, 1H), 7.56 (dd, $J$ = 8.9, 7.1 Hz, 1H), 7.42 (d, $J$ = 7.1 Hz, 1H), 3.62 (t, $J$ = 5.4 Hz, 4H), 1.66 (d, $J$ = 5.4 Hz, 2H), 1.58 (d, $J$ = 7.9 Hz, 4H). | MS (ESI, LR) Calc. for $C_{20}H_{17}F_2N_4O$ $[M+H]^+$: 367.1, found: 367.1. |
| **291** | 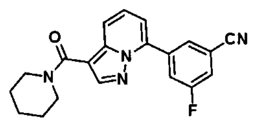 | $^1H$ NMR (400 MHz, DMSO-$d_6$) δ(ppm): 8.33 (t, $J$ = 1.5 Hz, 1H), 8.30-8.23 (m, 2H), 8.08-8.03 (m, 1H), 7.96 (dd, $J$ = 8.9, 1.4 Hz, 1H), 7.54 (dd, $J$ = 8.9, 7.0 Hz, 1H), 7.37 (dd, $J$ = 7.1, 1.4 Hz, 1H), 3.63 (t, $J$ = 5.4 Hz, 4H), 1.66 (d, $J$ = 5.5 Hz, 2H), 1.57 (d, $J$ = 7.4 Hz, 4H). | MS (ESI, LR) Calc. for $C_{20}H_{18}FN_4O$ $[M+H]^+$: 349.1, found: 349.1. |
| **292** | 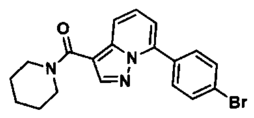 | $^1H$ NMR (400 MHz, DMSO-$d_6$) δ(ppm): 8.24 (s, 1H), 7.93-7.86 (m, 3H), 7.80-7.73 (m, 2H), 7.51 (dd, $J$ = 8.9, 7.1 Hz, 1H), 7.20 (dd, $J$ = 7.1, 1.4 Hz, 1H), 3.62 (t, $J$ = 5.4 Hz, 4H), 1.69-1.61 (m, 2H), 1.57 (td, $J$ = 6.8, 3.4 Hz, 4H). | MS (ESI, LR) Calc. for $C_{19}H_{19}BrN_3O$ $[M+H]^+$: 384.1, found: 384. |
| **293** | 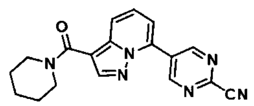 | $^1H$ NMR (400 MHz, DMSO-$d_6$) δ(ppm): 9.65 (s, 2H), 8.32 (s, 1H), 8.02 (dd, $J$ = 8.8, 1.5 Hz, 1H), 7.60 (dd, $J$ = 8.8, 7.0 Hz, 1H), 7.54 (dd, $J$ = 7.1, 1.5 Hz, 1H), 3.63 (dd, $J$ = 7.0, 4.1 Hz, 4H), 1.68-1.62 (m, 2H), 1.61-1.55 (m, 4H). | MS (ESI, LR) Calc. for $C_{18}H_{17}N_6O$ $[M+H]^+$: 333.1, found: 333.1. |

(continued)

| Example | Structure | $^1H$ NMR | MS |
|---|---|---|---|
| **294** | 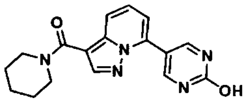 | $^1H$ NMR (400 MHz, DMSO-$d_6$) δ(ppm): 8.66 (s, 2H), 8.20 (s, 1H), 7.64 (dd, *J* = 8.8, 1.3 Hz, 1H), 7.39 (dd, *J* = 8.8, 7.2 Hz, 1H), 6.99 (dd, *J* = 7.2, 1.4 Hz, 1H), 3.61 (t, *J* = 5.4 Hz, 4H), 1.72-1.61 (m, 2H), 1.56 (d, *J* = 7.0 Hz, 4H). | MS (ESI, LR) Calc. for $C_{17}H_{18}N_5O_2$ $[M+H]^+$: 324.1, found: 324.1 |
| **298** | 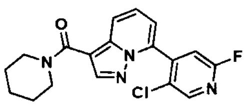 | $^1H$ NMR (400 MHz, DMSO-$d_6$) δ(ppm): 8.58 (s, 1H), 8.22 (s, 1H), 8.02 (dd, *J* = 8.9, 1.3 Hz, 1H), 7.70 (d, *J* = 2.6 Hz, 1H), 7.57 (dd, *J* = 9.0, 6.9 Hz, 1H), 7.26-7.22 (m, 1H), 3.62 (t, *J* = 5.4 Hz, 4H), 1.65 (d, *J* = 7.8 Hz, 2H), 1.61-1.55 (m, 4H). | MS (ESI, LR) Calc. for $C_{13}H_{17}ClFN_4O$ $[M+H]^+$: 359.1, found: 359.1 |
| **312** | 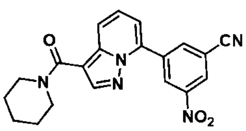 | $^1H$ NMR (400 MHz, DMSO-$d_6$) δ(ppm): 9.18 (t, *J* = 1.9 Hz, 1H), 8.90 (dt, *J* = 7.0, 1.6 Hz, 2H), 8.33 (s, 1H), 7.99 (dd, *J* = 8.8, 1.4 Hz, 1H), 7.58 (dd, *J* = 8.9, 7.1 Hz, 1H), 7.48 (dd, *J* = 7.1, 1.4 Hz, 1H), 3.64 (t, *J* = 5.4 Hz, 4H), 1.66 (d, *J* = 5.7 Hz, 2H), 1.58 (s, 4H). | MS (ESI, LR) Calc. for $C_{20}H_{18}N_5O_3$ $[M+H]^+$: 376.1, found: 376.1 |
| **313** | 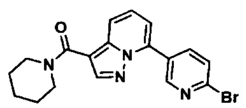 | $^1H$ NMR (400 MHz, DMSO-$d_6$) δ(ppm): 8.92 (d, *J* = 2.5 Hz, 1H), 8.37 (dd, *J* = 8.3, 2.6 Hz, 1H), 8.26 (s, 1H), 7.94 (dd, J= 8.9, 1.3 Hz, 1H), 7.87 (d, *J* = 8.4 Hz, 1H), 7.54 (dd, *J* = 8.9, 7.0 Hz, 1H), 7.32 (dd, *J* = 7.1, 1.3 Hz, 1H), 3.62 (t, *J= 5.4* Hz, 4H), 1.69-1.61 (m, 2H), 1.61-1.53 (m, 4H). | MS (ESI, LR) Calc. for $C_{18}H_{18}BrN_4O$ $[M+H]^+$: 385.1, found: 385 |
| **314** | 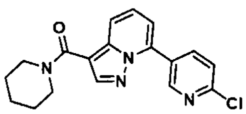 | $^1H$ NMR (400 MHz, DMSO-$d_6$) δ(ppm): 8.95 (d, *J* = 2.5 Hz, 1H), 8.48 (dd, *J* = 8.4, 2.5 Hz, 1H), 8.26 (s, 1H), 7.94 (dd, *J* = 8.9, 1.4 Hz, 1H), 7.74 (d, *J* = 8.4 Hz, 1H), 7.54 (dd, *J* = 8.9, 7.0 Hz, 1H), 7.33 (dd, *J* = 7.1, 1.4 Hz, 1H), 3.62 (t, *J*= 5.4 Hz, 4H), 1.65 (d, *J* = 5.5 Hz, 2H), 1.57 (s, 4H). | MS (ESI, LR) Calc. for $C_{18}H_{18}ClN_4O$ $[M+H]^+$: 341.1, found: 341.1 |
| **315** | 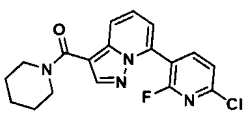 | $^1H$ NMR (400 MHz, DMSO-$d_6$) δ(ppm): 8.41 (dd, *J* = *9.2,* 7.8 Hz, 1H), 8.23 (s, 1H), 7.98 (dd, *J* = 8.9, 1.4 Hz, 1H), 7.76 (dd, *J* = 7.9, 1.1 Hz, 1H), 7.55 (dd, *J* = 8.9, 7.0 Hz, 1H), 7.28 (dd, *J* = 7.1, 1.4 Hz, 1H), 3.62 (t, *J* = 5.4 Hz, 4H), 1.65 (q, *J* = 5.0 Hz, 2H), 1.57 (q, *J* = 6.0 Hz, 4H). | MS (ESI, LR) Calc. for $C_{18}H_{17}ClFN_4O$ $[M+H]^+$: 359.1, found: 359.0 |
| **318** | 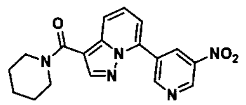 | $^1H$ NMR (400 MHz, DMSO-$d_6$) δ(ppm): 9.50 (dd, *J* = 6.2, 2.2 Hz, 2H), 9.27 (t, *J* = 2.3 Hz, 1H), 8.32 (s, 1H), 8.00 (dd, *J* = 8.9, 1.4 Hz, 1H), 7.58 (dd, *J* = 8.9, 7.1 Hz, 1H), 7.50 (dd, *J* = 7.0, 1.4 Hz, 1H), 3.64 (t, *J* = 5.4 Hz, 4H), 1.82-1.62 (m, 2H), 1.57 (d, *J* = 7.0 Hz, 4H). | MS (ESI, LR) Calc. for $C_{18}H_{18}N_5O_3$ $[M+H]^+$: 352.1, found: 352.1 |

Example 7. **(7-(3-aminophenyl)pyrazolo[1,5-a]pyridin-3-yl)(piperidin-1-yl)methanone**

<Scheme 2>

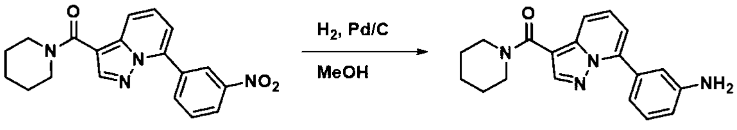


5 7

Compound 5 (0.331 g, 0.94 mmol) was dissolved in methanol (16.5 mL), and palladium/carbon (0.025 g, 0.24

mmol) was added. The reaction solution was filled with hydrogen gas and stirred for 12 hours. After completion of the reaction, the remaining palladium was removed using diatomaceous earth (Celite), and the filtrate was concentrated. Dichloromethane (30 mL) was added, and the mixture was washed with water and brine. The organic layer was dried over anhydrous magnesium sulfate, filtered, concentrated, and separated using column chromatography to obtain the target compound 7 (0.297 g, 95%).

$^1$H NMR (400 MHz, DMSO-$d_6$) δ(ppm): 8.21 (s, 1H), 7.83 (dd, *J* = 8.9, 1.4 Hz, 1H), 7.47 (dd, *J* = 8.9, 7.0 Hz, 1H), 7.17 (d, *J* = 7.8 Hz, 1H), 7.09 (t, *J* = 2.0 Hz, 1H), 7.05 (dd, *J* = 7.1, 1.4 Hz, 1H), 6.99-6.94 (m, 1H), 6.75-6.68 (m, 1H), 5.27 (s, 2H), 3.62 (t, *J* = 5.4 Hz, 4H), 1.65 (d, *J* = 5.2 Hz, 2H), 1.57 (s, 4H).

MS (ESI, LR) Calc. for $C_{19}H_{21}N_4O$ [M+H]$^+$: 321.2, found: 321.2.

Example **9. (7-(5-aminopyridin-3-yl)pyrazolo[1,5-*a*]pyridin-3-yl)(piperidin-1-yl)methanone**

**Example 14. (7-(4-aminophenyl)pyrazolo[1,5-*a*]pyridin-3-yl)(piperidin-1-yl)methanone**

**Example 17. (7-(6-aminopyridin-3-yl)pyrazolo[1,5-*a*]pyridin-3-yl)(piperidin-1-yl)methanone**

For Examples 9, 14, and 17, the compounds were obtained by sequentially using the synthetic method of step 1-3 in Example **1** and the synthetic method of Example **7,** using the corresponding compounds and compound **1-2.** The structural and spectral data are shown in Table 2 below.

[Table 2]

| Example | Structure | $^1$H NMR | MS |
|---|---|---|---|
| **9** | 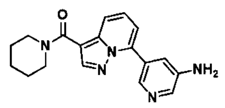 | $^1$H NMR (400 MHz, DMSO-$d_6$) δ(ppm): 8.23 (s, 1H), 8.16 (d, *J* = 1.9 Hz, 1H), 8.06 (d, *J* = 2.7 Hz, 1H), 7.87 (dd, *J* = 8.9, 1.3 Hz, 1H), 7.53-7.46 (m, 2H), 7.15 (dd, *J* = 7.0, 1.4 Hz, 1H), 5.54 (s, 2H), 3.61 (t, *J* = 5.4 Hz, 4H), 1.65 (d, *J* = 5.2 Hz, 2H), 1.56 (d, *J* = 7.7 Hz, 4H). | MS (ESI, LR) Calc. for $C_{18}H_{20}N_5O$ [M+H]$^+$: 322.2, found: 322.1. |
| **14** | 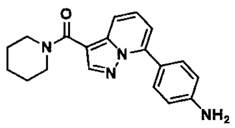 | $^1$H NMR (400 MHz, DMSO-$d_6$) δ(ppm): 8.21 (s, 1H), 7.75-7.68 (m, 3H), 7.43 (dd, *J* = 8.8, 7.1 Hz, 1H), 7.03 (dd, *J* = 7.2, 1.4 Hz, 1H), 6.68 (d, *J* = 8.6 Hz, 2H), 5.59 (s, 2H), 3.61 (t, J = 5.4 Hz, 4H), 1.65 (d, *J* = 5.6 Hz, 2H), 1.56 (d, *J* = 7.5 Hz, 4H). | MS (ESI, LR) Calc. for $C_{19}H_{21}N_4O$ [M+H]$^+$: 321.2, found: 321.2. |
| **17** | 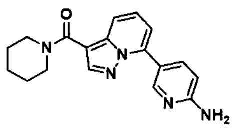 | $^1$H NMR (400 MHz, DMSO-$d_6$) δ(ppm): 8.51 (d, *J* = 2.4 Hz, 1H), 8.22 (s, 1H), 8.02 (dd, *J* = 8.7, 2.5 Hz, 1H), 7.78 (dd, *J* = 8.8, 1.3 Hz, 1H), 7.46 (dd, *J* = 8.8, 7.1 Hz, 1H), 7.11 (dd, *J* = 7.1, 1.4 Hz, 1H), 6.57 (d, *J* = 8.7 Hz, 1H), 6.43 (s, 2H), 3.61 (t, *J* = 5.4 Hz, 4H), 1.65 (d, *J* = 5.6 Hz, 2H), 1.56 (d, *J* = 6.5 Hz, 4H). | MS (ESI, LR) Calc. for $C_{18}H_{20}N_5O$ [M+H]$^+$: 322.2, found: 322.1. |

**Example 26. (7-(3-(morpholine-4-carbonyl)phenyl)pyrazolo[1,5-*a*]pyridin-3-yl)(piperidin-1-yl)methanone**

<Scheme 3>

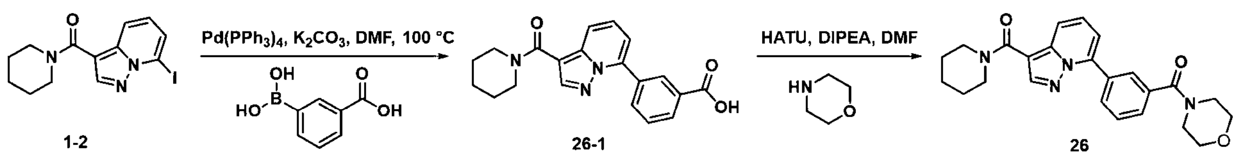


1-2 26-1 26

Step 26-1: Using compound **1-2** and 3-boronobenzoic acid, the target compound **26-1** was obtained by the same synthetic method as step 1-3 in Example **1.**

Step 26-2: Using compound **26-1** and morpholine, the target compound **26** was obtained by the same synthetic method as step 1-2 in Example 1.

$^1$H NMR (400 MHz, DMSO-$d_6$) δ(ppm): 8.03-7.92 (m, 3H), 7.65-7.54 (m, 2H), 7.38 (dd, *J* = 8.9, 7.0 Hz, 1H), 6.98 (dd, *J* = 7.0, 1.4 Hz, 1H), 3.78-3.60 (m, 12H), 1.74-1.66 (m, 6H).

MS (ESI, LR) Calc. for $C_{24}H_{27}N_4O_3$ [M+H]$^+$: 419.2, found: 419.2.

**Example 27. *N*-methyl-3-(3-(piperidine-1-carbonyl)pyrazolo[1,5-*a*]pyridin-7-yl)benzamide**

**Example 28. *N*-isopropyl-3-(3-(piperidine-1-carbonyl)pyrazolo[1,5-*a*]pyridin-7-yl)benzamide**

**Example 29. azetidin-1-yl(3-(3-(piperidine-1-carbonyl)pyrazolo[1,5-*a*]pyridin-7-yl)phenyl)methanone**

**Example 30. *N*-(2-methoxyethyl)-3-(3-(piperidine-1-carbonyl)pyrazolo[1,5-*a*]pyridin-7-yl)benzamide**

**Example 31. N-(2-(dimethylamino)ethyl)-3-(3-(piperidine-1-carbonyl)pyrazolo[1,5-*a*]pyridin-7-yl)benzamide**

**Example 32. *N*-(cyclopropylmethyl)-3-(3-(piperidine-1-carbonyl)pyrazolo[1,5-*a*]pyridin-7-yl)benzamide**

**Example 33. *N*-(1-methylpiperidin-4-yl)-3-(3-(piperidine-1-carbonyl)pyrazolo[1,5-*a*]pyridin-7-yl)benzamide**

**Example 35. *tert*-butyl 4-(3-(3-(piperidine-1-carbonyl)pyrazolo[1,5-*a*]pyridin-7-yl)benzoyl)piperazine-1-carboxylate**

**Example 36. *N*-(2-cyanoethyl)-3-(3-(piperidine-1-carbonyl)pyrazolo[1,5-*a*]pyridin-7-yl)benzamide**

**Example 37. 3-(3-(piperidine-1-carbonyl)pyrazolo[1,5-*a*]pyridin-7-yl)-N-(pyridin-3-yl)benzamide**

**Example 38. 3-(3-(piperidine-1-carbonyl)pyrazolo[1,5-*a*]pyridin-7-yl)-N-(pyridin-4-yl)benzamide**

For Examples 27, 28, 29, 30, 31, 32, 33, 35, 36, 37, and 38, the compounds were obtained by the same synthetic method as step 1-2 in Example **1** using the corresponding compounds and compound **26-1.** The structural and spectral data are shown in Table 3 below.

[Table 3]

| Example | Structure | $^1$H NMR | MS |
|---|---|---|---|
| **27** | 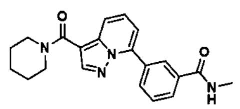 | $^1$H NMR (400 MHz, DMSO-$d_6$) δ(ppm): 8.26 (t, *J* = 1.8 Hz, 1H), 8.06 (s, 1H), 7.98 (ddd, *J* = 8.9, 6.9, 1.4 Hz, 2H), 7.89 (dt, *J* = 7.7, 1.5 Hz, 1H), 7.60 (t, *J* =7.8 Hz, 1H), 7.38 (dd, *J* = 8.9, 7.0 Hz, 1H), 6.98 (dd, *J* = 7.0, 1.4 Hz, 1H), 6.33 (s, 1H), 3.04 (d, *J* = 4.9 Hz, 3H), 1.70 (d, J = 27.1 Hz, 10H). | MS (ESI, LR) Calc. for $C_{21}H_{23}N_4O_2$ [M+H]$^+$: 363.2, found: 363.2. |
| **28** | 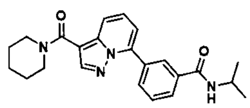 | $^1$H NMR (400 MHz, DMSO-$d_6$) δ(ppm): 8.21 (t, *J* = 1.8 Hz, 1H), 8.06 (s, 1H), 8.02-7.93 (m, 2H), 7.87 (dt, *J* = 7.8, 1.5 Hz, 1H), 7.60 (t, *J* = 7.8 Hz, 1H), 7.39 (dd, *J* = 8.9, 7.0 Hz, 1H), 6.98 (dd, *J* = 6.9, 1.4 Hz, 1H), 6.01 (d, *J* = 7.7 Hz, 1H), 4.31 (dq, *J* = 13.4, 6.7 Hz, 1H), 3.72 (t, *J* = 5.4 Hz, 4H), 1.73-1.72 (m, 2H), 1.59 (s, 4H), 1.28 (d, *J* = 6.6 Hz, 6H). | MS (ESI, LR) Calc. for $C_{23}H_{27}N_4O_2$ [M+H]$^+$: 391.2, found: 391.2. |
| **29** | 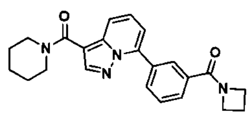 | $^1$H NMR (400 MHz, DMSO-$d_6$) δ(ppm): 8.16 (t, *J* = 1.8 Hz, 1H), 8.06 (s, 1H), 8.02-7.93 (m, 2H), 7.77 (dt, *J* = 7.7, 1.5 Hz, 1H), 7.58 (t, *J* = 7.8 Hz, 1H), 7.38 (dd, *J* = 8.9, 7.0 Hz, 1H), 6.98 (dd, *J* = 7.0, 1.4 Hz, 1H), 4.40 (t, *J* = 7.8 Hz, 2H), 4.26 (t, *J* = 7.9 Hz, 2H), 3.72 (t, *J* = 5.4 Hz, 4H), 2.43-2.32 (m, 2H), 1.78-1.70 (m, 2H), 1.70-1.64 (m, 4H). | MS (ESI, LR) Calc. for $C_{23}H_{25}N_4O_2$ [M+H]$^+$: 389.2, found: 389.2. |

(continued)

| Example | Structure | $^1H$ NMR | MS |
|---|---|---|---|
| **30** | 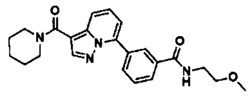 | $^1H$ NMR (400 MHz, DMSO-$d_6$) δ(ppm): 8.06 (s, 1H), 8.03-7.96 (m, 2H), 7.91 (dt, *J* = 7.9, 1.5 Hz, 1H), 7.61 (t, *J* = 7.8 Hz, 1H), 7.39 (dd, *J* = 8.9, 7.0 Hz, 1H), 6.98 (dd, *J* = 7.0, 1.4 Hz, 1H), 6.64 (s, 1H), 3.72 (t, *J* = 5.4 Hz, 4H), 3.68 (t, *J* = 5.2 Hz, 2H), 3.58 (t, *J* = 5.0 Hz, 2H), 3.39 (s, 3H), 1.72 (t, *J* = 4.7 Hz, 2H), 1.67 (s, 4H). | MS (ESI, LR) Calc. for $C_{23}H_{27}N_4O_3$ $[M+H]^+$: 407.2, found: 407.2. |
| **31** | 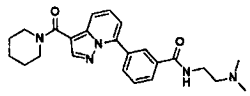 | $^1H$ NMR (400 MHz, DMSO-$d_6$) δ(ppm): 8.26 (t, *J* = 1.8 Hz, 1H), 8.04 (s, 1H), 8.00 (dt, *J* = 7.8, 1.4 Hz, 1H), 7.98-7.88 (m, 2H), 7.59 (t, *J* = 7.8 Hz, 1H), 7.38 (dd, *J* = 8.9, 7.0 Hz, 1H), 6.99 (dd, *J* = 7.0, 1.4 Hz, 1H), 3.69 (t, *J* = 5.3 Hz, 4H), 3.60 (q, *J* = 5.5 Hz, 2H), 2.76 (t, *J* = 5.7 Hz, 2H), 2.45 (s, 6H), 1.80-1.57 (m, 6H). | MS (ESI, LR) Calc. for $C_{24}H_{30}N_3O_2$ $[M+H]^+$: 420.2, found: 420.2. |
| **32** | 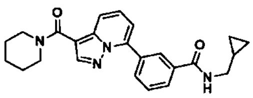 | $^1H$ NMR (400 MHz, DMSO-$d_6$) δ(ppm): 8.25 (t, *J* = 1.9 Hz, 1H), 8.06 (s, 1H), 8.04-7.95 (m, 2H), 7.91 (dt, *J* = 8.0, 1.4 Hz, 1H), 7.61 (t, *J* = 7.8 Hz, 1H), 7.39 (dd, *J* = 8.9, 7.0 Hz, 1H), 6.99 (dd, *J* = 7.0, 1.4 Hz, 1H), 6.32 (s, 1H), 3.72 (t, *J* = 5.3 Hz, 4H), 3.34 (dd, *J* = 7.2, 5.4 Hz, 2H), 1.74-1.67 (m, 6H), 1.06 (dt, *J* = 7.7, 4.4 Hz, 1H), 0.62-0.50 (m, 2H), 0.31-0.26 (m, *J* = 5.0 Hz, 2H). | MS (ESI, LR) Calc. for $C_{24}H_{27}N_4O_2$ $[M+H]^+$: 403.2, found: 403.2. |
| **33** | 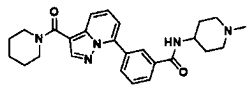 | $^1H$ NMR (400 MHz, DMSO-$d_6$) δ(ppm): 8.44 (d, *J* = 7.5 Hz, 1H), 8.33 (d, *J* = 2.0 Hz, 1H), 8.25 (s, 1H), 8.07 (d, *J* = 7.7 Hz, 1H), 8.01 (d, *J* = 7.8 Hz, 1H), 7.92 (d, *J* = 8.9 Hz, 1H), 7.66 (t, *J* = 7.8 Hz, 1H), 7.55 (dd, *J* = 8.9, 7.0 Hz, 1H), 7.23 (d, *J* = 7.0 Hz, 1H), 3.91 (s, 1H), 3.63 (t, *J* = 5.4 Hz, 4H), 3.36-3.31 (m, 2H), 3.12 (d, *J* = 23.3 Hz, 2H), 2.46 (s, 3H), 1.98-1.85 (m, 2H), 1.77-1.61 (m, 4H), 1.58 (q, *J* = 5.7 Hz, 4H). | MS (ESI, LR) Calc. for $C_{26}H_{32}N_5O_2$ $[M+H]^+$: 446.3, found: 446.2. |
| **35** | 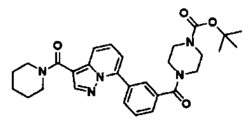 | $^1H$ NMR (400 MHz, DMSO-$d_6$) δ(ppm): 8.03-7.92 (m, 3H), 7.66-7.52 (m, 2H), 7.43-7.34 (m, 1H), 7.28-7.26 (m, 1H), 6.98 (d, *J* = 7.0 Hz, 1H), 3.72 (t, *J* = 5.3 Hz, 6H), 3.51 (s, 6H), 1.73-1.67 (m, 6H), 1.48 (s, 9H). | MS (ESI, LR) Calc. for $C_{29}H_{36}N_5O_4$ $[M+H]^+$: 518.3, found: 518.2. |
| **36** | 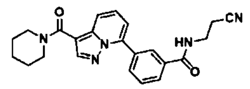 | $^1H$ NMR (400 MHz, DMSO-$d_6$) δ(ppm): 8.97 (t, *J* = 5.6 Hz, 1H), 8.37 (d, *J* = 1.8 Hz, 1H), 8.26 (s, 1H), 8.11 (d, *J* = 7.8 Hz, 1H), 8.02 (d, *J* = 7.8 Hz, 1H), 7.92 (dd, *J* = 8.9, 1.3 Hz, 1H), 7.69 (t, *J* = 7.8 Hz, 1H), 7.55 (dd, *J* = 8.9, 7.0 Hz, 1H), 7.24 (dd, *J* = 7.0, 1.4 Hz, 1H), 3.63 (t, *J* = 5.4 Hz, 4H), 3.54 (q, *J* = 6.2 Hz, 2H), 2.81 (t, *J* = 6.5 Hz, 2H), 1.66 (d, *J* = 5.7 Hz, 2H), 1.57 (d, *J* = 6.8 Hz, 4H). | MS (ESI, LR) Calc. for $C_{23}H_{24}N_5O_2$ $[M+H]^+$: 402.2, found: 402.2. |
| **37** | 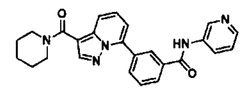 | $^1H$ NMR (400 MHz, DMSO-$d_6$) δ(ppm): 10.55 (s, 1H), 8.94 (d, *J* = 2.6 Hz, 1H), 8.51 (t, *J* = 1.7 Hz, 1H), 8.34 (dd, *J* = 4.7, 1.5 Hz, 1H), 8.27 (s, 1H), 8.24-8.09 (m, 3H), 7.93 (dd, *J* = 8.9, 1.3 Hz, 1H), 7.75 (t, *J* = 7.8 Hz, 1H), 7.56 (dd, *J* = 8.9, 7.0 Hz, 1H), 7.42 (dd, *J* = 8.3, 4.7 Hz, 1H), 7.30 (dd, *J* = 7.0, 1.4 Hz, 1H), 3.63 (t, *J* = 5.4 Hz, 4H), 1.65 (d, *J* = 6.4 Hz, 2H), 1.57 (s, 4H). | MS (ESI, LR) Calc. for $C_{25}H_{24}N_5O_2$ $[M+H]^+$: 426.2, found: 426.1. |

(continued)

| Example | Structure | $^1$H NMR | MS |
|---|---|---|---|
| **38** | 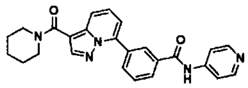 | $^1$H NMR (400 MHz, DMSO-$d_6$) δ(ppm): 9.00 (s, 1H), 8.52 (d, $J$ = 5.5 Hz, 2H), 8.39 (t, $J$ = 1.8 Hz, 1H), 8.11-7.89 (m, 4H), 7.72-7.58 (m, 3H), 7.35 (dd, $J$ = 8.9, 7.0 Hz, 1H), 6.96 (dd, $J$ = 7.1, 1.4 Hz, 1H), 3.69 (t, $J$ = 5.4 Hz, 4H), 1.65 (d, $J$ = 5.7 Hz, 6H). | MS (ESI, LR) Calc. for $C_{25}H_{24}N_5O_2$ $[M+H]^+$: 426.2, found: 426.2. |

**Example 34. piperazin-1-yl(3-(3-(piperidine-1-carbonyl)pyrazolo[1,5-*a*]pyridin-7-yl)phenyl)methanone**

<Scheme 4>

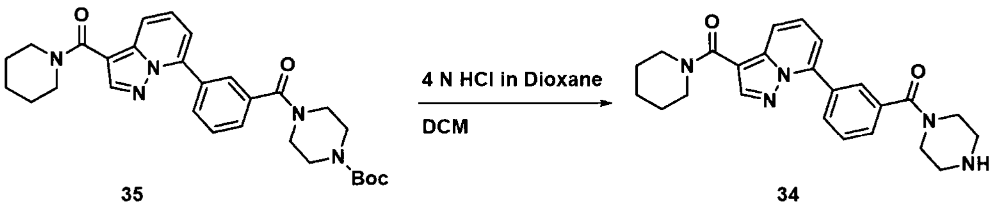


35 34

Compound **35** (0.1 g, 0.19 mmol) was dissolved in dichloromethane (1 mL) and stirred at 0 °C for 10 minutes. 1,4-dioxane dissolved in 4 N hydrochloric acid (0.48 mL) was added, and the mixture was stirred at room temperature for 16 hours. After completion of the reaction, the reaction solution was concentrated and separated using column chromatography to obtain the target compound **34** (36 mg, 44%).

$^1$H NMR (400 MHz, DMSO-$d_6$) δ(ppm): 8.25 (s, 1H), 8.07 (d, $J$ = 1.7 Hz, 1H), 8.01 (dt, $J$ = 7.5, 1.7 Hz, 1H), 7.91 (dd, $J$ = 8.9, 1.4 Hz, 1H), 7.71-7.60 (m, 2H), 7.54 (dd, $J$ = 8.9, 7.0 Hz, 1H), 7.23 (dd, $J$ = 7.0, 1.4 Hz, 1H), 3.76 (s, 4H), 3.62 (t, $J$ = 5.5 Hz, 4H), 3.20 (t, $J$ = 5.1 Hz, 4H), 1.66 (d, $J$ = 5.2 Hz, 2H), 1.57 (s, 4H).

MS (ESI, LR) Calc. for $C_{24}H_{28}N_5O_2$ $[M+H]^+$: 418.2, found: 418.2.

**Example 39. (7-(5-(morpholine-4-carbonyl)pyridin-3-yl)pyrazolo[1,5-a]pyridin-3-yl)(piperidin-1-yl)methanone**

<Scheme 5>

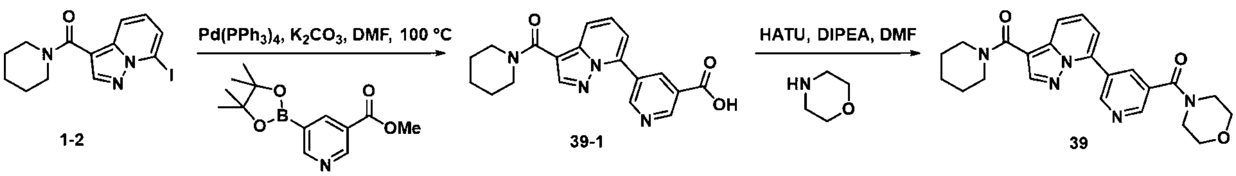


1-2 39-1 39

Step 39-1: Using compound **1-2** and methyl 5-(4,4,5,5-tetramethyl-1,3,2-dioxaborolan-2-yl)pyridine-3-carboxylate, the target compound **39-1** was obtained by the same synthetic method as step 1-3 in Example **1.**

Step 39-2: Using compound **39-1** and morpholine, the target compound **39** was obtained **by** the same synthetic method as step 1-2 in Example **1.**

$^1$H NMR (400 MHz, DMSO-$d_6$) δ(ppm): 9.15 (d, $J$ = 2.2 Hz, 1H), 8.77 (d, $J$ = 2.0 Hz, 1H), 8.48 (t, $J$ = 2.1 Hz, 1H), 8.27 (s, 1H), 7.95 (dd, $J$ = 8.9, 1.3 Hz, 1H), 7.55 (dd, $J$ = 8.9, 7.0 Hz, 1H), 7.36 (dd, $J$ = 7.1, 1.4 Hz, 1H), 3.67 (s, 4H), 3.63 (t, $J$ = 5.4 Hz, 6H), 3.47 (s, 2H), 1.66 (d, $J$ = 5.7 Hz, 2H), 1.57 (d, $J$ = 7.4 Hz, 4H).

MS (ESI, LR) Calc. for $C_{23}H_{26}N_5O_3$ $[M+H]^+$: 420.2, found: 420.2.

**Example 40. *N*-methyl-5-(3-(piperidine-1-carbonyl)pyrazolo[1,5-*a*]pyridin-7-yl)nicotinamide**

**Example 41. azetidin-1-yl(5-(3-(piperidine-1-carbonyl)pyrazolo[1,5-*a*]pyridin-7-yl)pyridin-3-yl)methanone**

**Example 42. (3-hydroxyazetidin-1-yl)(5-(3-(piperidine-1-carbonyl)pyrazolo[1,5-*a*]pyridin-7-yl)pyridin-3-yl) methanone**

**Example 43. (7-(5-(2-azaspiro[3.3]heptane-2-carbonyl)pyridin-3-yl)pyrazolo[1,5-*a*]pyridin-3-yl)(piperidin-1-yl) methanone**

**Example 44. (3,3-difluoroazetidin-1-yl)(5-(3-(piperidine-1-carbonyl)pyrazolo[1,5-*a*]pyridin-7-yl)pyridin-3-yl) methanone**

**Example 45. *N*-(2-methoxyethyl)-5-(3-(piperidine-1-carbonyl)pyrazolo[1,5-a]pyridin-7-yl)nicotinamide**

**Example 46. *N*-(cyclopropylmethyl)-5-(3-(piperidine-1-carbonyl)pyrazolo[1,5-*a*]pyridin-7-yl)nicotinamide**

**Example 47. *N*-(1-methylpiperidin-4-yl)-5-(3-(piperidine-1-carbonyl)pyrazolo [1,5-*a*]pyridin-7-yl)nicotinamide**

**Example 51. *N*-(2-cyanoethyl)-5-(3-(piperidine-1-carbonyl)pyrazolo[1,5-*a*]pyridin-7-yl)nicotinamide**

**Example 52. *N*-(cyanomethyl)-5-(3-(piperidine-1-carbonyl)pyrazolo[1,5-*a*]pyridin-7-yl)nicotinamide**

**Example 53. 5-(3-(piperidine-1-carbonyl)pyrazolo[1,5-*a*]pyridin-7-yl)-*N*-(pyridin-3-yl)nicotinamide**

**Example 54. 5-(3-(piperidine-1-carbonyl)pyrazolo[1,5-*a*]pyridin-7-yl)-*N*-(pyridin-4-yl)nicotinamide**

**Example 55. *N*-(isoxazol-3-ylmethyl)-5-(3-(piperidine-1-carbonyl)pyrazolo[1,5-*a*]pyridin-7-yl)nicotinamide**

**Example 56. 5-(3-(piperidine-1-carbonyl)pyrazolo[1,5-*a*]pyridin-7-yl)-*N*-(pyridazin-4-yl)nicotinamide**

**Example 57. 5-(3-(piperidine-1-carbonyl)pyrazolo[1,5-*a*]pyridin-7-yl)-*N*-(thiazol-5-ylmethyl)nicotinamide**

**Example 192. (3-fluoroazetidin-1-yl)(5-(3-(piperidine-1-carbonyl)pyrazolo[1,5-*a*]pyridin-7-yl)pyridin-3-yl)methanone**

**Example 193. *N*-(1-methyl-1H-pyrazol-3-yl)-5-(3-(piperidine-1-carbonyl)pyrazolo[1,5-*a*]pyridin-7-yl)nicotinamide**

**Example 201. *N*-methylsulfonyl-5-[3-(piperidine-1-carbonyl)pyrazolo[1,5-*a*]pyridin-7-yl]pyridine-3-carboxamide**

**Example 203. 4-[5-[3-(piperidine-1-carbonyl)pyrazolo[1,5-*a*]pyridin-7-yl]pyridine-3-carbonyl]piperazin-2-one**

**Example 204. 4-[5-[3-(piperidine-1-carbonyl)pyrazolo[1,5-*a*]pyridin-7-yl]pyridine-3-carbonyl]piperazine-2,6-dione**

**Example 225. (6-hydroxy-2-azaspiro[3.3]heptan-2-yl)-[5-[3-(piperidine-1-carbonyl)pyrazolo[1,5-*a*]pyridin-7-yl]-3-pyridyl]methanone**

**Example 226. 2-oxa-7-azaspiro[3.5]nonan-7-yl-[5-[3-(piperidine-1-carbonyl)pyrazolo[1,5-*a*]pyridin-7-yl]-3-pyridyl]methanone**

For Examples 40, 41, 42, 43, 44, 45, 46, 47, 51, 52, 53, 54, 55, 56, 57, 192, 193, 201, 203, 204, 225, and 226, the compounds were obtained by the same synthetic method as step 1-2 in Example **1** using the corresponding compounds and compound **39-1.** The structural and spectral data are shown in Table 4 below.

[Table 4]

| Example | Structure | $^1$H NMR | MS |
|---|---|---|---|
| **40** | 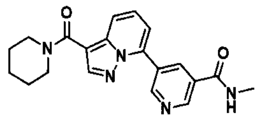 | $^1$H NMR (400 MHz, DMSO-$d_6$) δ(ppm): 9.15 (d, *J* = 2.2 Hz, 1H), 8.90 (d, *J* = 5.1 Hz, 1H), 8.58 (dd, *J* = 8.2, 2.2 Hz, 1H), 8.28 (s, 1H), 8.20 (d, *J* = 8.2 Hz, 1H), 7.95 (dd, *J* = 8.9, 1.3 Hz, 1H), 7.56 (dd, *J* = 8.9, 7.0 Hz, 1H), 7.37 (dd, *J* = 7.1, 1.4 Hz, 1H), 3.63 (t, *J* = 5.4 Hz, 4H), 2.87 (d, *J* = 4.8 Hz, 3H), 1.65 (d, *J* = 6.1 Hz, 2H), 1.57 (d, *J* = 7.4 Hz, 4H). | MS (ESI, LR) Calc. for $C_{20}H_{22}N_5O_2$ $[M+H]^+$: 364.2, found: 364.2. |
| **41** | 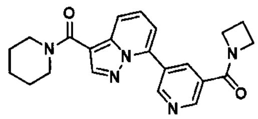 | $^1$H NMR (400 MHz, DMSO-$d_6$) δ(ppm): 9.17 (d, *J* = 2.2 Hz, 1H), 8.92 (d, *J* = 2.1 Hz, 1H), 8.62 (t, *J* = 2.2 Hz, 1H), 8.28 (s, 1H), 7.95 (dd, *J*= 8.9, 1.3 Hz, 1H), 7.55 (dd, *J* = 8.9, 7.0 Hz, 1H), 7.36 (dd, *J* = 7.0, 1.4 Hz, 1H), 4.42 (t, *J* = 7.7 Hz, 2H), 4.11 (t, *J* = 7.8 Hz, 2H), 3.63 *(t, J*= 5.4 Hz, 4H), 2.30 (p, *J* = 7.7 Hz, 2H), 1.66 (q, *J* = 5.9 Hz, 2H), 1.57 (q, *J* = 5.7 Hz, 4H). | MS (ESI, LR) Calc. for $C_{22}H_{24}N_5O_2$ $[M+H]^+$: 390.2, found: 390.2. |
| **42** | 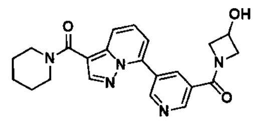 | $^1$H NMR (400 MHz, DMSO-$d_6$) δ(ppm): 9.18 (d, *J* = 2.2 Hz, 1H), 8.92 (d, *J* = 2.0 Hz, 1H), 8.62 (t, *J* = 2.1 Hz, 1H), 8.27 (s, 1H), 7.95 (dd, *J* = 8.9, 1.3 Hz, 1H), 7.55 (dd, *J* = 8.9, 7.0 Hz, 1H), 7.37 (dd, *J* = 7.0, 1.4 Hz, 1H), 5.82 (s, 1H), 4.57 (d, *J* = 8.4 Hz, 2H), 4.31 (dd, *J* = 10.1, 6.4 Hz, 1H), 4.21-4.11 (m, 1H), 3.85 (dd, *J* = 11.0, 3.4 Hz, 1H), 3.63 (t, *J* = 5.4 Hz, 4H), 1.65 (q, *J* = 5.8 Hz, 2H), 1.58 (t, *J* = 6.8 Hz, 4H). | MS (ESI, LR) Calc. for $C_{22}H_{24}N_5O_3$ $[M+H]^+$: 406.2, found: 406.2. |
| **43** | 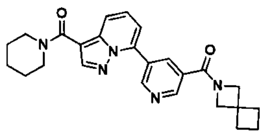 | $^1$H NMR (400 MHz, DMSO-$d_6$) δ(ppm): 9.18 (d, *J* = 2.2 Hz, 1H), 8.92 (d, *J* = 2.1 Hz, 1H), 8.59 (t, *J* = 2.1 Hz, 1H), 8.28 (s, 1H), 7.95 (dd, *J*= 9.0, 1.3 Hz, 1H), 7.55 (dd, *J* = 8.9, 7.0 Hz, 1H), 7.36 (dd, *J* = 7.1, 1.4 Hz, 1H), 4.39 (s, 2H), 4.08 (s, 2H), 3.63 (t, *J* = 5.4 Hz, 4H), 2.18 (t, *J* = 7.6 Hz, 4H), 1.78 (td, *J* = 7.9, 5.9 Hz, 2H), 1.71-1.61 (m, 2H), 1.58 (q, *J* = 5.7 Hz, 4H). | MS (ESI, LR) Calc. for $C_{25}H_{28}N_5O_2$ $[M+H]^+$: 430.2, found: 430.2. |
| **44** | 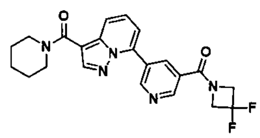 | $^1$H NMR (400 MHz, DMSO-$d_6$) δ(ppm): 9.23 (d, *J* = 2.1 Hz, 1H), 8.99 (d, *J* = 2.1 Hz, 1H), 8.67 (t, *J* = 2.2 Hz, 1H), 8.29 (s, 1H), 7.96 (dd, *J* = 8.9, 1.4 Hz, 1H), 7.56 (dd, *J* = 8.9, 7.0 Hz, 1H), 7.37 (dd, *J* = 7.1, 1.4 Hz, 1H), 4.93 (s, 2H), 4.56 (s, 2H), 3.63 (t, *J* = 5.4 Hz, 4H), 1.66 (d, *J* = 5.8 Hz, 2H), 1.57 (d, *J* = 7.4 Hz, 4H). | MS (ESI, LR) Calc. for $C_{22}H_{22}F_2N_5O_2$ $[M+H]^+$: 426.2, found: 426.1. |
| **45** | 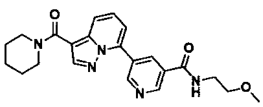 | $^1$H NMR (400 MHz, DMSO-$d_6$) δ(ppm): 9.16 (d, *J* = 2.2 Hz, 1H), 8.82 (s, 1H), 8.60 (dd, *J* = 8.2, 2.2 Hz, 1H), 8.28 (s, 1H), 8.21 (d, *J* = 8.2 Hz, 1H), 7.96 (dd, *J* = 8.8, 1.4 Hz, 1H), 7.57 (dd, *J* = 8.9, 7.0 Hz, 1H), 7.37 (dd, *J* = 7.1, 1.4 Hz, 1H), 3.63 (t, *J* = 5.4 Hz, 4H), 3.52 (d, *J* = 2.7 Hz, 4H), 3.30 (s, 3H), 1.66 (d, *J* = 5.6 Hz, 2H), 1.57 (d, *J* = 6.6 Hz, 4H). | MS (ESI, LR) Calc. for $C_{22}H_{26}N_5O_3$ $[M+H]^+$: 408.2, found: 408.1. |

(continued)

| Example | Structure | $^1H$ NMR | MS |
|---|---|---|---|
| **46** | 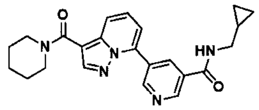 | $^1H$ NMR (400 MHz, DMSO-$d_6$) δ(ppm): 8.97 (d, *J* = 2.1 Hz, 1H), 8.88 (d, *J* = 2.1 Hz, 1H), 8.63 (t, *J* = 5.6 Hz, 1H), 8.51 (t, *J* = 2.2 Hz, 1H), 8.04 (s, 1H), 7.70 (dd, *J* = 8.9, 1.4 Hz, 1H), 7.32 (dd, *J* = 8.9, 7.0 Hz, 1H), 7.12 (dd, *J* = 7.1, 1.4 Hz, 1H), 3.38 (t, *J* = 5.4 Hz, 4H), 2.95 (t, *J* = 6.2 Hz, 2H), 1.40 (d, *J* = 5.5 Hz, 2H), 1.32 (s, 4H), 0.87-0.75 (m, 1H), 0.29 - 0.17 (m, 2H), 0.01 (dd, *J* = 4.8, 1.6 Hz, 2H). | MS (ESI, LR) Calc. for $C_{23}H_{26}N_5O_2$ $[M+H]^+$: 404.2, found: 404.1. |
| **47** | 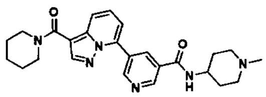 | $^1H$ NMR (400 MHz, DMSO-$d_6$) δ(ppm): 9.21 (d, *J* = 2.1 Hz, 1H), 9.11 (d, *J* = 2.1 Hz, 1H), 8.73 (t, *J* = 2.1 Hz, 1H), 8.56 (d, *J* = 7.6 Hz, 1H), 8.28 (s, 1H), 7.95 (dd, *J* = 8.9, 1.3 Hz, 1H), 7.56 (dd, *J* = 9.0, 7.0 Hz, 1H), 7.36 (dd, *J* = 7.1, 1.3 Hz, 1H), 3.87-3.73 (m, 1H), 3.63 (t, *J* = 5.4 Hz, 4H), 2.79 (d, *J* = 11.0 Hz, 2H), 2.19 (d, *J* = 4.9 Hz, 3H), 2.06 - 1.93 (m, 2H), 1.88-1.77 (m, 2H), 1.65 (dd, *J* = 7.9, 4.3 Hz, 2H), 1.57 (q, *J* = 7.6 Hz, 6H). | MS (ESI, LR) Calc. for $C_{25}H_{31}N_6O_2$ $[M+H]^+$: 447.2, found: 447.2. |
| **51** | 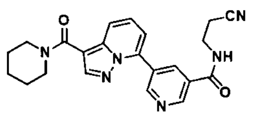 | $^1H$ NMR (400 MHz, DMSO-$d_6$) δ(ppm): 9.25 (d, *J* = 2.2 Hz, 1H), 9.22-9.12 (m, 2H), 8.78 (t, *J* = 2.1 Hz, 1H), 8.29 (s, 1H), 7.96 (dd, *J* = 8.9, 1.3 Hz, 1H), 7.57 (dd, *J* = 8.9, 7.0 Hz, 1H), 7.37 (dd, *J* = 7.0, 1.3 Hz, 1H), 3.63 (t, *J* = 5.4 Hz, 4H), 3.57 (q, *J* = 6.3 Hz, 2H), 2.82 (t, *J* = 6.5 Hz, 2H), 1.66 (d, *J* = 5.3 Hz, 2H), 1.57 (d, *J* = 7.6 Hz, 4H). | MS (ESI, LR) Calc. for $C_{22}H_{23}N_6O_2$ $[M+H]^+$: 403.2, found: 403.1. |
| **52** | 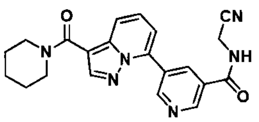 | $^1H$ NMR (400 MHz, DMSO-$d_6$) δ(ppm): 9.55 (s, 1H), 9.28 (d, *J* = 2.1 Hz, 1H), 9.14 (d, *J* = 2.1 Hz, 1H), 8.82 (t, *J* = 2.2 Hz, 1H), 8.29 (s, 1H), 7.96 (dd, *J* = 8.9, 1.3 Hz, 1H), 7.57 (dd, *J* = 8.9, 7.0 Hz, 1H), 7.39 (dd, *J*= 7.1, 1.4 Hz, 1H), 4.41 (d, *J* = 4.3 Hz, 2H), 3.63 (t, *J* = 5.4 Hz, 4H), 1.65 (d, *J* = 5.7 Hz, 2H), 1.57 (d, *J* = 7.4 Hz, 4H). | MS (ESI, LR) Calc. for $C_{21}H_{21}N_6O_2$ $[M+H]^+$: 389.2, found: 389.1. |
| **53** | 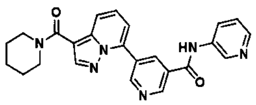 | $^1H$ NMR (400 MHz, DMSO-$d_6$) δ(ppm): 10.73 (s, 1H), 9.32 (d, *J* = 2.1 Hz, 1H), 9.26 (d, *J* = 2.1 Hz, 1H), 8.95 (d, *J* = 2.5 Hz, 1H), 8.90 (t, *J* = 2.2 Hz, 1H), 8.36 (dd, *J* = 4.7, 1.5 Hz, 1H), 8.30 (s, 1H), 8.21 (dt, *J* = 8.4, 1.9 Hz, 1H), 7.97 (dd, *J* = 8.9, 1.3 Hz, 1H), 7.59 (dd, *J* = 8.9, 7.0 Hz, 1H), 7.48-7.39 (m, 2H), 3.63 (t, *J* = 5.4 Hz, 4H), 1.66 (d, *J* = 4.9 Hz, 2H), 1.58 (q, *J* = 5.8 Hz, 4H). | MS (ESI, LR) Calc. for $C_{24}H_{23}N_6O_2$ $[M+H]^+$: 427.2, found: 427.1. |
| **54** | 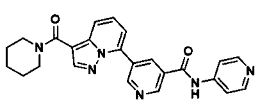 | $^1H$ NMR (400 MHz, DMSO-$d_6$) δ(ppm): 10.17 (s, 1H), 9.15 (d, *J* = 2.1 Hz, 1H), 8.64 - 8.57 (m, 2H), 8.53 (dd, *J* = 8.2, 2.1 Hz, 1H), 8.46 (d, *J* = 8.2 Hz, 1H), 8.12-8.05 (m, 2H), 7.77-7.71 (m, 2H), 7.43 (dd, J=9.0, 7.0 Hz, 1H), 7.07 (dd, *J* = 7.0, 1.4 Hz, 1H), 3.73 (t, *J* = 5.3 Hz, 4H), 1.79-1.72 (m, 2H), 1.69 (s, 4H). | MS (ESI, LR) Calc. for $C_{24}H_{23}N_6O_2$ $[M+H]^+$: 427.2, found: 427.1. |

(continued)

| Example | Structure | $^1$H NMR | MS |
|---|---|---|---|
| **55** | 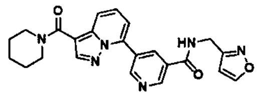 | $^1$H NMR (400 MHz, DMSO-$d_6$) δ(ppm): 9.41 (t, *J* = 5.9 Hz, 1H), 9.26 (d, *J*= 2.1 Hz, 1H), 9.16 (d, *J* = 2.1 Hz, 1H), 8.87 (d, *J* = 1.7 Hz, 1H), 8.80 (t, *J* = 2.1 Hz, 1H), 8.29 (s, 1H), 7.96 (dd, *J* = 8.9, 1.3 Hz, 1H), 7.57 (dd, *J* = 8.9, 7.0 Hz, 1H), 7.37 (dd, *J* = 7.1, 1.4 Hz, 1H), 6.62-6.55 (m, 1H), 4.62 (d, *J* = 5.8 Hz, 2H), 3.63 (t, *J* = 5.4 Hz, 4H), 1.66 (d, *J* = 5.6 Hz, 2H), 1.58 (d, *J* = 7.4 Hz, 4H). | MS (ESI, LR) Calc. for $C_{23}H_{23}N_6O_3$ [M+H]$^+$: 431.2, found: 431.1. |
| **56** | 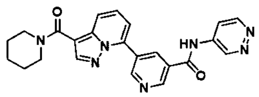 | $^1$H NMR (400 MHz, DMSO-$d_6$) δ(ppm): 11.10 (s, 1H), 9.52 (d, *J* = 2.7 Hz, 1H), 9.35 (d, *J* = 2.1 Hz, 1H), 9.27 (d, *J* = 2.1 Hz, 1H), 9.14 (d, *J* = 5.9 Hz, 1H), 8.91 (t, *J* = 2.1 Hz, 1H), 8.30 (s, 1H), 8.12 (dd, *J* = 5.9, 2.7 Hz, 1H), 7.98 (dd, *J* = 8.9, 1.3 Hz, 1H), 7.59 (dd, *J* = 8.9, 7.0 Hz, 1H), 7.42 (dd, *J* = 7.1, 1.4 Hz, 1H), 3.64 (t, *J* = 5.4 Hz, 4H), 1.73-1.63 (m, 2H), 1.58 (d, *J* = 7.2 Hz, 4H). | MS (ESI, LR) Calc. for $C_{23}H_{22}N_7O_2$ [M+H]$^+$: 428.2, found: 428.1. |
| **57** | 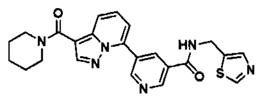 | $^1$H NMR (400 MHz, DMSO-$d_6$) δ(ppm): 9.49 (t, *J* = 5.7 Hz, 1H), 9.24 (d, *J* = 2.1 Hz, 1H), 9.14 (d, *J* = 2.1 Hz, 1H), 9.00 (s, 1H), 8.78 (t, *J* = 2.2 Hz, 1H), 8.28 (s, 1H), 7.95 (dd, *J* = 8.9, 1.3 Hz, 1H), 7.87 (s, 1H), 7.56 (dd, *J* = 8.9, 7.0 Hz, 1H), 7.36 (dd, *J* = 7.1, 1.4 Hz, 1H), 4.75 (d, *J* = 5.7 Hz, 2H), 3.62 (t, *J* = 5.4 Hz, 4H), 1.71-1.61 (m, 2H), 1.57 (s, 4H). | MS (ESI, LR) Calc. for $C_{23}H_{23}N_6O_2S$ [M+H]$^+$: 447.2, found: 447.1. |
| **192** | 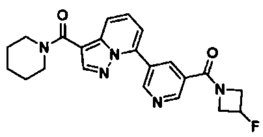 | $^1$H NMR (400 MHz, DMSO-$d_6$) δ(ppm): 9.20 (d, *J* = 2.2 Hz, 1H), 8.95 (d, *J* = 2.1 Hz, 1H), 8.64 (t, *J* = 2.1 Hz, 1H), 8.28 (s, 1H), 7.95 (dd, *J* = 8.9, 1.4 Hz, 1H), 7.55 (dd, *J* = 8.9, 7.0 Hz, 1H), 7.37 (dd, *J* = 7.0, 1.4 Hz, 1H), 5.48 (ddt, *J* = 57.6, 6.1, 2.9 Hz, 1H), 4.72-4.42 (m, 3H), 4.16 (dd, *J* = 24.6, 11.8 Hz, 1H), 3.63 (t, *J* = 5.4 Hz, 4H), 1.65 (d, *J* = 5.6 Hz, 2H), 1.57 (d, *J* = 7.7 Hz, 4H). | MS (ESI, LR) Calc. for $C_{22}H_{23}FN_5O_2$ [M+H]$^+$: 408.1, found: 408.1. |
| **193** | 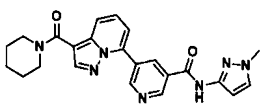 | $^1$H NMR (400 MHz, DMSO-$d_6$) δ(ppm): 11.22 (s, 1H), 9.31 (d, *J* = 2.1 Hz, 1H), 9.22 (d, *J* = 2.1 Hz, 1H), 8.92 (t, *J* = 2.2 Hz, 1H), 8.29 (s, 1H), 7.96 (dd, *J* = 9.0, 1.4 Hz, 1H), 7.65 (d, *J* = 2.2 Hz, 1H), 7.58 (dd, *J* = 8.9, 7.0 Hz, 1H), 7.42 (dd, *J* = 7.1, 1.3 Hz, 1H), 6.64 (d, *J* = 2.2 Hz, 1H), 3.80 (s, 3H), 3.63 (t, *J* = 5.4 Hz, 4H), 1.65 (d, *J* = 6.9 Hz, 2H), 1.57 (d, *J* = 7.4 Hz, 4H). | MS (ESI, LR) Calc. for $C_{23}H_{24}N_7O_2$ [M+H]$^+$: 430.1, found: 430.1. |
| **201** | 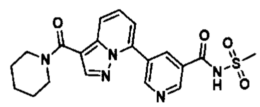 | 1H NMR (400 MHz, DMSO-*d6*) δ(ppm): 12.49 (s, 1H), 9.36 (d, *J*= 2.1 Hz, 1H), 9.18 (d, *J* = 2.1 Hz, 1H), 8.88 (t, *J* = 2.1 Hz, 1H), 8.30 (s, 1H), 7.96 (dd, *J* = 8.9, 1.3 Hz, 1H), 7.58 (dd, *J* = 8.9, 7.0 Hz, 1H), 7.40 (dd, *J*= 7.1, 1.4 Hz, 1H), 3.63 (t, *J* = 5.4 Hz, 4H), 3.41 (s, 3H), 1.70-1.62 (m, 2H), 1.57 (t, *J* = 6.1 Hz, 4H). | MS (ESI, LR) Calc. for $C_{20}H_{22}N_5O_4S$[M+H ]$^+$: 428.1, found: 428.1. |

(continued)

| Example | Structure | $^1$H NMR | MS |
|---|---|---|---|
| **203** | 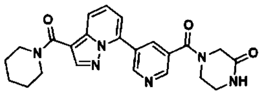 | 1H NMR (400 MHz, DMSO-*d6*) δ(ppm): 9.11 (d, *J* = 2.2 Hz, 1H), 8.84 (d, *J* = 2.1 Hz, 1H), 8.52 (t, *J* = 2.1 Hz, 1H), 8.11-7.98 (m, 2H), 7.41 (dd, *J* = 9.0, 7.0 Hz, 1H), 7.06 (dd, *J* = 7.0, 1.4 Hz, 1H), 6.34 (s, 1H), 4.38 (s, 2H), 3.90 (s, 2H), 3.72 (t, *J* = 5.3 Hz, 4H), 3.54 (s, 2H), 1.74 (s, 2H), 1.67 (d, *J* = 6.5 Hz, 4H). | MS (ESI, LR) Calc. for $C_{23}H_{25}N_6O_3[M+H]^+$: 433.2, found: 433.2. |
| **204** | 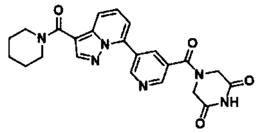 | 1H NMR (400 MHz, DMSO-*d6*) δ(ppm): 11.48 (s, 1H), 9.19 (d, *J* = 2.1 Hz, 1H), 8.82 (d, *J* = 2.1 Hz, 1H), 8.54 (t, *J* = 2.1 Hz, 1H), 8.26 (s, 1H), 7.95 (dd, *J* = 8.9, 1.4 Hz, 1H), 7.56 (dd, *J* = 8.9, 7.0 Hz, 1H), 7.37 (dd, *J* = 7.1, 1.4 Hz, 1H), 4.41 (s, 4H), 3.63 (t, *J* = 5.4 Hz, 4H), 1.65 (d, *J* = 6.5 Hz, 2H), 1.57 (d, *J* = 7.3 Hz, 4H). | MS (ESI, LR) Calc. for $C_{23}H_{23}N_6O_4[M+H]^+$ : 447.2, found: 447.1. |
| **225** | 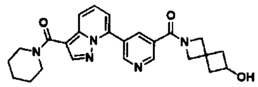 | 1H NMR (400 MHz, DMSO-*d6*) δ(ppm): 9.18 (t, *J* = 1.6 Hz, 1H), 8.91 (t, *J* = 1.8 Hz, 1H), 8.58 (q, *J* = 2.2 Hz, 1H), 8.28 (s, 1H), 7.94 (dd, *J* = 9.0, 1.4 Hz, 1H), 7.55 (dd, *J* = 8.9, 7.0 Hz, 1H), 7.35 (dd, *J* = 7.0, 1.4 Hz, 1H), 5.04 (d, *J* = 6.0 Hz, 1H), 4.36 (d, *J* = 17.6 Hz, 2H), 4.06 (d, *J* = 21.1 Hz, 2H), 3.96 (dq, *J* = 20.5, 6.8 Hz, 1H), 3.63 (t, *J* = 5.4 Hz, 4H), 2.47 (td, *J* = 6.7, 3.4 Hz, 2H), 1.99 (s, 2H), 1.66 (d, *J* = 5.7 Hz, 2H), 1.57 (s, 4H). | MS (ESI, LR) Calc. for $C_{25}H_{28}N_3O_3[M+H]^+$ : 446.2, found: 446.1. |
| **226** | 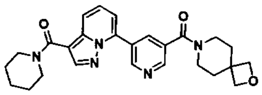 | $^1$H NMR (400 MHz, DMSO-*d6*) δ(ppm): 9.14 (d, *J* = 2.2 Hz, 1H), 8.73 (d, *J* = 2.0 Hz, 1H), 8.44 (t, *J* = 2.1 Hz, 1H), 8.27 (s, 1H), 7.94 (dd, *J* = 8.9, 1.3 Hz, 1H), 7.55 (dd, *J* = 8.9, 7.0 Hz, 1H), 7.36 (dd, *J* = 7.1, 1.4 Hz, 1H), 4.35 (d, *J* = 5.7 Hz, 4H), 3.73-3.51 (m, 6H), 3.36 (s, 2H), 1.86 (s, 4H), 1.65 (d, *J* = 6.3 Hz, 2H), 1.57 (d, *J* = 7.4 Hz, 4H). | MS (ESI, LR) Calc. for $C_{26}H_{30}N_5O_3[M+H]^+$ : 460.2, found: 460.1. |

**Example 48. piperazin-1-yl(5-(3-(piperidine-1-carbonyl)pyrazolo[1,5-*a*]pyridin-7-yl)pyridin-3-yl)methanone**

**Example 200. *N*-(azetidin-3-yl)-5-(3-(piperidine-1-carbonyl)pyrazolo[1,5-*a*]pyridin-7-yl)nicotinamide**

The target compound was obtained by sequentially using the same synthetic method of step 1-2 in Example **1** and the synthetic method of Example 34 using the corresponding compounds and compound **39-1.** The structural and spectral data are shown in Table 5 below.

[Table 5]

| Example | Structure | $^1$H NMR | MS |
|---|---|---|---|
| **48** | 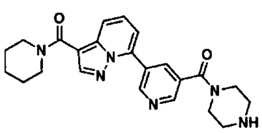 | $^1$H NMR (400 MHz, DMSO-$d_6$) δ(ppm): 9.17 (d, *J* = 2.1 Hz, 1H), 8.81 (d, *J* = 2.0 Hz, 1H), 8.54 (t, *J* = 2.1 Hz, 1H), 8.28 (s, 1H), 7.95 (dd, *J* = 8.9, 1.3 Hz, 1H), 7.57 (dd, *J* = 8.9, 7.0 Hz, 1H), 7.36 (dd, *J* = 7.1, 1.3 Hz, 1H), 3.63 (t, *J* = 5.5 Hz, 8H), 3.20 (t, *J* = 5.3 Hz, 4H), 1.66 (q, *J* = 5.9 Hz, 2H), 1.57 (q, *J* = 5.8 Hz, 4H). | MS (ESI, LR) Calc. for $C_{23}H_{28}N_6O_2$ $[M+H]^+$: 419.2, found: 419.1. |

(continued)

| Example | Structure | $^1$H NMR | MS |
|---|---|---|---|
| **200** | 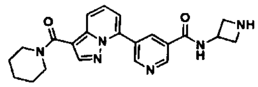 | 1H NMR (400 MHz, DMSO-*d6*) δ(ppm): 9.61 (d, *J* = 6.6 Hz, 1H), 9.27 (d, *J* = 2.1 Hz, 1H), 9.19 (d, *J* = 2.1 Hz, 1H), 9.05 (s, 2H), 8.82 (t, *J* = 2.1 Hz, 1H), 8.29 (s, 1H), 7.96 (dd, *J* = 8.9, 1.3 Hz, 1H), 7.57 (dd, *J* = 8.9, 7.0 Hz, 1H), 7.39 (dd, *J* = 7.0, 1.4 Hz, 1H), 4.88 (h, *J* = 7.6 Hz, 1H), 4.26-4.09 (m, 4H), 3.63 (t, *J* = 5.4 Hz, 4H), 1.66 (d, *J* = 6.8 Hz, 2H), 1.57 (s, 4H). | MS (ESI, LR) Calc. for $C_{22}H_{25}N_6O_2$ [M+H]$^+$: 405.2, found: 405.1. |

**Example 49. (4-methylpiperazin-1-yl)(5-(3-(piperidine-1-carbonyl)pyrazolo[1,5-*a*]pyridin-7-yl)pyridin-3-yl) methanone**

<Scheme 6>

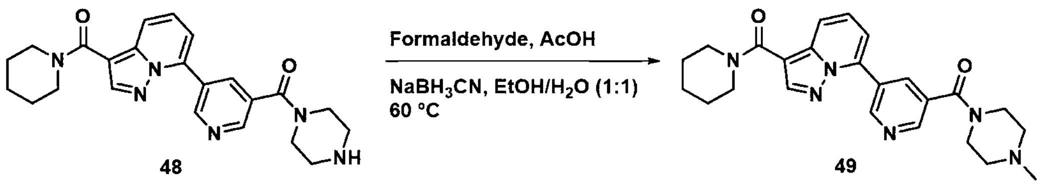


Compound **48** (50 mg, 0.12 mmol) was dissolved in ethyl alcohol (0.6 mL) and water (0.6 mL), 37% formaldehyde (17.8 μL, 0.24 mmol) was added, and the mixture was stirred at 60 °C for 1 hour. Sodium cyanoborohydride (15 mg, 0.24 mmol) and acetic acid (6.8 μL, 0.12 mmol) were added to the reaction solution, and the mixture was stirred at 60 °C for 12 hours. The reaction solution was concentrated and separated using column chromatography to obtain the target compound **49** (30 mg, 57%).

$^1$H NMR (400 MHz, DMSO-$d_6$) δ(ppm): 9.14 (d, *J* = 2.1 Hz, 1H), 8.74 (*d, J* = 2.0 Hz, 1H), 8.45 (t, *J* = 2.1 Hz, 1H), 8.27 (s, 1H), 7.94 (dd, *J* = 8.9, 1.3 Hz, 1H), 7.55 (dd, *J* = 8.9, 7.0 Hz, 1H), 7.36 (dd, *J* = 7.0, 1.4 Hz, 1H), 3.64 (q, *J* = 8.5 Hz, 6H), 3.44 (s, 2H), 2.37 (d, *J* = 19.9 Hz, 4H), 2.21 (s, 3H), 1.65 (d, *J* = 5.5 Hz, 2H), 1.57 (d, *J* = 7.2 Hz, 4H).

MS (ESI, LR) Calc. for $C_{24}H_{29}N_6O_2$ [M+H]$^+$: 433.2, found: 433.1.

**Example 202. *N*-(1-methylazetidin-3-yl)-5-[3-(piperidine-1-carbonyl)pyrazolo[1,5-*a*]pyridin-7-yl]pyridine-3-carboxamide**

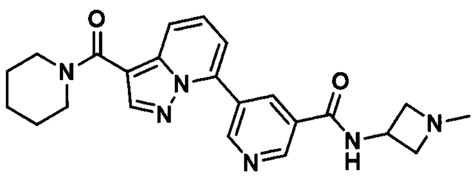

Using compound **200** and the corresponding compounds, the target compound 202 was obtained using the same synthetic method as Example 49.

$^1$H NMR (400 MHz, DMSO-$d_6$) δ(ppm): 9.68-9.54 (m, 1H), 9.26 (t, *J* = 2.4 Hz, 1H), 9.18 (d, *J* = 5.6 Hz, 1H), 8.81 (t, *J* = 2.0 Hz, 1H), 7.98-7.93 (m, 1H), 7.56 (dd, *J* = 8.9, 7.0 Hz, 1H), 7.38 (dd, *J* = 7.1, 2.9 Hz, 1H), 4.49 (s, 1H), 4.16 (dt, *J* = 18.1, 8.5 Hz, 2H), 3.62 (t, *J* = 5.4 Hz, 4H), 2.89 (d, *J* = 5.5 Hz, 4H), 2.73 (s, 1H), 1.65 (q, *J* = 5.6 Hz, 2H), 1.60-1.51 (m, 4H).

MS (ESI, LR) Calc. for $C_{23}H_{27}N_6O_2$ [M+H]$^+$: 419.1, found: 419.1.

**Example 50. (4-isopropylpiperazin-1-yl)(5-(3-(piperidine-1-carbonyl)pyrazolo[1,5-*a*]pyridin-7-yl)pyridin-3-yl) methanone**

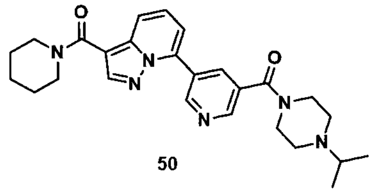

50

Using compound **48** and acetone, the target compound **50** was obtained by the synthetic method used in Example **49.**

$^1H$ NMR (400 MHz, DMSO-$d_6$) δ(ppm): 9.14 (d, *J* = 2.2 Hz, 1H), 8.75 (d, *J* = 2.1 Hz, 1H), 8.45 (t, *J* = 2.1 Hz, 1H), 8.27 (s, 1H), 7.94 (dd, *J* = 8.9, 1.3 Hz, 1H), 7.55 (dd, *J* = 8.9, 7.1 Hz, 1H), 7.36 (dd, *J* = 7.0, 1.3 Hz, 1H), 3.63 (t, *J* = 5.5 Hz, 6H), 3.42 (s, 2H), 2.70 (p, *J* = 6.5 Hz, 1H), 2.49 (d, *J* = 12.9 Hz, 4H), 1.65 (d, *J* = 5.1 Hz, 2H), 1.57 (s, 4H), 0.98 (d, *J* = 6.5 Hz, 6H).

MS (ESI, LR) Calc. for $C_{26}H_{33}N_6O_2$ $[M+H]^+$: 461.3, found: 461.2.

**Example 58. *N*-methyl-4-(3-(piperidine-1-carbonyl)pyrazolo[1,5-*a*]pyridin-7-yl)benzamide**

**Example 59. *N,N*-dimethyl-4-(3-(piperidine-1-carbonyl)pyrazolo[1,5-*a*]pyridin-7-yl)benzamide**

**Example 60. *N*-isopropyl-4-(3-(piperidine-1-carbonyl)pyrazolo[1,5-*a*]pyridin-7-yl)benzamide**

**Example 61. azetidin-1-yl(4-(3-(piperidine-1-carbonyl)pyrazolo[1,5-*a*]pyridin-7-yl)phenyl)methanone**

**Example 62. *N*-(2-methoxyethyl)-4-(3-(piperidine-1-carbonyl)pyrazolo[1,5-*a*]pyridin-7-yl)benzamide**

**Example 63. *N*-(2-(dimethylamino)ethyl)-4-(3-(piperidine-1-carbonyl)pyrazolo[1,5-*a*]pyridin-7-yl)benzamide**

**Example 64. *N*-(cyclopropylmethyl)-4-(3-(piperidine-1-carbonyl)pyrazolo[1,5-*a*]pyridin-7-yl)benzamide**

**Example 65. *N*-(1-methylpiperidin-4-yl)-4-(3-(piperidine-1-carbonyl)pyrazolo[1,5-*a*]pyridin-7-yl)benzamide**

**Example 69. *N*-(2-cyanoethyl)-4-(3-(piperidine-1-carbonyl)pyrazolo[1,5-*a*]pyridin-7-yl)benzamide**

**Example 70. *N*-(cyanomethyl)-4-(3-(piperidine-1-carbonyl)pyrazolo[1,5-*a*]pyridin-7-yl)benzamide**

**Example 71. 4-(3-(piperidine-1-carbonyl)pyrazolo[1,5-*a*]pyridin-7-yl)-*N*-(pyridin-3-yl)benzamide**

**Example 72. 4-(3-(piperidine-1-carbonyl)pyrazolo[1,5-*a*]pyridin-7-yl)-*N*-(pyridin-4-yl)benzamide**

**Example 73. *N*-(isoxazol-3-ylmethyl)-4-(3-(piperidine-1-carbonyl)pyrazolo[1,5-*a*]pyridin-7-yl)benzamide**

For Examples 58, 59, 60, 61, 62, 63, 64, 65, 69, 70, 71, 72, and 73, the compounds were obtained by the same synthetic method as step 1-2 in Example **1** using the corresponding compounds and compound **10.** The structural and spectral data are shown in Table 6 below.

[Table 6]

| Example | Structure | $^1H$ NMR | MS |
|---|---|---|---|
| **58** | 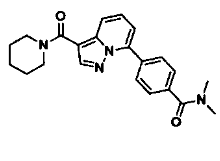 | $^1H$ NMR (400 MHz, DMSO-$d_6$) δ(ppm): 8.59 (d, *J* = 4.8 Hz, 1H), 8.25 (s, 1H), 8.01 (q, *J* = 8.6 Hz, 4H), 7.91 (dd, *J* = 8.9, 1.4 Hz, 1H), 7.53 (dd, *J* = 8.9, 7.0 Hz, 1H), 7.24 (dd, *J* = 7.1, 1.4 Hz, 1H), 3.63 (t, *J* = 5.4 Hz, 4H), 2.84 (d, *J* = 4.5 Hz, 3H), 1.71-1.61 (m, 2H), 1.57 (d, *J* = 7.5 Hz, 4H). | MS (ESI, LR) Calc. for $C_{21}H_{23}N_4O_2$ $[M+H]^+$: 363.2, found: 363.2. |

(continued)

| Example | Structure | $^1$H NMR | MS |
|---|---|---|---|
| **59** | 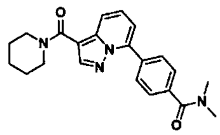 | $^1$H NMR (400 MHz, DMSO-$d_6$) δ(ppm): 8.25 (s, 1H), 8.04-7.97 (m, 2H), 7.90 (dd, *J* = 8.9, 1.3 Hz, 1H), 7.62-7.56 (m, 2H), 7.53 (dd, *J* = 8.9, 7.0 Hz, 1H), 7.23 (dd, *J* = 7.1, 1.4 Hz, 1H), 3.63 (t, *J* = 5.4 Hz, 4H), 3.01 (d, *J* = 17.6 Hz, 6H), 1.65 (d, *J* = 5.5 Hz, 2H), 1.57 (s, 4H). | MS (ESI, LR) Calc. for $C_{22}H_{25}N_4O_2$ $[M+H]^+$: 377.2, found: 377.3. |
| **60** | 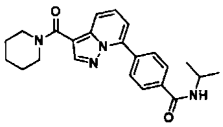 | $^1$H NMR (400 MHz, DMSO-$d_6$) δ(ppm): 8.05 (s, 1H), 7.99 (dd, *J* = 8.9, 1.4 Hz, 1H), 7.96 - 7.86 (m, 4H), 7.38 (dd, *J* = 8.9, 7.0 Hz, 1H), 6.97 (dd, *J* = 7.0, 1.4 Hz, 1H), 5.98 (d, *J* = 7.8 Hz, 1H), 4.32 (dq, *J* = 13.4, 6.7 Hz, 1H), 3.72 (t, *J* = 5.3 Hz, 4H), 1.73-1.65 (m, 6H), 1.29 (d, *J* = 6.5 Hz, 6H). | MS (ESI, LR) Calc. for $C_{23}H_{27}N_4O_2$ $[M+H]^+$: 391.2, found: 391.2. |
| **61** | 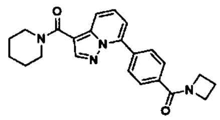 | $^1$H NMR (400 MHz, DMSO-$d_6$) δ(ppm): 8.25 (s, 1H), 8.06-7.98 (m, 2H), 7.91 (dd, *J* = 8.9, 1.4 Hz, 1H), 7.84-7.74 (m, 2H), 7.53 (dd, *J* = 8.9, 7.0 Hz, 1H), 7.23 (dd, *J* = 7.1, 1.4 Hz, 1H), 4.38 (t, *J* = 7.7 Hz, 2H), 4.10 (t, *J* = 7.8 Hz, 2H), 3.63 (t, *J* = 5.4 Hz, 4H), 2.30 (p, *J* = 7.7 Hz, 2H), 1.72-1.61 (m, 2H), 1.57 (q, *J* = 6.0 Hz, 4H). | MS (ESI, LR) Calc. for $C_{23}H_{25}N_4O_2$ $[M+H]^+$: 389.2, found: 389.2. |
| **62** | 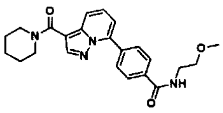 | $^1$H NMR (400 MHz, DMSO-$d_6$) δ(ppm): 8.68 (d, *J* = 6.3 Hz, 1H), 8.25 (s, 1H), 8.09-7.97 (m, 4H), 7.91 (dd, *J* = 8.9, 1.4 Hz, 1H), 7.53 (dd, *J* = 8.9, 7.0 Hz, 1H), 7.24 (dd, *J* = 7.1, 1.4 Hz, 1H), 3.63 (t, *J* = 5.4 Hz, 4H), 3.57-3.44 (m, 4H), 3.30 (s, 3H), 1.65 (d, *J* = 5.4 Hz, 2H), 1.57 (d, *J* = 7.4 Hz, 4H). | MS (ESI, LR) Calc. for $C_{23}H_{27}N_4O_3$ $[M+H]^+$: 407.2, found: 407.2. |
| **63** | 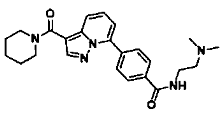 | $^1$H NMR (400 MHz, DMSO-$d_6$) δ(ppm): 8.53 (s, 1H), 8.25 (s, 1H), 8.01 (q, *J* = 8.6 Hz, 4H), 7.91 (dd, *J* = 8.9, 1.3 Hz, 1H), 7.53 (dd, *J* = 8.9, 7.0 Hz, 1H), 7.23 (dd, *J* = 7.1, 1.4 Hz, 1H), 3.63 (t, *J* = 5.5 Hz, 4H), 3.40 (q, *J* = 6.5 Hz, 2H), 2.44 (t, *J* = 6.9 Hz, 2H), 2.20 (s, 6H), 1.65 (d, *J* = 6.5 Hz, 2H), 1.57 (d, *J* = 6.9 Hz, 4H). | MS (ESI, LR) Calc. for $C_{24}H_{30}N_5O_2$ $[M+H]^+$: 420.2, found: 420.2. |
| **64** | 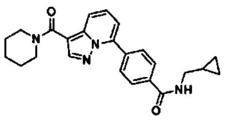 | $^1$H NMR (400 MHz, DMSO-$d_6$) δ(ppm): 8.06 (s, 1H), 7.99 (dd, *J* = 8.9, 1.4 Hz, 1H), 7.94 (s, 4H), 7.39 (dd, *J* = 8.9, 7.0 Hz, 1H), 6.98 (dd, *J* = 7.0, 1.4 Hz, 1H), 6.27 (s, 1H), 3.72 (t, *J* = 5.4 Hz, 4H), 3.36 (dd, *J* = 7.2, 5.3 Hz, 2H), 1.73-1.66 (m, 6H), 1.09 (ddd, *J* = 12.6, 7.8, 4.9 Hz, 1H), 0.63-0.53 (m, 2H), 0.35-0.24 (m, 2H). | MS (ESI, LR) Calc. for $C_{24}H_{27}N_4O_2$ $[M+H]^+$: 403.2, found: 403.2. |
| **65** | 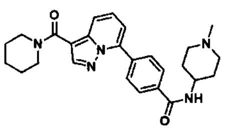 | $^1$H NMR (400 MHz, DMSO-$d_6$) δ(ppm): 8.38 (d, *J* = 7.7 Hz, 1H), 8.25 (s, 1H), 8.06-7.96 (m, 4H), 7.91 (dd, *J* = 8.9, 1.3 Hz, 1H), 7.53 (dd, *J* = 8.9, 7.0 Hz, 1H), 7.22 (dd, *J* = 7.2, 1.4 Hz, 1H), 3.83-3.71 (m, 1H), 3.62 (t, *J* = 5.4 Hz, 4H), 2.79 (d, *J* = 11.0 Hz, 2H), 2.18 (s, 3H), 1.96 (dd, *J* = 12.8, 10.3 Hz, 2H), 1.79 (d, *J* = 12.3 Hz, 2H), 1.70 - 1.53 (m, 8H). | MS (ESI, LR) Calc. for $C_{26}H_{32}N_5O_2$ $[M+H]^+$: 446.3, found: 446.2. |
| **69** | 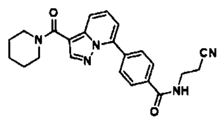 | $^1$H NMR (400 MHz, DMSO-$d_6$) δ(ppm): 9.00 (t, *J* = 5.7 Hz, 1H), 8.25 (s, 1H), 8.07 (d, *J* = 8.3 Hz, 2H), 8.02 (d, *J*= 8.5 Hz, 2H), 7.92 (dd, *J* = 8.9, 1.3 Hz, 1H), 7.54 (dd, *J* = 8.9, 7.1 Hz, 1H), 7.25 (dd, *J* = 7.0, 1.4 Hz, 1H), 3.63 (t, *J* = 5.4 Hz, 4H), 3.55 (q, *J* = 6.3 Hz, 2H), 2.83 (t, *J* = 6.5 Hz, 2H), 1.65 (d, *J* = 8.1 Hz, 2H), 1.57 (d, *J* = 7.2 Hz, 4H). | MS (ESI, LR) Calc. for $C_{23}H_{24}N_5O_2$ $[M+H]^+$: 402.2, found: 402.1. |

(continued)

| Example | Structure | $^1H$ NMR | MS |
|---|---|---|---|
| **70** | 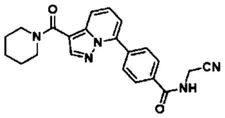 | $^1H$ NMR (400 MHz, DMSO-$d_6$) δ(ppm): 9.36 (t, $J$ = 5.4 Hz, 1H), 8.25 (s, 1H), 8.08 (d, $J$ = 8.5 Hz, 2H), 8.03 (d, $J$ = 8.3 Hz, 2H), 7.92 (dd, $J$ = 8.9, 1.3 Hz, 1H), 7.53 (dd, $J$ = 8.9, 7.1 Hz, 1H), 7.25 (dd, $J$ = 7.1, 1.4 Hz, 1H), 4.37 (d, $J$ = 5.4 Hz, 2H), 3.62 (t, $J$ = 5.4 Hz, 4H), 1.65 (d, $J$ = 5.3 Hz, 2H), 1.57 (s, 4H). | MS (ESI, LR) Calc. for $C_{22}H_{22}N_5O_2$ $[M+H]^+$: 388.2, found: 388.1. |
| **71** | 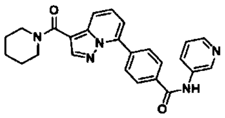 | $^1H$ NMR (400 MHz, DMSO-$d_6$) δ(ppm): 10.61 (s, 1H), 8.98 (d, $J$ = 2.5 Hz, 1H), 8.35 (dd, $J$ = 4.7, 1.5 Hz, 1H), 8.30-8.21 (m, 2H), 8.19-8.06 (m, 4H), 7.93 (dd, $J$ = 8.9, 1.4 Hz, 1H), 7.55 (dd, $J$ = 8.9, 7.0 Hz, 1H), 7.43 (dd, $J$ = 8.3, 4.7 Hz, 1H), 7.28 (dd, $J$ = 7.0, 1.4 Hz, 1H), 3.63 (t, $J$ = 5.4 Hz, 4H), 1.66 (d, $J$ = 6.6 Hz, 2H), 1.58 (d, $J$ = 7.0 Hz, 4H). | MS (ESI, LR) Calc. for $C_{25}H_{24}N_5O_2$ $[M+H]^+$: 426.2, found: 426.1. |
| **72** | 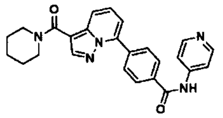 | $^1H$ NMR (400 MHz, DMSO-$d_6$) δ(ppm): 10.75 (s, 1H), 8.57-8.48 (m, 2H), 8.27 (s, 1H), 8.13 (s, 4H), 7.93 (dd, $J$ = 8.9, 1.3 Hz, 1H), 7.88-7.79 (m, 2H), 7.55 (dd, $J$ = 8.9, 7.0 Hz, 1H), 7.28 (dd, $J$ = 7.1, 1.3 Hz, 1H), 3.63 (t, $J$ = 5.4 Hz, 4H), 1.66 (d, $J$ = 5.8 Hz, 2H), 1.62-1.49 (m, 4H). | MS (ESI, LR) Calc. for $C_{25}H_{24}N_5O_2$ $[M+H]^+$: 426.2, found: 426.2. |
| **73** | 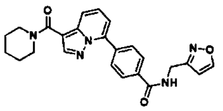 | $^1H$ NMR (400 MHz, DMSO-$d_6$) δ(ppm): 9.26 (t, $J$ = 5.8 Hz, 1H), 8.87 (d, $J$ = 1.7 Hz, 1H), 8.25 (s, 1H), 8.05 (s, 4H), 7.91 (dd, $J$ = 8.8, 1.4 Hz, 1H), 7.53 (dd, $J$ = 8.9, 7.1 Hz, 1H), 7.24 (dd, $J$ = 7.1, 1.4 Hz, 1H), 6.55 (d, $J$ = 1.7 Hz, 1H), 4.61 (d, $J$ = 5.9 Hz, 2H), 3.63 (t, $J$ = 5.5 Hz, 4H), 1.65 (d, $J$ = 5.6 Hz, 2H), 1.57 (s, 4H). | MS (ESI, LR) Calc. for $C_{24}H_{24}N_5O_3$ $[M+H]^+$: 430.2, found: 430.2. |

**Example 66. piperazin-1-yl(4-(3-(piperidine-1-carbonyl)pyrazolo[1,5-*a*]pyridin-7-yl)phenyl)methanone**

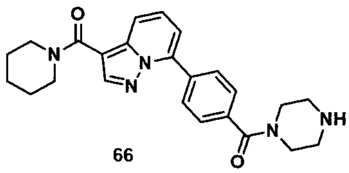

**66**

Using compound **10** and 1-Boc-piperazine, the target compound 66 was obtained by sequentially using the synthetic method of step 1-2 in Example 1 and the synthetic method of Example **34.**

$^1H$ NMR (400 MHz, DMSO-$d_6$) δ(ppm): 8.24 (s, 1H), 8.03 (d, $J$ = 8.1 Hz, 2H), 7.90 (dd, $J$ = 8.9, 1.3 Hz, 1H), 7.65 (d, $J$ = 8.3 Hz, 2H), 7.53 (dd, $J$ = 8.9, 7.0 Hz, 1H), 7.22 (dd, $J$ = 7.1, 1.4 Hz, 1H), 3.62 (t, $J$ = 5.4 Hz, 8H), 3.18 (s, 4H), 1.66 (d, $J$ = 5.8 Hz, 2H), 1.57 (d, $J$ = 7.4 Hz, 4H).

MS (ESI, LR) Calc. for $C_{24}H_{28}N_5O_2$ $[M+H]^+$: 418.2, found: 418.1.

**Example 67. (4-methylpiperazin-1-yl)(4-(3-(piperidine-1-carbonyl)pyrazolo[1,5-*a*]pyridin-7-yl)phenyl)methanone**

**Example 68. (4-isopropylpiperazin-1-yl)(4-(3-(piperidine-1-carbonyl)pyrazolo[1,5-*a*]pyridin-7-yl)phenyl)methanone**

For Example 67 and 68, the compounds were obtained by the same synthetic method as Example **49** using the corresponding compounds and compound **66.** The structural and spectral data are shown in Table 7 below.

[Table 7]

| Example | Structure | $^1H$ NMR | MS |
|---|---|---|---|
| **67** | 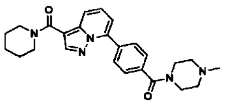 | $^1H$ NMR (400 MHz, DMSO-$d_6$) δ(ppm): 8.24 (s, 1H), 8.06-7.96 (m, 2H), 7.90 (dd, *J* = 8.9, 1.3 Hz, 1H), 7.63-7.48 (m, 3H), 7.22 (dd, *J* = 7.0, 1.3 Hz, 1H), 3.62 (t, *J* = 5.3 Hz, 8H), 2.36 (d, *J* = 19.6 Hz, 4H), 2.22 (s, 3H), 1.70-1.62 (m, 2H), 1.57 (d, *J* = 6.9 Hz, 4H). | MS (ESI, LR) Calc. for $C_{25}H_{30}N_3O_2$ $[M+H]^+$: 432.2, found: 432.1. |
| **68** | 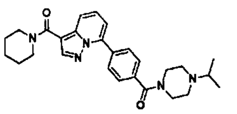 | $^1H$ NMR (400 MHz, DMSO-$d_6$) δ(ppm): 8.24 (s, 1H), 8.05-7.96 (m, 2H), 7.90 (dd, *J* = 8.9, 1.3 Hz, 1H), 7.54 (dd, *J* = 16.2, 8.6 Hz, 3H), 7.22 (dd, *J* = 7.1, 1.4 Hz, 1H), 3.74-3.54 (m, 6H), 3.41 (s, 2H), 2.70 (p, *J* = 6.5 Hz, 1H), 2.51-2.35 (m, 4H), 1.65 (d, *J* = 5.8 Hz, 2H), 1.57 (d, *J* = 7.2 Hz, 4H), 0.99 (d, *J* = 6.5 Hz, 6H). | MS (ESI, LR) Calc. for $C_{27}H_{34}N_5O_2$ $[M+H]^+$: 460.3, found: 460.2. |

**Example 74. (7-(6-(morpholine-4-carbonyl)pyridin-3-yl)pyrazolo[1,5-*a*]pyridin-3-yl)(piperidin-1-yl)methanone**

<Scheme 7>

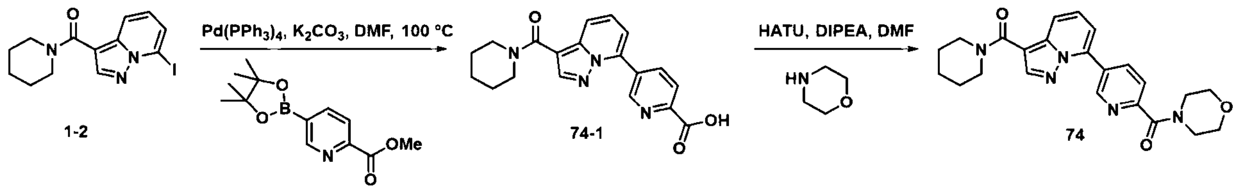


1-2 74-1 74

Using compound **1-2** and methyl 5-(4,4,5,5-tetramethyl-1,3,2-dioxaborolan-2-yl)pyridine-2-carboxylate, the target compound **74** was obtained by the same synthetic method as Example **39.**

$^1H$ NMR (400 MHz, DMSO-$d_6$) δ(ppm): 9.15 (d, *J* = 2.1 Hz, 1H), 8.77 (d, *J* = 2.0 Hz, 1H), 8.48 (t, *J* = 2.1 Hz, 1H), 8.27 (s, 1H), 7.95 (dd, *J* = 9.0, 1.3 Hz, 1H), 7.55 (dd, *J* = 8.9, 7.0 Hz, 1H), 7.36 (dd, *J* = 7.0, 1.4 Hz, 1H), 3.64 (q, *J* = 9.3 Hz, 10H), 3.46 (s, 2H), 1.72-1.61 (m, 2H), 1.58 (t, *J* = 6.5 Hz, 4H).

MS (ESI, LR) Calc. for $C_{23}H_{26}N_5O_3$ $[M+H]^+$: 420.2, found: 420.1.

**Example 75. *N*-methyl-5-(3-(piperidine-1-carbonyl)pyrazolo[1,5-*a*]pyridin-7-yl)picolinamide**

**Example 76. *N*-isopropyl-5-(3-(piperidine-1-carbonyl)pyrazolo[1,5-*a*]pyridin-7-yl)picolinamide**

**Example 77. azetidin-1-yl(5-(3-(piperidine-1-carbonyl)pyrazolo[1,5-*a*]pyridin-7-yl)pyridin-2-yl)methanone**

**Example 78. *N*-(2-methoxyethyl)-5-(3-(piperidine-1-carbonyl)pyrazolo[1,5-*a*]pyridin-7-yl)picolinamide**

**Example 79. *N*-(2-(dimethylamino)ethyl)-5-(3-(piperidine-1-carbonyl)pyrazolo[1,5-*a*]pyridin-7-yl)picolinamide**

**Example 80. *N*-(cyclopropylmethyl)-5-(3-(piperidine-1-carbonyl)pyrazolo[1,5-*a*]pyridin-7-yl)picolinamide**

**Example 81. *N*-(1-methylpiperidin-4-yl)-5-(3-(piperidine-1-carbonyl)pyrazolo [1,5-*a*]pyridin-7-yl)picolinamide**

**Example 85. *N*-(2-cyanoethyl)-5-(3-(piperidine-1-carbonyl)pyrazolo[1,5-*a*]pyridin-7-yl)picolinamide**

**Example 86. *N*-(cyanomethyl)-5-(3-(piperidine-1-carbonyl)pyrazolo[1,5-*a*]pyridin-7-yl)picolinamide**

**Example 87. 5-(3-(piperidine-1-carbonyl)pyrazolo[1,5-*a*]pyridin-7-yl)-*N*-(pyridin-3-yl)picolinamide**

**Example 88. 5-(3-(piperidine-1-carbonyl)pyrazolo[1,5-*a*]pyridin-7-yl)-*N*-(pyridin-4-yl)picolinamide**

**Example 89. *N*-(isoxazol-3-ylmethyl)-5-(3-(piperidine-1-carbonyl)pyrazolo[1,5-*a*]pyridin-7-yl)picolinamide**

**Example 213. (3-fluoroazetidin-1-yl)-[5-[3-(piperidine-1-carbonyl)pyrazolo[1,5-*a*]pyridin-7-yl]-2-pyridyl]methanone**

**Example 214. 2-azaspiro[3.3]heptan-2-yl-[5-[3-(piperidine-1-carbonyl)pyrazolo[1,5-*a*]pyridin-7-yl]-2-pyridyl] methanone**

**Example 237. (3,3-difluoroazetidin-1-yl)-[5-[3-(piperidine-1-carbonyl)pyrazolo[1,5-*a*]pyridin-7-yl]-2-pyridyl] methanone**

For Examples 75, 76, 77, 78, 79, 80, 81, 85, 86, 87, 88, 89, 213, 214, and 237, the compounds were obtained by the same synthetic method as step 1-2 in Example **1** using the corresponding compounds and compound **74-1.** The structural and spectral data are shown in Table 8 below.

[Table 8]

| Example | Structure | $^1$H NMR | MS |
|---|---|---|---|
| **75** | 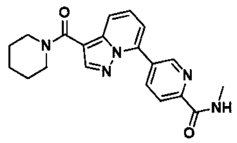 | $^1$ H NMR (400 MHz, DMSO-$d_6$) δ(ppm): 9.22 (d, *J* = 2.1 Hz, 1H), 9.10 (d, *J* = 2.1 Hz, 1H), 8.81-8.70 (m, 2H), 8.28 (s, 1H), 7.95 (dd, *J* = 8.9, 1.3 Hz, 1H), 7.56 (dd, *J* = 8.9, 7.0 Hz, 1H), 7.36 (dd, *J* = 7.1, 1.3 Hz, 1H), 3.63 (t, *J* = 5.4 Hz, 4H), 2.85 (d, *J* = 4.5 Hz, 3H), 1.65 (d, *J* = 6.2 Hz, 2H), 1.57 (s, 4H). | MS (ESI, LR) Calc. for $C_{20}H_{22}N_5O_2$ [M+H]$^+$: 364.2, found: 364.2. |
| **76** | 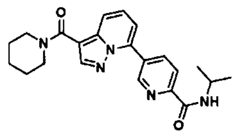 | $^1$H NMR (400 MHz, DMSO-$d_6$) δ(ppm): 9.20 (d, *J* = 2.1 Hz, 1H), 9.11 (d, *J* = 2.1 Hz, 1H), 8.73 (t, *J* = 2.1 Hz, 1H), 8.53 (d, *J* = 7.6 Hz, 1H), 8.28 (s, 1H), 7.95 (dd, *J* = 8.9, 1.3 Hz, 1H), 7.56 (dd, *J* = 8.9, 7.0 Hz, 1H), 7.36 (dd, *J* = 7.0, 1.3 Hz, 1H), 4.14 (dq, *J* = 13.4, 6.6 Hz, 1H), 3.63 (t, *J* = 5.4 Hz, 4H), 1.65 (d, *J* = 6.5 Hz, 2H), 1.57 (s, 4H), 1.20 (d, *J* = 6.6 Hz, 6H). | MS (ESI, LR) Calc. for $C_{22}H_{26}N_5O_2$ [M+H]$^+$: 392.2, found: 392.2. |
| **77** | 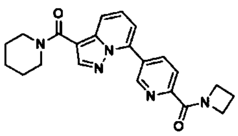 | $^1$H NMR (400 MHz, DMSO-$d_6$) δ(ppm): 9.17 (d, *J* = 2.2 Hz, 1H), 8.92 (d, *J* = 2.1 Hz, 1H), 8.62 (t, *J* = 2.1 Hz, 1H), 8.27 (s, 1H), 7.95 (dd, *J* = 8.9, 1.3 Hz, 1H), 7.55 (dd, *J* = 8.9, 7.1 Hz, 1H), 7.36 (dd, *J* = 7.1, 1.4 Hz, 1H), 4.42 (t, *J* = 7.7 Hz, 2H), 4.11 (t, *J* = 7.8 Hz, 2H), 3.63 (t, *J* = 5.4 Hz, 4H), 2.30 (p, *J* = 7.6 Hz, 2H), 1.65 (d, *J* = 5.4 Hz, 2H), 1.57 (d, *J* = 6.4 Hz, 4H). | MS (ESI, LR) Calc. for $C_{22}H_{24}N_5O_2$ [M+H]$^+$: 390.2, found: 390.1. |
| **78** | 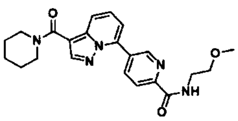 | $^1$H NMR (400 MHz, DMSO-$d_6$) δ(ppm): 9.22 (d, *J* = 2.1 Hz, 1H), 9.12 (d, *J* = 2.1 Hz, 1H), 8.86 (s, 1H), 8.76 (t, *J* = 2.2 Hz, 1H), 8.28 (s, 1H), 7.95 (dd, *J* = 8.9, 1.3 Hz, 1H), 7.56 (dd, *J* = 8.9, 7.1 Hz, 1H), 7.36 (dd, *J* = 7.1, 1.4 Hz, 1H), 3.63 (t, *J* = 5.4 Hz, 4H), 3.49 (q, *J* = 2.8 Hz, 4H), 3.29 (s, 3H), 1.71-1.61 (m, 2H), 1.57 (d, *J* = 7.3 Hz, 4H). | MS (ESI, LR) Calc. for $C_{22}H_{26}N_5O_3$ [M+H]$^+$: 408.2, found: 408.2. |
| **79** | 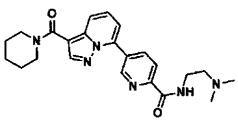 | $^1$H NMR (400 MHz, DMSO-$d_6$) δ(ppm): 9.22 (d, *J* = 2.1 Hz, 1H), 9.11 (d, *J* = 2.1 Hz, 1H), 8.79-8.67 (m, 2H), 8.29 (s, 1H), 7.95 (dd, *J* = 8.9, 1.3 Hz, 1H), 7.57 (dd, *J* = 8.9, 7.0 Hz, 1H), 7.36 (dd, *J* = 7.1, 1.3 Hz, 1H), 3.63 (t, *J* = 5.4 Hz, 4H), 3.41 (q, *J* = 6.5 Hz, 2H), 2.43 (t, *J* = 6.8 Hz, 2H), 2.19 (s, 6H), 1.66 (d, *J* = 6.4 Hz, 2H), 1.57 (d, *J* = 6.6 Hz, 4H). | MS (ESI, LR) Calc. for $C_{23}H_{29}N_6O_2$ [M+H]$^+$: 421.2, found: 421.2. |

(continued)

| Example | Structure | $^1$H NMR | MS |
|---|---|---|---|
| **80** | 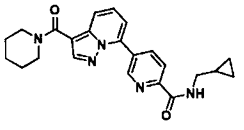 | $^1$H NMR (400 MHz, DMSO-$d_6$) δ(ppm): 9.22 (d, $J$ = 2.1 Hz, 1H), 9.13 (d, $J$ = 2.1 Hz, 1H), 8.88 (t, $J$ = 5.6 Hz, 1H), 8.77 (t, $J$ = 2.1 Hz, 1H), 8.29 (s, 1H), 7.96 (dd, $J$ = 8.9, 1.3 Hz, 1H), 7.57 (dd, $J$ = 8.9, 7.0 Hz, 1H), 7.37 (dd, $J$ = 7.1, 1.4 Hz, 1H), 3.63 (t, $J$ = 5.4 Hz, 4H), 3.20 (t, $J$ = 6.2 Hz, 2H), 1.66 (d, $J$= 5.4 Hz, 2H), 1.58 (d, $J$ = 4.4 Hz, 4H), 1.12 - 1.00 (m, 1H), 0.51-0.42 (m, 2H), 0.30-0.21 (m, 2H). | MS (ESI, LR) Calc. for $C_{23}H_{26}N_5O_2$ $[M+H]^+$: 404.2, found: 404.2. |
| **81** | 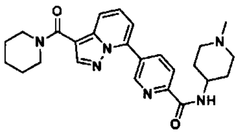 | $^1$H NMR (400 MHz, DMSO-$d_6$) δ(ppm): 9.20 - 9.12 (m, 1H), 8.66 (d, $J$ = 8.4 Hz, 1H), 8.58 (dd, $J$ = 8.2, 2.2 Hz, 1H), 8.27 (s, 1H), 8.23-8.14 (m, 1H), 7.95 (dd, $J$ = 8.9, 1.3 Hz, 1H), 7.56 (dd, $J$ = 8.9, 7.0 Hz, 1H), 7.36 (dd, $J$ = 7.0, 1.4 Hz, 1H), 3.80 (dd, $J$ = 9.4, 4.9 Hz, 1H), 3.63 (t, $J$ = 5.4 Hz, 4H), 2.76 (d, $J$ = 11.2 Hz, 2H), 2.18 (s, 3H), 1.99 (t, $J$ = 11.0 Hz, 2H), 1.74 (dt, $J$ = 14.0, 10.0 Hz, 4H), 1.65 (d, $J$ = 6.1 Hz, 2H), 1.62 - 1.51 (m, 4H). | MS (ESI, LR) Calc. for $C_{25}H_{31}N_6O_2$ $[M+H]^+$: 447.2, found: 447.3. |
| **85** | 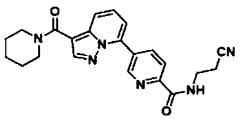 | $^1$H NMR (400 MHz, DMSO-$d_6$) δ(ppm): 9.25 (d, $J$ = 2.1 Hz, 1H), 9.17 (t, $J$ = 5.6 Hz, 1H), 9.14 (d, $J$ = 2.1 Hz, 1H), 8.78 (t, $J$ = 2.1 Hz, 1H), 8.29 (s, 1H), 7.96 (dd, $J$ = 8.9, 1.3 Hz, 1H), 7.57 (dd, $J$ = 8.9, 7.0 Hz, 1H), 7.37 (dd, $J$ = 7.1, 1.4 Hz, 1H), 3.63 (t, $J$ = 5.4 Hz, 4H), 3.57 (q, $J$ = 6.2 Hz, 2H), 2.82 (t, $J$ = 6.5 Hz, 2H), 1.66 (d, $J$ = 5.5 Hz, 2H), 1.62-1.52 (m, 4H). | MS (ESI, LR) Calc. for $C_{22}H_{23}N_6O_2$ $[M+H]^+$: 403.2, found: 403.2. |
| **86** | 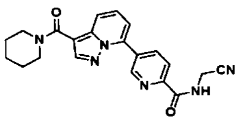 | $^1$H NMR (400 MHz, DMSO-$d_6$) δ(ppm): 9.54 (s, 1H), 9.28 (d, $J$ = 2.1 Hz, 1H), 9.14 (d, $J$ = 2.1 Hz, 1H), 8.81 (t, $J$ = 2.1 Hz, 1H), 8.29 (s, 1H), 7.96 (dd, $J$ = 9.0, 1.4 Hz, 1H), 7.57 (dd, $J$ = 8.9, 7.1 Hz, 1H), 7.38 (dd, $J$ = 7.0, 1.4 Hz, 1H), 4.41 (d, $J$ = 4.6 Hz, 2H), 3.63 (t, $J$ = 5.4 Hz, 4H), 1.65 (d, $J$ = 5.7 Hz, 2H), 1.57 (d, $J$ = 6.8 Hz, 4H). | MS (ESI, LR) Calc. for $C_{21}H_{21}N_6O_2$ $[M+H]^+$: 389.2, found: 389.1. |
| **87** | 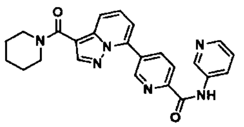 | $^1$H NMR (400 MHz, DMSO-$d_6$) δ(ppm): 10.74 (s, 1H), 9.29 (dd, $J$ = 22.2, 2.1 Hz, 2H), 8.99-8.87 (m, 2H), 8.37 (dd, $J$ = 4.7, 1.5 Hz, 1H), 8.30 (s, 1H), 8.22 (dt, $J$ = 8.5, 2.0 Hz, 1H), 7.97 (dd, $J$ = 8.9, 1.3 Hz, 1H), 7.59 (dd, $J$ = 8.9, 7.0 Hz, 1H), 7.44 (td, $J$ = 8.1, 3.0 Hz, 2H), 3.64 (t, $J$ = 5.4 Hz, 4H), 1.66 (d, $J$ = 7.1 Hz, 2H), 1.57 (d, $J$ = 7.4 Hz, 4H). | MS (ESI, LR) Calc. for $C_{24}H_{23}N_6O_2$ $[M+H]^+$: 427.2, found: 427.1. |
| **88** | 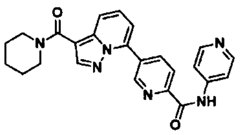 | $^1$H NMR (400 MHz, DMSO-$d_6$) δ(ppm): 10.86 (s, 1H), 9.32 (d, $J$ = 2.1 Hz, 1H), 9.24 (d, $J$ = 2.1 Hz, 1H), 8.88 (t, $J$ = 2.2 Hz, 1H), 8.58 - 8.48 (m, 2H), 8.30 (s, 1H), 7.97 (dd, $J$ = 8.9, 1.4 Hz, 1H), 7.84-7.74 (m, 2H), 7.58 (dd, $J$ = 8.9, 7.0 Hz, 1H), 7.42 (dd, $J$ = 7.1, 1.4 Hz, 1H), 3.63 (t, $J$ = 5.4 Hz, 4H), 1.65 (d, $J$ = 6.0 Hz, 2H), 1.57 (d, $J$ = 7.4 Hz, 4H). | MS (ESI, LR) Calc. for $C_{24}H_{23}N_6O_2$ $[M+H]^+$: 427.2, found: 427.1. |
| **89** | 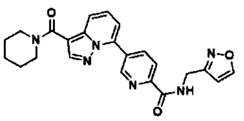 | $^1$H NMR (400 MHz, DMSO-$d_6$) δ(ppm): 9.42 (t, $J$ = 5.9 Hz, 1H), 9.26 (d, $J$ = 2.1 Hz, 1H), 9.16 (d, $J$ = 2.1 Hz, 1H), 8.87 (d, $J$ = 1.7 Hz, 1H), 8.80 (t, $J$ = 2.1 Hz, 1H), 8.29 (s, 1H), 7.95 (dd, $J$ = 8.9, 1.4 Hz, 1H), 7.56 (dd, $J$ = 8.9, 7.0 Hz, 1H), 7.37 (dd, $J$ = 7.1, 1.4 Hz, 1H), 6.58 (d, $J$ = 1.7 Hz, 1H), 4.62 (d, $J$ = 5.8 Hz, 2H), 3.63 (t, $J$ = 5.4 Hz, 4H), 1.65 (d, $J$ = 5.8 Hz, 2H), 1.56 (t, $J$ = 5.8 Hz, 4H). | MS (ESI, LR) Calc. for $C_{23}H_{23}N_6O_3$ $[M+H]^+$: 431.2, found: 431.1. |

(continued)

| Example | Structure | $^1$H NMR | MS |
|---|---|---|---|
| **213** | 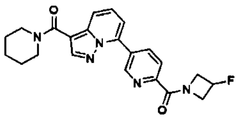 | $^1$H NMR (400 MHz, DMSO-$d_6$) δ(ppm): 9.18 (t, $J$ = 1.6 Hz, 1H), 8.91 (t, $J$ = 1.8 Hz, 1H), 8.58 (q, $J$ = 2.2 Hz, 1H), 8.28 (s, 1H), 7.94 (dd, $J$ = 9.0, 1.4 Hz, 1H), 7.55 (dd, $J$ = 8.9, 7.0 Hz, 1H), 7.35 (dd, $J$= 7.0, 1.4 Hz, 1H), 5.04 (d, $J$ = 6.0 Hz, 1H), 4.36 (d, $J$ = 17.6 Hz, 2H), 4.06 (d, $J$ = 21.1 Hz, 2H), 3.96 (dq, $J$ = 20.5, 6.8 Hz, 1H), 3.63 (t, $J$ = 5.4 Hz, 4H), 2.47 (td, $J$ = 6.7, 3.4 Hz, 2H), 1.99 (s, 2H), 1.66 (d, $J$ = 5.7 Hz, 2H), 1.57 (s, 4H). | MS (ESI, LR) Calc. for $C_{22}H_{23}FN_5O_2$ $[M+H]^+$: 408.2, found: 408.1. |
| **214** | 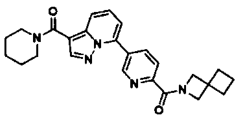 | $^1$H NMR (400 MHz, DMSO-$d_6$) δ(ppm): 9.14 (d, $J$ = 2.2 Hz, 1H), 8.54 (dd, $J$ = 8.2, 2.3 Hz, 1H), 8.27 (s, 1H), 8.11 (d, $J$ = 8.2 Hz, 1H), 7.95 (dd, $J$ = 8.9, 1.3 Hz, 1H), 7.55 (dd, $J$ = 8.9, 7.1 Hz, 1H), 7.35 (dd, $J$ = 7.0, 1.4 Hz, 1H), 4.62 (s, 2H), 4.09 (s, 2H), 3.63 (t, $J$ = 5.4 Hz, 4H), 2.19 (t, $J$ = 7.6 Hz, 4H), 1.86-1.75 (m, 2H), 1.66 (t, $J$ = 5.7 Hz, 2H), 1.57 (d, $J$ = 6.3 Hz, 4H). | MS (ESI, LR) Calc. for $C_{25}H_{28}N_5O_2$ $[M+H]^+$: 430.2, found: 430.2. |
| **237** | 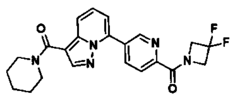 | $^1$H NMR (400 MHz, DMSO-$d_6$) δ(ppm): 9.19 (d, $J$ = 2.2 Hz, 1H), 8.59 (dd, $J$ = 8.2, 2.2 Hz, 1H), 8.28 (s, 1H), 8.20 (d, $J$ = 8.3 Hz, 1H), 7.96 (dd, $J$ = 8.9, 1.3 Hz, 1H), 7.66-7.62 (m, 1H), 7.37 (dd, $J$ = 7.1, 1.4 Hz, 1H), 5.09 (t, $J$ = 12.5 Hz, 2H), 4.57 (t, $J$ = 12.5 Hz, 2H), 3.63 (t, $J$ = 5.4 Hz, 4H), 1.65 (d, $J$ = 5.8 Hz, 2H), 1.57 (d, $J$ = 7.6 Hz, 4H). | MS (ESI, LR) Calc. for $C_{22}H_{22}F_2N_5O_2$ $[M+H]^+$: 426.2, found: 426.1. |

**Example 82. piperazin-1-yl(5-(3-(piperidine-1-carbonyl)pyrazolo[1,5-*a*]pyridin-7-yl)pyridin-2-yl)methanone**

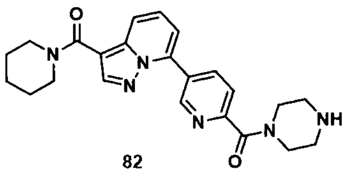

82

Using compound **74-1** and 1-Boc-piperazine, the target compound 82 was obtained by sequentially using the synthetic method of step 1-2 in Example 1 and the synthetic method of Example **34.**

$^1$H NMR (400 MHz, DMSO-$d_6$) δ(ppm): 9.13 (d, $J$ = 2.1 Hz, 1H), 9.02 (s, 2H), 8.56 (dd, $J$ = 8.1, 2.2 Hz, 1H), 8.27 (s, 1H), 7.95 (dd, $J$ = 8.9, 1.3 Hz, 1H), 7.87 (d, $J$ = 8.2 Hz, 1H), 7.57 (dd, $J$ = 8.9, 7.1 Hz, 1H), 7.35 (dd, $J$ = 7.1, 1.4 Hz, 1H), 3.91 (d, $J$ = 5.6 Hz, 2H), 3.78 (d, $J$ = 6.2 Hz, 2H), 3.63 (t, $J$ = 5.4 Hz, 4H), 3.21 (d, $J$ = 6.3 Hz, 2H), 3.18-3.08 (m, 2H), 1.66 (q, $J$ = 5.6 Hz, 2H), 1.58 (q, $J$ = 5.5 Hz, 4H).

MS (ESI, LR) Calc. for $C_{23}H_{27}N_6O_2$ $[M+H]^+$: 419.2, found: 419.1.

**Example 83. (4-methylpiperazin-1-yl)(5-(3-(piperidine-1-carbonyl)pyrazolo[1,5-*a*]pyridin-7-yl)pyridin-2-yl) methanone**

**Example 84. (4-isopropylpiperazin-1-yl)(5-(3-(piperidine-1-carbonyl)pyrazolo[1,5-*a*]pyridin-7-yl)pyridin-2-yl) methanone**

For Examples 83 and 84, the compounds were obtained by the same synthetic method as Example **49** using the corresponding compounds and compound **74-1.** The structural and spectral data are shown in Table 9 below.

[Table 9]

| Example | Structure | $^1$H NMR | MS |
|---|---|---|---|
| **83** | 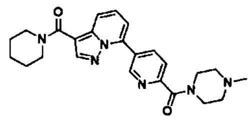 | $^1$H NMR (400 MHz, DMSO-$d_6$) δ(ppm): 9.10 (dd, $J$ = 2.3, 0.9 Hz, 1H), 8.52 (dd, $J$ = 8.2, 2.2 Hz, 1H), 8.27 (s, 1H), 7.94 (dd, $J$ = 8.9, 1.4 Hz, 1H), 7.76 (dd, $J$ = 8.2, 0.9 Hz, 1H), 7.55 (dd, $J$ = 8.9, 7.0 Hz, 1H), 7.35 (dd, $J$ = 7.0, 1.4 Hz, 1H), 3.69 (t, $J$ = 5.1 Hz, 2H), 3.63 (t, $J$ = 5.5 Hz, 4H), 3.47 (t, $J$ = 4.9 Hz, 2H), 2.42 (t, $J$ = 5.1 Hz, 2H), 2.33 (t, $J$ = 5.0 Hz, 2H), 1.66 (q, $J$ = 6.2 Hz, 2H), 1.57 (p, $J$ = 5.3 Hz, 4H). | MS (ESI, LR) Calc. for $C_{24}H_{29}N_6O_2$ [M+H]$^+$: 433.2, found: 433.1. |
| **84** | 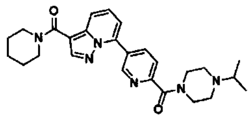 | $^1$H NMR (400 MHz, DMSO-$d_6$) δ(ppm): 9.10 (dd, $J$ = 2.2, 0.9 Hz, 1H), 8.52 (dd, $J$ = 8.2, 2.2 Hz, 1H), 8.27 (s, 1H), 7.94 (dd, $J$ = 8.9, 1.3 Hz, 1H), 7.76 (dd, $J$ = 8.2, 0.9 Hz, 1H), 7.55 (dd, $J$ = 8.9, 7.0 Hz, 1H), 7.35 (dd, $J$ = 7.1, 1.4 Hz, 1H), 3.67 (t, $J$ = 5.0 Hz, 2H), 3.63 (t, $J$ = 5.5 Hz, 4H), 3.45 (t, $J$ = 4.9 Hz, 2H), 2.71 (p, $J$ = 6.5 Hz, 1H), 2.54 (t, $J$ = 5.1 Hz, 2H), 2.45 (t, $J$ = 5.0 Hz, 2H), 1.65 (d, $J$ = 4.9 Hz, 2H), 1.57 (d, $J$ = 7.0 Hz, 4H), 0.99 (d, $J$ = 6.5 Hz, 6H). | MS (ESI, LR) Calc. for $C_{26}H_{33}N_6O_2$ [M+H]$^+$: 461.3, found: 461.2. |

**Example 243. 3-fluoro-5-[3-(piperidine-1-carbonyl)pyrazolo[1,5-*a*]pyridin-7-yl]pyridine-2-carboxylic acid**

<Scheme 8>

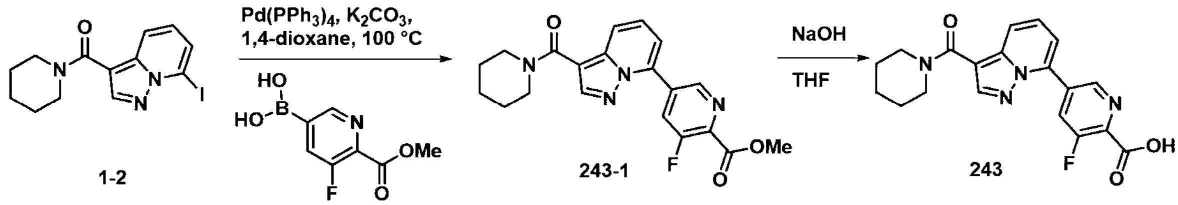

1-2 243-1 243

Step 243-1: Using compound **1-2** and (5-fluoro-6-(methoxycarbonyl)pyridin-3-yl)boronic acid, target compound **243-1** was obtained by the same synthetic method as step 1-3 in Example 1.

Step 243-2: Compound **243-1** (19 mg, 49 μM) was dissolved in tetrahydrofuran (1 mL), 1 N sodium hydroxide (0.3 mL) was added, and the mixture was stirred at 0 °C for 2 hours. After completion of the reaction, water (3 mL) was added to the reaction solution and washed with dichloromethane solution. The aqueous layer was acidified with 1 N hydrochloric acid to adjust the pH of the aqueous solution to 2, and then extracted with dichloromethane (5 mL x 3). The organic layer was dried over anhydrous magnesium sulfate, filtered, and concentrated to obtain the target compound **243** (12 mg, 66%).

$^1$H NMR (400 MHz, DMSO-$d_6$) δ(ppm): 13.82 (s, 1H), 9.07 (d, $J$ = 1.6 Hz, 1H), 8.57 (dd, $J$ = 11.5, 1.7 Hz, 1H), 8.31 (s, 1H), 7.98 (dd, $J$ = 8.9, 1.3 Hz, 1H), 7.57 (dd, $J$ = 8.9, 7.1 Hz, 1H), 7.46 (dd, $J$ = 7.1, 1.4 Hz, 1H), 3.63 (t, $J$ = 5.4 Hz, 4H), 1.65 (q, $J$ = 5.6 Hz, 2H), 1.57 (q, $J$ = 5.7 Hz, 4H).

MS (ESI, LR) Calc. for $C_{19}H_{18}FN_4O_3$ [M+H]$^+$: 369.1, found: 369.1.

**Example 248. [7-[6-(azetidine-1-carbonyl)-5-fluoro-3-pyridyl]pyrazolo[1,5-*a*]pyridin-3-yl]-(1-piperidyl)methanone**

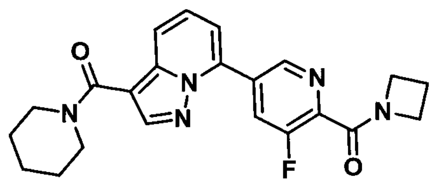

Using compound **243** and azetidine, the target compound **248** was obtained by the same synthetic method as step 1-2 in Example 1.

$^1$H NMR (400 MHz, DMSO-$d_6$) δ(ppm): 9.02 (t, *J* = 1.5 Hz, 1H), 8.53 (dd, *J* = 11.2, 1.7 Hz, 1H), 8.30 (s, 1H), 7.97 (dd, *J* = 8.9, 1.4 Hz, 1H), 7.56 (dd, *J* = 8.9, 7.1 Hz, 1H), 7.43 (dd, *J* = 7.0, 1.3 Hz, 1H), 4.28 (t, *J* = 7.7 Hz, 2H), 4.13 (t, *J* = 7.8 Hz, 2H), 3.63 (t, *J* = 5.4 Hz, 4H), 2.32 (p, *J* = 7.7 Hz, 2H), 1.66 (d, *J* = 5.2 Hz, 2H), 1.57 (d, *J* = 6.7 Hz, 4H).

MS (ESI, LR) Calc. for $C_{22}H_{23}FN_5O_2$ $[M+H]^+$: 408.2, found: 408.1.

**Example 249. [7-[5-fluoro-6-(morpholine-4-carbonyl)-3-pyridyl]pyrazolo[1,5-*a*]pyridin-3-yl]-(1-piperidyl)methanone**

**Example 250. 3-fluoro-*N*-methyl-5-[3-(piperidine-1-carbonyl)pyrazolo[1,5-*a*]pyridin-7-yl]pyridine-2-carboxamide**

For Examples 249 and 250, the target compounds were obtained using the same synthetic method as step 1-2 in Example **1** using the corresponding compounds and compound **243.** The structural and spectral data are shown in Table 10 below.

[Table 10]

| Example | Structure | $^1$H NMR | MS |
|---|---|---|---|
| **249** | 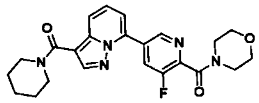 | $^1$H NMR (400 MHz, DMSO-$d_6$) δ(ppm): 9.03 (t, *J* = 1.6 Hz, 1H), 8.56 (dd, *J* = 10.5, 1.7 Hz, 1H), 8.30 (s, 1H), 7.97 (dd, *J* = 8.9, 1.3 Hz, 1H), 7.57 (dd, *J* = 8.9, 7.1 Hz, 1H), 7.42 (dd, *J* = 7.1, 1.4 Hz, 1H), 3.72 (s, 4H), 3.61 (dt, *J* = 13.6, 5.2 Hz, 6H), 3.35 (d, *J* = 4.9 Hz, 2H), 1.65 (d, *J* = 4.7 Hz, 2H), 1.57 (s, 4H). | MS (ESI, LR) Calc. for $C_{23}H_{25}FN_5O_3$ $[M+H]^+$: 438.2, found: 438.2. |
| 250 | 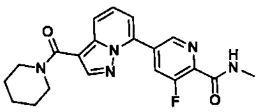 | $^1$H NMR (400 MHz, DMSO-$d_6$) δ(ppm): 9.03 (t, *J* = 1.5 Hz, 1H), 8.78 (d, *J* = 5.1 Hz, 1H), 8.53 (dd, *J* = 11.7, 1.7 Hz, 1H), 8.30 (s, 1H), 7.98 (dd, *J*= 8.9, 1.3 Hz, 1H), 7.57 (dd, *J* = 8.9, 7.1 Hz, 1H), 7.45 (dd, *J* = 7.1, 1.4 Hz, 1H), 3.63 (t, *J* = 5.4 Hz, 4H), 2.84 (d, *J* = 4.7 Hz, 3H), 1.66 (d, *J* = 5.7 Hz, 2H), 1.57 (s, 4H). | MS (ESI, LR) Calc. for $C_{20}H_{21}FN_5O_2$ $[M+H]^+$: 382.2, found: 382.1. |

**Example 277. [7-[2-(azetidine-1-carbonyl)pyrimidin-5-yl]pyrazolo[1,5-*a*]pyridin-3-yl]-(1-piperidyl)methanone**

<Scheme 9>

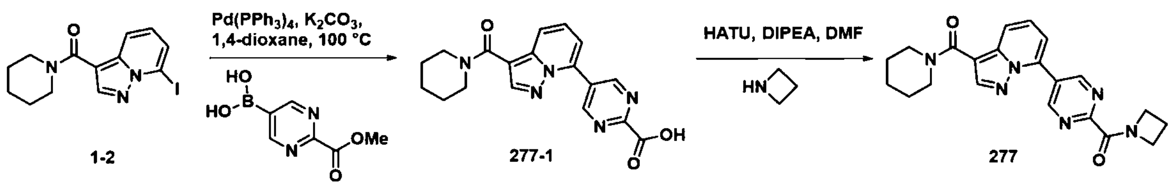


Compound **277-1** was obtained by the same synthetic method as step 1-3 in Example 1 using compound **1-2** and (2-(methoxycarbonyl)pyrimidin-5-yl)boronic acid, and then the target compound **277** was obtained by the same synthetic method as step 1-2 in Example 1 using azetidine.

$^1$H NMR (400 MHz, DMSO-$d_6$) δ(ppm): 9.51 (s, 2H), 8.31 (s, 1H), 7.98 (dd, *J* = 8.9, 1.4 Hz, 1H), 7.58 (dd, *J* = 8.9, 7.0 Hz, 1H), 7.48 (dd, *J* = 7.1, 1.4 Hz, 1H), 4.48 (t, *J* = 7.7 Hz, 2H), 4.14 *(t, J* = 7.7 Hz, 2H), 3.63 (t, *J* = 5.4 Hz, 4H), 2.39-2.27 (m, 2H), 1.66 (d, *J* = 5.4 Hz, 2H), 1.57 (d, *J* = 6.9 Hz, 4H).

MS (ESI, LR) Calc. for $C_{21}H_{23}N_6O_2$ $[M+H]^+$: 391.2, found: 391.1.

**Example 258. 3-chloro-5-[3-(piperidine-1-carbonyl)pyrazolo[1,5-*a*]pyridin-7-yl]pyridine-2-carboxylic acid**

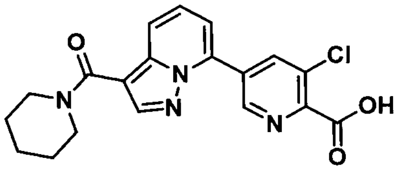

Using (5-chloro-6-(methoxycarbonyl)pyridin-3-yl)boronic acid, the intermediate was obtained by the same synthetic method as step 1-3 in Example 1, and then the target compound **258** was obtained by the same synthetic method as step 243-2 in Example 243.

$^1$H NMR (400 MHz, DMSO-$d_6$) δ(ppm): 14.07 (s, 1H), 9.08 (d, *J* = 1.8 Hz, 1H), 8.71 (d, *J* = 1.9 Hz, 1H), 8.30 (s, 1H), 7.97 (dd, *J* = 8.9, 1.4 Hz, 1H), 7.56 (dd, *J* = 8.9, 7.1 Hz, 1H), 7.43 (dd, *J* = 7.0, 1.4 Hz, 1H), 3.63 (t, *J* = 5.4 Hz, 4H), 1.73-1.62 (m, 2H), 1.57 (d, *J* = 7.4 Hz, 4H).

MS (ESI, LR) Calc. for $C_{19}H_{18}ClN_4O_3$ [M+H]$^+$: 385.1, found: 385.1.

**Example 262. 3-chloro-*N*-methyl-5-[3-(piperidine-1-carbonyl)pyrazolo[1,5-*a*]pyridin-7-yl]pyridine-2-carboxamide**

**Example 286. [7-[6-(azetidine-1-carbonyl)-5-chloro-3-pyridyl]pyrazolo[1,5-*a*]pyridin-3-yl]-(1-piperidyl)methanone**

For examples 262 and 286, the compounds were obtained by the same synthetic method as step 1-2 in Example 1 using the corresponding compounds and compound **258.** The structural and spectral data are shown in Table 11 below.

[Table 11]

| Example | Structure | $^1$H NMR | MS |
|---|---|---|---|
| **262** | 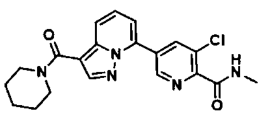 | $^1$H NMR (400 MHz, DMSO-$d_6$) δ(ppm): 9.06 (d, *J* = 1.8 Hz, 1H), 8.72 (d, *J* = 5.0 Hz, 1H), 8.67 (d, *J* = 1.8 Hz, 1H), 8.30 (s, 1H), 7.97 (dd, *J* = 8.9, 1.3 Hz, 1H), 7.56 (dd, *J* = 8.9, 7.0 Hz, 1H), 7.42 (dd, *J* = 7.1, 1.4 Hz, 1H), 3.63 (t, *J* = 5.4 Hz, 4H), 2.83 (d, *J* = 4.7 Hz, 3H), 1.65 (d, *J* = 5.3 Hz, 2H), 1.57 (d, *J* = 5.1 Hz, 4H). | MS (ESI, LR) Calc. for $C_{20}H_{21}ClN_3O_2$ [M+H]$^+$: 398.1, found: 398.1. |
| **286** | 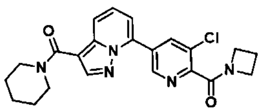 | $^1$H NMR (400 MHz, DMSO-$d_6$) δ(ppm): 9.08 (d, *J* = 1.8 Hz, 1H), 8.71 (d, *J* = 1.8 Hz, 1H), 8.31 (s, 1H), 7.97 (dd, *J* = 8.9, 1.4 Hz, 1H), 7.56 (dd, *J* = 8.9, 7.0 Hz, 1H), 7.46-7.40 (m, 1H), 4.12 (dt, *J* = 22.1, 7.8 Hz, 4H), 3.63 (t, *J* = 5.4 Hz, 4H), 2.34 (p, *J* = 7.7 Hz, 2H), 1.66 (d, *J* = 5.8 Hz, 2H), 1.58 (d, *J* = 7.8 Hz, 4H). | MS (ESI, LR) Calc. for $C_{22}H_{23}ClN_3O_2$ [M+H]$^+$: 424.1, found: 424.1. |

MS (ESI, LR) Calc. for $C_{22}H_{23}ClN_5O_2$ [M+H]$^+$: 424.1, found: 424.1.

**Example 90. *N*-(2-cyanoethyl)-5-(3-(piperidine-1-carbonyl)pyrazolo[1,5-*a*]pyridin-7-yl)furan-2-carboxamide**

<Scheme 10>

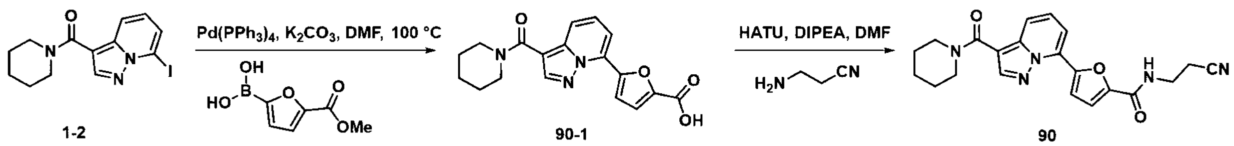


Using compound **1-2** and methyl (5-methoxycarbonyl-2-furyl)boronic acid, compound **90-1** was obtained by the synthetic method used in Example 39, and then the target compound **90** was obtained by the same synthetic method as step 1-2 in Example 1 using 3-aminopropanenitrile.

$^1$H NMR (400 MHz, DMSO-$d_6$) δ(ppm): 9.11 (t, *J* = 5.9 Hz, 1H), 8.42 (s, 1H), 8.05-7.91 (m, 3H), 7.64 (dd, *J* = 8.8, 7.3 Hz, 1H), 7.39 (d, *J* = 3.6 Hz, 1H), 3.64 (t, *J* = 5.4 Hz, 4H), 3.56 (q, *J* = 6.3 Hz, 2H), 2.83 (t, *J* = 6.5 Hz, 2H), 1.66 (d, *J* = 4.9 Hz, 2H), 1.58 (d, *J* = 6.0 Hz, 4H).

MS (ESI, LR) Calc. for $C_{21}H_{22}N_5O_3$ $[M+H]^+$: 392.2, found: 392.1.

**Example 230. (3-hydroxyiminoazetidin-1-yl)-[5-[3-(piperidine-1-carbonyl)pyrazolo[1,5-*a*]pyridin-7-yl]-3-pyridyl] methanone**

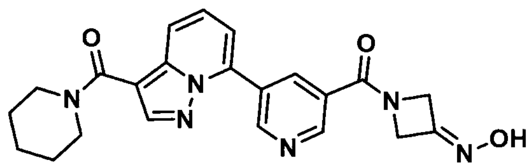

After obtaining the intermediate by the same synthetic method as step 1-2 in Example 1 using compound **39-1** and azetidin-3-one, the target compound **230** was obtained by the same synthetic method as step 236-2 in Example 236.

$^1$H NMR (400 MHz, DMSO-$d_6$) δ(ppm): 11.07 (d, *J* = 12.6 Hz, 1H), 9.22 (d, *J* = 2.2 Hz, 1H), 9.00 (d, *J* = 2.1 Hz, 1H), 8.68 (s, 1H), 8.27 (d, *J* = 6.5 Hz, 1H), 7.95 (d, *J* = 8.9 Hz, 1H), 7.56 (dd, *J* = 8.9, 7.0 Hz, 1H), 7.37 (d, *J* = 7.0 Hz, 1H), 5.13 (s, 2H), 4.79 (d, *J* = 17.8 Hz, 2H), 3.63 (s, 4H), 1.65 (d, *J* = 6.1 Hz, 2H), 1.57 (s, 4H).

MS (ESI, LR) Calc. for $C_{22}H_{23}N_6O_3$ $[M+H]^+$: 419.2, found: 419.1.

**Example 91. 3-methoxy-*N*-(3-(3-(piperidine-1-carbonyl)pyrazolo[1,5-*a*]pyridin-7-yl)phenyl)propanamide**

**Example 93. 1-methyl-*N*-(3-(3-(piperidine-1-carbonyl)pyrazolo[1,5-*a*]pyridin-7-yl)phenyl)piperidine-4-carboxamide**

**Example 94. *N*-(3-(3-(piperidine-1-carbonyl)pyrazolo[1,5-*a*]pyridin-7-yl)phenyl)cyclopropanecarboxamide**

**Example 95. *N*-(3-(3-(piperidine-1-carbonyl)pyrazolo[1,5-*a*]pyridin-7-yl)phenyl)nicotinamide**

**Example 96. *N*-(3-(3-(piperidine-1-carbonyl)pyrazolo[1,5-*a*]pyridin-7-yl)phenyl)cyclohexanecarboxamide**

For Examples 91, 93, 94, 95, and 96, the compounds were obtained by the same synthetic method as step 1-2 in Example 1 using the corresponding compounds and compound 7. The structural and spectral data are shown in Table 12 below.

[Table 12]

| Example | Structure | $^1$H NMR | MS |
|---|---|---|---|
| **91** | 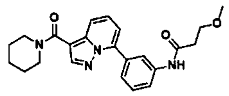 | $^1$H NMR (400 MHz, DMSO-$d_6$) δ(ppm): 10.14 (s, 1H), 8.23 (s, 1H), 8.18 (t, *J* = 2.0 Hz, 1H), 7.88 (dd, *J* = 8.9, 1.4 Hz, 1H), 7.76 (d, *J* = 8.0 Hz, 1H), 7.57-7.45 (m, 3H), 7.12 (dd, *J* = 7.0, 1.4 Hz, 1H), 3.63 (dq, *J* = 5.6, 3.0 Hz, 6H), 3.25 (s, 3H), 2.58 (t, *J* = 6.1 Hz, 2H), 1.65 (d, *J* = 5.6 Hz, 2H), 1.57 (d, *J* = 7.8 Hz, 4H). | MS (ESI, LR) Calc. for $C_{23}H_{27}N_4O_3$ $[M+H]^+$: 407.2, found: 407.2. |
| **93** | 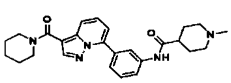 | $^1$H NMR (400 MHz, DMSO-$d_6$) δ(ppm): 10.24 (s, 1H), 8.23 (s, 1H), 8.18 (s, 1H), 7.88 (d, *J* = 8.7 Hz, 1H), 7.75 (d, *J* = 8.0 Hz, 1H), 7.52 (dq, *J* = 15.8, 7.9 Hz, 3H), 7.11 (d, *J* = 7.0 Hz, 1H), 3.62 (s, 4H), 2.98 (s, 2H), 2.78 (s, 2H), 2.03 (s, 3H), 1.66 (s, 2H), 1.57 (s, 4H), 1.25 (s, 2H). | MS (ESI, LR) Calc. for $C_{26}H_{32}N_5O_2$ $[M+H]^+$: 446.3, found: 446.2. |

(continued)

| Example | Structure | $^1$H NMR | MS |
|---|---|---|---|
| **94** | 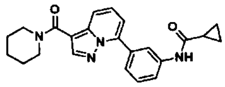 | $^1$H NMR (400 MHz, DMSO-$d_6$) δ(ppm): 10.39 (s, 1H), 8.23 (s, 1H), 8.16 (d, $J$ = 2.0 Hz, 1H), 7.88 (dd, $J$ = 8.9, 1.4 Hz, 1H), 7.74 (d, $J$ = 8.3 Hz, 1H), 7.57-7.44 (m, 3H), 7.12 (dd, $J$ = 7.0, 1.4 Hz, 1H), 3.62 (t, $J$ = 5.4 Hz, 4H), 1.85-1.78 (m, 1H), 1.65 (d, $J$ = 7.7 Hz, 2H), 1.57 (s, 4H), 0.82 (d, $J$ = 5.1 Hz, 4H). | MS (ESI, LR) Calc. for $C_{23}H_{25}N_4O_2$ $[M+H]^+$: 389.2, found: 389.2. |
| **95** | 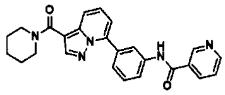 | $^1$H NMR (400 MHz, DMSO-$d_6$) δ(ppm): 10.64 (s, 1H), 9.14 (d, $J$ = 2.3 Hz, 1H), 8.78 (dd, $J$ = 4.8, 1.7 Hz, 1H), 8.39-8.28 (m, 2H), 8.25 (s, 1H), 7.92 (ddd, $J$ = 17.9, 8.5, 1.7 Hz, 2H), 7.66 (d, $J$ = 7.8 Hz, 1H), 7.63-7.48 (m, 3H), 7.17 (dd, $J$ = 7.1, 1.4 Hz, 1H), 3.63 (t, $J$ = 5.4 Hz, 4H), 1.65 (d, $J$= 5.6 Hz, 2H), 1.57 (d, $J$ = 4.8 Hz, 4H). | MS (ESI, LR) Calc. for $C_{25}H_{24}N_5O_2$ $[M+H]^+$: 426.2, found: 426.1. |
| **96** | 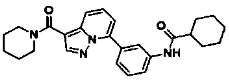 | $^1$H NMR (400 MHz, DMSO-$d_6$) δ(ppm): 9.99 (s, 1H), 8.23 (s, 1H), 8.18 (d, $J$ = 2.0 Hz, 1H), 7.88 (dd, $J$ = 9.0, 1.4 Hz, 1H), 7.75 (d, $J$ = 8.1 Hz, 1H), 7.56-7.42 (m, 3H), 7.11 (dd, $J$ = 7.0, 1.4 Hz, 1H), 3.62 (t, $J$ = 5.4 Hz, 4H), 1.79 (dd,$J$ = 22.7, 12.6 Hz, 4H), 1.65 (d, $J$ = 7.6 Hz, 2H), 1.57 (s, 4H), 1.42 (q, $J$ = 12.0 Hz, 2H), 1.25 (dq, $J$ = 24.3, 12.0 Hz, 4H). | MS (ESI, LR) Calc. for $C_{26}H_{3\ 1}N_4O_2$ $[M+H]^+$: 431.2, found: 431.2. |

**Example 92. 1-(3-(3-(piperidine-1-carbonyl)pyrazolo[1,5-*a*]pyridin-7-yl)phenyl)urea**

<Scheme 11>

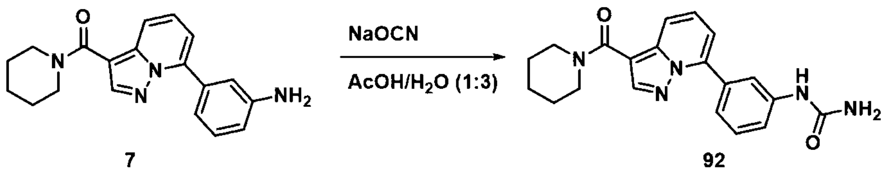


7 92

Sodium cyanate (28.4 mg, 0.44 mmol) was dissolved in warm water (3 mL). Compound **7** (70 mg, 0.22 mmol) dissolved in acetic acid (0.5 mL) and water (1.5 mL) was added to the reaction solution and stirred at 50 °C for 12 hours. After completion of the reaction, the temperature was lowered using ice water and the precipitated solid was filtered to obtain. The target compound **92** (35.6 mg, 45%) was obtained by recrystallization using boiling water.

$^1$H NMR (400 MHz, DMSO-$d_6$) δ(ppm): 8.74 (s, 1H), 8.22 (s, 1H), 7.95 (d, $J$ = 2.1 Hz, 1H), 7.86 (dd, $J$ = 8.9, 1.3 Hz, 1H), 7.60-7.54 (m, 1H), 7.50 (dd, $J$ = 8.9, 7.0 Hz, 1H), 7.42-7.37 (m, 2H), 7.11 (dd, $J$ = 7.0, 1.4 Hz, 1H), 5.91 (s, 2H), 3.62 (t, $J$ = 5.3 Hz, 4H), 1.65 (d, $J$ = 7.7 Hz, 2H), 1.57 (s, 4H).

MS (ESI, LR) Calc. for $C_{20}H_{22}N_5O_2$ $[M+H]^+$: 364.2, found: 364.2.

**Example 97. *N*-(4-(3-(piperidine-1-carbonyl)pyrazolo[1,5-*a*]pyridin-7-yl)phenyl)acetamide**

**Example 98. 3-methoxy-*N*-(4-(3-(piperidine-1-carbonyl)pyrazolo[1,5-*a*]pyridin-7-yl)phenyl)propanamide**

**Example 99. 1-methyl-*N*-(4-(3-(piperidine-1-carbonyl)pyrazolo[1,5-*a*]pyridin-7-yl)phenyl)piperidine-4-carboxamide**

**Example 100. *N*-(4-(3-(piperidine-1-carbonyl)pyrazolo[1,5-*a*]pyridin-7-yl)phenyl)cyclopropanecarboxamide**

**Example 101. *N*-(4-(3-(piperidine-1-carbonyl)pyrazolo[1,5-*a*]pyridin-7-yl)phenyl)nicotinamide**

**Example 102. *N*-(4-(3-(piperidine-1-carbonyl)pyrazolo[1,5-*a*]pyridin-7-yl)phenyl)cyclohexanecarboxamide**

**Example 274. *N*-(4-(3-(piperidine-1-carbonyl)pyrazolo[1,5-*a*]pyridin-7-yl)phenyl)-2-(pyridin-3-yl)acetamide**

For Examples 97, 98, 99, 100, 101, 102, and 274, the compounds were obtained by the same synthetic method as step 1-2 in Example 1 using the corresponding compounds and compound **14.** The structural and spectral data are shown in Table 13 below.

[Table 13]

| Example | Structure | $^1$H NMR | MS |
|---|---|---|---|
| **97** | 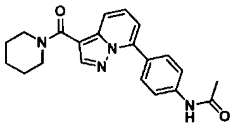 | $^1$H NMR (400 MHz, DMSO-$d_6$) δ(ppm): 10.19 (s, 1H), 8.23 (s, 1H), 7.94-7.87 (m, 2H), 7.84 (dd, *J* = 8.9, 1.4 Hz, 1H), 7.75 (d, *J* = 8.6 Hz, 2H), 7.49 (dd, *J* = 8.9, 7.1 Hz, 1H), 7.14 (dd, *J* = 7.1, 1.4 Hz, 1H), 3.62 (t, *J* = 5.4 Hz, 4H), 2.10 (s, 3H), 1.65 (d, *J* = 6.0 Hz, 2H), 1.57 (s, 4H). | MS (ESI, LR) Calc. for $C_{21}H_{23}N_4O_2$ $[M+H]^+$: 363.2, found: 363.2. |
| **98** | 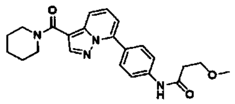 | $^1$H NMR (400 MHz, DMSO-$d_6$) δ(ppm): 10.20 (s, 1H), 8.23 (s, 1H), 7.93-7.88 (m, 2H), 7.84 (dd, *J* = 8.9, 1.4 Hz, 1H), 7.77 (d, *J* = 8.7 Hz, 2H), 7.49 (dd, *J* = 8.9, 7.1 Hz, 1H), 7.14 (dd, *J* = 7.1, 1.3 Hz, 1H), 3.63 (dt, *J* = 11.6, 5.8 Hz, 6H), 3.26 (s, 3H), 2.60 (t, *J* = 6.2 Hz, 2H), 1.65 (d, *J* = 5.9 Hz, 2H), 1.56 (s, 4H). | MS (ESI, LR) Calc. for $C_{23}H_{27}N_4O_3$ $[M+H]^+$: 407.2, found: 407.2. |
| **99** | 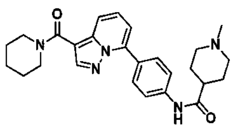 | $^1$H NMR (400 MHz, DMSO-$d_6$) δ(ppm): 10.46 (s, 1H), 8.23 (s, 1H), 7.92 (d, *J* = 8.5 Hz, 2H), 7.82 (dd, *J* = 15.7, 8.7 Hz, 3H), 7.49 (dd, *J* = 8.9, 7.1 Hz, 1H), 7.14 (d, *J* = 7.0 Hz, 1H), 3.62 (t, *J* = 5.5 Hz, 4H), 2.73 (s, 3H), 2.09 - 1.91 (m, 4H), 1.64 (t, *J* = 5.5 Hz, 2H), 1.56 (q, *J* = 5.4 Hz, 4H), 1.31-1.16 (m, 3H). | MS (ESI, LR) Calc. for $C_{26}H_{32}N_5O_2$ $[M+H]^+$: 446.3, found: 446.4. |
| **100** | 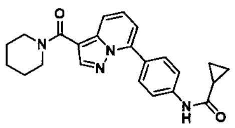 | $^1$H NMR (400 MHz, DMSO-$d_6$) δ(ppm): 10.45 (s, 1H), 8.23 (s, 1H), 7.91 (d, *J* = 8.6 Hz, 2H), 7.84 (dd, *J* = 8.9, 1.3 Hz, 1H), 7.76 (d, *J*= 8.7 Hz, 2H), 7.49 (dd, *J* = 8.9, 7.1 Hz, 1H), 7.15 (dd, *J* = 7.0, 1.4 Hz, 1H), 3.62 (t, *J* = 5.4 Hz, 4H), 1.87-1.78 (m, 1H), 1.65 (d, *J* = 6.9 Hz, 2H), 1.57 (d, *J* = 7.6 Hz, 4H), 0.87-0.83 (m, 4H). | MS (ESI, LR) Calc. for $C_{23}H_{25}N_4O_2$ $[M+H]^+$: 389.2, found: 389.2. |
| **101** | 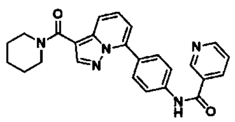 | $^1$H NMR (400 MHz, DMSO-$d_6$) δ(ppm): 10.69 (s, 1H), 9.16 (d, *J* = 2.2 Hz, 1H), 8.80 (dd, *J* = 4.8, 1.7 Hz, 1H), 8.37-8.32 (m, 1H), 8.25 (s, 1H), 8.06-7.92 (m, 4H), 7.89 - 7.84 (m, 1H), 7.61 (dd, *J* = 8.0, 4.8 Hz, 1H), 7.52 (dd, *J* = 8.9, 7.1 Hz, 1H), 7.20 (d, *J* = 7.0 Hz, 1H), 3.64 (d, *J* = 5.9 Hz, 4H), 1.66 (s, 2H), 1.57 (s, 4H). | MS (ESI, LR) Calc. for $C_{25}H_{24}N_5O_2$ $[M+H]^+$: 426.2, found: 426.1. |
| **102** | 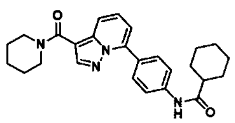 | $^1$H NMR (400 MHz, DMSO-$d_6$) δ(ppm): 10.05 (s, 1H), 8.23 (s, 1H), 7.90 (d, *J* = 8.7 Hz, 2H), 7.84 (dd, *J* = 9.0, 1.3 Hz, 1H), 7.78 (d, *J* = 8.7 Hz, 2H), 7.49 (dd, *J* = 8.9, 7.1 Hz, 1H), 7.14 (dd, *J* = 7.1, 1.4 Hz, 1H), 3.62 (t, *J* = 5.4 Hz, 4H), 1.81 (dd, *J* = 23.4, 12.8 Hz, 4H), 1.66 (s, 2H), 1.57 (s, 4H), 1.44 (q, *J* = 11.9 Hz, 2H), 1.27 (dq, *J* = 23.5, 12.0 Hz, 4H). | MS (ESI, LR) Calc. for $C_{26}H_{31}N_4O_2$ $[M+H]^+$: 431.2, found: 431.2. |
| **274** | 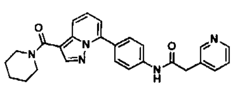 | $^1$H NMR (400 MHz, DMSO-$d_6$) δ(ppm): 10.49 (s, 1H), 8.56 (d, *J*= 2.4 Hz, 1H), 8.52-8.43 (m, 1H), 8.23 (s, 1H), 7.93 (d, *J* = 8.5 Hz, 2H), 7.85 (d, *J* = 8.9 Hz, 1H), 7.80-7.72 (m, 3H), 7.49 (dd, *J* = 8.9, 7.0 Hz, 1H), 7.39 (dd, *J* = 7.9, 4.8 Hz, 1H), 7.15 (d, *J* = 7.0 Hz, 1H), 3.78 (s, 2H), 3.62 (t, *J* = 5.3 Hz, 4H), 1.65 (d, *J* = 5.6 Hz, 2H), 1.57 (s, 4H). | MS (ESI, LR) Calc. for $C_{26}H_{26}N_5O_2$ $[M+H]^+$: 440.2, found: 440.1. |

**Example 196. 2-(dimethylamino)-*N*-(5-(3-(piperidine-1-carbonyl)pyrazolo[1,5-*a*]pyridin-7-yl)pyridin-3-yl)acetamide**

**Example 197. 3-methoxy-*N*-(5-(3-(piperidine-1-carbonyl)pyrazolo[1,5-*a*]pyridin-7-yl)pyridin-3-yl)propenamide**

**Example 198. 2-oxo-*N*-(5-(3-(piperidine-1-carbonyl)pyrazolo[1,5-*a*]pyridin-7-yl)pyridin-3-yl)piperidine-4-carboxamide**

**Example 326. *N*-(5-(3-(piperidine-1-carbonyl)pyrazolo[1,5-*a*]pyridin-7-yl)pyridin-3-yl)nicotinamide**

**Example 327. *N*-(5-(3-(piperidine-1-carbonyl)pyrazolo[1,5-*a*]pyridin-7-yl)pyridin-3-yl)-2-(pyridin-3-yl)acetamide**

**Example 333. 1,2-dimethyl-*N*-(5-(3-(piperidine-1-carbonyl)pyrazolo[1,5-*a*]pyridin-7-yl)pyridin-3-yl)-1*H*-imidazole-5-carboxamide**

**Example 334. *N*-(5-(3-(piperidine-1-carbonyl)pyrazolo[1,5-*a*]pyridin-7-yl)pyridin-3-yl)pyrimidine-5-carboxamide**

**Example 335. 5-fluoro-*N*-(5-(3-(piperidine-1-carbonyl)pyrazolo[1,5-*a*]pyridin-7-yl)pyridin-3-yl)nicotinamide**

**Example 388. *N*-(5-(3-(piperidine-1-carbonyl)pyrazolo[1,5-*a*]pyridin-7-yl)pyridin-3-yl)-2-(6-(trifluoromethyl)pyridin-3-yl)acetamide**

**Example 389. 2-phenyl-*N*-(5-(3-(piperidine-1-carbonyl)pyrazolo[1,5-*a*]pyridin-7-yl)pyridin-3-yl)acetamide**

**Example 402. 2-(5-fluoropyridin-3-yl)-*N*-(5-(3-(piperidine-1-carbonyl)pyrazolo[1,5-*a*]pyridin-7-yl)pyridin-3-yl)acetamide**

**Example 403. *N*-(5-(3-(piperidine-1-carbonyl)pyrazolo[1,5-*a*]pyridin-7-yl)pyridin-3-yl)-2-(pyrimidin-5-yl)acetamide**

**Example 406. 2-(4-chlorophenoxy)-*N*-(5-(3-(piperidine-1-carbonyl)pyrazolo[1,5-*a*]pyridin-7-yl)pyridin-3-yl)acetamide**

For Examples 196, 197, 198, 326, 327, 333, 334, 335, 388, 389, 402, 403, and 406, the compounds were obtained by the same synthetic method as step 1-2 in Example **1** using the corresponding compounds and compound **9.** The structural and spectral data are shown in Table 14 below.

[Table 14]

| Example | Structure | $^1$H NMR | MS |
|---|---|---|---|
| **196** | 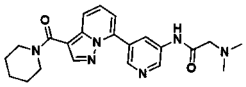 | $^1$H NMR (400 MHz, DMSO-$d_6$) δ(ppm): 10.18 (s, 1H), 8.99 (d, *J* = 2.4 Hz, 1H), 8.75 (d, *J* = 2.0 Hz, 1H), 8.70 (t, *J* = 2.2 Hz, 1H), 8.27 (s, 1H), 7.93 (dd, *J* = 8.9, 1.4 Hz, 1H), 7.54 (dd, *J* = 8.9, 7.0 Hz, 1H), 7.26 (dd, *J* = 7.1, 1.4 Hz, 1H), 3.63 (t, *J* = 5.4 Hz, 4H), 3.15 (s, 2H), 2.31 (s, 6H), 1.65 (d, *J* = 5.4 Hz, 2H), 1.57 (d, *J* = 7.2 Hz, 4H). | MS (ESI, LR) Calc. for $C_{22}H_{27}N_6O_2$ $[M+H]^+$: 407.2, found: 407.1. |
| **197** | 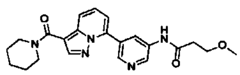 | $^1$H NMR (400 MHz, DMSO-$d_6$) δ(ppm): 10.40 (s, 1H), 8.89 (d, *J* = 2.4 Hz, 1H), 8.72 (d, *J* = 2.0 Hz, 1H), 8.67 (t, *J* = 2.2 Hz, 1H), 8.27 (s, 1H), 7.92 (dd, *J* = 8.9, 1.3 Hz, 1H), 7.54 (dd, *J* = 8.9, 7.0 Hz, 1H), 7.26 (dd, *J* = 7.1, 1.4 Hz, 1H), 3.63 (q, *J* = 6.1 Hz, 6H), 3.26 (s, 3H), 2.63 (t, *J* = 6.1 Hz, 2H), 1.65 (d, *J* = 6.5 Hz, 2H), 1.57 (s, 4H). | MS (ESI, LR) Calc. for $C_{22}H_{26}N_5O_3$ $[M+H]^+$: 408.2, found: 408.1. |

(continued)

| Example | Structure | $^1H$ NMR | MS |
|---|---|---|---|
| **198** | 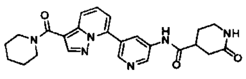 | $^1H$ NMR (400 MHz, DMSO-$d_6$) δ(ppm): 10.45 (s, 1H), 8.90 (d, *J* = 2.4 Hz, 1H), 8.73 (d, *J* = 2.0 Hz, 1H), 8.68 (t, *J* = 2.2 Hz, 1H), 8.27 (s, 1H), 7.93 (dd, *J* = 8.9, 1.4 Hz, 1H), 7.58-7.51 (m, 2H), 7.26 (dd, *J* = 7.1, 1.4 Hz, 1H), 3.63 (t, *J* = 5.4 Hz, 4H), 3.26-3.13 (m, 2H), 2.98-2.89 (m, 1H), 2.36 (d, *J* = 7.7 Hz, 2H), 1.65 (d, *J* = 5.8 Hz, 2H), 1.57 (s, 4H), 1.25 (s, 2H). | MS (ESI, LR) Calc. for $C_{24}H_{27}N_6O_3$ $[M+H]^+$: 447.2, found: 447.2. |
| **326** | 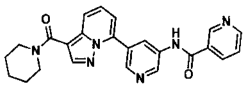 | $^1H$ NMR (400 MHz, DMSO-$d_6$) δ(ppm): 10.86 (s, 1H), 9.17 (d, *J* = 2.2 Hz, 1H), 9.08 (d, *J* = 1.5 Hz, 1H), 8.86-8.77 (m, 3H), 8.36 (dt, *J* = 8.0, 2.0 Hz, 1H), 8.27 (s, 1H), 7.94 (dd, *J* = 9.0, 1.3 Hz, 1H), 7.61 (dd, *J* = 8.0, 4.8 Hz, 1H), 7.56 (dd, *J* = 8.9, 7.0 Hz, 1H), 7.30 (dd, *J* = 7.1, 1.3 Hz, 1H), 3.63 (t, *J* = 5.4 Hz, 4H), 1.66 (q, *J* = 5.8 Hz, 2H), 1.61-1.47 (m, 4H). | MS (ESI, LR) Calc. for $C_{24}H_{23}N_6O_3$ $[M+H]^+$: 427.2, found: 427.1. |
| **327** | 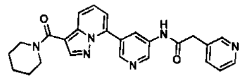 | $^1H$ NMR (400 MHz, DMSO-$d_6$) δ(ppm): 10.67 (s, 1H), 8.90 (d, *J* = 2.4 Hz, 1H), 8.74 (d, *J* = 2.0 Hz, 1H), 8.67 (t, *J* = 2.2 Hz, 1H), 8.55 (d, *J* = 2.3 Hz, 1H), 8.48 (dd, *J* = 4.8, 1.7 Hz, 1H), 8.25 (s, 1H), 7.92 (dd, *J* = 8.9, 1.4 Hz, 1H), 7.77 (dt, *J* = 8.0, 2.0 Hz, 1H), 7.53 (dd, *J* = 8.9, 7.1 Hz, 1H), 7.37 (dd, *J* = 7.8, 4.8 Hz, 1H), 7.26 (dd, *J* = 7.0, 1.4 Hz, 1H), 3.80 (s, 2H), 3.62 (t, *J* = 5.5 Hz, 4H), 1.65 (m, 2H), 1.57 (m, 4H). | MS (ESI, LR) Calc. for $C_{25}H_{25}N_6O_2$ $[M+H]^+$: 441.2, found: 441.1. |
| **333** | 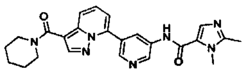 | $^1H$ NMR (400 MHz, DMSO-$d_6$) δ(ppm): 10.32 (s, 1H), 9.01 (d, *J* = 2.4 Hz, 1H), 8.77 (d, *J* = 2.0 Hz, 1H), 8.72 (t, *J* = 2.2 Hz, 1H), 8.27 (s, 1H), 7.94 (dd, *J* = 8.9, 1.4 Hz, 1H), 7.78 (s, 1H), 7.55 (dd, *J* = 8.9, 7.0 Hz, 1H), 7.28 (dd, *J* = 7.1, 1.4 Hz, 1H), 3.80 (s, 3H), 3.63 (t, *J* = 5.4 Hz, 4H), 2.37 (s, 3H), 1.66 (m, 2H), 1.58 (m, 4H). | MS (ESI, LR) Calc. for $C_{24}H_{26}N_7O_2$ $[M+H]^+$: 444.2, found: 444.1. |
| **334** | 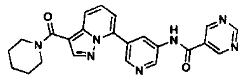 | $^1H$ NMR (400 MHz, DMSO-$d_6$) δ(ppm): 11.01 (s, 1H), 9.40 (s, 1H), 9.33 (s, 2H), 9.07 (d, *J* = 2.3 Hz, 1H), 8.84 (dd, *J* = 7.5, 2.1 Hz, 2H), 8.28 (s, 1H), 7.95 (dd, *J* = 8.9, 1.4 Hz, 1H), 7.56 (dd, *J* = 8.9, 7.0 Hz, 1H), 7.31 (dd, *J* = 7.0, 1.4 Hz, 1H), 3.63 (t, *J* = 5.4 Hz, 4H), 1.65 (m, 2H), 1.58 (m, 4H). | MS (ESI, LR) Calc. for $C_{23}H_{22}N_7O_2$ $[M+H]^+$: 428.2, found: 428.1. |
| **335** | 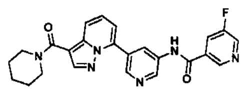 | $^1H$ NMR (400 MHz, DMSO-$d_6$) δ(ppm): 10.90 (s, 1H), 9.12-8.98 (m, 2H), 8.83 (dt, *J* = 9.0, 2.0 Hz, 3H), 8.34-8.20 (m, 2H), 7.94 (dd, *J* = 8.9, 1.3 Hz, 1H), 7.55 (dd, *J* = 8.9, 7.1 Hz, 1H), 7.30 (dd, *J* = 7.0, 1.4 Hz, 1H), 3.63 (t, *J* = 5.4 Hz, 4H), 1.71-1.59 (m, 2H), 1.60-1.46 (m, 4H). | MS (ESI, LR) Calc. for $C_{24}H_{22}FN_6O_2$ $[M+H]^+$: 445.2, found: 445.1. |
| **388** | 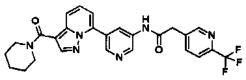 | $^1H$ NMR (400 MHz, DMSO-$d_6$) δ(ppm): 10.75 (s, 1H), 8.90 (d, *J* = 2.4 Hz, 1H), 8.75 (t, *J* = 2.4 Hz, 2H), 8.67 (t, *J* = 2.3 Hz, 1H), 8.26 (s, 1H), 8.12-7.99 (m, 1H), 7.96-7.84 (m, 2H), 7.53 (dd, *J* = 8.9, 7.0 Hz, 1H), 7.27 (dd, *J* = 7.1, 1.4 Hz, 1H), 3.97 (s, 2H), 3.62 (t, *J* = 5.5 Hz, 4H), 1.65 (m, 2H), 1.57 (m, 2H), 1.25 (m, 2H). | MS (ESI, LR) Calc. for $C_{26}H_{24}F_3N_6O_2$ $[M+H]^+$: 509.2, found: 509.1. |

(continued)

| Example | Structure | $^1$H NMR | MS |
|---|---|---|---|
| **389** | 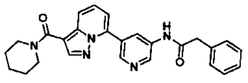 | $^1$H NMR (400 MHz, DMSO-$d_6$) δ(ppm): 10.62 (s, 1H), 8.90 (d, *J* = 2.4 Hz, 1H), 8.72 (d, *J* = 2.0 Hz, 1H), 8.67 (t, *J* = 2.2 Hz, 1H), 8.26 (s, 1H), 7.92 (dd, *J* = 8.9, 1.3 Hz, 1H), 7.52 (dd, *J* = 8.9, 7.0 Hz, 1H), 7.42-7.16 (m, 5H), 3.73 (s, 2H), 3.62 (t, *J* = 5.4 Hz, 3H), 1.65 (m, 2H), 1.56 (m, 2H), 1.25 (m, 2H). | MS (ESI, LR) Calc. for $C_{26}H_{26}N_5O_2$ $[M+H]^+$: 440.2, found: 440.1. |
| **402** | 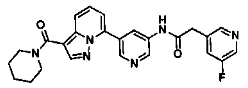 | $^1$H NMR (400 MHz, DMSO-$d_6$) δ(ppm): 10.71 (s, 1H), 8.90 (d, *J* = 2.4 Hz, 1H), 8.75 (d, *J* = 2.0 Hz, 1H), 8.67 (t, *J* = 2.2 Hz, 1H), 8.50 (d, *J* = 2.8 Hz, 1H), 8.43 (t, *J* = 1.8 Hz, 1H), 8.26 (s, 1H), 7.92 (dd, *J* = 8.9, 1.3 Hz, 1H), 7.73 (dt, *J* = 10.0, 2.2 Hz, 1H), 7.53 (dd, *J* = 8.9, 7.0 Hz, 1H), 7.26 (dd, *J* = 7.0, 1.4 Hz, 1H), 3.88 (s, 2H), 3.62 (m, 4H), 1.65 (m, 2H), 1.56 (m, 4H). | MS (ESI, LR) Calc. for $C_{25}H_{24}FN_6O_2$ $[M+H]^+$: 459.2, found: 459.1. |
| **403** | 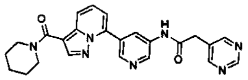 | $^1$H NMR (400 MHz, DMSO-$d_6$) δ(ppm): 10.75 (s, 1H), 9.10 (s, 1H), 8.89 (d, *J* = 2.4 Hz, 1H), 8.78 (s, 2H), 8.75 (d, *J* = 1.9 Hz, 1H), 8.68 (d, *J* = 2.2 Hz, 1H), 8.25 (s, 1H), 7.92 (dd, *J* = 8.9, 1.3 Hz, 1H), 7.53 (dd, *J* = 8.9, 7.0 Hz, 1H), 7.26 (dd, *J* = 7.0, 1.4 Hz, 1H), 3.87 (s, 2H), 3.62 (t, *J* = 5.4 Hz, 5H), 1.65 (m, 2H), 1.56 (m, 4H). | MS (ESI, LR) Calc. for $C_{24}H_{24}N_7O_2$ $[M+H]^+$: 442.2, found: 442.2. |
| **406** | 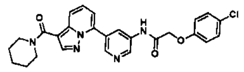 | $^1$H NMR (400 MHz, DMSO-$d_6$) δ(ppm): 10.56 (s, 1H), 8.96 (d, *J* = 2.4 Hz, 1H), 8.78 (d, *J* = 2.0 Hz, 1H), 8.70 (t, *J* = 2.2 Hz, 1H), 8.26 (s, 1H), 7.93 (dd, *J* = 8.9, 1.4 Hz, 1H), 7.54 (dd, *J* = 8.9, 7.0 Hz, 1H), 7.42-7.35 (m, 2H), 7.27 (dd, *J* = 7.1, 1.4 Hz, 1H), 7.12-7.02 (m, 2H), 4.81 (s, 2H), 3.62 (t, *J* = 5.4 Hz, 4H), 1.73-1.62 (m, 2H), 1.57 (m, 4H). | MS (ESI, LR) Calc. for $C_{26}H_{25}ClN_3O_3$ $[M+H]^+$: 490.2, found: 490.1. |

**Example 103. 3-methoxy-*N*-(5-(3-(piperidine-1-carbonyl)pyrazolo[1,5-*a*]pyridin-7-yl)pyridin-2-yl)propanamide**

<Scheme 12>

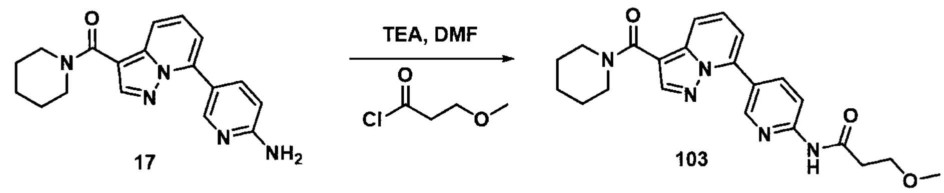


Compound 17 (50 mg, 0.16 mmol) was dissolved in *N,N*-dimethylformamide (5 mL), and triethylamine (64 μL, 0.47 mmol) was added. 3-Methoxypropionyl chloride (28 mg, 0.23 mmol) was added to the reaction solution, and the mixture was stirred at room temperature for 12 hours. After completion of the reaction, the reaction solution was concentrated, diluted with ethyl acetate (20 mL), and washed with water and brine. The organic layer was dried over anhydrous magnesium sulfate, filtered, concentrated, and then separated using column chromatography to obtain the target compound **103** (27.2 mg, 43%).

$^1$H NMR (400 MHz, DMSO-$d_6$) δ(ppm): 10.75 (s, 1H), 8.89 (d, *J* = 2.4 Hz, 1H), 8.40 (dd, *J* = 8.8, 2.4 Hz, 1H), 8.28 - 8.25 (m, 1H), 7.89 (dd, *J* = 8.8, 1.3 Hz, 1H), 7.52 (dd, *J* = 8.9, 7.1 Hz, 1H), 7.27 (dd, *J* = 7.1, 1.4 Hz, 1H), 5.75 (s, 1H), 3.64 (dt, *J* = 10.7, 5.7 Hz, 6H), 3.26 (s, 3H), 2.70 (t, *J* = 6.2 Hz, 2H), 1.65 (d, *J* = 5.6 Hz, 2H), 1.57 (s, 4H).

MS (ESI, LR) Calc. for $C_{23}H_{28}N_5O_3$ $[M+H]^+$: 408.2, found: 408.1.

**Example 104. 2-(dimethylamino)-*N*-(5-(3-(pip eridine-1-carbonyl)pyrazolo[1,5-*a*]pyridin-7-yl)pyridin-2-yl)acetamide**

<Scheme 13>

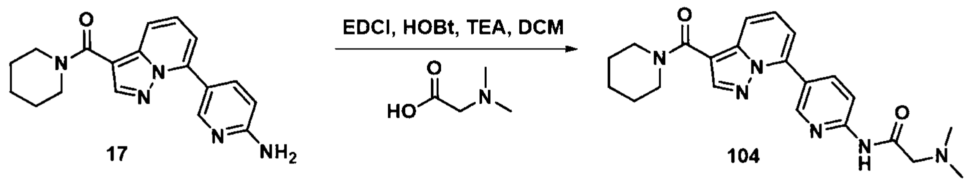


17

104

HOBt (21 mg, 0.16 mmol) and EDCI·HCl (71.6 mg, 0.37 mmol) were dissolved in dichloromethane (10 mL), triethylamine (64.7 μL, 0.47 mmol) was added, and stirred for 1 hour. Compound 17 (0.1 g, 0.31 mmol) was added to the reaction solution, and stirred for 3 hours. After completion of the reaction, the reaction solution was diluted with dichloromethane (20 mL) and washed with water. The organic layer was dried over anhydrous magnesium sulfate, filtered, concentrated, and separated using column chromatography to obtain the target compound **104** (36.3 mg, 29%). [HOBt (Hydroxybenzotriazole), EDCI (1-Ethyl-3-(3-dimethylaminopropyl)carbodiimide)]

$^1$H NMR (400 MHz, DMSO-$d_6$) δ(ppm): 10.17 (s, 1H), 8.88 (d, *J* = 2.4 Hz, 1H), 8.43 (dd, *J* = 8.7, 2.4 Hz, 1H), 8.30-8.22 (m, 2H), 7.89 (dd, *J* = 8.9, 1.4 Hz, 1H), 7.52 (dd, *J* = 8.9, 7.0 Hz, 1H), 7.27 (dd, *J* = 7.1, 1.4 Hz, 1H), 3.62 (t, *J* = 5.4 Hz, 4H), 3.20 (s, 2H), 2.33 (s, 6H), 1.65 (d, *J* = 7.6 Hz, 2H), 1.57 (q, J = 5.8 Hz, 4H).

MS (ESI, LR) Calc. for $C_{22}H_{27}N_6O_2$ $[M+H]^+$: 407.2, found: 407.1.

**Example 105. 2-oxo-*N*-(5-(3-(piperidine-1-carbonyl)pyrazolo[1,5-*a*]pyridin-7-yl)pyridin-2-yl)piperidine-4-carboxamide**

<Scheme 14>

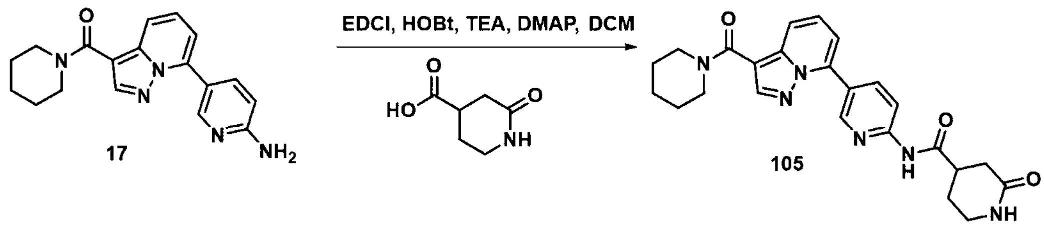


17

105

HOBt (42 mg, 0.31 mmol), EDCI·HCl (58 mg, 0.37 mmol), and 4-dimethylaminopyridine (19 mg, 0.15 mmol) were dissolved in dichloromethane (7 mL), triethylamine (86 μL, 0.62 mmol) was added, and stirred for 1 hour. Compound **17** (0.1 mg, 0.31 mmol) was added to the reaction solution, and stirred for 3 hours. After completion of the reaction, the reaction solution was diluted with dichloromethane (20 mL) and washed with water. The organic layer was dried over anhydrous magnesium sulfate, filtered, concentrated, and then separated using column chromatography to obtain the target compound **105** (29.8 mg, 22%).

$^1$H NMR (400 MHz, DMSO-$d_6$) δ(ppm): 10.87 (s, 1H), 8.90 (d, *J* = 2.4 Hz, 1H), 8.41 (dd, *J* = 8.8, 2.4 Hz, 1H), 8.26 (d, *J* = 7.7 Hz, 2H), 7.91-7.86 (m, 1H), 7.52 (t, *J* = 7.9 Hz, 2H), 7.27 (d, *J* = 7.0 Hz, 1H), 3.62 (t, *J* = 5.5 Hz, 4H), 3.24-3.00 (m, 4H), 2.35 (d, *J* = 7.7 Hz, 2H), 2.05-1.96 (m, 1H), 1.65 (s, 2H), 1.57 (s, 4H).

MS (ESI, LR) Calc. for $C_{24}H_{27}N_6O_3$ $[M+H]^+$: 447.2, found: 447.1

**Example 106. *N*-(5-(3-(piperidine-1-carbonyl)pyrazolo[1,5-*a*]pyridin-7-yl)pyridin-2-yl)-2-(pyridin-3-yl)acetamide**

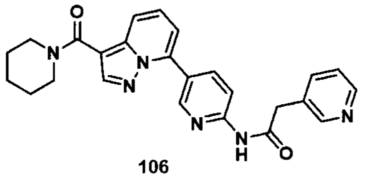

106

The target compound **106** was obtained by the same synthetic method in Example **105** using compound **17** and 2-(3-pyridyl)acetic acid.

$^1$H NMR (400 MHz, DMSO-$d_6$) δ(ppm): 11.10 (s, 1H), 8.91 (dd, *J* = 2.4, 0.9 Hz, 1H), 8.56 (d, *J* = 2.3 Hz, 1H), 8.48 (dd, *J* = 4.8, 1.7 Hz, 1H), 8.40 (dd, *J* = 8.7, 2.4 Hz, 1H), 8.26 (s, 1H), 8.21 (d, *J* = 8.7 Hz, 1H), 7.89 (dd, *J* = 8.8, 1.4 Hz, 1H), 7.78 (dt, *J* = 7.8, 2.1 Hz, 1H), 7.52 (dd, *J* = 8.9, 7.1 Hz, 1H), 7.42-7.34 (m, 1H), 7.27 (dd, *J* = 7.1, 1.3 Hz, 1H), 3.85 (s, 2H), 3.62 (t, *J* = 5.4 Hz, 4H), 1.65 (d, *J* = 5.6 Hz, 2H), 1.56 (d, *J* = 7.1 Hz, 4H).

MS (ESI, LR) Calc. for $C_{25}H_{25}N_6O_2$ [M+H]$^+$: 441.2, found: 441.1

**Example 407. *N*-[5-[3-(piperidine-1-carbonyl)pyrazolo[1,5-*a*]pyridin-7-yl]-2-pyridyl]-2-pyrimidin-5-yl-acetamide**

**Example 408. *N*-[5-[3-(piperidine-1-carbonyl)pyrazolo[1,5-*a*]pyridin-7-yl]-2-pyridyl]-2-[6-(trifluoromethyl)-3-pyridyl]acetamide**

**Example 409. 2-(5-fluoropyridin-3-yl)-*N*-(5-(3-(piperidine-1-carbonyl)pyrazolo[1,5-*a*]pyridin-7-yl)pyridin-2-yl) acetamide**

**Example 410. 2-(4-chlorophenoxy)-*N*-[5-[3-(piperidine-1-carbonyl)pyrazolo[1,5-*a*]pyridin-7-yl]-2-pyridyl]acetamide**

For Examples 407, 408, 409, and 410, the compounds were obtained by the same synthetic method as step 1-2 in Example 1 using compound **17** and the corresponding compounds. The structural and spectral data are shown in Table 15 below.

[Table 15]

| Example | Structure | $^1$H NMR | MS |
|---|---|---|---|
| **407** | 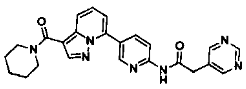 | $^1$H NMR (400 MHz, DMSO-$d_6$) δ(ppm): 11.15 (s, 1H), 9.11 (s, 1H), 8.95-8.90 (m, 1H), 8.79 (s, 2H), 8.41 (dd, *J* = 8.8, 2.4 Hz, 1H), 8.27 (s, 1H), 8.21 (d, *J* = 8.8 Hz, 1H), 7.89 (dd, *J* = 8.9, 1.3 Hz, 1H), 7.52 (dd, *J* = 8.9, 7.1 Hz, 1H), 7.28 (dd, *J* = 7.1, 1.3 Hz, 1H), 3.93 (s, 2H), 3.62 (t, *J* = 5.5 Hz, 4H), 1.65 (d, *J* = 5.8 Hz, 2H), 1.57 (s, 5H). | MS (ESI, LR) Calc. for $C_{24}H_{24}N_7O_2$ [M+H]$^+$: 442.2, found: 442.1. |
| **408** | 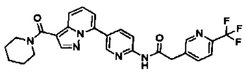 | $^1$H NMR (400 MHz, DMSO-$d_6$) δ(ppm): 11.17 (s, 1H), 8.96-8.87 (m, 1H), 8.75 (d, *J* = 2.1 Hz, 1H), 8.41 (dd, *J* = 8.8, 2.4 Hz, 1H), 8.27 (s, 1H), 8.21 (d, *J* = 8.8 Hz, 1H), 8.08 (dd, *J* = 8.5, 2.1 Hz, 1H), 7.94-7.84 (m, 2H), 7.52 (dd, *J* = 8.9, 7.1 Hz, 1H), 7.28 (dd, *J* = 7.0, 1.3 Hz, 1H), 4.02 (s, 2H), 3.62 (d, *J* = 5.3 Hz, 4H), 1.65 (d, *J* = 5.9 Hz, 2H), 1.56 (d, *J* = 7.0 Hz, 4H). | MS (ESI, LR) Calc. for $C_{26}H_{24}F_3N_6O_2$[M+ H]$^+$: 509.2, found: 509.1. |
| **409** | 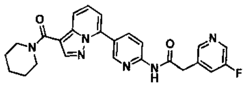 | $^1$H NMR (400 MHz, DMSO-$d_6$) δ(ppm): 11.11 (s, 1H), 8.92 (d, *J* = 2.4 Hz, 1H), 8.50 (d, *J* = 2.8 Hz, 1H), 8.44 (t, *J* = 1.9 Hz, 1H), 8.40 (dd, *J* = 8.8, 2.4 Hz, 1H), 8.26 (s, 1H), 8.21 (d, *J* = 8.7 Hz, 1H), 7.89 (dd, *J* = 8.9, 1.4 Hz, 1H), 7.73 (dt, *J* = 9.8, 2.3 Hz, 1H), 7.52 (dd, *J* = 8.9, 7.1 Hz, 1H), 7.27 (dd, *J* = 7.1, 1.4 Hz, 1H), 3.93 (s, 2H), 3.62 (t, *J* = 5.4 Hz, 4H), 1.64 (d, *J* = 7.1 Hz, 2H), 1.57 (d, *J* = 7.0 Hz, 4H). | MS (ESI, LR) Calc. for $C_{25}H_{24}FN_6O_2$[M+H ]$^+$: 459.2, found: 459.2. |

(continued)

| Example | Structure | $^1H$ NMR | MS |
|---|---|---|---|
| **410** | 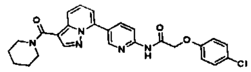 | $^1H$ NMR (400 MHz, DMSO-$d_6$) δ(ppm): 10.88 (s, 1H), 8.97-8.88 (m, 1H), 8.43 (dd, *J* = 8.8, 2.4 Hz, 1H), 8.27 (s, 1H), 8.21 (d, *J* = 8.7 Hz, 1H), 7.89 (dd, *J* = 8.9, 1.3 Hz, 1H), 7.52 (dd, *J* = 8.9, 7.1 Hz, 1H), 7.43-7.32 (m, 2H), 7.28 (dd, *J* = 7.1, 1.4 Hz, 1H), 7.08-6.97 (m, 2H), 4.88 (s, 2H), 3.62 (t, *J* = 5.4 Hz, 4H), 1.65 (d, *J* = 5.3 Hz, 2H), 1.57 (d, *J* = 7.2 Hz, 4H). | MS (ESI, LR) Calc. for $C_{26}H_{25}ClN_5O_3$[M+ H]$^+$: 490.2, found: 490.1. |

**Example 278. *N*-(2,6-difluoro-4-(3-(piperidine-1-carbonyl)pyrazolo[1,5-*a*]pyridin-7-yl)phenyl)-2-(pyridin-3-yl) acetamide**

<Scheme 15>

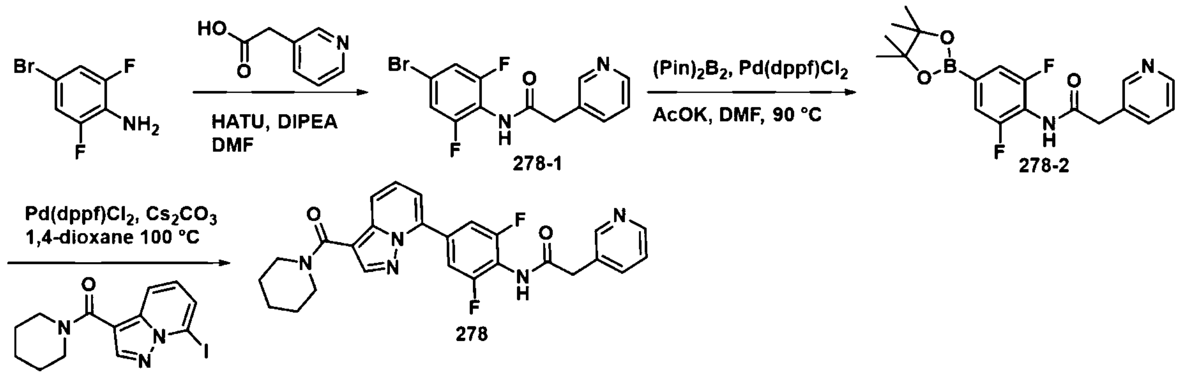


Step 278-1: The target compound **278-1** was obtained by the same synthetic method as step 1-2 in Example 1, using 4-bromo-2,6-difluoro-aniline and 2-(3-pyridyl)acetic acid.

Step 278-2: Using compound **278-1,** the target compound **278-2** was obtained by the same synthetic method as step 351-1 in Example 351.

Step 278-3: Using compound **278-2,** the target compound **278** was obtained by the same synthetic method as step 159-3 in Example 159.

$^1H$ NMR (400 MHz, DMSO-$d_6$) δ(ppm): 10.21 (s, 1H), 8.56 (d, *J* = 2.3 Hz, 1H), 8.49 (dd, *J* = 4.8, 1.7 Hz, 1H), 8.27 (s, 1H), 7.92 (dd, *J* = 8.9, 1.3 Hz, 1H), 7.84 (d, *J* = 8.9 Hz, 2H), 7.82-7.72 (m, 1H), 7.52 (dd, *J* = 8.9, 7.1 Hz, 1H), 7.40 (dd, *J* = 7.9, 4.8 Hz, 1H), 7.32 (dd, *J* = *7.1,* 1.3 Hz, 1H), 3.82 (s, 2H), 3.62 (t, *J* = 5.4 Hz, 4H), 1.65 (d, *J* = 5.6 Hz, 2H), 1.56 (d, *J* = 6.9 Hz, 4H).

MS (ESI, LR) Calc. for $C_{26}H_{24}F_2N_5O_2$ [M+H]$^+$: 476.2, found: 476.1.

**Example 107. (7-(6-((3-methoxypropyl)amino)pyridin-3-yl)pyrazolo[1,5-*a*]pyridin-3-yl)(piperidin-1-yl)methanone**

<Scheme 16>

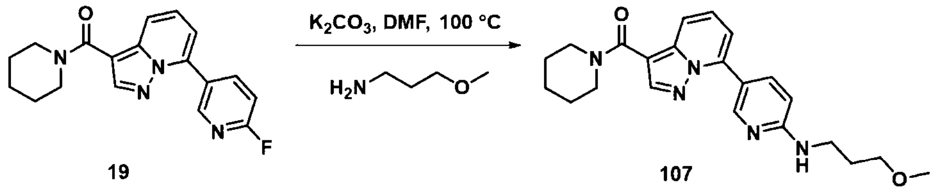


19 107

Potassium carbonate (0.106 g, 0.77 mmol) was added to a solution of 3-methoxypropylamine (47 μL, 0.46 mmol) in N,N-dimethylformamide (6 mL), and stirred for 10 minutes. Compound **19** (0.1 g, 0.31 mmol) was added to the reaction solution, and the mixture was stirred at 100 °C for 12 hours. After completion of the reaction, the reaction solution was concentrated, diluted with dichloromethane (20 mL), and washed with water and brine. The organic layer was dried over anhydrous magnesium sulfate, filtered, concentrated, and then separated using column chromatography to obtain the target compound **107** (0.059 g, 49%).

$^1$H NMR (400 MHz, DMSO-$d_6$) δ(ppm): 8.55 (d, *J* = 2.4 Hz, 1H), 8.22 (s, 1H), 8.02 (dd, *J* = 8.9, 2.5 Hz, 1H), 7.78 (dd, *J* = 8.9, 1.4 Hz, 1H), 7.46 (dd, *J* = 8.9, 7.1 Hz, 1H), 7.11 (dd, *J* = 7.2, 1.4 Hz, 1H), 6.59 (d, *J* = 8.9 Hz, 1H), 3.61 (t, *J* = 5.4 Hz, 4H), 3.42 (t, *J* = 6.3 Hz, 2H), 3.38 (d, *J* = 6.7 Hz, 2H), 3.26 (s, 3H), 1.80 (p, *J* = 6.6 Hz, 2H), 1.65 (d, *J* = 6.0 Hz, 2H), 1.56 (d, *J* = 7.1 Hz, 4H).

MS (ESI, LR) Calc. for $C_{22}H_{28}N_5O_2$ [M+H]$^+$: 394.2, found: 394.2.

**Example 108. (7-(6-morpholinopyridin-3-yl)pyrazolo[1,5-*a*]pyridin-3-yl)(piperidin-1-yl)methanone**

**Example 109. (7-(6-(isopropylamino)pyridin-3-yl)pyrazolo[1,5-*a*]pyridin-3-yl)(piperidin-1-yl)methanone**

**Example 110. piperidin-1-yl(7-(6-(piperidin-1-yl)pyridin-3-yl)pyrazolo[1,5-*a*]pyridin-3-yl)methanone**

**Example 111. (7-(6-(4-methylpiperazin-1-yl)pyridin-3-yl)pyrazolo[1,5-*a*]pyridin-3-yl)(piperidin-1-yl)methanone**

**Example 206. (7-(6-(azetidin-1-yl)pyridin-3-yl)pyrazolo[1,5-a]pyridin-3-yl)(piperidin-1-yl)methanone**

**Example 208. (7-(6-(3-fluoroazetidin-1-yl)pyridin-3-yl)pyrazolo[1,5-a]pyridin-3-yl)(piperidin-1-yl)methanone**

**Example 209. (7-(6-(3,3-difluoroazetidin-1-yl)pyridin-3-yl)pyrazolo[1,5-*a*]pyridin-3-yl)(piperidin-1-yl)methanone**

**Example 210. (7-(6-(3-hydroxyazetidin-1-yl)pyridin-3-yl)pyrazolo[1,5-*a*]pyridin-3-yl)(piperidin-1-yl)methanone**

**Example 215. (7-(6-(2-azabicyclo[2.2.1]heptan-2-yl)pyridin-3-yl)pyrazolo[1,5-*a*]pyridin-3-yl)(piperidin-1-yl)methanone**

**Example 216. (7-(6-(2-azabicyclo[2.2.2]octan-2-yl)pyridin-3-yl)pyrazolo[1,5-*a*]pyridin-3-yl)(piperidin-1-yl)methanone**

**Example 217. -(6-(7-azabicyclo[2.2.1]heptan-7-yl)pyridin-3-yl)pyrazolo[1,5-*a*]pyridin-3-yl)(piperidin-1-yl)methanone**

**Example 218. (7-(6-(3-(dimethylamino)azetidin-1-yl)pyridin-3-yl)pyrazolo[1,5-*a*]pyridin-3-yl)(piperidin-1-yl)methanone**

**Example 219. methyl 1-(5-(3-(piperidine-1-carbonyl)pyrazolo[1,5-*a*]pyridin-7-yl)pyridin-2-yl)azetidine-3-carboxylate**

**Example 220. (7-(6-(2-azaspiro[3.3]heptan-2-yl)pyridin-3-yl)pyrazolo[1,5-*a*]pyridin-3-yl)(piperidin-1-yl)methanone**

**Example 221. (7-(6-(3-(hydroxymethyl)azetidin-1-yl)pyridin-3-yl)pyrazolo[1,5-*a*]pyridin-3-yl)(piperidin-1-yl)methanone**

**Example 222. (7-(6-(7-azaspiro[3.5]nonan-7-yl)pyridin-3-yl)pyrazolo[1,5-*a*]pyridin-3-yl)(piperidin-1-yl)methanone**

**Example 223. (7-(6-(6-azaspiro[3.4]octan-6-yl)pyridin-3-yl)pyrazolo[1,5-*a*]pyridin-3-yl)(piperidin-1-yl)methanone**

**Example 332. piperidin-1-yl(7-(6-((2-(pyridin-3-yl)ethyl)amino)pyridin-3-yl)pyrazolo[1,5-a]pyridin-3-yl)methanone**

**Example 344. (7-(6-(4-amino-*1H*-pyrazol-1-yl)pyridin-3-yl)pyrazolo[1,5-*a*]pyridin-3-yl)(piperidin-1-yl)methanone**

For Examples 108, 109, 110, 111, 206, 208, 209, 210, 215, 216, 217, 218, 219, 220, 221, 222, 223, 332, and 344, the compounds were obtained by the same synthetic method used in Example **107** using the corresponding compounds and compound **19.** The structural and spectral data are presented in Table 16 below.

[Table 16]

| Example | Structure | $^1$H NMR | MS |
|---|---|---|---|
| **108** | 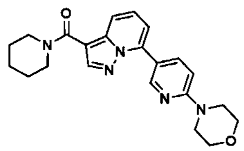 | $^1$H NMR (400 MHz, DMSO-$d_6$) δ(ppm): 8.70 (d, *J* = 2.5 Hz, 1H), 8.22 (d, *J* = 9.5 Hz, 2H), 7.81 (dd, *J* = 8.8, 1.3 Hz, 1H), 7.48 (dd, *J* = 8.9, 7.1 Hz, 1H), 7.18 (dd, *J* = 7.1, 1.3 Hz, 1H), 6.99 (d, *J* = 9.0 Hz, 1H), 3.74 (t, *J* = 4.8 Hz, 4H), 3.60 (dt, *J* = 9.9, 5.0 Hz, 8H), 1.65 (d, *J* = 5.6 Hz, 2H), 1.57 (d, J = 7.0 Hz, 4H). | MS (ESI, LR) Calc. for $C_{22}H_{26}N_3O_2$ $[M+H]^+$: 392.2, found: 392.2. |
| **109** | 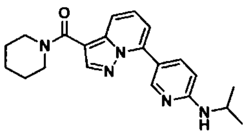 | $^1$H NMR (400 MHz, DMSO-$d_6$) δ(ppm): 8.55 (d, *J* = 2.5 Hz, 1H), 8.22 (s, 1H), 8.00 (dd, *J* = 8.9, 2.5 Hz, 1H), 7.77 (dd, *J* = 8.8, 1.4 Hz, 1H), 7.46 (dd, *J* = 8.9, 7.1 Hz, 1H), 7.11 (dd, *J* = 7.1, 1.4 Hz, 1H), 6.89 (d, *J* = 7.6 Hz, 1H), 6.57 (d, *J* = 8.9 Hz, 1H), 4.11 (h, *J* = 6.6 Hz, 1H), 3.62 (t, *J* = 5.4 Hz, 4H), 1.65 (d, *J* = 5.7 Hz, 2H), 1.62-1.53 (m, 4H), 1.19 (d, *J* = 6.5 Hz, 6H). | MS (ESI, LR) Calc. for $C_{21}H_{26}N_3O$ $[M+H]^+$: 364.2, found: 364.2. |
| **110** | 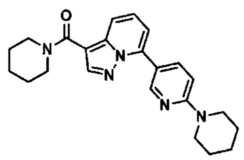 | $^1$H NMR (400 MHz, DMSO-$d_6$) δ(ppm): 8.67 (d, *J* = 2.5 Hz, 1H), 8.23 (s, 1H), 8.16 (dd, *J* = 9.0, 2.6 Hz, 1H), 7.80 (dd, *J* = 8.8, 1.4 Hz, 1H), 7.47 (dd, *J* = 8.8, 7.1 Hz, 1H), 7.16 (dd, *J* = 7.2, 1.4 Hz, 1H), 6.96 (d, *J* = 9.1 Hz, 1H), 3.64 (dt, *J* = 13.9, 5.4 Hz, 8H), 1.66 (dt, *J* = 9.2, 5.6 Hz, 4H), 1.58 (p, *J* = 5.8 Hz, 8H). | MS (ESI, LR) Calc. for $C_{23}H_{28}N_3O$ $[M+H]^+$: 390.2, found: 390.2. |
| **111** | 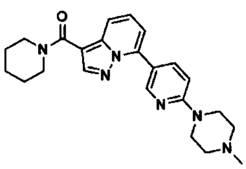 | $^1$H NMR (400 MHz, DMSO-$d_6$) δ(ppm): 8.68 (d, *J* = 2.5 Hz, 1H), 8.23 (s, 1H), 8.19 (dd, *J* = 9.0, 2.5 Hz, 1H), 7.81 (dd, *J* = 8.9, 1.3 Hz, 1H), 7.47 (dd, *J* = 8.8, 7.1 Hz, 1H), 7.17 (dd, *J* = 7.1, 1.4 Hz, 1H), 6.98 (d, *J* = 9.1 Hz, 1H), 3.63 (dd, *J* = 6.4, 3.2 Hz, 8H), 2.42 (t, *J* = 5.1 Hz, 4H), 2.24 (s, 3H), 1.65 (d, *J* = 8.5 Hz, 2H), 1.56 (d, *J* = 7.5 Hz, 4H). | MS (ESI, LR) Calc. for $C_{23}H_{29}N_6O$ $[M+H]^+$: 405.2, found: 405.2. |
| **206** | 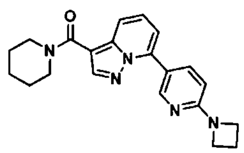 | $^1$H NMR (400 MHz, DMSO-$d_6$) δ(ppm): 8.61 (d, *J* = 2.4 Hz, 1H), 8.22 (s, 1H), 8.15 (dd, *J* = 8.7, 2.4 Hz, 1H), 7.80 (dd, *J* = 8.8, 1.4 Hz, 1H), 7.47 (dd, *J* = 8.9, 7.1 Hz, 1H), 7.13 (dd, *J* = 7.1, 1.4 Hz, 1H), 6.48 (d, *J* = 8.8 Hz, 1H), 4.05 (t, *J* = 7.4 Hz, 4H), 3.61 (t, *J* = 5.4 Hz, 4H), 2.38 (p, *J* = 7.4 Hz, 2H), 1.65 (q, *J* = 5.8 Hz, 2H), 1.57 (q, *J* = 5.6 Hz, 4H). | MS (ESI, LR) Calc. for $C_{21}H_{24}N_5O$ $[M+H]^+$: 362.2, found: 362.1. |
| **208** | 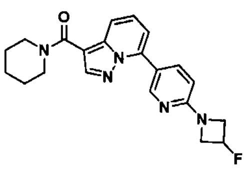 | $^1$H NMR (400 MHz, DMSO-$d_6$) δ(ppm): 8.64 (d, *J* = 2.3 Hz, 1H), 8.23 (s, 1H), 8.19 (dd, *J* = *8.7,* 2.4 Hz, 1H), 7.82 (dd, *J* = 8.9, 1.4 Hz, 1H), 7.48 (dd, *J* = 8.8, 7.1 Hz, 1H), 7.16 (dd, *J* = 7.1, 1.4 Hz, 1H), 6.61 (d, *J* = 8.7 Hz, 1H), 5.64-5.47 (m, 1H), 4.45-4.32 (m, 2H), 4.17-4.06 (m, 2H), 3.62 (t, *J* = 5.4 Hz, 4H), 1.61 (dd, *J* = 34.5, 6.4 Hz, 6H). | MS (ESI, LR) Calc. for $C_{21}H_{23}FN_5O$ $[M+H]^+$: 380.2, found: 380.1. |

(continued)

| Example | Structure | $^1$H NMR | MS |
|---|---|---|---|
| **209** | 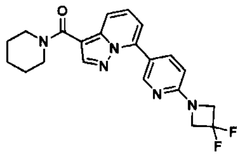 | $^1$H NMR (400 MHz, DMSO-$d_6$) δ(ppm): 8.69 (d, $J$ = 2.3 Hz, 1H), 8.27-8.21 (m, 2H), 7.84 (dd, $J$ = 8.8, 1.4 Hz, 1H), 7.49 (dd, $J$ = 8.9, 7.1 Hz, 1H), 7.18 (dd, $J$ = 7.0, 1.4 Hz, 1H), 6.74 (d, $J$ = 8.7 Hz, 1H), 4.50 (t, $J$ = 12.5 Hz, 4H), 3.62 (t, $J$ = 5.4 Hz, 4H), 1.65 (d, $J$ = 5.0 Hz, 2H), 1.56 (d, $J$ = 7.7 Hz, 4H). | MS (ESI, LR) Calc. for $C_{21}H_{22}F_2N_5O$ $[M+H]^+$: 398.2, found: 398.1. |
| **210** | 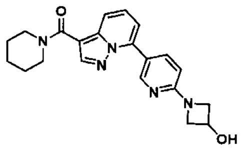 | $^1$H NMR (400 MHz, DMSO-$d_6$) δ(ppm): 8.61 (d, $J$ = 2.4 Hz, 1H), 8.22 (s, 1H), 8.15 (dd, $J$ = 8.7, 2.4 Hz, 1H), 7.80 (dd, $J$ = 8.9, 1.4 Hz, 1H), 7.47 (dd, $J$ = 8.9, 7.1 Hz, 1H), 7.14 (dd, $J$ = 7.1, 1.4 Hz, 1H), 6.52 (d, $J$ = 8.7 Hz, 1H), 5.73 (d, $J$ = 6.4 Hz, 1H), 4.63 (q, $J$ = 5.4 Hz, 1H), 4.26 (dd, $J$ = 8.9, 6.6 Hz, 2H), 3.78 (dd, $J$ = 9.0, 4.6 Hz, 2H), 3.62 (t, $J$ = 5.4 Hz, 4H), 1.65 (d, $J$ = 5.4 Hz, 2H), 1.56 (d, $J$ = 4.6 Hz, 4H). | MS (ESI, LR) Calc. for $C_{21}H_{24}N_3O_2$ $[M+H]^+$: 378.2, found: 378.1. |
| **215** | 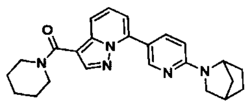 | $^1$H NMR (400 MHz, DMSO-$d_6$) δ(ppm): 8.61 (d, $J$ = 2.4 Hz, 1H), 8.22 (s, 1H), 8.11 (dd, $J$ = 8.9, 2.5 Hz, 1H), 7.78 (dd, $J$ = 8.9, 1.4 Hz, 1H), 7.46 (dd, $J$ = 8.8, 7.1 Hz, 1H), 7.12 (dd, $J$ = 7.1, 1.4 Hz, 1H), 4.60 (s, 1H), 3.62 (t, $J$ = 5.4 Hz, 4H), 3.45 (d, $J$ = 9.5 Hz, 1H), 3.10 (s, 1H), 2.66 (s, 1H), 1.76-1.50 (m, 11H), 1.39 (t, $J$ = 8.5 Hz, 1H). | MS (ESI, LR) Calc. for $C_{24}H_{28}N_5O$ $[M+H]^+$: 402.2, found: 402.1. |
| **216** | 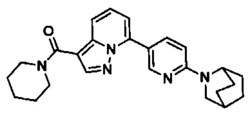 | $^1$H NMR (400 MHz, DMSO-$d_6$) δ(ppm): 8.62 (d, $J$ = 2.5 Hz, 1H), 8.22 (s, 1H), 8.15 (dd, $J$ = 9.0, 2.5 Hz, 1H), 7.78 (dd, $J$ = 8.9, 1.3 Hz, 1H), 7.47 (dd, $J$ = 8.9, 7.1 Hz, 1H), 7.13 (dd, $J$ = 7.0, 1.4 Hz, 1H), 6.62 (d, $J$ = 9.0 Hz, 1H), 3.62 (t, $J$ = 5.4 Hz, 4H), 3.43 (s, 2H), 2.03 (s, 1H), 1.88-1.52 (m, 16H). | MS (ESI, LR) Calc. for $C_{25}H_{30}N_5O$ $[M+H]^+$: 416.2, found: 416.1. |
| **217** | 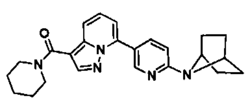 | $^1$H NMR (400 MHz, DMSO-$d_6$) δ(ppm): 8.66 (d, $J$ = 2.4 Hz, 1H), 8.23 (s, 1H), 8.17 (dd, $J$ = 8.8, 2.5 Hz, 1H), 7.81 (dd, $J$ = 8.8, 1.4 Hz, 1H), 7.48 (dd, $J$ = 8.9, 7.0 Hz, 1H), 7.17 (dd, $J$ = 7.1, 1.3 Hz, 1H), 7.01 (d, $J$ = 8.8 Hz, 1H), 4.64-4.56 (m, 2H), 3.62 (t, $J$ = 5.5 Hz, 4H), 1.76-1.46 (m, 14H). | MS (ESI, LR) Calc. for $C_{24}H_{28}N_5O$ $[M+H]^+$: 402.2, found: 402.1. |
| **218** | 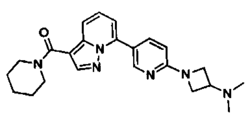 | $^1$H NMR (400 MHz, DMSO-$d_6$) δ(ppm): 8.62 (d, $J$ = 2.4 Hz, 1H), 8.22 (s, 1H), 8.16 (dd, $J$ = 8.8, 2.4 Hz, 1H), 7.80 (dd, $J$ = 8.9, 1.4 Hz, 1H), 7.47 (dd, $J$ = 8.8, 7.1 Hz, 1H), 7.14 (dd, $J$ = 7.1, 1.4 Hz, 1H), 6.52 (d, $J$ = 8.7 Hz, 1H), 4.08 (dd, $J$ = 8.6, 6.9 Hz, 2H), 3.82 (dd, $J$ = 8.7, 5.2 Hz, 2H), 3.61 (t, $J$ = 5.4 Hz, 4H), 3.27-3.20 (m, 1H), 2.14 (s, 6H), 1.65 (d, $J$ = 6.0 Hz, 2H), 1.57 (t, $J$ = 6.7 Hz, 4H). | MS (ESI, LR) Calc. for $C_{23}H_{29}N_6O$ $[M+H]^+$: 405.2, found: 405.1. |
| **219** | 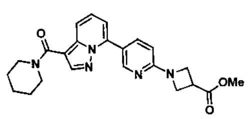 | $^1$H NMR (400 MHz, DMSO-$d_6$) δ(ppm): 8.63 (d, $J$ = 2.3 Hz, 1H), 8.22 (s, 1H), 8.18 (dd, $J$ = 8.7, 2.4 Hz, 1H), 7.81 (dd, $J$ = 8.9, 1.4 Hz, 1H), 7.48 (dd, $J$ = 8.9, 7.1 Hz, 1H), 7.15 (dd, $J$ = 7.1, 1.3 Hz, 1H), 6.58 (d, $J$ = 8.8 Hz, 1H), 4.26 (t, $J$ = 8.6 Hz, 2H), 4.12 (dd, $J$ = 8.3, 5.8 Hz, 2H), 3.71 (s, 4H), 3.62 (t, $J$ = 5.5 Hz, 4H), 1.65 (d, $J$ = 8.2 Hz, 2H), 1.56 (d, $J$ = 7.6 Hz, 4H). | MS (ESI, LR) Calc. for $C_{23}H_{26}N_5O_3$ $[M+H]^+$: 420.2, found: 420.1. |
| **220** | 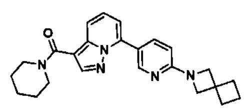 | $^1$H NMR (400 MHz, DMSO-$d_6$) δ(ppm): 8.61 (d, $J$ = 2.4 Hz, 1H), 8.22 (s, 1H), 8.14 (dd, $J$ = 8.8, 2.4 Hz, 1H), 7.80 (dd, $J$ = 8.9, 1.4 Hz, 1H), 7.47 (dd, $J$ = 8.9, 7.1 Hz, 1H), 7.13 (dd, $J$ = 7.1, 1.4 Hz, 1H), 6.49 (d, $J$ = 8.8 Hz, 1H), 4.02 (s, 4H), 3.62 (t, $J$ = 5.4 Hz, 4H), 2.22 (t, $J$ = 7.6 Hz, 4H), 1.84 (p, $J$ = 7.5 Hz, 2H), 1.69-1.61 (m, 2H), 1.56 (s, 4H). | MS (ESI, LR) Calc. for $C_{24}H_{28}N_5O$ $[M+H]^+$: 420.2, found: 420.1. |

(continued)

| Example | Structure | $^1$H NMR | MS |
|---|---|---|---|
| **221** | 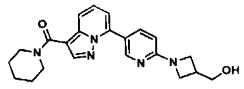 | $^1$H NMR (400 MHz, DMSO-$d_6$) δ(ppm): 8.61 (d, *J* = 2.4 Hz, 1H), 8.22 (s, 1H), 8.14 (dd, *J* = 8.7, 2.4 Hz, 1H), 7.80 (dd, *J* = 8.9, 1.4 Hz, 1H), 7.47 (dd, *J* = 8.8, 7.1 Hz, 1H), 7.13 (dd, *J* = 7.1, 1.4 Hz, 1H), 6.49 (d, *J* = 8.8 Hz, 1H), 4.81 (t, *J* = 5.3 Hz, 1H), 4.06 (t, *J* = 8.2 Hz, 2H), 3.79 (dd, *J* = 8.3, 5.4 Hz, 2H), 3.61 (t, *J* = 5.8 Hz, 6H), 2.85 (ddd, *J* = 13.7, 7.5, 5.3 Hz, 1H), 1.61 (dd, *J* = 35.4, 6.3 Hz, 6H). | MS (ESI, LR) Calc. for $C_{22}H_{26}N_5O_2$ $[M+H]^+$: 392.2, found: 392.1. |
| **222** | 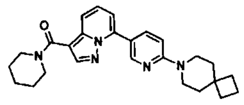 | $^1$H NMR (400 MHz, DMSO-$d_6$) δ(ppm): 8.66 (d, *J* = 2.5 Hz, 1H), 8.22 (s, 1H), 8.15 (dd, *J* = 9.0, 2.5 Hz, 1H), 7.79 (dd, *J* = 8.8, 1.4 Hz, 1H), 7.47 (dd, *J* = 8.8, 7.1 Hz, 1H), 7.15 (dd, *J* = 7.1, 1.3 Hz, 1H), 6.97 (d, *J* = 9.1 Hz, 1H), 3.66-3.54 (m, 8H), 1.96-1.76 (m, 6H), 1.69-1.51 (m, 10H). | MS (ESI, LR) Calc. for $C_{26}H_{32}N_5O$ $[M+H]^+$: 430.2, found: 430.1. |
| **223** | 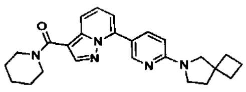 | $^1$H NMR (400 MHz, DMSO-$d_6$) δ(ppm): 8.64 (d, *J* = 2.4 Hz, 1H), 8.22 (s, 1H), 8.14 (dd, *J* = 8.9, 2.5 Hz, 1H), 7.78 (dd, *J* = 8.9, 1.4 Hz, 1H), 7.46 (dd, *J* = 8.8, 7.1 Hz, 1H), 7.13 (dd, *J* = 7.2, 1.4 Hz, 1H), 6.57 (d, *J* = 8.9 Hz, 1H), 3.62 (t, *J* = 5.4 Hz, 4H), 3.47 (d, *J* = 6.5 Hz, 4H), 2.09-1.87 (m, 8H), 1.65 (d, *J* = 5.6 Hz, 2H), 1.56 (d, *J* = 7.5 Hz, 4H). | MS (ESI, LR) Calc. for $C_{25}H_{30}N_5O$ $[M+H]^+$: 416.2, found: 416.1. |
| **332** | 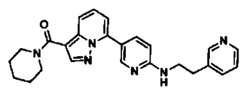 | $^1$H NMR (400 MHz, DMSO-$d_6$) δ(ppm): 8.59 (d, *J* = 2.4 Hz, 1H), 8.50 (d, *J* = 2.3 Hz, 1H), 8.43 (dd, *J* = 4.8, 1.7 Hz, 1H), 8.22 (s, 1H), 8.03 (dd, *J* = 8.8, 2.5 Hz, 1H), 7.78 (dd, *J* = 8.9, 1.4 Hz, 1H), 7.70 (dt, *J* = 7.9, 2.0 Hz, 1H), 7.46 (dd, *J* = 8.9, 7.1 Hz, 1H), 7.34 (dd, **J** = 7.7, 4.8 Hz, 1H), 7.19-7.05 (m, 2H), 6.63 (d, *J* = 8.8 Hz, 1H), 3.71-3.54 (m, 6H), 2.92 (t, *J* = 7.1 Hz, 2H), 1.65 (q, *J* = 5.8 Hz, 2H), 1.57 (q, *J* = 5.8 Hz, 4H). | MS (ESI, LR) Calc. for $C_{25}H_{27}N_6O$ $[M+H]^+$: 427.2, found: 427.2. |
| **344** | 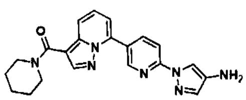 | $^1$H NMR (400 MHz, DMSO-$d_6$) δ(ppm): 8.93 (d, *J* = 2.3 Hz, 1H), 8.50 (dd, *J* = 8.7, 2.4 Hz, 1H), 8.27 (s, 1H), 7.98-7.88 (m, 3H), 7.56-7.51 (m, 1H), 7.44 (s, 1H), 7.34-7.29 (m, 1H), 4.39 (s, 2H), 3.63 (t, *J* = 5.4 Hz, 4H), 1.66 (s, 2H), 1.58 (s, 4H). | MS (ESI, LR) Calc. for $C_{21}H_{22}N_7O$ $[M+H]^+$: 388.2, found: 388.1. |

**Example 287. *N*-(5-(3-(piperidine-1-carbonyl)pyrazolo[1,5-*a*]pyridin-7-yl)pyridin-2-yl)nicotinamide**

<Scheme 17>

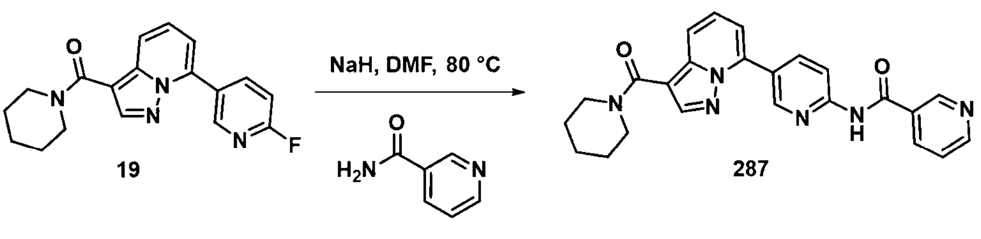


19

287

Compound **19** (36.5 mg, 0.11 mmol), pyridine-3-carboxamide (20.6 mg, 0.17 mmol), and sodium hydride (6.8 mg, 0.17 mmol) were added to N,N-dimethylformamide (3 mL) , and the mixture was stirred at 80 °C for 12 hours. After completion of the reaction, the reaction solution was concentrated, diluted with dichloromethane (10 mL), and washed with water and brine. The organic layer was dried over anhydrous magnesium sulfate, filtered, concentrated, and then separated using column chromatography to obtain the target compound 287 (39.4 mg, 82%).

$^1$H NMR (400 MHz, DMSO-$d_6$) δ(ppm): 11.36 (s, 1H), 9.19 (d, *J* = 2.3 Hz, 1H), 8.98 (d, *J* = 2.3 Hz, 1H), 8.79 (dd, *J* =

4.8, 1.7 Hz, 1H), 8.50 (dd, *J* = 8.8, 2.4 Hz, 1H), 8.46-8.35 (m, 2H), 8.28 (s, 1H), 7.91 (dd, *J* = 8.8, 1.4 Hz, 1H), 7.62-7.50 (m, 2H), 7.32 (dd, *J* = 7.1, 1.4 Hz, 1H), 3.63 (t, *J* = 5.4 Hz, 4H), 1.66 (d, *J* = 6.0 Hz, 2H), 1.58 (d, *J* = 7.4 Hz, 4H).

MS (ESI, LR) Calc. for $C_{24}H_{23}N_6O_2$ [M+H]$^+$: 427.2, found: 427.1.

**Example 316. 3-(5-(3-(piperidine-1-carbonyl)pyrazolo[1,5-*a*]pyridin-7-yl)pyridin-2-yl)oxazolidin-2-one**

**Example 355. (7-(6-(4-nitro-*1H*-pyrazol-1-yl)pyridin-3-yl)pyrazolo[1,5-*a*]pyridin-3-yl)(piperidin-1-yl)methanone**

For Examples 316 and 355, the compounds were obtained by the synthetic method used in Example **287** using compound **19** and the corresponding compounds. The structural and spectral data are shown in Table 17 below.

[Table 17]

| Example | Structure | $^1$H NMR | MS |
|---|---|---|---|
| **316** | 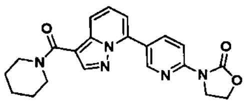 | $^1$H NMR (400 MHz, DMSO-$d_6$) δ(ppm): 8.92 (d, *J* = 2.4 Hz, 1H), 8.45 (dd, *J* = 8.9, 2.4 Hz, 1H), 8.25 (t, *J* = 4.4 Hz, 2H), 7.90 (dd, *J*= 8.9, 1.4 Hz, 1H), 7.53 (dd, *J* = 8.9, 7.0 Hz, 1H), 7.27 (dd, *J* = 7.1, 1.4 Hz, 1H), 4.52 (t, *J* = 8.0 Hz, 2H), 4.27 (dd, *J* = 8.7, 7.3 Hz, 2H), 3.63 (t, *J* = 5.4 Hz, 4H), 1.70-1.62 (m, 2H), 1.57 (d, *J* = 7.5 Hz, 4H). | MS (ESI, LR) Calc. for $C_{21}H_{22}N_3O_3$ [M+H]$^+$: 392.2, found: 392.1. |
| **355** | 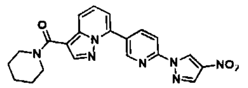 | $^1$H NMR (400 MHz, DMSO-$d_6$) δ(ppm): 9.55 (s, 1H), 9.13 (d, *J* = 2.3 Hz, 1H), 8.80-8.60 (m, 2H), 8.29 (s, 1H), 8.21 (d, *J* = 8.6 Hz, 1H), 7.96 (dd, *J* = 8.9, 1.3 Hz, 1H), 7.57 (dd, *J* = 8.9, 7.0 Hz, 1H), 7.39 (dd, *J* = 7.2, 1.3 Hz, 1H), 3.63 (t, *J* = 5.4 Hz, 4H), 1.66 (m, 2H), 1.63-1.44 (m, 4H). | MS (ESI, LR) Calc. for $C_{21}H_{20}N_7O_3$ [M+H]$^+$: 418.2, found: 418.1. |

**Example 301. *N*-(3-chloro-5-(3-(piperidine-1-carbonyl)pyrazolo[1,5-*a*]pyridin-7-yl)pyridin-2-yl)nicotinamide**

**Example 311. *N*-(3-chloro-5-(3-(piperidine-1-carbonyl)pyrazolo[1,5-*a*]pyridin-7-yl)pyridin-2-yl)-2-(pyridin-3-yl) acetamide**

For Examples 301 and 311, the compounds were obtained by the synthetic method in Example **287** using compound **289** and the corresponding compounds. The structural and spectral data are shown in Table 18 below.

[Table 18]

| Example | Structure | $^1$H NMR | MS |
|---|---|---|---|
| **301** | 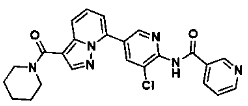 | $^1$H NMR (400 MHz, DMSO-$d_6$) δ(ppm): 11.19 (s, 1H), 9.19 (d, *J* = 2.3 Hz, 1H), 9.02-8.97 (m, 1H), 8.82 (d, *J* = 4.7 Hz, 1H), 8.76-8.71 (m, 1H), 8.41-8.35 (m, 1H), 8.30 (s, 1H), 7.96 (d, *J* = 8.8 Hz, 1H), 7.62 (dd, *J* = 7.9, 4.9 Hz, 1H), 7.56 (dd, *J* = 8.9, 7.1 Hz, 1H), 7.43 (d, *J* = 7.0 Hz, 1H), 3.64 (t, *J* = 5.4 Hz, 4H), 1.66 (m, 3H), 1.58 (m, 4H). | MS (ESI, LR) Calc. for $C_{24}H_{22}ClN_6O_2$ [M+H]$^+$: 461.1, found: 461.0. |
| **311** | 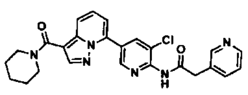 | $^1$H NMR (400 MHz, DMSO-$d_6$) δ(ppm): 10.71 (s, 1H), 8.91 (d, *J* = 2.1 Hz, 1H), 8.65 (d, *J* = 2.1 Hz, 1H), 8.57 (d, *J* = 2.2 Hz, 1H), 8.49 (dd, *J* = 4.8, 1.7 Hz, 1H), 8.28 (s, 1H), 7.94 (dd, *J* = 8.9, 1.3 Hz, 1H), 7.79 (dt, *J* = 7.8, 2.1 Hz, 1H), 7.54 (dd, *J* = 8.9, 7.1 Hz, 1H), 7.38 (ddd, *J* = 7.2, 4.9, 3.1 Hz, 2H), 3.83 (s, 2H), 3.63 (t, *J* = 5.4 Hz, 4H), 1.65 (d, *J* = 5.3 Hz, 2H), 1.58 (t, *J* = 6.7 Hz, 4H). | MS (ESI, LR) Calc. for $C_{25}H_{24}ClN_6O_2$ [M+H]$^+$: 475.2, found: 475.1. |

**Example 323. 3-(3-fluoro-5-(3-(piperidine-1-carbonyl)pyrazolo[1,5-*a*]pyridin-7-yl)pyridin-2-yl)oxazolidin-2-one**

**Example 362. *N*-(3-fluoro-5-(3-(piperidine-1-carbonyl)pyrazolo[1,5-*a*]pyridin-7-yl)pyridin-2-yl)-2-(pyridin-3-yl) acetamide**

For Examples 323 and 362, the compounds were obtained by the synthetic method in Example 287 using compound **288** and the corresponding compounds. The structural and spectral data are shown in Table 19 below.

[Table 19]

| Example | Structure | $^1$H NMR | MS |
|---|---|---|---|
| **323** | 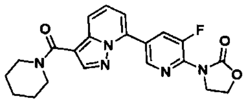 | $^1$H NMR (400 MHz, DMSO-$d_6$) δ(ppm): 8.92-8.80 (m, 1H), 8.53 (dd, *J* = 11.3, 1.9 Hz, 1H), 8.28 (s, 1H), 7.95 (dd, *J* = 8.9, 1.4 Hz, 1H), 7.55 (dd, *J* = 8.9, 7.1 Hz, 1H), 7.38 (dd, *J* = 7.1, 1.4 Hz, 1H), 4.60 (dd, *J* = 8.6, 6.9 Hz, 2H), 4.25 (dd, *J* = 8.4, 7.0 Hz, 2H), 3.63 (t, *J* = 5.4 Hz, 4H), 1.66 (m, 2H), 1.58 (m, 4H). | MS (ESI, LR) Calc. for $C_{21}H_{21}FN_3O_3$ $[M+H]^+$: 410.2, found: 410.1. |
| **362** | 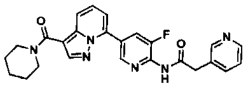 | $^1$H NMR (400 MHz, DMSO-$d_6$) δ(ppm): 10.80 (s, 1H), 8.81 (dd, *J* = 1.9, 0.9 Hz, 1H), 8.56 (d, *J* = 2.3 Hz, 1H), 8.49 (dd, *J* = 4.8, 1.7 Hz, 1H), 8.44 (dd, *J* = 11.2, 1.9 Hz, 1H), 8.27 (s, 1H), 7.93 (dd, *J* = 8.9, 1.3 Hz, 1H), 7.78 (dt, *J* = 7.9, 2.0 Hz, 1H), 7.54 (dd, *J* = 8.9, 7.1 Hz, 1H), 7.43-7.31 (m, 2H), 3.85 (s, 2H), 3.62 (t, *J* = 5.4 Hz, 4H), 1.65 (m, 2H), 1.57 (m, 4H). | MS (ESI, LR) Calc. for $C_{25}H_{24}FN_6O_2$ $[M+H]^+$: 459.2, found: 459.1. |

**Example 308. 3-(5-(3-(piperidine-1-carbonyl)pyrazolo[1,5-*a*]pyridin-7-yl)pyrimidin-2-yl)oxazolidin-2-one**

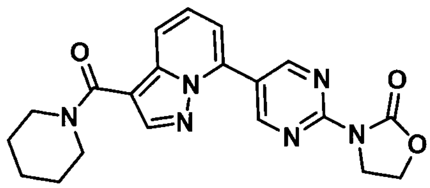

The target compound **308** was obtained by the synthetic method in Example 287 using compound **267** and oxazolidin-2-one.

$^1$H NMR (400 MHz, DMSO-$d_6$) δ(ppm): 9.30 (s, 2H), 8.27 (s, 1H), 7.93 (dd, *J* = 8.9, 1.4 Hz, 1H), 7.55 (dd, *J* = 8.9, 7.0 Hz, 1H), 7.38 (dd, *J* = 7.1, 1.4 Hz, 1H), 4.48 (t, *J* = 7.9 Hz, 2H), 4.27 (t, *J* = 7.9 Hz, 2H), 3.62 (d, *J* = 5.3 Hz, 4H), 1.73-1.63 (m, 2H), 1.58 (d, *J* = 7.2 Hz, 4H).

MS (ESI, LR) Calc. for $C_{20}H_{21}N_6O_3$ $[M+H]^+$: 393.2, found: 393.1.

**Example 257. (7-(2-(3-fluoroazetidin-1-yl)pyridin-4-yl)pyrazolo[1,5-*a*]pyridin-3-yl)(piperidin-1-yl)methanone**

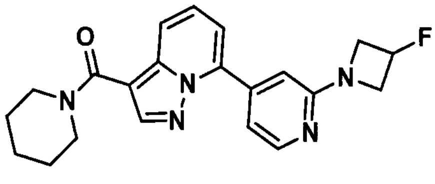

After obtaining the intermediate by the same synthetic method as step 1-3 in Example 1 using compound **1-2** and (2-fluoro-4-pyridyl)boronic acid, the target compound **257** was obtained by the synthetic method in Example 107 using azetidine.

$^1$H NMR (400 MHz, DMSO-$d_6$) δ(ppm): 8.65 (d, *J* = 2.3 Hz, 1H), 8.23 (s, 1H), 8.19 (dd, *J* = 8.7, 2.4 Hz, 1H), 7.82 (dd, *J* = 8.9, 1.4 Hz, 1H), 7.48 (dd, *J* = 8.9, 7.1 Hz, 1H), 7.16 (dd, *J* = 7.1, 1.4 Hz, 1H), 6.61 (d, *J* = 8.8 Hz, 1H), 5.56 (ddt, *J* =

57.4, 5.9, 2.9 Hz, 1H), 4.39 (dddd, *J* = 21.3, 10.3, 5.9, 1.5 Hz, 2H), 4.12 (dddd, *J* = 24.5, 10.4, 3.2, 1.5 Hz, 2H), 3.62 (t, *J* = 5.4 Hz, 4H), 1.65 (d, *J* = 5.4 Hz, 2H), 1.56 (d, *J* = 7.6 Hz, 4H).

MS (ESI, LR) Calc. for $C_{21}H_{23}FN_5O$ $[M+H]^+$: 380.2, found: 380.1.

**Example 112. *N*-(5-(3-(piperidine-1-carbonyl)pyrazolo[1,5-*a*]pyridin-7-yl)pyridin-2-yl)methanesulfonamide**

<Scheme 18>

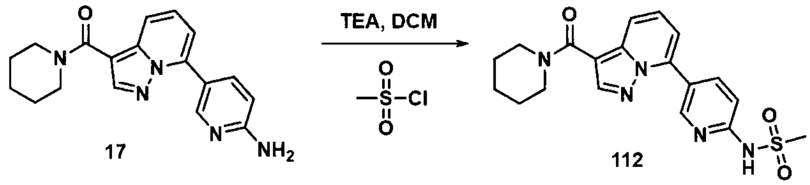


Compound **17** (0.1 g, 0.31 mmol) and triethylamine (97 μL, 0.7 mmol) were dissolved in dichloromethane (5 mL), the temperature was lowered to 0 °C using ice water, and methanesulfonyl chloride (52 μL, 0.7 mmol) was added dropwise. The temperature was raised to room temperature and the reaction mixture was stirred for 12 hours. After completion of the reaction, the reaction solution was diluted with dichloromethane (20 mL) and washed with water (30 mL), saturated aqueous sodium bicarbonate solution (30 mL), and brine (30 mL). The organic layer was dried over anhydrous magnesium sulfate, filtered, concentrated, and separated using column chromatography to obtain the target compound 112 (29 mg, 24%).

$^1H$ NMR (400 MHz, DMSO-$d_6$) δ(ppm): 8.80 (d, *J* = 2.4 Hz, 1H), 8.33 (dd, *J* = 8.7, 2.4 Hz, 1H), 8.25 (s, 1H), 7.88 (dd, *J* = 8.9, 1.3 Hz, 1H), 7.51 (dd, *J* = 8.9, 7.1 Hz, 1H), 7.24 (dd, *J* = 7.0, 1.4 Hz, 1H), 7.12 (d, *J* = 8.7 Hz, 1H), 3.62 (t, *J* = 5.4 Hz, 4H), 3.36 (s, 3H), 1.65 (d, *J* = 7.5 Hz, 2H), 1.56 (d, *J* = 7.6 Hz, 4H).

MS (ESI, LR) Calc. for $C_{20}H_{22}N_5O_4S$ $[M+H]^+$: 400.1, found: 400.0.

**Example 113. (7-(3-(1*H*-tetrazol-5-yl)phenyl)pyrazolo[1,5-*a*]pyridin-3-yl)(piperidin-1-yl)methanone**

<Scheme 19>

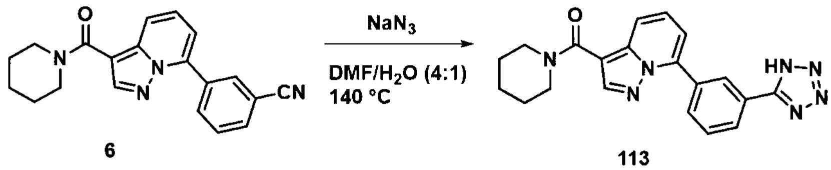


Compound **6** (0.1 g, 0.3 mmol) was dissolved in N,N-dimethylformamide (8 mL). Sodium azide (0.039 g, 0.61 mmol) dissolved in water (2 mL) was added, and stirred at 140 °C for 12 hours. After completion of the reaction, the reaction solution was concentrated and separated using column chromatography to obtain the target compound **113** (7 mg, 6%).

$^1H$ NMR (400 MHz, DMSO-$d_6$) δ(ppm): 8.51 (t, *J* = 1.7 Hz, 1H), 8.25 (s, 1H), 8.12 (dt, *J* = 7.7, 1.4 Hz, 1H), 7.89 (dd, *J* = 8.9, 1.3 Hz, 1H), 7.78 (dt, *J* = 7.7, 1.5 Hz, 1H), 7.55 (dt, *J* = 10.8, 8.3 Hz, 2H), 7.20 (dd, *J* = 7.0, 1.3 Hz, 1H), 3.64 (t, *J* = 5.4 Hz, 4H), 1.65 (d, *J* = 7.4 Hz, 2H), 1.58 (s, 4H).

MS (ESI, LR) Calc. for $C_{20}H_{20}N_7O$ $[M+H]^+$: 374.2, found: 374.2.

**Example 114. (7-(4-(1*H*-tetrazol-5-yl)phenyl)pyrazolo[1,5-*a*]pyridin-3-yl)(piperidin-1-yl)methanone**

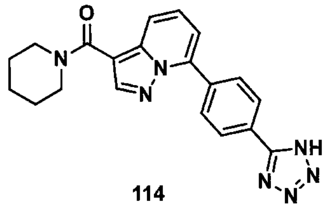

114

The target compound **114** was obtained by the synthetic method in Example **113** using compound **15** and the corresponding compounds.

$^1H$ NMR (400 MHz, DMSO-$d_6$) δ(ppm): 8.26 (s, 1H), 8.14 (d, *J* = 8.1 Hz, 2H), 7.97 (d, *J* = 9.2 Hz, 2H), 7.90-7.83 (m, 1H), 7.52 (dd, *J* = 8.9, 7.1 Hz, 1H), 7.22 (d, *J* = 7.0 Hz, 1H), 3.63 (t, *J* = 5.4 Hz, 4H), 1.65 (s, 2H), 1.58 (s, 4H).

MS (ESI, LR) Calc. for $C_{20}H_{20}N_7O$ $[M+H]^+$: 374.2, found: 374.2.

**Example 115. 3-(3-(3-(piperidine-1-carbonyl)pyrazolo[1,5-*a*]pyridin-7-yl)phenyl)-1,2,4-oxadiazol-5(4*H*)-one**

<Scheme 20>

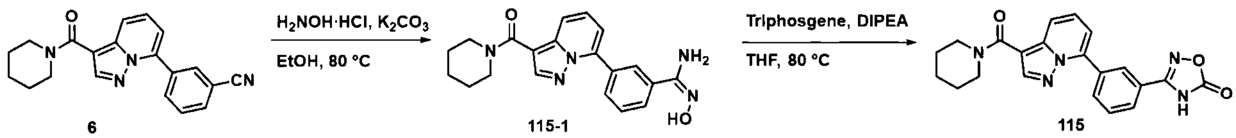


6 115-1 115

Step 115-1: After dissolving compound **6** (0.15 g, 0.45 mmol) in ethanol (6.5 mL), hydroxyamine (47 mg, 0.68 mmol) was added and stirred at 80 °C for 16 hours. After completion of the reaction, the reaction solution was concentrated, diluted with ethyl acetate (20 mL), and washed with water (10 mL). The organic layer was dried over anhydrous magnesium sulfate, filtered, and concentrated to obtain the target compound **115-1** (0.079 g, 24%).

Step 115-2: Compound **115-1** (0.2 g, 0.55 mmol) was dissolved in tetrahydrofuran (20 mL) and DIPEA (0.19 mL, 1.1 mmol) was added. Triphosgene (0.065 g, 0.22 mmol) dissolved in tetrahydrofuran (5 mL) was slowly added to the reaction solution and stirred at room temperature for 30 minutes. The temperature was increased to 80 °C and stirred for 1 hour. After completion of the reaction, the reaction solution was concentrated and separated using column chromatography to obtain the target compound **115** (0.093 g, 43%).

$^1H$ NMR (400 MHz, DMSO-$d_6$) δ(ppm): 8.36 (d, *J* = 2.0 Hz, 1H), 8.26 (s, 1H), 8.09 (d, *J* = 7.8 Hz, 1H), 7.94 (dd, *J* = 15.0, 8.4 Hz, 2H), 7.71 (t, *J* = 7.5 Hz, 1H), 7.55 (dd, *J* = 8.9, 7.0 Hz, 1H), 7.24 (d, *J* = 6.7 Hz, 1H), 6.53 (s, 1H), 3.63 (t, *J* = 5.4 Hz, 3H), 1.65 (s, 2H), 1.57 (s, 4H).

MS (ESI, LR) Calc. for $C_{21}H_{20}N_5O_3$ $[M+H]^+$: 390.2, found: 390.1.

**Example 116. 3-(4-(3-(piperidine-1-carbonyl)pyrazolo[1,5-*a*]pyridin-7-yl)phenyl)-1,2,4-oxadiazol-5(4*H*)-one**

**Example 117. 3-(5-(3-(piperidine-1-carbonyl)pyrazolo[1,5-*a*]pyridin-7-yl)pyridin-2-yl)-1,2,4-oxadiazol-5(4*H*)-one**

**Example 247. 3-[3-fluoro-5-[3-(piperidine-1-carbonyl)pyrazolo[1,5-*a*]pyridin-7-yl]-2-pyridyl]-4*H*-1,2,4-oxadiazol-5-one**

**Example 275. 3-[2-fluoro-5-[3-(piperidine-1-carbonyl)pyrazolo[1,5-*a*]pyridin-7-yl]phenyl]-4*H*-1,2,4-oxadiazol-5-one**

**Example 295. 3-[3-fluoro-4-[3-(piperidine-1-carbonyl)pyrazolo[1,5-*a*]pyridin-7-yl]phenyl]-4*H*-1,2,4-oxadiazol-5-one**

**Example 297. 3-(5-(3-(piperidine-1-carbonyl)pyrazolo[1,5-*a*]pyridin-7-yl)pyridin-3-yl)-1,2,4-oxadiazol-5(4*H*)-one**

**Example 299. 3-(3-fluoro-5-(3-(piperidine-1-carbonyl)pyrazolo[1,5-*a*]pyridin-7-yl)phenyl)-1,2,4-oxadiazol-5(4*H*)-one**

**Example 304. 3-[2-fluoro-4-[3-(piperidine-1-carbonyl)pyrazolo[1,5-*a*]pyridin-7-yl]phenyl]-4*H*-1,2,4-oxadiazol-5-one**

**Example 321. 3-[5-[3-(piperidine-1-carbonyl)pyrazolo[1,5-*a*]pyridin-7-yl]pyrimidin-2-yl]-4*H*-1,2,4-oxadiazol-5-one**

**Example 329. 3-(3-nitro-5-(3-(piperidine-1-carbonyl)pyrazolo[1,5-*a*]pyridin-7-yl)phenyl)-1,2,4-oxadiazol-5(4*H*)-one**

**Example 331. 3-(2,6-difluoro-4-(3-(piperidine-1-carbonyl)pyrazolo[1,5-*a*]pyridin-7-yl)phenyl)-1,2,4-oxadiazol-5(4*H*)-one**

For Examples 116, 117, 247, 275, 295, 297, 299, 304, 321, 329, and 331, the compounds were obtained by sequentially using the synthetic method as step 1-3 of Example 1 and the synthetic method of Example **115** using compound **1-2** and the corresponding compounds. The structural and spectral data are shown in Table 20 below.

[Table 20]

| Example | Structure | $^1$H NMR | MS |
|---|---|---|---|
| **116** | 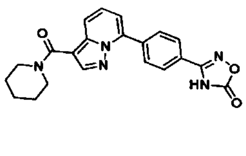 | $^1$H NMR (400 MHz, DMSO-$d_6$) δ(ppm): 8.25 (d, *J* = 4.8 Hz, 1H), 8.11 (d, *J* = 7.9 Hz, 2H), 7.97 (d, *J* = 7.9 Hz, 2H), 7.91 (d, *J* = 8.9 Hz, 1H), 7.54 (t, *J* = 8.1 Hz, 1H), 7.27 (d, *J* = 7.1Hz, 1H), 6.53 (s, 1H), 3.62 (d, *J* = 5.3 Hz, 3H), 1.65 (s, 2H), 1.57 (s, 4H). | MS (ESI, LR) Calc. for $C_{21}H_{20}N_5O_3$ $[M+H]^+$: 390.2, found: 390.1. |
| **117** | 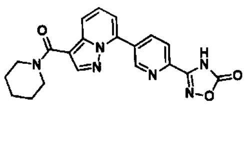 | $^1$H NMR (400 MHz, DMSO-$d_6$) δ(ppm): 9.27 (d, *J* = 2.2 Hz, 1H), 8.64 (dd, *J* = 8.3, 2.2 Hz, 1H), 8.28 (s, 1H), 8.17 (d, *J* = 8.3 Hz, 1H), 7.97 (dd, *J* = 8.8, 1.3 Hz, 1H), 7.57 (dd, *J* = 8.9, 7.0 Hz, 1H), 7.41 (dd, *J* = 7.0, 1.3 Hz, 1H), 3.63 (t, *J* = 5.4 Hz, 4H), 1.66 (s, 2H), 1.57 (s, 4H). | MS (ESI, LR) Calc. for $C_{20}H_{19}N_6O_3$ $[M+H]^+$: 391.1, found: 391.1. |
| **247** | 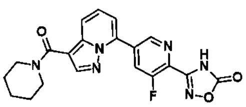 | $^1$H NMR (400 MHz, DMSO-$d_6$) δ(ppm): 13.36 (s, 1H),9.19 (s, 1H), 8.70 (d, *J* = 11.5 Hz, 1H), 8.31 (s, 1H), 8.00 (dd, *J* = 8.9, 1.3 Hz, 1H), 7.59 (dd, *J* = 8.9, 7.1 Hz, 1H), 7.50 (d, *J* = 7.1 Hz, 1H), 3.63 (t, *J* = 5.3 Hz, 4H), 1.66 (d, *J* = 6.0 Hz, 2H), 1.57 (q, *J* = 5.4 Hz, 4H). | MS (ESI, LR) Calc. for $C_{20}H_{18}FN_6O_3$ $[M+H]^+$: 409.1, found: 409.1. |
| **275** | 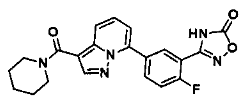 | $^1$H NMR (400 MHz, DMSO-$d_6$) δ(ppm): 12.97 (s, 1H), 8.36 (dd, *J* = 6.8, 2.3 Hz, 1H), 8.33-8.24 (m, 2H), 7.93 (dd, *J* = 8.9, 1.3 Hz, 1H), 7.69 (dd, *J* = 10.4, 8.8 Hz, 1H), 7.55 (dd, *J* = 8.9, 7.0 Hz, 1H), 7.27 (dd, *J* = 7.1, 1.4 Hz, 1H), 3.63 (t, *J* = 5.5 Hz, 4H), 1.66 (q, *J* = 5.7 Hz, 2H), 1.57 (q, *J* = 5.7 Hz, 4H). | MS (ESI, LR) Calc. for $C_{21}H_{19}FN_3O_3$ $[M+H]^+$: 408.1, found: 408.1. |
| **295** | 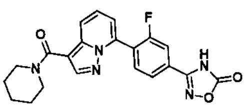 | $^1$H NMR (400 MHz, DMSO-$d_6$) δ(ppm): 13.19 (s, 1H), 8.21 (s, 1H), 7.98 (dd, *J* = 8.9, 1.4 Hz, 1H), 7.92 (t, *J* = 7.5 Hz, 1H), 7.88-7.77 (m, 2H), 7.55 (dd, *J* = 9.0, 7.0 Hz, 1H), 7.24 (dd, *J* = 7.0, 1.4 Hz, 1H), 3.62 (t, *J* = 5.4 Hz, 4H), 1.65 (d, *J* = 5.4 Hz, 2H), 1.57 (d, *J* = 7.0 Hz, 4H). | MS (ESI, LR) Calc. for $C_{21}H_{19}FN_5O_3$ $[M+H]^+$: 408.1, found: 408.1. |
| **297** | 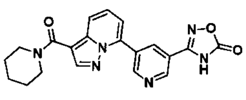 | $^1$H NMR (400 MHz, DMSO-$d_6$) δ(ppm): 9.14 (d, *J* = 2.2 Hz, 1H), 9.07 (d, *J* = 2.0 Hz, 1H), 8.74 (t, *J* = 2.1 Hz, 1H), 8.28 (s, 1H), 7.95 (dd, *J* = 8.9, 1.4 Hz, 1H), 7.55 (dd, *J* = 8.9, 7.0 Hz, 1H), 7.36 (dd, *J* = 6.9, 1.4 Hz, 1H), 3.63 (t, *J* = 5.5 Hz, 4H), 1.69-1.62 (m, 2H), 1.58 (d, *J* = 7.3 Hz, 4H). | MS (ESI, LR) Calc. for $C_{20}H_{19}N_6O_3$ $[M+H]^+$: 391.1, found: 391.1. |
| **299** | 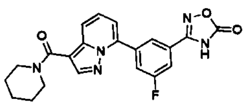 | $^1$H NMR (400 MHz, DMSO-$d_6$) δ(ppm): 8.28 (s, 1H), 8.23 (t, *J* = 1.5 Hz, 1H), 8.04-7.99 (m, 1H), 7.94 (dd, *J* = 9.0, 1.3 Hz, 1H), 7.74 (dd, *J* = 9.1, 2.3 Hz, 1H), 7.55 (dd, *J* = 8.9, 7.0 Hz, 1H), 7.31 (dd, *J* = 7.2, 1.4 Hz, 1H), 3.63 (t, *J* = 5.4 Hz, 4H), 1.66 (d, *J* = 5.7 Hz, 2H), 1.58 (d, *J* = 4.6 Hz, 4H). | MS (ESI, LR) Calc. for $C_{21}H_{19}FN_5O_3$ $[M+H]^+$: 408.1, found: 408.1. |

(continued)

| Example | Structure | $^1$H NMR | MS |
|---|---|---|---|
| **304** | 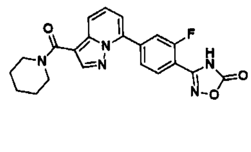 | $^1$H NMR (400 MHz, DMSO-$d_6$) δ(ppm): 13.01 (s, 1H), 8.28 (s, 1H), 8.15 (dd, *J* = 12.0, 1.6 Hz, 1H), 8.01 (dd, *J* = 8.2, 1.6 Hz, 1H), 7.96 (dt, *J* = 7.9, 3.3 Hz, 2H), 7.55 (dd, *J* = 8.9, 7.1 Hz, 1H), 7.41-7.33 (m, 1H), 3.63 (t, *J* = 5.4 Hz, 4H), 1.71-1.62 (m, 2H), 1.58 (q, *J* = 5.6 Hz, 4H). | MS (ESI, LR) Calc. for $C_{21}H_{19}FN_3O_3$ $[M+H]^+$: 408.1, found: 408.1. |
| **321** | 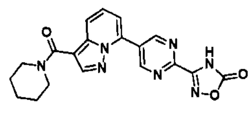 | $^1$H NMR (400 MHz, DMSO-$d_6$) δ(ppm): 13.43 (s, 1H), 9.62 (s, 2H), 8.32 (s, 1H), 8.01 (dd, *J* = 8.8, 1.4 Hz, 1H), 7.60 (dd, *J* = 8.9, 7.0 Hz, 1H), 7.57-7.50 (m, 1H), 3.63 (t, *J* = 5.3 Hz, 4H), 1.75-1.64 (m, 2H), 1.57 (d, *J* = 7.9 Hz, 4H). | MS (ESI, LR) Calc. for $C_{19}H_{18}N_7O_3$ $[M+H]^+$: 392.1, found: 392.1. |
| **329** | 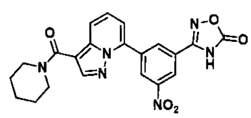 | $^1$H NMR (400 MHz, DMSO-$d_6$) δ(ppm): 9.09 (t, *J* = 1.9 Hz, 1H), 8.79 (t, *J* = 1.6 Hz, 1H), 8.77 (t, *J* = 1.8 Hz, 1H), 8.33 (s, 1H), 8.00 (dd, *J* = 8.9, 1.3 Hz, 1H), 7.59 (dd, *J* = 8.9, 7.1 Hz, 1H), 7.45 (dd, *J* = 7.1, 1.4 Hz, 1H), 3.64 (t, *J* = 5.4 Hz, 4H), 1.66 (d, *J* = 7.4 Hz, 2H), 1.58 (d, *J* = 7.3 Hz, 4H). | MS (ESI, LR) Calc. for $C_{21}H_{19}N_6O_5$ $[M+H]^+$: 435.1, found: 435.1. |
| **331** | 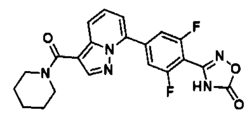 | $^1$H NMR (400 MHz, DMSO-$d_6$) δ(ppm): 13.10 (s, 1H), 8.30 (s, 1H), 8.08 (d, *J* = 1.6 Hz, 1H), 8.06 (s, 1H), 7.98 (dd, *J* = 9.0, 1.3 Hz, 1H), 7.56 (dd, *J* = 8.9, 7.1 Hz, 1H), 7.43 (dd, *J* =7.1, 1.4 Hz, 1H), 3.63 (t, *J* = 5.4 Hz, 4H), 1.66 (d, *J* = 5.7 Hz, 2H), 1.57 (d, *J* = 4.7 Hz, 4H). | MS (ESI, LR) Calc. for $C_{21}H_{18}F_2N_5O_3$ $[M+H]^+$: 426.1, found: 426.1. |

**Example 118. (7-(4-(5-methyl-1,2,4-oxadiazol-3-yl)phenyl)pyrazolo[1,5-*a*]pyridin-3-yl)(piperidin-1-yl)methanone**

<Scheme 21>

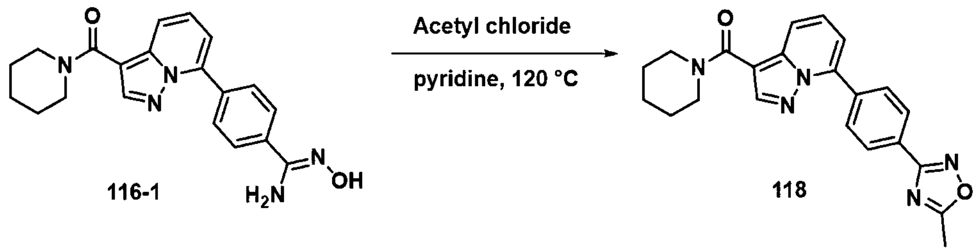


116-1 118

After dissolving compound **116-1** (0.014 g, 0.038 mmol) in pyridine (2 mL), acetyl chloride (5.5 μL, 47 mg, 0.077 mmol) was added and stirred at 120 °C for 24 hours. After completion of the reaction, the reaction solution was diluted with ethyl acetate (10 mL) and washed with water (10 mL x 2) and brine (10 mL x 2). The organic layer was dried over anhydrous magnesium sulfate, filtered, concentrated, and separated using column chromatography to obtain the target compound **118** (5.4 mg, 36%).

$^1$H NMR (400 MHz, DMSO-$d_6$) δ(ppm): 8.27 (s, 1H), 8.20-8.11 (m, 4H), 7.92 (dd, *J* = 8.8, 1.3 Hz, 1H), 7.54 (dd, *J* = 8.9, 7.0 Hz, 1H), 7.28 (d, *J* = 7.0 Hz, 1H), 3.63 (t, *J* = 5.5 Hz, 4H), 2.71 (s, 3H), 1.65 (s, 2H), 1.57 (s, 4H).

MS (ESI, LR) Calc. for $C_{22}H_{22}N_5O_2$ $[M+H]^+$: 388.2, found: 388.1.

**Example 263. [7-[5-fluoro-6-(5-methyl-1,2,4-oxadiazol-3-yl)-3-pyridyl]pyrazolo[1,5-*a*]pyridin-3-yl]-(1-piperidyl) methanone**

**Example 276. [7-[4-fluoro-3-(5-methyl-1,2,4-oxadiazol-3-yl)phenyl]pyrazolo[1,5-*a*]pyridin-3-yl]-(1-piperidyl) methanone**

**Example 296. [7-[2-fluoro-4-(5-methyl-1,2,4-oxadiazol-3-yl)phenyl]pyrazolo[1,5-*a*]pyridin-3-yl]-(1-piperidyl) methanone**

**Example 303. [7-[3-fluoro-4-(5-methyl-1,2,4-oxadiazol-3-yl)phenyl]pyrazolo[1,5-*a*]pyridin-3-yl]-(1-piperidyl) methanone**

**Example 317. [7-[2-(5-methyl-1,2,4-oxadiazol-3-yl)pyrimidin-5-yl]pyrazolo[1,5-*a*]pyridin-3-yl]-(1-piperidyl) methanone**

**Example 319. (7-(5-(5-methyl-1,2,4-oxadiazol-3-yl)pyridin-3-yl)pyrazolo[1,5-*a*]pyridin-3-yl)(piperidin-1-yl) methanone**

**Example 322. (7-(3-fluoro-5-(5-methyl-1,2,4-oxadiazol-3-yl)phenyl)pyrazolo[1,5-*a*]pyridin-3-yl)(piperidin-1-yl) methanone**

**Example 328. (7-(3,5-difluoro-4-(5-methyl-1,2,4-oxadiazol-3-yl)phenyl)pyrazolo[1,5-*a*]pyridin-3-yl)(piperidin-1-yl)methanone**

**Example 330. (7-(3-(5-methyl-1,2,4-oxadiazol-3-yl)-5-nitrophenyl)pyrazolo[1,5-*a*]pyridin-3-yl)(piperidin-1-yl) methanone**

For Examples 263, 276, 296, 303, 317, 319, 322, 328, and 330, the compounds were obtained by sequentially using the same synthetic method as step 159-3 of Example 159, the synthetic method in step 115-1 of Example 115, and the synthetic method of Example 118 using compound **1-2** and the corresponding compounds. The structural and spectral data are shown in Table 21 below.

[Table 21]

| Example | Structure | $^1H$ NMR | MS |
|---|---|---|---|
| **263** | 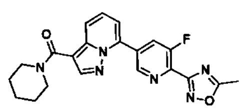 | $^1H$ NMR (400 MHz, DMSO-$d_6$) δ(ppm): 9.20 (t, *J* = 1.6 Hz, 1H), 8.70 (dd, *J* = 11.5, 1.8 Hz, 1H), 8.32 (s, 1H), 8.00 (dd, *J* = 8.9, 1.4 Hz, 1H), 7.59 (dd, *J* = 8.8, 7.1 Hz, 1H), 7.51 (dd, *J* = 7.1, 1.4 Hz, 1H), 3.63 (t, *J* = 5.4 Hz, 4H), 2.75 (s, 3H), 1.66 (d, *J* = 5.6 Hz, 2H), 1.58 (d, *J* = 7.5 Hz, 4H). | MS (ESI, LR) Calc. for $C_{21}H_{20}FN_6O_2$ $[M+H]^+$: 407.2, found: 407.1. |
| **276** | 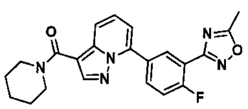 | $^1H$ NMR (400 MHz, DMSO-$d_6$) δ(ppm): 8.62 (dd, *J* = 6.8, 2.4 Hz, 1H), 8.28 (s, 1H), 8.19 (ddd, *J* = 8.7, 4.8, 2.4 Hz, 1H), 7.92 (dd, *J* = 8.9, 1.3 Hz, 1H), 7.66 (dd, *J* = 10.5, 8.7 Hz, 1H), 7.54 (dd, *J* = 8.9, 7.0 Hz, 1H), 7.27 (dd, *J* = 7.1, 1.4 Hz, 1H), 3.63 (t, *J* = 5.4 Hz, 4H), 2.71 (s, 3H), 1.66 (d, *J*= 5.6 Hz, 2H), 1.57 (d, *J* = 7.4 Hz, 4H). | MS (ESI, LR) Calc. for $C_{22}H_{21}FN_3O_2$ $[M+H]^+$: 406.2, found: 406.1. |
| **296** | 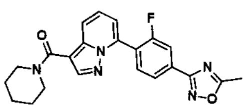 | $^1H$ NMR (400 MHz, DMSO-$d_6$) δ(ppm): 8.22 (s, 1H), 8.06-7.86 (m, 4H), 7.55 (dd, *J* = 8.9, 7.0 Hz, 1H), 7.24 (dd, *J* = 7.0, 1.4 Hz, 1H), 3.63 (t, *J* = 5.4 Hz, 4H), 2.73 (s, 3H), 1.65 (d, *J* = 7.0 Hz, 2H), 1.62-1.51 (m, 4H). | MS (ESI, LR) Calc. for $C_{22}H_{21}FN_3O_2$ $[M+H]^+$: 406.2, found: 406.1. |
| **303** | 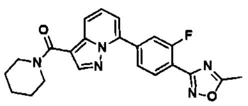 | $^1H$ NMR (400 MHz, DMSO-$d_6$) δ(ppm): 8.30 (s, 1H), 8.18 (t, *J* = 7.8 Hz, 1H), 8.13 (d, *J* = 12.0 Hz, 1H), 8.01 (d, *J* = 8.2 Hz, 1H), 7.95 (d, *J* = 8.9 Hz, 1H), 7.55 (t, *J* = 8.0 Hz, 1H), 7.37 (d, *J* = 7.1 Hz, 1H), 3.63 (t, *J* = 5.4 Hz, 4H), 2.80-2.67 (m, 3H), 1.66 (d, *J* = 6.6 Hz, 2H), 1.58 (d, *J* = 7.2 Hz, 4H). | MS (ESI, LR) Calc. for $C_{22}H_{21}FN_3O_2$ $[M+H]^+$: 406.2, found: 406.1. |

(continued)

| Example | Structure | $^1$H NMR | MS |
|---|---|---|---|
| **317** | 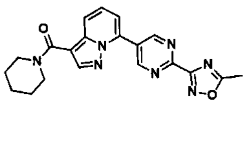 | $^1$H NMR (400 MHz, DMSO-$d_6$) δ(ppm): 9.53 (s, 2H), 8.11 (dd, *J* = 9.0, 1.3 Hz, 1H), 8.09 (s, 1H), 7.43 (dd, *J* = 9.0, 7.0 Hz, 1H), 7.15-7.09 (m, 1H), 3.73 (t, *J* = 5.3 Hz, 4H), 2.77 (s, 3H), 1.79-1.72 (m, 2H), 1.72-1.65 (m, 4H). | MS (ESI, LR) Calc. for $C_{20}H_{20}N_7O_2$ $[M+H]^+$: 390.2, found: 390.1. |
| **319** | 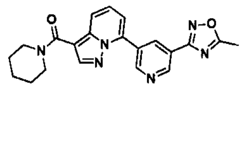 | $^1$H NMR (400 MHz, DMSO-$d_6$) δ(ppm): 9.27 (dd, *J* = 8.0, 2.1 Hz, 2H), 9.00 (t, *J* = 2.1 Hz, 1H), 8.30 (s, 1H), 7.97 (dd, *J* = 8.9, 1.3 Hz, 1H), 7.57 (dd, *J* = 8.9, 7.0 Hz, 1H), 7.43 (dd, *J* = 7.0, 1.4 Hz, 1H), 3.64 (t, *J* = 5.4 Hz, 4H), 2.73 (s, 3H), 1.66 (d, *J* = 5.7 Hz, 2H), 1.63-1.55 (m, 4H). | MS (ESI, LR) Calc. for $C_{21}H_{21}N_6O_2$ $[M+H]^+$: 389.2, found: 389.1. |
| **322** | 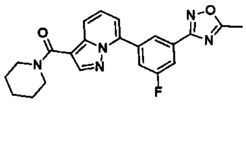 | $^1$H NMR (400 MHz, DMSO-$d_6$) δ(ppm): 8.46 (t, *J* = 1.5 Hz, 1H), 8.30 (s, 1H), 8.07 (dt, *J* = 9.6, 2.1 Hz, 1H), 7.97-7.89 (m, 2H), 7.55 (dd, *J* = 8.9, 7.1 Hz, 1H), 7.36 (dd, *J* = 7.1, 1.4 Hz, 1H), 3.64 (t, *J* = 5.4 Hz, 4H), 2.71 (s, 3H), 1.66 (d, *J* = 5.0 Hz, 2H), 1.58 (s, 4H). | MS (ESI, LR) Calc. for $C_{22}H_{21}FN_3O_2$ $[M+H]^+$: 406.2, found: 406.1. |
| **328** | 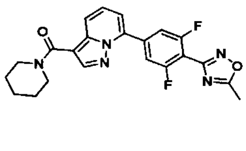 | $^1$H NMR (400 MHz, DMSO-$d_6$) δ(ppm): 8.32 (s, 1H), 8.04 (d, *J* = 9.6 Hz, 2H), 7.98 (dd, *J* = 8.9, 1.4 Hz, 1H), 7.59-7.54 (m, 1H), 7.44 (d, *J* = 7.0 Hz, 1H), 3.64 (d, *J* = 5.7 Hz, 4H), 2.75 (s, 3H), 1.66 (s, 2H), 1.58 (s, 4H). | MS (ESI, LR) Calc. for $C_{22}H_{20}F_2N_5O_2$ $[M+H]^+$: 424.2, found: 424.1. |
| **330** | 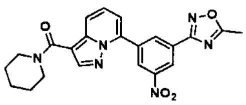 | $^1$H NMR (400 MHz, DMSO-$d_6$) δ(ppm): 9.01 (t, *J* = 1.9 Hz, 1H), 8.98 (t, *J* = 1.6 Hz, 1H), 8.82 (t, *J* = 1.9 Hz, 1H), 8.33 (s, 1H), 7.99 (dd, *J* = 8.9, 1.4 Hz, 1H), 7.58 (dd, *J* = 8.9, 7.1 Hz, 1H), 7.48 (dd, *J* = 7.1, 1.4 Hz, 1H), 3.64 (t, *J* = 5.4 Hz, 4H), 2.75 (s, 3H), 1.66 (d, *J* = 7.3 Hz, 2H), 1.58 (s, 4H). | MS (ESI, LR) Calc. for $C_{22}H_{21}N_6O_4$ $[M+H]^+$: 433.2, found: 433.1. |

**Example 352. [7-[4-(1,2,4-oxadiazol-3-yl)phenyl]pyrazolo[1,5-*a*]pyridin-3-yl]-(1-piperidyl)methanone**

<Scheme 22>

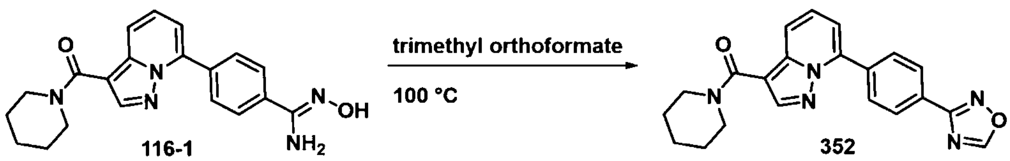


Compound **116-1** (50 mg, 0.14 mmol) was added to trimethyl orthoformate (0.13 mL, 0.78 mmol) and stirred at 100 °C for 16 hours. The reaction solution was concentrated and separated using column chromatography to obtain the target compound **352** (35 mg, 65%).

$^1$H NMR (400 MHz, DMSO-$d_6$) δ(ppm): 9.78 (s, 1H), 8.27 (s, 1H), 8.22 (d, *J* = 8.5 Hz, 2H), 8.17 (d, *J* = 8.5 Hz, 2H), 7.93 (dd, *J* = 8.8, 1.3 Hz, 1H), 7.55 (dd, *J* = 8.9, 7.0 Hz, 1H), 7.28 (dd, *J* = 7.1, 1.4 Hz, 1H), 3.63 (t, *J* = 5.4 Hz, 4H), 1.66 (d, *J* = 5.2 Hz, 2H), 1.58 (s, 4H).

MS (ESI, LR) Calc. for $C_{21}H_{20}N_5O_2$ $[M+H]^+$: 374.2, found: 374.1.

**Example 341. (7-(3-fluoro-5-(1,2,4-oxadiazol-3-yl)phenyl)pyrazolo[1,5-*a*]pyridin-3-yl)(piperidin-1-yl)methanone**

**Example 342. (7-(3-nitro-5-(1,2,4-oxadiazol-3-yl)phenyl)pyrazolo[1,5-*a*]pyridin-3-yl)(piperidin-1-yl)methanone**

**Example 374. (7-(5-(1,2,4-oxadiazol-3-yl)pyridin-3-yl)pyrazolo[1,5-*a*]pyridin-3-yl)(piperidin-1-yl)methanone**

For Examples 341, 342, and 374, the compounds were obtained by sequentially using the same synthetic method in step 159-3 of Example 159, the synthetic method in step 115-1 of Example 115 and the synthetic method of 352 using compound **1-2** and the corresponding compounds. The structural and spectral data are shown in Table 22 below.

[Table 22]

| Example | Structure | $^1$H NMR | MS |
|---|---|---|---|
| **341** | 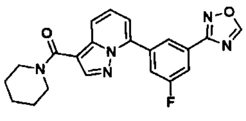 | $^1$H NMR (400 MHz, DMSO-$d_6$) δ(ppm): 9.81 (s, 1H), 8.51 (t, *J* = 1.5 Hz, 1H), 8.29 (s, 1H), 8.09 (dt, *J* = 9.8, 2.2 Hz, 1H), 7.97 (ddd, *J* = 9.0, 7.9, 1.8 Hz, 2H), 7.55 (dd, *J* = 8.9, 7.1 Hz, 1H), 7.37 (dd, *J* = 7.1, 1.4 Hz, 1H), 3.64 (t, *J* = 5.4 Hz, 4H), 1.66 (d, *J* = 5.5 Hz, 2H), 1.58 (d, *J* = 7.4 Hz, 4H). | MS (ESI, LR) Calc. for $C_{21}H_{19}FN_3O_2$ $[M+H]^+$: 392.1, found: 392.1. |
| **342** | 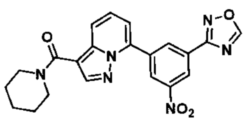 | $^1$H NMR (400 MHz, DMSO-$d_6$) δ(ppm): 9.89 (s, 1H), 9.03 (p, *J* = 1.7 Hz, 2H), 8.86 (t, *J* = 1.9 Hz, 1H), 8.32 (s, 1H), 8.00 (dd, *J* = 8.9, 1.4 Hz, 1H), 7.59 (dd, *J* = 8.9, 7.0 Hz, 1H), 7.49 (dd, *J* = 7.1, 1.4 Hz, 1H), 3.65 (t, *J* = 5.4 Hz, 4H), 1.67 (d, *J* = 5.6 Hz, 2H), 1.58 (d, *J* = 7.7 Hz, 4H). | MS (ESI, LR) Calc. for $C_{21}H_{19}N_6O_4$ $[M+H]^+$: 419.1, found: 419.0. |
| **374** | 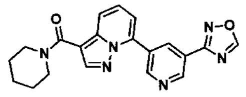 | $^1$H NMR (400 MHz, DMSO-$d_6$) δ(ppm): 9.86 (s, 1H), 9.33 (d, *J* = 2.0 Hz, 1H), 9.28 (d, *J* = 2.2 Hz, 1H), 9.05 (t, *J* = 2.1 Hz, 1H), 8.30 (s, 1H), 7.97 (dd, *J* = 8.9, 1.3 Hz, 1H), 7.57 (dd, *J* = 8.9, 7.1 Hz, 1H), 7.44 (dd, *J* = 7.0, 1.4 Hz, 1H), 3.64 (t, *J* = 5.4 Hz, 4H), 1.66 (d, *J* = 5.5 Hz, 2H), 1.58 (d, *J* = 7.2 Hz, 4H). | MS (ESI, LR) Calc. for $C_{20}H_{19}N_6O_2$ $[M+H]^+$: 375.1, found: 375.1. |

**Example 361. 1-piperidyl-[7-[4-[5-(trifluoromethyl)-1,2,4-oxadiazol-3-yl]phenyl]pyrazolo[1,5-*a*]pyridin-3-yl] methanone**

<Scheme 23>

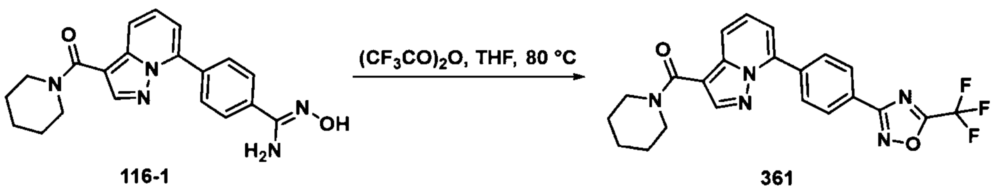


116-1 361

Compound **116-1** (0.040 g, 0.11 mmol) was dissolved in tetrahydrofuran (2 mL), trifluoroacetic anhydride (15.3 μL, 23 mg, 0.11 mmol) was added, and the mixture was stirred at 80 °C for 16 hours. After completion of the reaction, the reaction solution was diluted with dichloromethane (10 mL) and washed with water (10 mL x 2) and brine (10 mL x 2). The organic layer was dried over anhydrous magnesium sulfate, filtered, concentrated, and separated using column chromatography to obtain the target compound 361 (45 mg, 92%).

$^1$H NMR (400 MHz, DMSO-$d_6$) δ(ppm): 8.28 (s, 1H), 8.27-8.19 (m, 4H), 7.94 (dd, *J* = 8.9, 1.3 Hz, 1H), 7.56 (dd, *J* = 8.9, 7.1 Hz, 1H), 7.31 (dd, *J* = 7.0, 1.3 Hz, 1H), 3.63 (t, *J* = 5.4 Hz, 4H), 1.66 (d, *J* = 5.0 Hz, 2H), 1.62-1.51 (m, 4H).

MS (ESI, LR) Calc. for $C_{22}H_{19}F_3N_5O_2$ $[M+H]^+$: 442.1, found: 442.1.

**Example 359. [7-[4-(5-amino-1,2,4-oxadiazol-3-yl)phenyl]pyrazolo[1,5-*a*]pyridin-3-yl]-(1-piperidyl)methanone**

<Scheme 24>

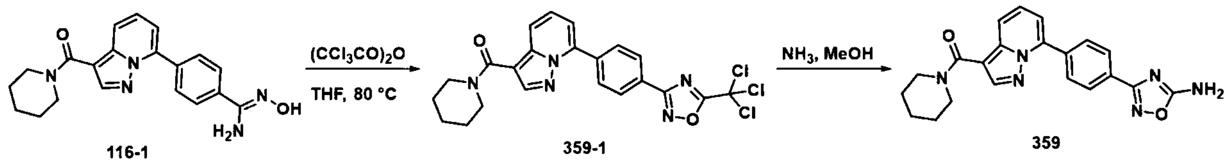


116-1 359-1 359

Step 359-1: The target compound **359-1** was obtained by the synthetic method in Example 361 using compound 116-1 and trichloroacetic anhydride.

Step 359-2: To compound 359-1 (130 mg, 0.26 mmol) was added 7 N ammonia solution in methanol (1.6 mL, 11.2 mmol), and stirred at room temperature for 16 hours. After concentrating the reaction solution, it was separated using column chromatography to obtain target compound 359 (92 mg, 89%).

$^1H$ NMR (400 MHz, DMSO-$d_6$) δ(ppm): 8.26 (s, 1H), 8.09 (d, $J$ = 8.6 Hz, 2H), 8.04 (d, $J$ = 8.6 Hz, 2H), 8.01 (s, 2H), 7.91 (dd, $J$ = 8.9, 1.3 Hz, 1H), 7.54 (dd, $J$ = 8.9, 7.1 Hz, 1H), 7.26 (dd, $J$ = 7.1, 1.4 Hz, 1H), 3.63 (t, $J$ = 5.4 Hz, 4H), 1.65 (d, $J$ = 5.1 Hz, 2H), 1.57 (s, 4H).

MS (ESI, LR) Calc. for $C_{21}H_{21}N_6O_2$ $[M+H]^+$: 389.2, found: 389.1.

**Example 348. [7-[4-(5-methyl-1,3,4-oxadiazol-2-yl)phenyl]pyrazolo[1,5-*a*]pyridin-3-yl]-(1-piperidyl)methanone**

<Scheme 25>

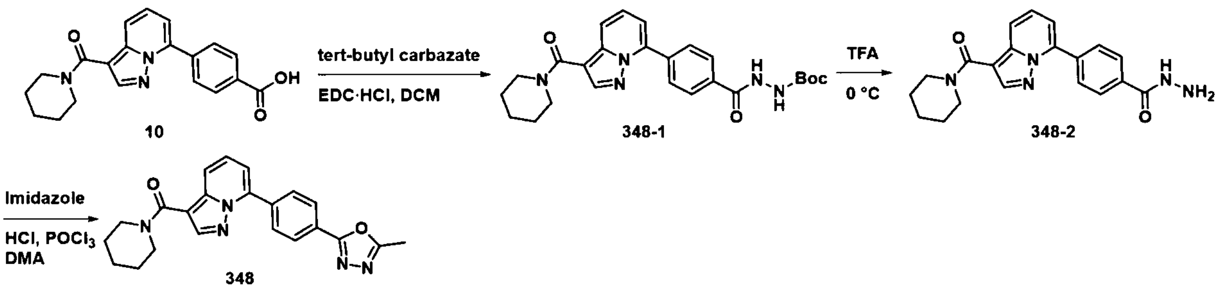


10 348-1 348-2 348

Step 348-1: Compound **10** (120 mg, 0.34 mmol) was dissolved in dichloromethane (4 mL), tertiary butyl carbazate (49.9 mg, 0.38 mmol) and EDC·HCl (72.4 mg, 0.38 mmol) were added, and stirred for 1 hour. After completion of the reaction, diluted with dichloromethane (10 mL), washed with aqueous sodium bicarbonate solution and brine, dried over anhydrous magnesium sulfate, filtered, and concentrated to obtain compound **348-1** (151 mg, 95%).

Step 348-2: Compound **348-1** (220 g, 0.47 mmol) was placed in a reaction vessel and the temperature was lowered to 0 °C. Trifluoroacetic acid (1 mL, 13.1 mmol) was added and stirred for 1 hour. After completion of the reaction, it was concentrated, diluted with diethyl ether (10 mL), recrystallized, and filtered. The filtrate was dissolved in aqueous sodium bicarbonate solution and extracted with dichloromethane. Drying over anhydrous magnesium sulfate, filtering, and concentration were performed to obtain compound **348-2** (140 mg, 65%).

Step 348-3: N,N-dimethylformamide (2 mL) was added to the reaction vessel, 4 N hydrochloric acid dissolved in 1,4-dioxane (0.1 mL, 0.41 mmol) was added, and stirred for 5 minutes. Imidazole (28.1 mg, 0.41 mmol) was added, and stirred for 30 minutes, compound **348-2** (50 mg, 0.14 mmol) was added, and stirred at 100 °C for 16 hours. Phosphorus oxychloride (15.4 μL, 0.17 mmol) was added, and stirred for 12 hours. After completion of the reaction, the temperature was lowered to room temperature, diluted with brine (5 mL), and extracted with dichloromethane (5 mL x 3). The combined organic layers were dried over anhydrous magnesium sulfate, filtered, concentrated, and recrystallized using *N,N*-dimethylformamide, filtered, and dried to obtain the target Compound 348 (14 mg, 26%).

$^1H$ NMR (400 MHz, DMSO-$d_6$) δ(ppm): 8.27 (s, 1H), 8.23-8.10 (m, 4H), 7.93 (dd, $J$ = 8.9, 1.3 Hz, 1H), 7.58-7.51 (m, 1H), 7.29 (dd, $J$ = 7.0, 1.4 Hz, 1H), 3.63 (t, $J$ = 5.4 Hz, 4H), 2.63 (s, 3H), 1.66 (s, 2H), 1.62-1.53 (m, 4H).

MS (ESI, LR) Calc. for $C_{22}H_{22}N_5O_2$ $[M+H]^+$: 388.2, found: 388.1.

**Example 364. [7-[6-(5-methyl-1,3,4-oxadiazol-2-yl)-3-pyridyl]pyrazolo[1,5-*a*]pyridin-3-yl]-(1-piperidyl)methanone**

**Example 379. [7-[5-fluoro-6-(5-methyl-1,3,4-oxadiazol-2-yl)-3-pyridyl]pyrazolo[1,5-*a*]pyridin-3-yl]-(1-piperidyl) methanone**

For Examples 364 and 379, the compounds were obtained by sequentially using the same synthetic method as in step 159-3 of Example 159 and the same synthetic method as in Example 348 using the corresponding compounds. The structural and spectral data are shown in Table 23 below.

[Table 23]

| Example | Structure | $^1$H NMR | MS |
|---|---|---|---|
| **364** | 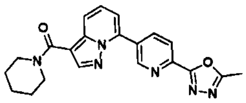 | $^1$H NMR (400 MHz, DMSO-$d_6$) δ(ppm): 9.32-9.27 (m, 1H), 8.68 (dd, *J* = 8.3, 2.2 Hz, 1H), 8.34 (dd, *J* = 8.2, 0.8 Hz, 1H), 8.30 (s, 1H), 7.97 (dd, *J* = 8.9, 1.3 Hz, 1H), 7.58 (dd, *J* = 8.9, 7.1 Hz, 1H), 7.42 (dd, *J* = 7.1, 1.4 Hz, 1H), 3.63 (t, *J* = 5.4 Hz, 4H), 2.66 (s, 3H), 1.66 (d, *J* = 5.3 Hz, 2H), 1.58 (d, *J* = 7.0 Hz, 4H). | MS (ESI, LR) Calc. for $C_{21}H_{21}N_6O_2$ $[M+H]^+$: 389.2, found: 389.1. |
| **379** | 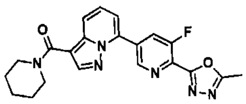 | $^1$H NMR (400 MHz, DMSO-$d_6$) δ(ppm): 9.22 (d, *J* = 1.6 Hz, 1H), 8.74 (dd, *J* = 11.6, 1.7 Hz, 1H), 8.33 (s, 1H), 8.00 (dd, *J* = 8.8, 1.4 Hz, 1H), 7.59 (dd, *J* = 8.8, 7.1 Hz, 1H), 7.51 (dd, *J* = 7.1, 1.5 Hz, 1H), 3.64 (t, *J* = 5.3 Hz, 4H), 2.68 (s, 3H), 1.66 (s, 2H), 1.58 (d, *J* = 7.1 Hz, 4H). | MS (ESI, LR) Calc. for $C_{21}H_{20}FN_6O_2$ $[M+H]^+$: 407.2, found: 407.1. |

**Example 350. [7-[4-(1,3,4-oxadiazol-2-yl)phenyl]pyrazolo[1,5-*a*]pyridin-3-yl]-(1-piperidyl)methanone**

**Example 380. [7-[5-fluoro-6-(1,3,4-oxadiazol-2-yl)-3-pyridyl]pyrazolo[1,5-*a*]pyridin-3-yl]-(1-piperidyl)methanone**

For Examples 350 and 380, the compounds were obtained by sequentially using the same synthetic method as in step 348-1, 348-2 of Example 348 and Example 352 using compounds **10** and **243.** The structural and spectral data are shown in Table 24 below.

[Table 24]

| Example | Structure | $^1$H NMR | MS |
|---|---|---|---|
| **350** | 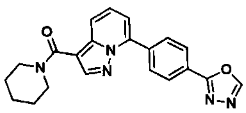 | $^1$H NMR (400 MHz, DMSO-$d_6$) δ(ppm): 9.42 (s, 1H), 8.27 (s, 1H), 8.24-8.17 (m, 4H), 7.94 (dd, *J* = 8.9, 1.3 Hz, 1H), 7.55 (dd, *J* = 8.9, 7.0 Hz, 1H), 7.30 (dd, *J* = 7.1, 1.4 Hz, 1H), 3.63 (t, *J* = 5.4 Hz, 4H), 1.66 (d, *J* = 5.6 Hz, 2H), 1.58 (s, 4H). | MS (ESI, LR) Calc. for $C_{21}H_{20}N_5O_2$ $[M+H]^+$: 374.2, found: 374.1. |
| **380** | 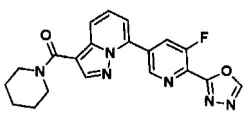 | $^1$H NMR (400 MHz, DMSO-$d_6$) δ(ppm): 9.56 (s, 1H), 9.25 (t, *J* = 1.5 Hz, 1H), 8.77 (dd, *J* = 11.6, 1.7 Hz, 1H), 8.33 (s, 1H), 8.01 (dd, *J* = 8.8, 1.4 Hz, 1H), 7.59 (dd, *J* = 8.8, 7.1 Hz, 1H), 7.52 (dd, *J* = 7.1, 1.4 Hz, 1H), 3.64 (t, *J* = 5.4 Hz, 4H), 1.66 (d, *J* = 5.7 Hz, 2H), 1.58 (d, *J* = 7.2 Hz, 4H). | MS (ESI, LR) Calc. for $C_{20}H_{18}FN_6O_2$ $[M+H]^+$: 393.1, found: 393.1. |

**Example 365. 1-piperidyl-[7-[6-(2-thioxo-3*H*-1,3,4-oxadiazol-5-yl)-3-pyridyl]pyrazolo[1,5-*a*]pyridin-3-yl]methanone**

<Scheme 26>

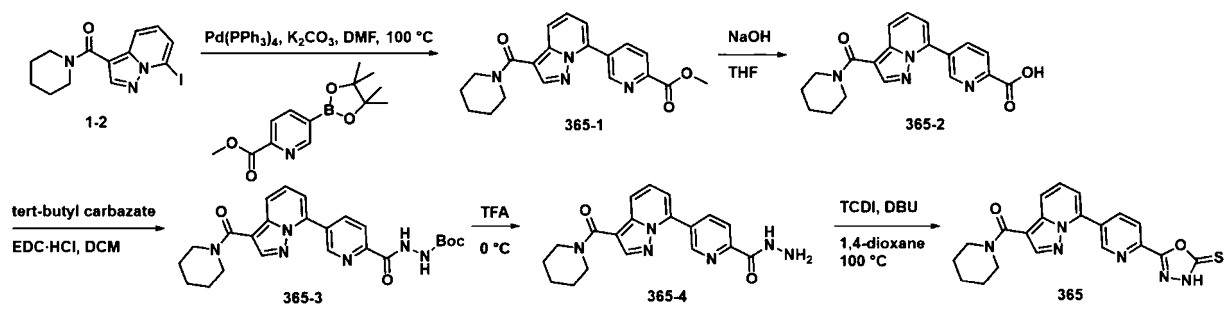


Step 365-1: Using compound 1-2, the target compound **365-1** was obtained by the same synthetic method as step 1-3 in Example 1.

Step 365-2: Using compound **365-1,** the target compound **365-2** was obtained by the same synthetic method as step 243-1 in Example 243.

Step 365-3: Using compound **365-2,** the target compound **365-3** was obtained by the same synthetic method as in step 348-1 in Example 348.

Step 365-4: Using compound **365-3,** target compound **365-4** was obtained by the same synthetic method as step 348-2 in Example 348.

Step 365-5: Compound 365-4 (70 mg, 0.15 mmol) was dissolved in 1,4-dioxane (2 mL), di(imidazol-1-yl) methanethione (39.5 mg, 0.22 mmol) and DBU (88 μL, 0.59 mmol) were added, and stirred at 100 °C for 16 hours. After completion of the reaction, the reaction solution was concentrated and separated using column chromatography to obtain the target compound **365** (57 mg, 95%).

$^1H$ NMR (400 MHz, DMSO-$d_6$) δ(ppm): 11.81 (s, 1H), 9.20 (d, $J$ = 2.2 Hz, 1H), 8.62 (dd, $J$ = 8.2, 2.2 Hz, 1H), 8.28 (s, 1H), 8.24 (d, $J$ = 8.2 Hz, 1H), 7.95 (dd, $J$ = 8.9, 1.3 Hz, 1H), 7.57 (dd, $J$ = 8.9, 7.0 Hz, 1H), 7.41 (dd, $J$ = 7.1, 1.4 Hz, 1H), 3.63 (t, $J$ = 5.5 Hz, 4H), 1.65 (d, $J$ = 5.3 Hz, 2H), 1.59 (d, $J$ = 4.7 Hz, 4H).

MS (ESI, LR) Calc. for $C_{20}H_{19}N_6O_2S$ $[M+H]^+$: 407.1, found: 407.1.

**Example 394. [7-[5-fluoro-6-[5-(trifluoromethyl)-1,3,4-oxadiazol-2-yl]-3-pyridyl]pyrazolo[1,5-*a*]pyridin-3-yl]-(1-piperidyl)methanone**

<Scheme 27>

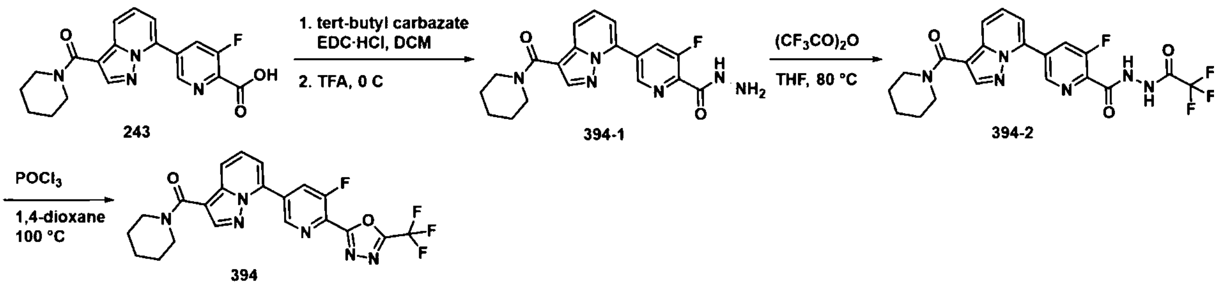


Step 394-1: Using compound **243,** the target compound **394-1** was obtained by the same synthetic method as in step 348-1 and 348-2 in Example 348.

Step 394-2: Using compound **394-1,** the target compound **394-2** was obtained by the same synthetic method as Example 361.

Step 394-3: Compound 394-2 (75 mg, 0.15 mmol) was dissolved in 1,4-dioxane (2 mL), phosphorus oxychloride (0.15 mL, 1.5 mmol) was added, and stirred at 100 °C for 16 hours. After completion of the reaction, the reaction solution was concentrated, diluted with dichloromethane (5 mL), and the pH of the solution was adjusted to 9 with aqueous sodium bicarbonate solution. After extraction with dichloromethane (5 mL x 3), the combined organic solution was washed with brine, dried over anhydrous magnesium sulfate, filtered, and concentrated. The residue was separated using column chromatography to obtain the target compound **394** (7 mg, 9.2%).

$^1$H NMR (400 MHz, DMSO-$d_6$) δ(ppm): 9.29 (t, *J* = 1.5 Hz, 1H), 8.83 (dd, *J* = 11.6, 1.7 Hz, 1H), 8.33 (s, 1H), 8.02 (dd, *J* = 8.8, 1.5 Hz, 1H), 7.60 (dd, *J* = 8.8, 7.1 Hz, 1H), 7.57-7.52 (m, 1H), 3.64 (t, *J* = 5.4 Hz, 4H), 1.66 (d, *J* = 6.1 Hz, 2H), 1.58 (d, *J* = 4.6 Hz, 4H).

MS (ESI, LR) Calc. for $C_{21}H_{17}F_4N_6O_2$ [M+H]$^+$: 461.1, found: 461.1.

**Example 349. 5-[4-[3-(piperidine-1-carbonyl)pyrazolo[1,5-*a*]pyridin-7-yl]phenyl]-3*H*-1,3,4-oxadiazol-2-one**

<Scheme 28>

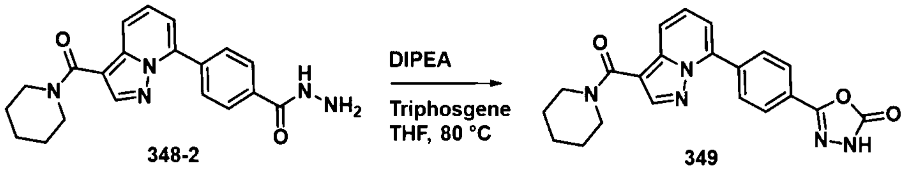


348-2

349

Compound 348-2 (50 mg, 0.14 mmol) was dissolved in tetrahydrofuran (2 mL), and DIPEA (48 μL, 0.28 mmol) and triphosgene (16.3 mg, 0.05 mmol) were added and stirred at 80 °C for 1 hour. After completion of the reaction, the reaction solution was concentrated and separated using column chromatography to obtain the target compound **349** (30 mg, 53%).

$^1$H NMR (400 MHz, DMSO-$d_6$) δ(ppm): 12.69 (s, 1H), 8.26 (s, 1H), 8.17-8.09 (m, 2H), 8.00-7.94 (m, 2H), 7.92 (dd, *J* = 8.9, 1.4 Hz, 1H), 7.54 (dd, *J* = 8.9, 7.0 Hz, 1H), 7.27 (dd, *J* = 7.1, 1.3 Hz, 1H), 3.63 (t, *J* = 5.4 Hz, 4H), 1.66 (q, *J* = 6.2 Hz, 2H), 1.57 (q, *J* = 5.7 Hz, 4H).

MS (ESI, LR) Calc. for $C_{21}H_{20}N_5O_3$ [M+H]$^+$: 390.1, found: 390.1.

**Example 360. 5-[5-[3-(piperidine-1-carbonyl)pyrazolo[1,5-*a*]pyridin-7-yl]-2-pyridyl]-3*H*-1,3,4-oxadiazol-2-one**

**Example 373. 5-[3-fluoro-5-[3-(piperidine-1-carbonyl)pyrazolo[1,5-*a*]pyridin-7-yl]-2-pyridyl]-3*H*-1,3,4-oxadiazol-2-one**

For Examples 360 and 373, the compounds were obtained by the same synthetic method as Example 349 using the corresponding compounds. The structural and spectral data are shown in Table 25 below.

[Table 25]

| Example | Structure | $^1$H NMR | MS |
|---|---|---|---|
| **360** | 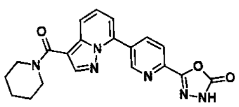 | $^1$H NMR (400 MHz, DMSO-$d_6$) δ(ppm): 12.88 (s, 1H), 9.24 (d, *J* = 2.2 Hz, 1H), 8.62 (dd, *J* = 8.3, 2.2 Hz, 1H), 8.29 (s, 1H), 8.10 (d, *J* = 8.3 Hz, 1H), 7.96 (dd, *J* = 8.9, 1.3 Hz, 1H), 7.57 (dd, *J* = 8.9, 7.1 Hz, 1H), 7.39 (dd, *J* = 7.1, 1.3 Hz, 1H), 3.63 (t, *J* = 5.4 Hz, 4H), 1.72-1.62 (m, 2H), 1.62-1.51 (m, 4H). | MS (ESI, LR) Calc. for $C_{20}H_{19}N_6O_3$ [M+H]$^+$: 391.1, found: 391.1. |
| **373** | 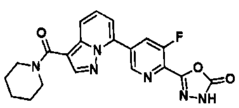 | $^1$H NMR (400 MHz, DMSO-$d_6$) δ(ppm): 12.98 (s, 1H), 9.16 (t, *J* = 1.6 Hz, 1H), 8.67 (dd, *J* = 11.9, 1.7 Hz, 1H), 8.32 (s, 1H), 7.99 (dd, *J* = 8.9, 1.4 Hz, 1H), 7.58 (dd, *J* = 8.8, 7.1 Hz, 1H), 7.49 (dd, *J* = 7.1, 1.4 Hz, 1H), 3.63 (t, *J* = 5.5 Hz, 4H), 1.71-1.63 (m, 2H), 1.58 (q, *J* = 5.7 Hz, 4H). | MS (ESI, LR) Calc. for $C_{20}H_{18}FN_6O_3$ [M+H]$^+$: 409.1, found: 409.1. |

**Example 300. [7-[4-(2-methylpyrazol-3-yl)phenyl]pyrazolo[1,5-*a*]pyridin-3-yl]-(1-piperidyl)methanone**

**Example 302. [7-[4-(3-furyl)phenyl]pyrazolo[1,5-*a*]pyridin-3-yl]-(1-piperidyl)methanone**

**Example 305. 5-[4-[3-(piperidine-1-carbonyl)pyrazolo[1,5-*a*]pyridin-7-yl]phenyl]pyrimidine-2-carbonitrile**

**Example 306. [7-[4-(2-hydroxypyrimidin-5-yl)phenyl]pyrazolo[1,5-*a*]pyridin-3-yl]-(1-piperidyl)methanone**

**Example 307. 5-[4-[3-(piperidine-1-carbonyl)pyrazolo[1,5-*a*]pyridin-7-yl]phenyl]pyrimidine-2-carboxamide**

**Example 324. [7-[4-(3,5-dimethylisoxazol-4-yl)phenyl]pyrazolo[1,5-*a*]pyridin-3-yl]-(1-piperidyl)methanone**

**Example 346. 1-piperidyl-[7-[4-(1*H*-pyrazol-4-yl)phenyl]pyrazolo[1,5-*a*]pyridin-3-yl]methanone**

**Example 347. 1-piperidyl-[7-[4-(3-thienyl)phenyl]pyrazolo[1,5-*a*]pyridin-3-yl]methanone**

For Examples 300, 302, 305, 306, 307, 324, 346, and 347, the compounds were obtained by using the same synthetic method as step 1-3 in Example 1 using compound 292 and the corresponding compounds. The structural and spectral data are shown in Table 26 below.

[Table 26]

| Example | Structure | $^1$H NMR | MS |
|---|---|---|---|
| **300** | 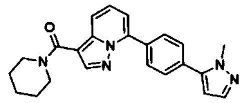 | $^1$H NMR (400 MHz, DMSO-$d_6$) δ(ppm): 8.26 (s, 1H), 8.07 (d, *J* = 8.0 Hz, 2H), 7.91 (dd, *J* = 8.9, 1.4 Hz, 1H), 7.74 (d, *J* = 8.1 Hz, 2H), 7.58-7.50 (m, 2H), 7.25 (d, *J* = 7.0 Hz, 1H), 6.53 (d, *J* = 1.9 Hz, 1H), 3.95 (s, 3H), 3.63 (t, *J* = 5.3 Hz, 4H), 1.66 (d, *J* = 4.9 Hz, 2H), 1.58 (d, *J* = 6.4 Hz, 4H). | MS (ESI, LR) Calc. for $C_{23}H_{24}N_5O$ $[M+H]^+$: 386.2, found: 386.1. |
| **302** | 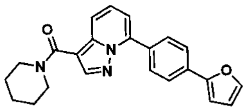 | $^1$H NMR (400 MHz, DMSO-$d_6$) δ(ppm): 8.34-8.31 (m, 1H), 8.25 (d, *J* = 5.8 Hz, 1H), 8.01-7.95 (m, 2H), 7.88 (dd, *J* = 8.9, 1.4 Hz, 1H), 7.83-7.78 (m, 3H), 7.52 (dd, *J* = 8.8, 7.0 Hz, 1H), 7.21 (dd, *J* = 7.1, 1.4 Hz, 1H), 7.09-7.05 (m, 1H), 3.63 (t, *J* = 5.4 Hz, 4H), 1.66 (s, 2H), 1.57 (d, *J* = 7.4 Hz, 4H). | MS (ESI, LR) Calc. for $C_{23}H_{22}N_3O_2$ $[M+H]^+$: 372.2, found: 372.1. |
| **305** | 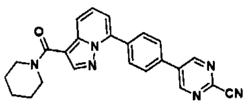 | $^1$H NMR (400 MHz, DMSO-$d_6$) δ(ppm): 9.51 (s, 2H), 8.26 (s, 1H), 8.16 (d, *J* = 8.4 Hz, 2H), 8.12 (d, *J* = 8.4 Hz, 2H), 7.92 (d, *J* = 8.8 Hz, 1H), 7.55 (dd, *J* = 8.9, 7.0 Hz, 1H), 7.28 (d, *J* = 7.0 Hz, 1H), 3.63 (t, *J* = 5.4 Hz, 4H), 1.66 (d, *J* = 5.3 Hz, 2H), 1.58 (d, *J* = 7.7 Hz, 4H). | MS (ESI, LR) Calc. for $C_{24}H_{21}N_6O$ $[M+H]^+$: 409.2, found: 409.1. |
| **306** | 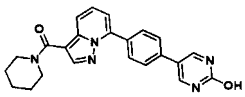 | $^1$H NMR (400 MHz, DMSO-$d_6$) δ(ppm): 12.29 (s, 1H), 8.75 (s, 2H), 8.24 (s, 1H), 8.02 (d, *J* = 8.4 Hz, 2H), 7.88 (d, *J* = 8.9 Hz, 1H), 7.83 (d, *J* = 8.2 Hz, 2H), 7.52 (dd, *J* = 8.9, 7.1 Hz, 1H), 7.22 (d, *J* = 7.0 Hz, 1H), 3.63 (t, *J* = 5.4 Hz, 4H), 1.66 (d, *J* = 5.1 Hz, 2H), 1.57 (d, *J* = 7.6 Hz, 4H). | MS (ESI, LR) Calc. for $C_{23}H_{22}N_5O_2$ $[M+H]^+$: 400.2, found: 400.2. |
| **307** | 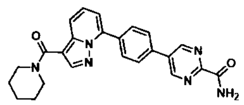 | $^1$H NMR (400 MHz, DMSO-$d_6$) δ(ppm): 9.38 (s, 2H), 8.26 (s, 2H), 8.14 (d, *J* = 8.1 Hz, 2H), 8.09 (d, *J* = 8.2 Hz, 2H), 7.96-7.89 (m, 1H), 7.85 (s, 1H), 7.55 (dd, *J* = 9.0, 7.1 Hz, 1H), 7.28 (d, *J* = 6.9 Hz, 1H), 3.64 (t, *J* = 5.4 Hz, 4H), 1.66 (s, 2H), 1.58 (s, 4H). | MS (ESI, LR) Calc. for $C_{24}H_{23}N_6O_2$ $[M+H]^+$: 427.2, found: 427.1. |
| **324** | 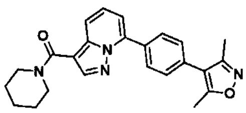 | $^1$H NMR (400 MHz, DMSO-$d_6$) δ(ppm): 8.25 (s, 1H), 8.04 (d, *J* = 8.2 Hz, 2H), 7.90 (dd, *J* = 8.9, 1.4 Hz, 1H), 7.59 (d, *J* = 8.1 Hz, 2H), 7.53 (dd, *J* = 8.9, 7.0 Hz, 1H), 7.27-7.21 (m, 1H), 3.63 (t, *J* = 5.4 Hz, 4H), 2.49 (s, 3H), 2.31 (s, 3H), 1.66 (d, *J* = 7.5 Hz, 2H), 1.57 (d, *J* = 7.5 Hz, 4H). | MS (ESI, LR) Calc. for $C_{24}H_{25}N_4O_2$ $[M+H]^+$: 401.2, found: 401.1. |

(continued)

| Example | Structure | $^1H$ NMR | MS |
|---|---|---|---|
| **346** | 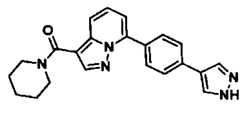 | $^1H$ NMR (400 MHz, DMSO-$d_6$) δ(ppm): 13.04 (s, 1H), 8.31 (s, 1H), 8.24 (s, 1H), 8.05 (s, 1H), 7.95 (d, *J* = 8.1 Hz, 2H), 7.87 (dd, *J* = 8.9, 1.4 Hz, 1H), 7.79 (d, *J* = 8.1 Hz, 2H), 7.51 (dd, *J* = 8.9, 7.0 Hz, 1H), 7.20 (dd, *J* = 7.0, 1.4 Hz, 1H), 3.63 (t, *J* = 5.4 Hz, 4H), 1.66 (s, 2H), 1.58 (d, *J* = 7.4 Hz, 4H). | MS (ESI, LR) Calc. for $C_{22}H_{22}N_5O$ $[M+H]^+$: 372.2, found: 372.1. |
| **347** | 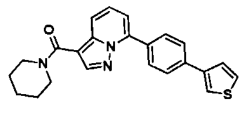 | $^1H$ NMR (400 MHz, DMSO-$d_6$) δ(ppm): 8.25 (s, 1H), 8.06-7.97 (m, 3H), 7.94-7.86 (m, 3H), 7.71 (dd, *J* = 5.0, 2.8 Hz, 1H), 7.67 (dd, *J* = 5.0, 1.5 Hz, 1H), 7.52 (dd, *J* = 8.9, 7.1 Hz, 1H), 7.23 (dd, *J* = 7.1, 1.4 Hz, 1H), 3.63 (t, *J* = 5.4 Hz, 4H), 1.66 (d, *J* = 5.5 Hz, 2H), 1.58 (d, *J* = 7.2 Hz, 4H). | MS (ESI, LR) Calc. for $C_{23}H_{22}N_3OS$ $[M+H]^+$: 388.1, found: 388.1. |

**Example 351. [7-[4-(5-methylthiazol-2-yl)phenyl]pyrazolo[1,5-*a*]pyridin-3-yl]-(1-piperidyl)methanone**

<Scheme 29 >

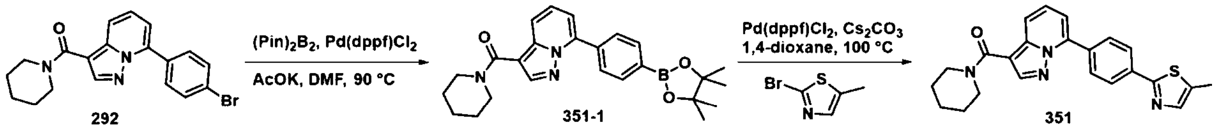


292 351-1 351

Step 351-1: Compound **292** (0.370 g, 0.96 mmol) was dissolved in *N,N*-dimethylformamide (10 mL), and bis(pinacolato)diboron (0.366 g, 1.44 mmol), [1,1'-bis(diphenylphosphino)ferrocene]dichloropalladium (0.040 g, 0.05 mmol), and potassium acetate (0.283 g, 2.89 mmol) were added. The reaction solution was bubbled with nitrogen for 10 minutes and stirred at 90 °C for 16 hours. After completion of the reaction, the temperature was lowered to room temperature, diluted with dichloromethane (10 mL), and palladium was removed using diatomaceous earth (Celite). The organic layer was washed with water and brine, dried over anhydrous magnesium sulfate, filtered, and concentrated. The residue was separated using column chromatography to obtain the target compound **351-1** (0.528 g, 57%).

Step 351-2: The target compound **351** was obtained by the same synthetic method as in step 159-3 of Example 159, using compound **351-1** and 2-bromo-5-methyl-thiazole.

$^1H$ NMR (400 MHz, DMSO-$d_6$) δ(ppm): 8.26 (s, 1H), 8.13-8.01 (m, 4H), 7.91 (dd, *J* = 8.8, 1.3 Hz, 1H), 7.69 (d, *J* = 1.5 Hz, 1H), 7.53 (dd, *J* = 8.9, 7.1 Hz, 1H), 7.26 (dd, *J* = 7.1, 1.4 Hz, 1H), 3.63 (t, *J* = 5.4 Hz, 4H), 1.66 (d, *J* = 6.8 Hz, 2H), 1.58 (d, *J* = 5.0 Hz, 4H).

MS (ESI, LR) Calc. for $C_{23}H_{23}N_4OS$ $[M+H]^+$: 403.2, found: 403.1.

**Example 358. 7-[4-(1,2-dimethylimidazol-4-yl)phenyl]pyrazolo[1,5-*a*]pyridin-3-yl]-(1-piperidyl)methanone**

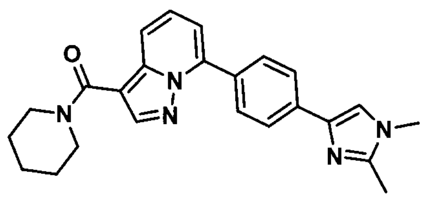

The target compound **358** was obtained by the same synthetic method as step 351-2 in Example 351 using compound **351-1** and 4-bromo-1,2-dimethyl-imidazole.

$^1H$ NMR (400 MHz, DMSO-$d_6$) δ(ppm): 8.07 (s, 1H), 7.93 (d, *J* = 8.8 Hz, 1H), 7.87 (s, 4H), 7.36 (dd, *J* = 8.9, 7.0 Hz, 1H), 7.17 (s, 1H), 6.97 (d, *J* = 7.1 Hz, 1H), 3.72 (t, *J* = 5.3 Hz, 4H), 3.62 (s, 3H), 2.45 (s, 3H), 1.72 (q, *J* = 5.6 Hz, 2H), 1.69-1.62

(m, 4H).

MS (ESI, LR) Calc. for $C_{24}H_{26}N_5O$ $[M+H]^+$: 400.2, found: 400.1.

**Example 343. (7-(3-amino-5-(5-methyl-1,2,4-oxadiazol-3-yl)phenyl)pyrazolo[1,5-*a*]pyridin-3-yl)(piperidin-1-yl) methanone**

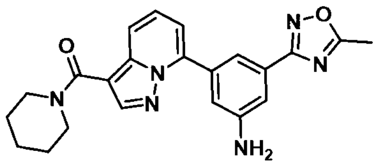

The target compound **343** was obtained by the same synthetic method as Example 7 using compound **330.**

$^1$H NMR (400 MHz, DMSO-$d_6$) δ(ppm): 8.24 (s, 1H), 7.88 (dd, *J* = 8.9, 1.3 Hz, 1H), 7.64 (t, *J* = 1.5 Hz, 1H), 7.50 (dd, *J* = 8.9, 7.0 Hz, 1H), 7.39 (t, *J* = 1.9 Hz, 1H), 7.25 (t, *J* = 1.9 Hz, 1H), 7.14 (dd, *J* = 7.1, 1.4 Hz, 1H), 5.64 (s, 2H), 3.63 (t, *J* = 5.4 Hz, 4H), 2.66 (s, 3H), 1.68-1.62 (m, 2H), 1.62-1.55 (m, 4H).

MS (ESI, LR) Calc. for $C_{22}H_{23}N_6O_2$ $[M+H]^+$: 403.2, found: 403.1.

**Example 119. (*Z*)-5-(3-(3-(piperidine-1-carbonyl)pyrazolo[1,5-*a*]pyridin-7-yl)benzylidene)thiazolidine-2,4-dione**

<Scheme 30>

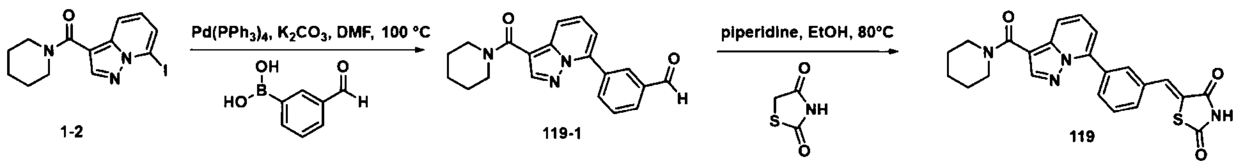


Step 119-1: Using compound **1-2** and 3-formylphenylboronic acid, the target compound **119-1** was obtained by the same synthetic method as step 1-3 in Example **1.**

Step 119-2: Compound **119-1** (0.15 g, 0.45 mmol) and thiazolidine-2,4-dione (0.063 g, 0.54 mmol) were dissolved in ethanol (5 mL), piperidine (35 μL, 0.36 mmol) was added, and the mixture was stirred at 80 °C for 16 hours. After completion of the reaction, the temperature was lowered to room temperature, diluted with water (5 mL), and acidified with 1 N citric acid (pH 4) to obtain a precipitated solid. The solid was filtered and washed with water, diethyl ether, and methyl *tert*-butyl ether successively to obtain the target compound **119** (50 mg, 25%).

$^1$H NMR (400 MHz, DMSO-$d_6$) δ(ppm): 8.09-8.04 (m, 2H), 8.01-7.83 (m, 3H), 7.67-7.64 (m, 2H), 7.45 (td, *J* = 8.7, 4.4 Hz, 1H), 7.06-6.96 (m, 1H), 3.76-3.72 (m, 4H), 1.76-1.69 (m, 6H).

MS (ESI, LR) Calc. for $C_{23}H_{21}N_4O_3S$ $[M+H]^+$: 433.1, found: 433.1.

**Example 253. 3-(3-(piperidine-1-carbonyl)pyrazolo[1,5-*a*]pyridin-7-yl)benzaldehyde oxime**

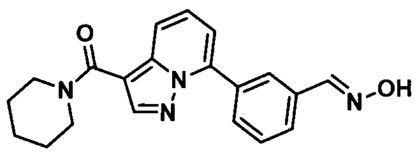

The target compound **253** was obtained by the synthetic method in step 236-2 of Example 236 using compound **119-1.**

$^1$H NMR (400 MHz, DMSO-$d_6$) δ(ppm): 11.35 (s, 1H), 8.16 (t, *J* = 1.8 Hz, 1H), 7.94-7.87 (m, 2H), 7.76 (dt, *J* = 7.9, 1.4 Hz, 1H), 7.66-7.48 (m, 4H), 7.20 (dd, *J* = 7.1, 1.4 Hz, 1H), 3.63 (t, *J* = 5.4 Hz, 4H), 1.65 (q, *J* = 5.7 Hz, 2H), 1.57 (q, *J* = 7.6

Hz, 4H).

MS (ESI, LR) Calc. for $C_{20}H_{21}N_4O_2$ $[M+H]^+$: 349.2, found: 349.1.

**Example 132. 2-methyl-7-(3-(piperidine-1-carbonyl)pyrazolo[1,5-*a*]pyridin-7-yl)-3,4-dihydroisoquinolin-1(2*H*)-one**

<Scheme 31>

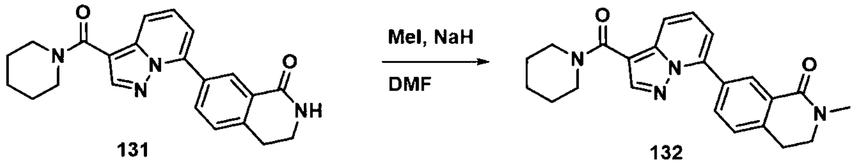


131 132

Compound **131** (30 mg, 0.08 mmol) was dissolved in N,N-dimethylformamide (3 mL), the temperature was lowered to 0 °C, and sodium hydride (60%, 8 mg, 0.2 mmol) was added. The reaction solution was stirred at room temperature for 15 minutes, methyl iodide (7.5 μL, 0.12 mmol) was added, and the mixture was stirred for 1 hour. After completion of the reaction, the temperature was lowered to 0 °C, and the reaction solution was diluted with water (10 mL), and extracted with dichloromethane (10 mL x 3). The combined organic layers were washed with brine, dried over anhydrous magnesium sulfate, filtered, and concentrated. The residue was separated using column chromatography to obtain the target compound **132** (13.8 mg, 44%).

$^1$H NMR (400 MHz, DMSO-$d_6$) δ(ppm): 8.41 (d, *J* = 2.0 Hz, 1H), 8.24 (s, 1H), 8.00 (dd, *J* = 7.9, 2.0 Hz, 1H), 7.89 (dd, *J* = 8.9, 1.4 Hz, 1H), 7.55-7.45 (m, 2H), 7.19 (dd, *J* = 7.0, 1.4 Hz, 1H), 3.63 (t, *J* = 5.5 Hz, 6H), 3.08 (d, *J* = 7.4 Hz, 5H), 1.69-1.62 (m, 2H), 1.62-1.55 (m, 4H).

MS (ESI, LR) Calc. for $C_{23}H_{25}N_4O_2$ $[M+H]^+$: 389.2, found: 389.2.

**Example 199. 6-methyl-3-(3-(piperidine-1-carbonyl)pyrazolo[1,5-*a*]pyridin-7-yl)-7,8-dihydro-1,6-naphthyridin-5(6*H*)-one**

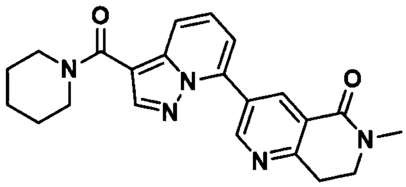

The target compound **199** was obtained by the synthetic method in Example 132 using compound **195** and the corresponding compounds.

$^1$H NMR (400 MHz, DMSO-$d_6$) δ(ppm): 9.09 (d, *J* = 2.3 Hz, 1H), 8.78 (d, *J* = 2.3 Hz, 1H), 8.27 (s, 1H), 7.93 (dd, *J* = 8.9, 1.4 Hz, 1H), 7.54 (dd, *J* = 8.9, 7.1 Hz, 1H), 7.33 (dd, *J* = 7.1, 1.3 Hz, 1H), 3.73 (t, *J* = 6.8 Hz, 2H), 3.63 (t, *J* = 5.4 Hz, 4H), 3.24 (t, *J* = 6.8 Hz, 2H), 3.09 (s, 3H), 1.66 (d, *J* = 6.1 Hz, 2H), 1.57 (d, *J* = 7.3 Hz, 4H).

MS (ESI, LR) Calc. for $C_{22}H_{24}N_5O_2$ $[M+H]^+$: 390.2, found: 390.1.

**Example 133. 2-isopropyl-7-(3-(piperidine-1-carbonyl)pyrazolo[1,5-*a*]pyridin-7-yl)-3,4-dihydroisoquinolin-1(2*H*)-one**

<Scheme 32>

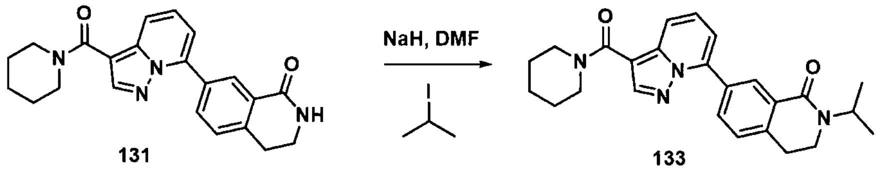


131

133

The target compound **133** was obtained by the synthetic method in Example **132** using compound **131** and isopropyl iodide.

$^1$H NMR (400 MHz, DMSO-d$_6$) δ(ppm): 8.41 (d, *J* = 2.0 Hz, 1H), 8.24 (s, 1H), 7.98 (dd, *J* = 7.9, 2.0 Hz, 1H), 7.88 (dd, *J* = 8.9, 1.3 Hz, 1H), 7.54-7.46 (m, 2H), 7.19 (dd, *J* = 7.0, 1.4 Hz, 1H), 4.89 (p, *J* = 6.8 Hz, 1H), 3.63 (t, *J* = 5.4 Hz, 4H), 3.50 (t, *J* = 6.5 Hz, 2H), 3.03 (t, *J* = 6.5 Hz, 2H), 1.65 (s, 2H), 1.57 (s, 4H), 1.17 (d, *J* = 6.8 Hz, 6H).

MS (ESI, LR) Calc. for $C_{25}H_{29}N_4O_2$ [M+H]$^+$: 417.2, found: 417.1.

**Example 135. 2-methyl-7-(3-(piperidine-1-carbonyl)pyrazolo[1,5-*a*]pyridin-7-yl)isoquinolin-1(2*H*)-one**

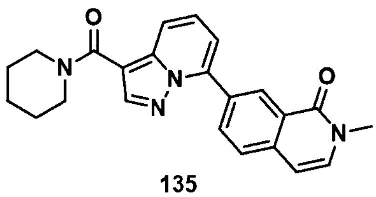

135

The target compound **135** was obtained by the synthetic method in Example **132** using compound **134** and methyl iodide.

$^1$H NMR (400 MHz, DMSO-*d*$_6$) δ(ppm): 8.80 (d, *J* = 1.9 Hz, 1H), 8.27 (s, 1H), 8.23 (dd, *J* = 8.3, 2.0 Hz, 1H), 7.91 (dd, *J* = 8.9, 1.3 Hz, 1H), 7.82 (d, *J* = 8.4 Hz, 1H), 7.60 (d, *J* = 7.3 Hz, 1H), 7.54 (dd, *J* = 8.9, 7.0 Hz, 1H), 7.28 (dd, *J* = 7.1, 1.4 Hz, 1H), 6.72 (d, *J* = 7.3 Hz, 1H), 3.63 (t, *J* = 5.4 Hz, 4H), 3.55 (s, 3H), 1.65 (d, *J* = 7.8 Hz, 2H), 1.57 (d, *J* = 7.7 Hz, 4H).

MS (ESI, LR) Calc. for $C_{23}H_{23}N_4O_2$ [M+H]$^+$: 387.2, found: 387.1.

**Example 137. 2-methyl-6-(3-(piperidine-1-carbonyl)pyrazolo[1,5-*a*]pyridin-7-yl)-3,4-dihydroisoquinolin-1(2*H*)-one**

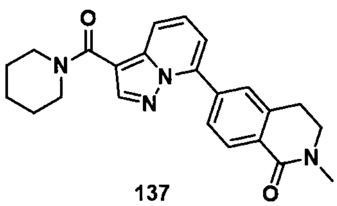

137

The target compound **137** was obtained by the synthetic method in Example **132** using compound **136** and methyl iodide.

$^1$H NMR (400 MHz, DMSO-*d*$_6$) δ(ppm): 8.26 (s, 1H), 8.04-7.99 (m, 1H), 7.93-7.86 (m, 3H), 7.52 (dd, *J* = 8.9, 7.0 Hz, 1H), 7.22 (dd, *J* = 7.1, 1.4 Hz, 1H), 3.62 (t, *J* = 5.5 Hz, 6H), 3.08 (s, 5H), 1.65 (q, *J* = 5.8 Hz, 2H), 1.57 (d, *J* = 6.8 Hz, 4H).

MS (ESI, LR) Calc. for $C_{23}H_{25}N_4O_2$ [M+H]$^+$: 389.2, found: 389.1.

**Example 139. 2-methyl-6-(3-(piperidine-1-carbonyl)pyrazolo[1,5-*a*]pyridin-7-yl)isoquinolin-1(2*H*)-one**

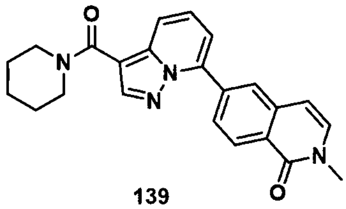

**139**

The target compound **139** was obtained by the synthetic method in Example **132** using compound **138** and methyl iodide.

$^1$H NMR (400 MHz, DMSO-$d_6$) δ(ppm): 8.35 (d, $J$ = 8.4 Hz, 1H), 8.28 (s, 1H), 8.23 (d, $J$ = 1.7 Hz, 1H), 8.01 (dd, $J$ = 8.4, 1.8 Hz, 1H), 7.94 (dd, $J$ = 8.9, 1.3 Hz, 1H), 7.56 (dd, $J$ = 8.9, 7.1 Hz, 2H), 7.30 (dd, $J$ = 7.0, 1.4 Hz, 1H), 6.72 (d, $J$ = 7.3 Hz, 1H), 3.63 (t, $J$ = 5.5 Hz, 4H), 3.56 (s, 3H), 1.66 (s, 2H), 1.57 (s, 4H).

MS (ESI, LR) Calc. for $C_{23}H_{23}N_4O_2$ [M+H]$^+$: 387.2, found: 387.1.

**Example 141. 2-methyl-6-(3-(piperidine-1-carbonyl)pyrazolo[1,5-*a*]pyridin-7-yl)isoindolin-1-one**

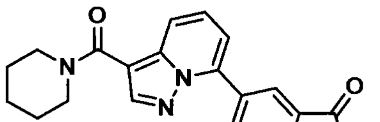

**141**

The target compound **141** was obtained by the synthetic method in Example **132** using compound **140** and methyl iodide.

$^1$H NMR (400 MHz, DMSO-$d_6$) δ(ppm): 8.28-8.22 (m, 2H), 8.12 (dd, $J$ = 7.9, 1.7 Hz, 1H), 7.91 (dd, $J$ = 8.9, 1.3 Hz, 1H), 7.77 (d, $J$ = 7.9 Hz, 1H), 7.53 (dd, $J$ = 8.9, 7.0 Hz, 1H), 7.26 (dd, $J$ = 7.1, 1.4 Hz, 1H), 4.58 (s, 2H), 3.63 (t, $J$ = 5.4 Hz, 4H), 3.13 (s, 3H), 1.65 (d, $J$ = 8.0 Hz, 2H), 1.57 (s, 4H).

MS (ESI, LR) Calc. for $C_{22}H_{23}N_4O_2$ [M+H]$^+$: 375.2, found: 375.1.

**Example 142. 2-isopropyl-6-(3-(piperidine-1-carbonyl)pyrazolo[1,5-*a*]pyridin-7-yl)isoindolin-1-one**

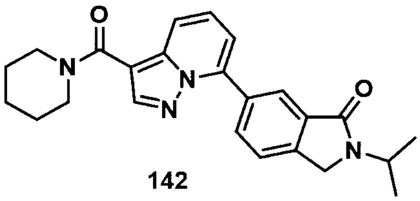

**142**

The target compound **142** was obtained by the synthetic method in Example **132** using compound **140** and isopropyl iodide.

$^1$H NMR (400 MHz, DMSO-$d_6$) δ(ppm): 8.29-8.21 (m, 2H), 8.11 (dd, $J$ = 7.9, 1.7 Hz, 1H), 7.91 (dd, $J$ = 8.9, 1.4 Hz, 1H), 7.78 (d, $J$ = 7.9 Hz, 1H), 7.53 (dd, $J$ = 8.9, 7.0 Hz, 1H), 7.25 (dd, $J$ = 7.1, 1.4 Hz, 1H), 4.56 (s, 2H), 4.47 (p, $J$ = 6.7 Hz, 1H), 3.63 (t, $J$ = 5.4 Hz, 4H), 1.72-1.63 (m, 2H), 1.57 (d, $J$ = 7.3 Hz, 4H), 1.28 (d, $J$ = 6.8 Hz, 6H).

MS (ESI, LR) Calc. for $C_{24}H_{27}N_4O_2$ [M+H]$^+$: 403.2, found: 403.1.

**Example 144. 2-methyl-5-(3-(piperidine-1-carbonyl)pyrazolo[1,5-*a*]pyridin-7-yl)isoindolin-1-one**

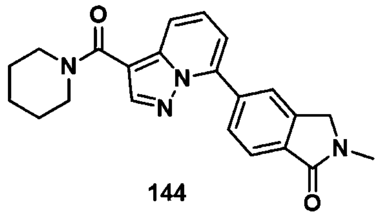

**144**

The target compound **144** was obtained by the synthetic method in Example **132** using compound **143** and methyl iodide.

$^1$H NMR (400 MHz, DMSO-$d_6$) δ(ppm): 8.26 (s, 1H), 8.15 (s, 1H), 8.01 (dd, *J* = 7.9, 1.6 Hz, 1H), 7.92 (dd, *J* = 9.0, 1.3 Hz, 1H), 7.83 (d, *J* = *7.9* Hz, 1H), 7.54 (dd, *J* = 8.9, 7.0 Hz, 1H), 7.24 (dd, *J* = 7.1, 1.4 Hz, 1H), 4.57 (s, 2H), 3.63 (t, *J* = 5.4 Hz, 4H), 3.13 (s, 3H), 1.66 (d, *J* = 5.4 Hz, 2H), 1.57 (s, 4H).

MS (ESI, LR) Calc. for $C_{22}H_{23}N_4O_2$ [M+H]$^+$: 375.2, found: 375.1.

**Example 259. [7-(3-amino-1,2-benzoxazol-5-yl)pyrazolo[1,5-*a*]pyridin-3-yl]-(1-piperidyl)methanone**

<Scheme 33>

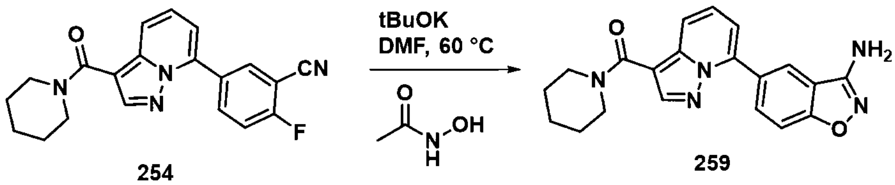


**254** **259**

Potassium *tert*-butyrate (64.4 mg, 0.57 mmol) and acetohydroxamic acid (43.1 mg, 0.57 mmol) were dissolved in *N,N*-dimethylformamide (3 mL) and stirred for 20 minutes. Compound **254** (100 mg, 0.29 mmol) was added and stirred for 1 hour, then the temperature was increased to 60 °C and stirred for 1 hour. After completion of the reaction, the temperature of the reaction solution was lowered to room temperature, water was added, and filtered. The filtrate was separated using column chromatography to obtain the target compound **259** (36 mg, 33%).

$^1$H NMR (400 MHz, DMSO-$d_6$) δ(ppm): 8.41 (d, *J* = 1.9 Hz, 1H), 8.26 (s, 1H), 8.06 (dd, *J* = 8.8, 1.8 Hz, 1H), 7.90 (dd, *J* = 8.9, 1.4 Hz, 1H), 7.62 (d, *J* = 8.8 Hz, 1H), 7.54 (dd, *J* = 8.9, 7.0 Hz, 1H), 7.18 (dd, *J* = 7.1, 1.4 Hz, 1H), 6.55 (s, 2H), 3.63 (t, *J* = 5.5 Hz, 4H), 1.66 (d, *J* = 5.6 Hz, 2H), 1.62-1.50 (m, 4H).

MS (ESI, LR) Calc. for $C_{20}H_{20}N_5O$, [M+H]$^+$: 362.2, found: 362.1.

**Example 320. [7-(3-amino-1,2-benzoxazol-6-yl)pyrazolo[1,5-*a*]pyridin-3-yl]-(1-piperidyl)methanone**

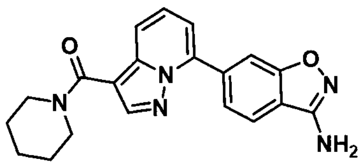

The target compound **320** was obtained by the synthetic method in Example 259 using compound **22.**

$^1$H NMR (400 MHz, DMSO-$d_6$) δ(ppm): 8.26 (s, 1H), 8.04 (s, 1H), 7.98 (d, *J* = 8.2 Hz, 1H), 7.92 (dd, *J* = 8.9, 1.4 Hz, 1H), 7.77 (dd, *J* = 8.2, 1.4 Hz, 1H), 7.54 (dd, *J* = 8.9, 7.0 Hz, 1H), 7.27 (dd, *J* = 7.0, 1.4 Hz, 1H), 6.54 (s, 2H), 3.63 (t, *J* = 5.4 Hz, 4H), 1.66 (s, 2H), 1.57 (d, *J* = 7.4 Hz, 4H).

MS (ESI, LR) Calc. for $C_{20}H_{20}N_5O_2$ [M+H]$^+$: 362.2, found: 362.1.

**Example 260. [7-(3-amino-1H-indazol-5-yl)pyrazolo[1,5-*a*]pyridin-3-yl]-(1-piperidyl)methanone**

<Scheme 34>

Compound 254 (100 mg, 0.29 mmol) was dissolved in ethanol (3 mL), hydrazine hydrate (35.9 mg, 0.57 mmol) was added, and the mixture was stirred in a microwave reactor at 150 °C for 35 minutes. The reaction solution was concentrated, diluted with water, and filtered. The filtrate was separated using column chromatography to obtain the target compound **260** (85 mg, 33%).

$^1$H NMR (400 MHz, DMSO-$d_6$) δ(ppm): 11.64 (s, 1H), 8.32-8.21 (m, 2H), 7.81 (ddd, *J* = 20.3, 8.8, 1.5 Hz, 2H), 7.51 (dd, *J* = 8.9, 7.0 Hz, 1H), 7.40-7.32 (m, 1H), 7.12 (dd, *J* = 7.1, 1.4 Hz, 1H), 5.51 (s, 2H), 3.63 (t, *J* = 5.4 Hz, 4H), 1.65 (d, *J* = 5.3 Hz, 2H), 1.57 (s, 4H).

MS (ESI, LR) Calc. for $C_{20}H_{21}N_6O$ [M+H]$^+$: 361.2, found: 361.1.

**Example 309. [7-(3-amino-1*H*-indazol-6-yl)pyrazolo[1,5-*a*]pyridin-3-yl]-(1-piperidyl)methanone**

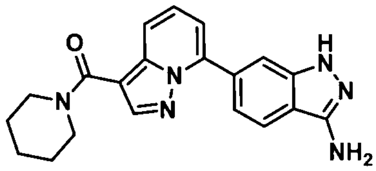

The target compound **309** was obtained by the synthetic method in Example 260 using compound **22.**

$^1$H NMR (400 MHz, DMSO-$d_6$) δ(ppm): 11.62 (s, 1H), 8.24 (d, *J* = 1.1 Hz, 1H), 7.90-7.85 (m, 2H), 7.83 (d, *J* = 8.4 Hz, 1H), 7.52 (dd, *J* = 8.8, 7.1 Hz, 1H), 7.40-7.34 (m, 1H), 7.22 (dd, *J* = 7.1, 1.4 Hz, 1H), 5.45 (s, 2H), 3.63 (t, *J* = 5.3 Hz, 4H), 1.66 (d, *J* = 6.9 Hz, 2H), 1.58 (d, *J* = 7.3 Hz, 4H).

MS (ESI, LR) Calc. for $C_{20}H_{21}N_6O$ [M+H]$^+$: 361.2, found: 361.1.

**Example 261. [7-(3-amino-1-methyl-indazol-5-yl)pyrazolo[1,5-*a*]pyridin-3-yl]-(1-piperidyl)methanone**

**Example 310. [7-(3-amino-1-methyl-indazol-6-yl)pyrazolo[1,5-*a*]pyridin-3-yl]-(1-piperidyl)methanone**

For Examples 261 and 310, the compounds were obtained from compounds **254** and **22,** respectively, using methylhydrazine by the same synthetic method as in Example 260. The structural and spectral data are shown in Table 27 below.

[Table 27]

| Example | Structure | $^1$H NMR | MS |
|---|---|---|---|
| **261** | 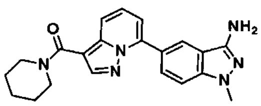 | $^1$H NMR (400 MHz, DMSO-$d_6$) δ(ppm): 8.37 (d, *J* = 2.3 Hz, 1H), 8.25 (d, *J* = 8.9 Hz, 2H), 7.88 (dd, *J* = 8.8, 1.3 Hz, 1H), 7.51 (dd, *J* = 8.9, 7.1 Hz, 1H), 7.43 (d, *J* = 9.0 Hz, 1H), 7.24 (dd, *J* = 7.1, 1.3 Hz, 1H), 4.32 (q, *J* = 7.0 Hz, 2H), 3.62 (t, *J* = 5.4 Hz, 4H), 1.65 (d, *J* = 5.4 Hz, 2H), 1.57 (s, 4H), 1.43 (t, *J* = 6.9 Hz, 3H). | MS (ESI, LR) Calc. for $C_{21}H_{23}N_6O$ [M+H]$^+$: 375.2, found: 375.1. |
| **310** | 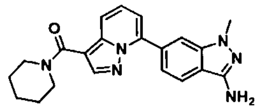 | $^1$H NMR (400 MHz, DMSO-$d_6$) δ(ppm): 8.26 (s, 1H), 7.94 (d, *J* = 8.9 Hz, 1H), 7.90 (d, *J* = 8.1 Hz, 1H), 7.78 (d, *J* = 1.5 Hz, 1H), 7.67-7.62 (m, 1H), 7.54 (dd, *J* = 8.9, 7.0 Hz, 1H), 7.31 (d, *J* = 7.0 Hz, 1H), 4.29 (q, *J* = 7.0 Hz, 2H), 3.62 (t, *J* = 5.4 Hz, 4H), 1.65 (d, *J* = 5.2 Hz, 2H), 1.57 (d, *J* = 5.8 Hz, 4H), 1.41 (t, *J* = 7.0 Hz, 3H). | MS (ESI, LR) Calc. for $C_{21}H_{23}N_6O$ [M+H]$^+$: 375.2, found: 375.1. |

**Example 395. (7-(1-((1,2,4-oxadiazol-3-yl)methyl)-1*H*-pyrazol-4-yl)pyrazolo[1,5-*a*]pyridin-3-yl)(piperidin-1-yl) methanone**

<Scheme 35>

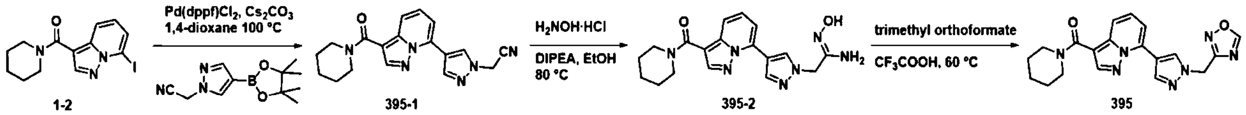


1-2 395-1 395-2 395

Step 395-1: Using compound **1-2** and 2-[4-(4,4,5,5-tetramethyl-1,3,2-dioxaborolan-2-yl)pyrazol-1-yl]acetonitrile, the synthetic method in step 159-3 of Example 159 was used to obtain the target compound **395-1.**

Step 395-2: The target compound **395-2** was obtained by the synthetic method in step 115-1 of Example 115 using compound **395-1** and DIPEA.

Step 395-3: To compound 395-2 (100 mg, 0.27 mmol), trimethyl orthoformate (1.49 mL, 13.6 mmol) and trifluoroacetic acid (8.3 µL) were added, and the mixture was stirred at 60 °C for 1 hour. After completion of the reaction, the reaction solution was diluted with dichloromethane (10 mL) and washed with water and brine. The organic layer was dried over anhydrous magnesium sulfate, filtered, concentrated, and then separated using column chromatography to obtain the target compound **395** (39 mg, 38%).

$^1$H NMR (400 MHz, DMSO-$d_6$) δ(ppm): 9.64 (s, 1H), 9.13 (s, 1H), 8.48 (s, 1H), 8.35 (s, 1H), 7.78 (dd, *J* = 8.7, 1.4 Hz, 1H), 7.58 (dd, *J* = 7.3, 1.4 Hz, 1H), 7.50 (dd, *J* = 8.7, 7.2 Hz, 1H), 5.80 (s, 2H), 3.62 (t, *J* = 5.4 Hz, 4H), 1.65 (d, *J* = 5.5 Hz, 2H), 1.57 (d, *J* = 5.6 Hz, 4H).

MS (ESI, LR) Calc. for $C_{19}H_{20}N_7O_2$ [M+H]$^+$: 378.2, found: 378.1.

**Example 396. 3-((4-(3-(piperidine-1-carbonyl)pyrazolo[1,5-*a*]pyridin-7-yl)-1*H*-pyrazol-1-yl)methyl)-1,2,4-oxadiazol-5(4*H*)-one**

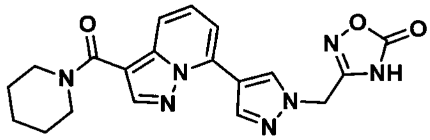

The target compound **396** was obtained by the synthetic method in Example 349 using compound **395-5.**

$^1$H NMR (400 MHz, DMSO-$d_6$) δ(ppm): 8.95 (s, 1H), 8.43 (s, 1H), 8.36 (s, 1H), 7.77 (d, *J* = 8.8 Hz, 1H), 7.56 (t, *J* = 6.3 Hz, 1H), 7.51-7.47 (m, 1H), 5.10 (s, 2H), 3.63 (t, *J* = 5.5 Hz, 4H), 1.66 (s, 2H), 1.57 (s, 4H).

MS (ESI, LR) Calc. for $C_{19}H_{20}N_7O_3$ [M+H]$^+$: 394.2, found: 394.1.

**Example 397. (7-(2-(1-((1,2,4-oxadiazol-3-yl)methyl)-1*H*-pyrazol-4-yl)pyridin-4-yl)pyrazolo[1,5-*a*]pyridin-3-yl)(piperidin-1-yl)methanone**

<Scheme 36>

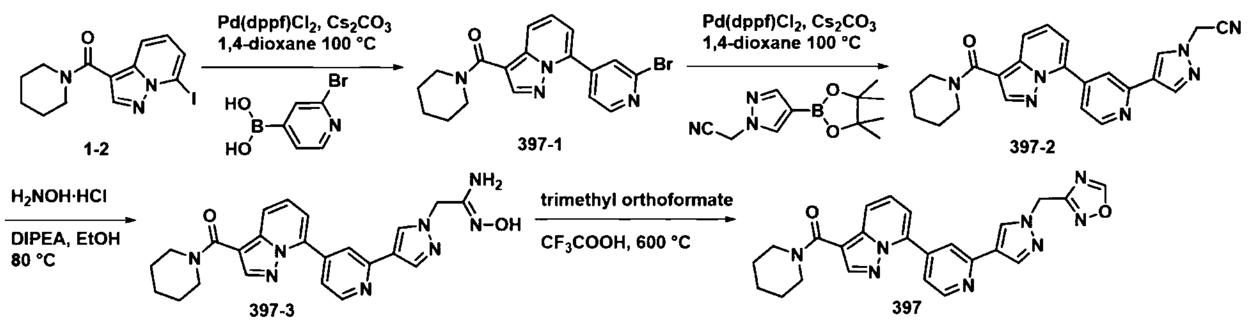


1-2 397-1 397-2 397-3 397

Step 397-1: The target compound **397-1** was obtained by the synthetic method in step 159-3 of Example 159 using compound **1-2.**

Step 397-2: The target compound **397-2** was obtained by the synthetic method in Step 395-1 of Example 395 using compound **397-1.**

Step 397-3: The target compound **397-3** was obtained by the synthetic method in step 395-2 of Example 395 using compound **397-2.**

Step 397-4: : The target compound **397** was obtained by the synthetic method in step 395-3 of Example 395 using compound **397-3.**

$^1$H NMR (400 MHz, DMSO-$d_6$) δ(ppm): 9.64 (s, 1H), 8.71 (d, *J* = 5.2 Hz, 1H), 8.56 (s, 1H), 8.29 (s, 1H), 8.21 (s, 1H), 8.15 (s, 1H), 7.97 (dd, *J* = 8.9, 1.4 Hz, 1H), 7.78 (dd, *J* = 5.2, 1.6 Hz, 1H), 7.56 (dd, *J* = 8.9, 7.0 Hz, 1H), 7.39 (dd, *J* = 7.0, 1.4 Hz, 1H), 5.69 (s, 2H), 3.63 (t, *J* = 5.4 Hz, 4H), 1.66 (d, *J* = 5.6 Hz, 2H), 1.57 (d, *J* = 6.7 Hz, 4H).

MS (ESI, LR) Calc. for $C_{24}H_{23}N_8O_2$ [M+H]$^+$: 455.2, found: 455.1.

**Example 146. 4-((3-(piperidine-1-carbonyl)pyrazolo[1,5-*a*]pyridin-7-yl)amino)benzonitrile**

<Scheme 37>

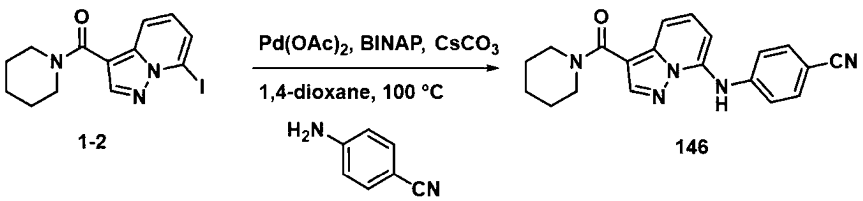


1-2 146

Compound **1-2** (0.05 g, 0.14 mmol), palladium acetate (1.58 mg, 0.007 mmol), BINAP (8.7 mg, 0.01 mmol), and cesium carbonate (183.4 mg, 0.56 mmol) were dissolved in 1,4-dioxane (2 mL), and the mixture was stirred at 100 °C for 16 hours after filling with nitrogen gas. After completion of the reaction, the reaction solution was diluted with dichloromethane (10 mL) and washed with water and brine. The organic layer was dried over anhydrous magnesium sulfate, filtered, concentrated, and separated using column chromatography to obtain the target compound **146** (16 mg, 32%). [BINAP (2,2'-bis(diphenylphosphino)-1,1'-binaphthyl)]

$^1$H NMR (400 MHz, DMSO-$d_6$) δ(ppm): 9.72 (s, 1H), 8.28 (s, 1H), 7.85-7.73 (m, 2H), 7.58-7.50 (m, 2H), 7.49-7.35 (m, 2H), 6.88 (dd, *J* = 7.1, 1.5 Hz, 1H), 3.61 (t, *J* = 5.4 Hz, 4H), 1.65 (d, *J* = 5.4 Hz, 2H), 1.57 (d, *J* = 7.3 Hz, 4H).

MS (ESI, LR) Calc. for $C_{20}H_{20}N_5O$ [M+H]$^+$: 346.2, found: 346.1.

**Example 147. 3-((3-(piperidine-1-carbonyl)pyrazolo[1,5-*a*]pyridin-7-yl)amino)pyridin-2(1*H*)-one**

**Example 148. piperidin-1-yl(7-((pyrimidin-2-ylmethyl)amino)pyrazolo[1,5-*a*]pyridin-3-yl)methanone**

**Example 149. piperidin-1-yl(7-((2-(pyridin-4-yl)ethyl)amino)pyrazolo[1,5-*a*]pyridin-3-yl)methanone**

**Example 150. (7-((1-methylpiperidin-4-yl)amino)pyrazolo[1,5-*a*]pyridin-3-yl)(piperidin-1-yl)methanone**

**Example 211. 7-[[3-(piperidine-1-carbonyl)pyrazolo[1,5-*a*]pyridin-7-yl]amino]-3,4-dihydro-2*H*-isoquinolin-1-one**

**Example 212. 6-[[3-(piperidine-1-carbonyl)pyrazolo[1,5-*a*]pyridin-7-yl]amino]isoindolin-1-one**

**Example 238. [7-[(4-chlorophenyl)methylamino]pyrazolo[1,5-*a*]pyridin-3-yl]-(1-piperidyl)methanone**

**Example 242. [7-[2-(4-chlorophenyl)ethylamino]pyrazolo[1,5-*a*]pyridin-3-yl]-(1-piperidyl)methanone**

For Examples 147, 148, 149, 150, 211, 212, 238, and 242, the compounds were obtained by the synthetic method in Example **146** using compound **1-2** and the corresponding compounds. The structural and spectral data are shown in Table 28 below.

[Table 28]

| Example | Structure | $^1$H NMR | MS |
|---|---|---|---|
| **147** | 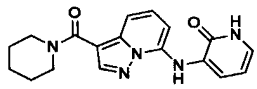 | $^1$H NMR (400 MHz, DMSO-$d_6$) δ(ppm): 12.12 (s, 1H), 9.19 (s, 1H), 8.31 (s, 1H), 7.60 (dd, *J* = 7.3, 1.6 Hz, 1H), 7.45 (t, *J* = 8.2 Hz, 1H), 7.37 (dd, *J* = 8.8, 1.1 Hz, 1H), 7.12 (dd, *J* = 6.7, 1.6 Hz, 1H), 7.00-6.87 (m, 1H), 6.33 (t, *J* = 6.9 Hz, 1H), 3.61 (t, *J* = 5.3 Hz, 4H), 1.65 (d, *J* = 7.8 Hz, 2H), 1.57 (d, *J* = 6.8 Hz, 4H). | MS (ESI, LR) Calc. for $C_{18}H_{20}N_5O_2$ $[M+H]^+$: 338.2, found: 338.1. |
| **148** | 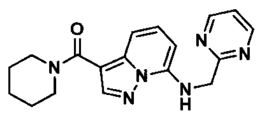 | $^1$H NMR (400 MHz, DMSO-$d_6$) δ(ppm): 8.84 (d, *J* = 4.9 Hz, 2H), 8.23 (s, 1H), 7.66 (t, *J* = 5.7 Hz, 1H), 7.46 (t, *J* = 4.9 Hz, 1H), 7.30 (t, *J* = 8.1 Hz, 1H), 7.13-7.04 (m, 1H), 6.09 (d, *J* = 7.6 Hz, 1H), 4.81 (d, *J* = 5.7 Hz, 2H), 3.60 (t, *J* = 5.4 Hz, 4H), 1.64 (d, *J* = 5.8 Hz, 2H), 1.56 (q, *J* = 5.8 Hz, 4H). | MS (ESI, LR) Calc. for $C_{18}H_{21}N_6O$ $[M+H]^+$: 337.2, found: 337.1. |
| **149** | 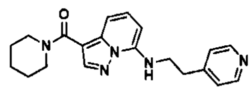 | $^1$H NMR (400 MHz, DMSO-$d_6$) δ(ppm): 8.53-8.42 (m, 2H), 8.15 (s, 1H), 7.39 - 7.26 (m, 3H), 7.12 (t, *J* = 6.1 Hz, 1H), 7.05 (dd, *J* = 8.7, 1.1 Hz, 1H), 6.21 (d, *J* = 7.6 Hz, 1H), 3.64 (q, *J* = 6.8 Hz, 2H), 3.57 (t, *J* = 5.4 Hz, 4H), 3.01 (t, *J* = 7.2 Hz, 2H), 1.63 (d, *J* = 5.9 Hz, 2H), 1.54 (d, *J* = 4.9 Hz, 4H). | MS (ESI, LR) Calc. for $C_{20}H_{24}N_5O$ $[M+H]^+$: 350.2, found: 350.1. |
| **150** | 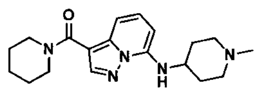 | $^1$H NMR (400 MHz, DMSO-$d_6$) δ(ppm): 8.15 (s, 1H), 7.31 (t, *J* = 8.2 Hz, 1H), 7.04 (dd, *J* = 8.7, 1.1 Hz, 1H), 6.63 (d, *J* = 8.5 Hz, 1H), 6.19 (d, *J* = 7.7 Hz, 1H), 3.58 (t, *J* = 5.4 Hz, 4H), 3.55-3.45 (m, 1H), 2.75 (d, *J* = 11.2 Hz, 2H), 2.19 (s, 3H), 2.13-2.02 (m, 2H), 1.98 - 1.88 (m, 2H), 1.72 (td, *J* = 10.9, 2.8 Hz, 2H), 1.64 (dt, *J* = 11.0, 4.7 Hz, 2H), 1.54 (p, *J* = 5.6 Hz, 4H). | MS (ESI, LR) Calc. for $C_{19}H_{28}N_5O$ $[M+H]^+$: 342.2, found: 342.2. |
| **211** | 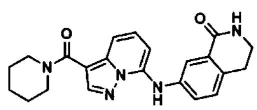 | $^1$H NMR (400 MHz, DMSO-$d_6$) δ(ppm): 8.09 (d, *J* = 1.5 Hz, 2H), 7.91 (s, 1H), 7.42 (dd, *J* = 8.1, 2.5 Hz, 1H), 7.37 (dd, *J* = 8.8, 1.2 Hz, 1H), 7.29-7.24 (m, 2H), 6.57 (dd, *J* = 7.7, 1.2 Hz, 1H), 6.07 (s, 1H), 3.71 (t, *J* = 5.3 Hz, 4H), 3.61 (td, *J* = 6.6, 2.8 Hz, 2H), 3.02 (t, *J* = 6.6 Hz, 2H), 1.78-1.64 (m, 6H). | MS (ESI, LR) Calc. for $C_{22}H_{24}N_5O_2$ $[M+H]^+$: 390.2, found: 390.1. |
| **212** | 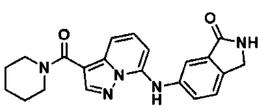 | $^1$H NMR (400 MHz, DMSO-$d_6$) δ(ppm): 8.11 (s, 1H), 8.00 (s, 1H), 7.90 (d, *J* = 1.6 Hz, 1H), 7.52 (d, *J* = 1.4 Hz, 2H), 7.45-7.36 (m, 1H), 7.29 (d, *J* = 8.0 Hz, 1H), 6.78 (s, 1H), 6.62 (d, *J* = 7.5 Hz, 1H), 4.49 (s, 2H), 3.71 (t, *J* = 5.3 Hz, 4H), 1.78-1.66 (m, 6H). | MS (ESI, LR) Calc. for $C_{21}H_{22}N_3O_2$ $[M+H]^+$: 376.2, found: 376.1. |

(continued)

| Example | Structure | $^1H$ NMR | MS |
|---|---|---|---|
| **238** | 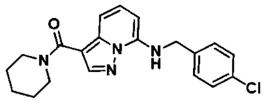 | $^1H$ NMR (400 MHz, DMSO-$d_6$) δ(ppm): 8.20 (s, 1H), 7.90 (t, *J* = 6.5 Hz, 1H), 7.47-7.35 (m, 4H), 7.23 (t, *J* = 8.1 Hz, 1H), 7.03 (dd, *J* = 8.7, 1.2 Hz, 1H), 5.95 (dd, *J* = 7.7, 1.1 Hz, 1H), 4.59 (d, *J* = 6.5 Hz, 2H), 3.58 (t, *J* = 5.4 Hz, 4H), 1.64 (d, *J* = 5.3 Hz, 2H), 1.54 (t, *J* = 5.9 Hz, 4H). | MS (ESI, LR) Calc. for $C_{20}H_{22}ClN_4O$ $[M+H]^+$: 369.1, found: 369.1. |
| **242** | 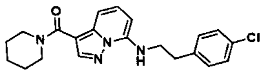 | $^1H$ NMR (400 MHz, DMSO-$d_6$) δ(ppm): 8.15 (s, 1H), 7.40-7.27 (m, 5H), 7.10-7.01 (m, 2H), 6.19 (dd, *J* = 7.8, 1.2 Hz, 1H), 3.67-3.52 (m, 6H), 2.98 (t, *J* = 7.3 Hz, 2H), 1.68-1.59 (m, 2H), 1.55 (q, *J* = 5.7 Hz, 4H). | MS (ESI, LR) Calc. for $C_{21}H_{24}ClN_4O$ $[M+H]^+$: 383.2, found: 383.1. |

**Example 194. (7-((3-methoxyphenyl)thio)pyrazolo[1,5-*a*]pyridin-3-yl)(piperidin-1-yl)methanone**

<Scheme 38>

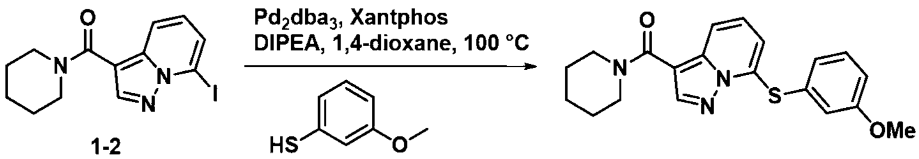


Compound **1-2** (0.06 g, 0.17 mmol), tris(dibenzylideneacetone)dipalladium(0) (3.86 mg, 0.025 mmol), Xantphos (4.88 mg, 0.05 mmol), DIPEA (43.6 mg, 0.34 mmol), and 3-methoxybenzinethiol (71.05 mg, 0.51 mmol) were dissolved in 1,4-dioxane (2 mL), and the mixture was stirred at 100 °C for 1 hour after filling with nitrogen gas. After completion of the reaction, the reaction solution was diluted with dichloromethane (10 mL) and washed with water and brine. The organic layer was dried over anhydrous magnesium sulfate, filtered, concentrated, and then separated using column chromatography to obtain the target compound **194** (61 mg, 98%).

$^1H$ NMR (400 MHz, DMSO-$d_6$) δ(ppm): 8.32 (s, 1H), 7.68 (dd, *J* = 8.8, 1.2 Hz, 1H), 7.50 (t, *J*= 8.2 Hz, 1H), 7.32 (dd, *J* = 8.8, 7.4 Hz, 1H), 7.29-7.22 (m, 2H), 7.20-7.14 (m, 1H), 6.34 (dd, *J* = 7.3, 1.2 Hz, 1H), 3.81 (s, 3H), 3.60 (t, *J* = 5.4 Hz, 4H), 1.72-1.60 (m, 2H), 1.60-1.48 (m, 4H).

MS (ESI, LR) Calc. for $C_{20}H_{22}N_3O_2S$ $[M+H]^+$: 368.1, found: 368.1.

**Example 235. 4-[[3-(piperidine-1-carbonyl)pyrazolo[1,5-*a*]pyridin-7-yl]amino]-1*H*-pyrimidin-6-one**

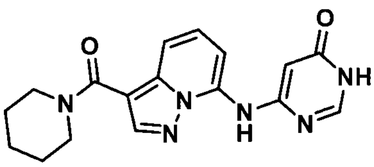

The target compound **235** was obtained by the synthetic method in Example 194 using compound **1-2,** palladium acetate, and the corresponding compounds.

$^1H$ NMR (400 MHz, DMSO-$d_6$) δ(ppm): 12.24 (s, 1H), 9.77 (s, 1H), 8.27 (s, 1H), 8.15 (s, 1H), 7.58-7.39 (m, 3H), 6.07 (s, 1H), 3.61 (t, *J*= 5.4 Hz, 4H), 1.71-1.61 (m, 2H), 1.56 (d, J = 7.9 Hz, 4H).

MS (ESI, LR) Calc. for $C_{17}H_{19}N_6O_2$ $[M+H]^+$: 339.1, found: 339.1.

**Example 236. 3-methyl-6-[[3-(piperidine-1-carbonyl)pyrazolo[1,5-*a*]pyridin-7-yl]amino]pyrimidin-4-one**

<Scheme 39>

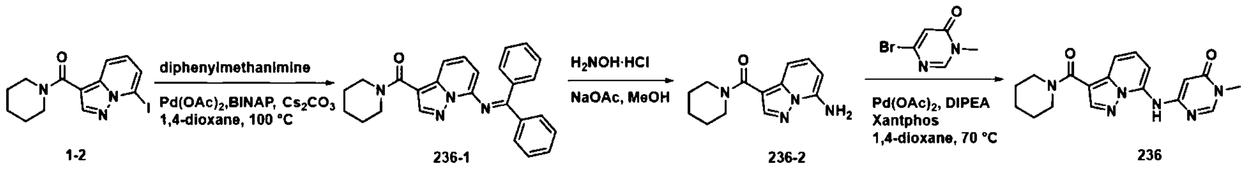


Step 236-1: Compound **236-1** was obtained using compound **1-2** and diphenylmethanimine by the synthetic method in Example 146.

Step 236-2: Compound **236-1** (2 g, 4.89 mmol), hydroxyamine hydrochloride (0.68 g, 9.79 mmol), and sodium acetate (1 g, 12.24 mmol) were dissolved in methanol (48 mL), and stirred at room temperature for 16 hours. The reaction solution was concentrated, and the residue was separated using column chromatography to obtain the target compound **236-2** (845 mg, 69%).

Step 236-3: The target compound **236** was obtained by the synthetic method in Example 235 using compound **236-2** and 6-bromo-3-methyl-pyrimidin-4-one.

$^1$H NMR (400 MHz, DMSO-$d_6$) δ(ppm): 9.79 (s, 1H), 8.43 (s, 1H), 8.27 (s, 1H), 7.56-7.40 (m, 3H), 6.16 (s, 1H), 3.61 (t, *J* = 5.4 Hz, 4H), 3.38 (s, 3H), 1.65 (q, *J* = 5.7 Hz, 2H), 1.57 (q, *J* = 5.8 Hz, 4H).

MS (ESI, LR) Calc. for $C_{18}H_{21}N_6O_2$ [M+H]$^+$: 353.2, found: 353.1.

**Example 239. 4-chloro-*N*-[3-(piperidine-1-carbonyl)pyrazolo[1,5-*a*]pyridin-7-yl]benzamide**

<Scheme 40>

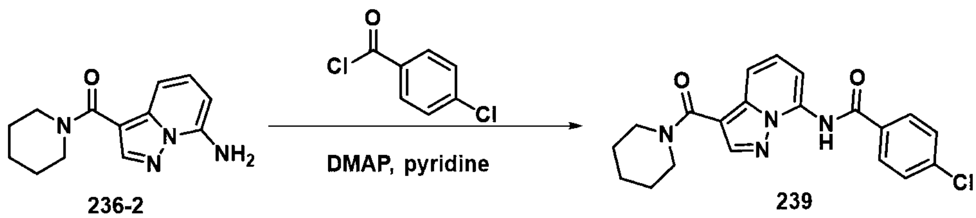


Compound 236-2 (70 mg, 0.28 mmol) was dissolved in pyridine (2.6 mL), dimethylaminopyridine (17.5 mg, 0.14 mmol) and 4-chlorophenzoyl chloride (75.2 mg) were added, and the mixture was stirred at room temperature for 16 hours. The reaction solution was concentrated, and the residue was separated using column chromatography to obtain the target compound **239** (60 mg, 53%).

$^1$H NMR (400 MHz, DMSO-$d_6$) δ(ppm): 8.25 (d, *J* = 8.2 Hz, 1H), 8.11-8.04 (m, 1H), 7.91-7.86 (m, 1H), 7.84-7.77 (m, 2H), 7.66-7.61 (m, 1H), 7.46-7.39 (m, 2H), 3.81-3.69 (m, 4H), 1.82-1.74 (m, 2H), 1.69-1.67 (m, 4H).

MS (ESI, LR) Calc. for $C_{20}H_{20}ClN_4O_2$ [M+H]$^+$: 383.1, found: 383.1.

**Example 240. 1,3,5-trimethyl-*N*-[3-(piperidine-1-carbonyl)pyrazolo[1,5-*a*]pyridin-7-yl]pyrazole-4-sulfonamide**

**Example 241. 5-fluoro-*N*-[3-(piperidine-1-carbonyl)pyrazolo[1,5-*a*]pyridin-7-yl]pyridine-3-carboxamide**

For Examples 240 and 241, the compounds were obtained by the synthetic method in Example 239 using compound **236-2** and the corresponding compounds. The structural and spectral data are shown in Table 29 below.

[Table 29]

| Example | Structure | $^1$H NMR | MS |
|---|---|---|---|
| **240** | 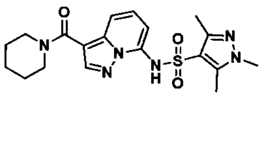 | $^1$H NMR (400 MHz, DMSO-$d_6$) δ(ppm): 11.01 (s, 1H), 8.19 (s, 1H), 7.59 (d, $J$ = 8.8 Hz, 1H), 7.38 (dd, $J$ = 8.9, 7.4 Hz, 1H), 6.88 (d, $J$ = 7.4 Hz, 1H), 3.60 (s, 3H), 3.57 (t, $J$ = 5.4 Hz, 4H), 2.35 (s, 3H), 2.18 (s, 3H), 1.63 (d, $J$ = 6.5 Hz, 2H), 1.55 (q, $J$ = 5.7 Hz, 4H). | MS (ESI, LR) Calc. for $C_{19}H_{25}N_6O_3S$ $[M+H]^+$: 417.2, found: 417.1. |
| **241** | 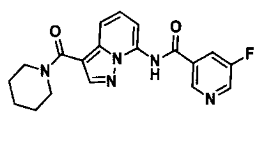 | $^1$H NMR (400 MHz, DMSO-$d_6$) δ(ppm): 11.13 (s, 1H), 9.06 (t, $J$ = 1.7 Hz, 1H), 8.88 (d, $J$ = 2.8 Hz, 1H), 8.32 (s, 1H), 8.29 (ddd, $J$ = 9.3, 2.8, 1.7 Hz, 1H), 7.74 (dd, $J$ = 7.8, 2.4 Hz, 1H), 7.58-7.50 (m, 2H), 3.62 (t, $J$ = 5.4 Hz, 4H), 1.65 (q, $J$ = 5.8 Hz, 2H), 1.57 (p, $J$ = 5.6 Hz, 4H). | MS (ESI, LR) Calc. for $C_{19}H_{19}FN_5O_2$ $[M+H]^+$: 368.1, found: 368.1. |

**Example 151. 1-(7-(quinolin-3-yl)pyrazolo[1,5-*a*]pyridin-3-yl)pentan-1-one**

<Scheme 41>

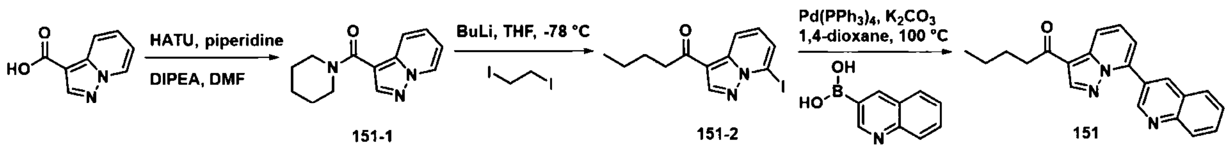


151-1 151-2 151

Step 151-1: The target compound **151-1** was obtained by the same synthetic method as step 1-2 in Example **1** using pyrazolo[1,5-*a*]pyridine-3-carboxylic acid.

Step 151-2: Compound **151-1** (1 g, 4.36 mmol) was dissolved in tetrahydrofuran (15 mL) , the temperature was lowered to -78 °C, and *n*-butyllithium (2.5 M solution, 2.44 mL, 6.1 mmol) was slowly added. After the reaction solution was stirred for 30 minutes, 1,2-iodoethane (1.47 g, 5.23 mmol) dissolved in tetrahydrofuran (9 mL) was added. After the reaction solution was stirred for 3 hours, the temperature was raised to room temperature, and the mixture was diluted with saturated sodium bicarbonate aqueous solution (20 mL) and dichloromethane (20 mL). The organic layer was washed with brine, dried over anhydrous magnesium sulfate, filtered, and concentrated. The residue was separated using column chromatography to obtain the target compound **151-2** (151 mg, 11%).

Step 151-3: Using compound **151-2,** the target compound **151** was obtained by the same synthetic method as steps 1-3 in Example 1.

$^1$H NMR (400 MHz, DMSO-$d_6$) δ(ppm): 9.35 (d, $J$ = 2.2 Hz, 1H), 8.78 (d, $J$ = 2.2 Hz, 1H), 8.54 (dd, $J$ = 8.8, 1.4 Hz, 1H), 8.41 (s, 1H), 8.20 (d, $J$ = 8.5 Hz, 1H), 7.95 (d, $J$ = 8.1 Hz, 1H), 7.82 (ddd, $J$ = 8.4, 6.9, 1.5 Hz, 1H), 7.70-7.56 (m, 2H), 7.23 (dd, $J$ = 7.1, 1.4 Hz, 1H), 2.93 (t, $J$ = 7.5 Hz, 2H), 1.79 (p, $J$ = 7.6 Hz, 2H), 1.46 (h, $J$ = 7.4 Hz, 2H), 0.98 (t, $J$ = 7.3 Hz, 3H).

MS (ESI, LR) Calc. for $C_{21}H_{20}N_3O$ $[M+H]^+$: 330.2, found: 330.2.

**Example 152. 4-(3-acetylpyrazolo[1,5-*a*]pyridin-7-yl)benzonitrile**

<Scheme 42>

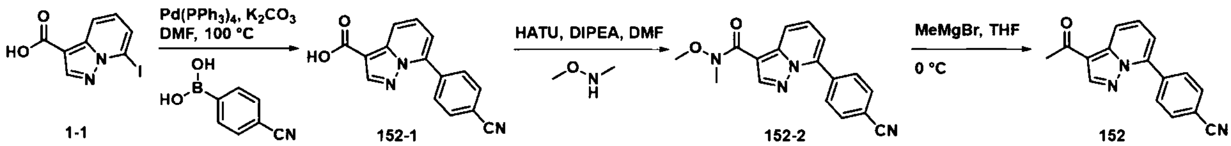


1-1 152-1 152-2 152

Step 152-1: The target compound **152-1** was obtained by the same synthetic method as step 1-3 in Example 1 using pyrazolo[1,5-*a*]pyridine-3-carboxylic acid and 4-cyanophenylboronic acid.

Step 152-2: The target compound **152-2** was obtained by the same synthetic method as step 1-2 in Example **1**

using compound **152-1** and dimethylhydroxylamine.

Step 152-3: Compound **152-2** (0.05 mg, 0.16 mmol) was dissolved in tetrahydrofuran (2 mL), the temperature was lowered to 0 °C, and methylmagnesium bromide (3 M solution, 0.1 mL, 0.33 mmol) was slowly added. The reaction solution was stirred for 3 hours, diluted with saturated ammonium chloride aqueous solution (10 mL) and ethyl acetate (10 mL). The organic layer was washed with brine, dried over anhydrous magnesium sulfate, filtered, and concentrated. The residue was separated using column chromatography to obtain the target compound **152** (11 mg, 24%).

$^1H$ NMR (400 MHz, DMSO-$d_6$) δ(ppm): 8.72 (s, 1H), 8.36 (dd, *J* = 8.8, 1.3 Hz, 1H), 8.14 (d, *J* = 8.3 Hz, 2H), 8.05 (d, *J* = 8.4 Hz, 2H), 7.76 (dd, *J* = 8.8, 7.2 Hz, 1H), 7.43 (dd, *J* = 7.2, 1.4 Hz, 1H), 2.54 (s, 3H).

MS (ESI, LR) Calc. for $C_{16}H_{12}N_3O$ $[M+H]^+$: 262.1, found: 262.1.

**Example 153. 4-(3-(2-hydroxypropan-2-yl)pyrazolo[1,5-*a*]pyridin-7-yl)benzonitrile**

<Scheme 43>

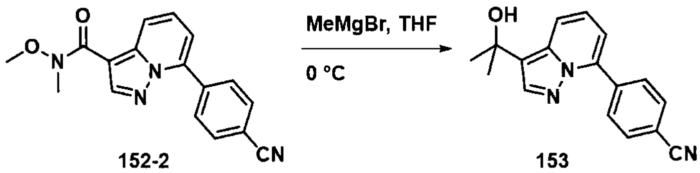


152-2 153

The target compound **153** was obtained by the same synthetic method as step 152-3 in Example **152** using compound **152-2.**

$^1H$ NMR (400 MHz, DMSO-$d_6$) δ(ppm): 8.72 (s, 1H), 8.35 (dd, *J* = 8.8, 1.3 Hz, 1H), 8.17-8.11 (m, 2H), 8.08 (d, *J* = 8.5 Hz, 2H), 7.76 (dd, *J* = 8.8, 7.1 Hz, 1H), 7.41 (dd, *J* = 7.2, 1.4 Hz, 1H), 2.67 (s, 3H), 2.55 (s, 3H).

MS (ESI, LR) Calc. for $C_{17}H_{15}N_2O_2$ $[M+H]^+$: 279.1, found: 279.1.

**Example 154. 7-(3-(4-fluoropiperidine-1-carbonyl)pyrazolo[1,5-*a*]pyridin-7-yl)-3,4-dihydroisoquinolin-1(2*H*)-one**

<Scheme 44>

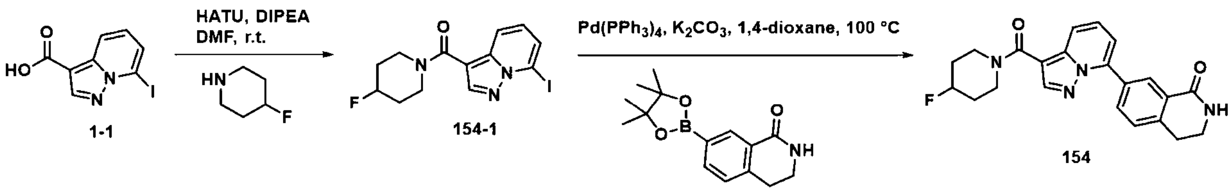


1-1 154-1 154

Step 154-1: The target compound **154-1** was obtained by the same synthetic method as step 1-2 in Example 1 using compound **1-1** and 4-fluoropiperidine.

Step 154-2: The target compound **154** was obtained by the same synthetic method as step 1-3 in Example **1** using compound **154-1** and 7-(4,4,5,5,-tetramethyl-1,3,2-dioxaborolan-2-yl)-3,4-dihydro-2*H*-isoquinolin-1-one.

$^1H$ NMR (400 MHz, DMSO-$d_6$) δ(ppm): 8.41 (d, *J* = 2.0 Hz, 1H), 8.30 (s, 1H), 8.07 (s, 1H), 8.01 (dd, *J* = 7.9, 2.0 Hz, 1H), 7.92 (dd, *J* = 8.9, 1.3 Hz, 1H), 7.57-7.48 (m, 2H), 7.21 (dd, *J* = 7.1, 1.4 Hz, 1H), 3.80-3.62 (m, 4H), 3.45 (td, *J* = 6.6, 2.7 Hz, 2H), 3.02 (t, *J* = 6.5 Hz, 2H), 2.06-1.82 (m, 3H), 1.82 - 1.67 (m, 2H).

MS (ESI, LR) Calc. for $C_{22}H_{22}FN_4O_2$ $[M+H]^+$: 393.2, found: 393.1.

**Example 155. 6-(3-(4-fluoropiperidine-1-carbonyl)pyrazolo[1,5-*a*]pyridin-7-yl)isoindolin-1-one**

**Example 156. 4-(3-(4-fluoropiperidine-1-carbonyl)pyrazolo[1,5-*a*]pyridin-7-yl)benzonitrile**

**Example 157. (4-fluoropiperidin-1-yl)(7-(4-nitrophenyl)pyrazolo[1,5-*a*]pyridin-3-yl)methanone**

**Example 231. 6-(3-(4-fluoropiperidine-1-carbonyl)pyrazolo[1,5-*a*]pyridin-7-yl)-2-methylisoindolin-1-one**

For Examples 155, 156, 157, and 231, the compounds were obtained by the same synthetic method as step 1-3 in Example **1** using compound **154-1** and the corresponding compounds. The structural and spectral data are shown in Table 30 below.

[Table 30]

| Example | Structure | $^1$H NMR | MS |
|---|---|---|---|
| **155** | 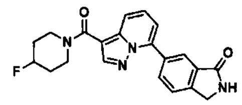 | $^1$H NMR (400 MHz, DMSO-$d_6$) δ(ppm): 8.71 (s, 1H), 8.31 (s, 1H), 8.25 (d, *J* = 1.7 Hz, 1H), 8.12 (dd, *J* = 7.9, 1.7 Hz, 1H), 7.93 (dd, *J* = 8.8, 1.4 Hz, 1H), 7.77 (d, *J* = 7.9 Hz, 1H), 7.55 (dd, *J* = 8.9, 7.1 Hz, 1H), 7.27 (dd, *J* = 7.1, 1.4 Hz, 1H), 4.50 (s, 2H), 3.81-3.62 (m, 4H), 2.06-1.81 (m, 3H), 1.78 (d, *J* = 3.5 Hz, 2H). | MS (ESI, LR) Calc. for $C_{21}H_{20}FN_4O$ $[M+H]^+$: 379.2, found: 379.1. |
| **156** | 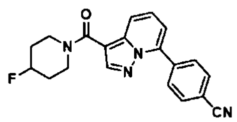 | $^1$H NMR (400 MHz, DMSO-$d_6$) δ(ppm): 8.31 (s, 1H), 8.18-8.12 (m, 2H), 8.07-8.01 (m, 2H), 7.96 (dd, *J* = 8.9, 1.3 Hz, 1H), 7.56 (dd, *J* = 8.9, 7.0 Hz, 1H), 7.30 (dd, *J* = 7.0, 1.3 Hz, 1H), 3.80-3.60 (m, 4H), 2.12-1.82 (m, 3H), 1.77 (td, *J* = 6.8, 3.5 Hz, 2H). | MS (ESI, LR) Calc. for $C_{20}H_{17}FN_4O$ $[M+H]^+$: 349.1, found: 349.1. |
| **157** | 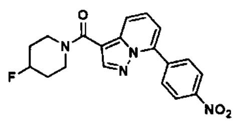 | $^1$H NMR (400 MHz, DMSO-$d_6$) δ(ppm): 8.43-8.38 (m, 2H), 8.33 (s, 1H), 8.27-8.23 (m, 2H), 7.99 (dd, *J* = 8.9, 1.3 Hz, 1H), 7.58 (dd, *J* = 8.9, 7.1 Hz, 1H), 7.34 (dd, *J* = 7.1, 1.4 Hz, 1H), 3.81-3.61 (m, 4H), 2.15-1.60 (m, 5H). | MS (ESI, LR) Calc. for $C_{19}H_{18}FN_4O_3$ $[M+H]^+$: 369.1, found: 369.0. |
| **231** | 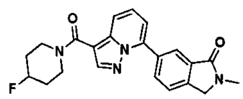 | $^1$H NMR (400 MHz, DMSO-$d_6$) δ(ppm): 8.31 (s, 1H), 8.25 (d, *J* = 1.7 Hz, 1H), 8.12 (dd, *J* = 7.9, 1.7 Hz, 1H), 7.93 (dd, *J* = 8.9, 1.3 Hz, 1H), 7.77 (d, *J* = 7.9 Hz, 1H), 7.54 (dd, *J* = 8.9, 7.0 Hz, 1H), 7.27 (dd, *J* = 7.1, 1.3 Hz, 1H), 5.05-4.85 (m, 1H), 4.58 (s, 2H), 3.82-3.61 (m, 4H), 3.13 (s, 3H), 2.04-1.71 (m, 5H). | MS (ESI, LR) Calc. for $C_{22}H_{22}FN_4O_2$ $[M+H]^+$: 393.2, found: 393.1. |

**Example 158. cyclohexyl(7-phenylpyrazolo[1,5-*a*]pyridin-3-yl)methanone**

<Scheme 45>

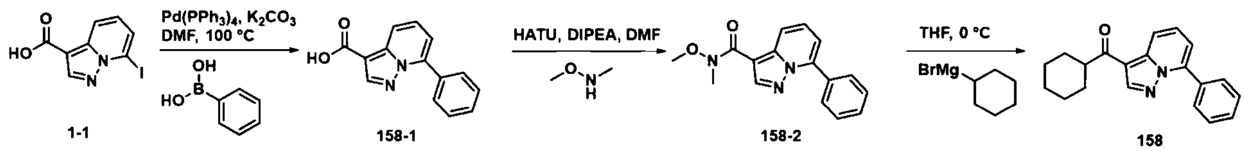


1-1 158-1 158-2 158

Step 158-1: The target compound **158-1** was obtained by the same synthetic method as step 1-3 in Example **1** using pyrazolo[1,5-*a*]pyridine-3-carboxylic acid and phenylboronic acid.

Step 158-2: The target compound **158-2** was obtained by the same synthetic method as Step 1-2 in Example **1** using compound **158-1** and dimethylhydroxylamine.

Step 158-3: The target compound 158 was obtained by the same synthetic method as step 152-3 in Example **152** using compound **158-2** and cyclohexylmagnesium bromide.

$^1$H NMR (400 MHz, DMSO-$d_6$) δ(ppm): 8.75 (s, 1H), 8.33 (dd, *J* = 8.8, 1.4 Hz, 1H), 7.97-7.86 (m, 2H), 7.72 (dd, *J* = 8.8, 7.2 Hz, 1H), 7.57 (dd, *J* = 5.2, 2.0 Hz, 3H), 7.31 (dd, *J* = 7.1, 1.4 Hz, 1H), 3.23 (s, 1H), 1.91-1.64 (m, 5H), 1.53-1.35 (m, 4H), 1.22 (d, *J* = 11.4 Hz, 1H).

MS (ESI, LR) Calc. for $C_{20}H_{21}N_2O$ $[M+H]^+$: 305.2, found: 305.1.

**Example 227. (2-azabicyclo[2.2.1]heptan-2-yl)(7-phenylpyrazolo[1,5-*a*]pyridin-3-yl)methanone**

**Example 228. (2-azabicyclo[2.2.2]octan-2-yl)(7-phenylpyrazolo[1,5-*a*]pyridin-3-yl)methanone**

**Example 229. (7-azabicyclo[2.2.1]heptan-7-yl)(7-phenylpyrazolo[1,5-*a*]pyridin-3-yl)methanone**

For Examples 227, 228, and 229, the compounds were obtained by the same synthetic method as step 1-2 in Example 1 using compound **158-1** and the corresponding compounds. The structural and spectral data are shown in Table 31 below.

[Table 31]

| Example | Structure | $^1$H NMR | MS |
|---|---|---|---|
| **227** | 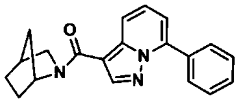 | $^1$H NMR (400 MHz, DMSO-$d_6$) δ(ppm): 8.42-8.25 (m, 2H), 7.91 (s, 2H), 7.59-7.49 (m, 4H), 7.19 (dd, *J* = 7.1, 1.5 Hz, 1H), 4.60 (d, *J* = 20.8 Hz, 1H), 3.80 (s, 1H), 3.44 (dd, *J* = 37.2, 9.6 Hz, 1H), 2.65 (s, 1H), 1.87-1.61 (m, 4H), 1.46 (d, *J* = 10.2 Hz, 2H). | MS (ESI, LR) Calc. for $C_{20}H_{20}N_3O$ $[M+H]^+$: 318.2, found: 318.1. |
| **228** | 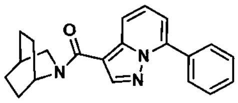 | $^1$H NMR (400 MHz, DMSO-$d_6$) δ(ppm): 8.28-8.12 (m, 1H), 7.91 (dd, *J* = 7.4, 2.3 Hz, 3H), 7.60-7.45 (m, 4H), 7.17 (s, 1H), 4.29 (d, *J* = 143.2 Hz, 1H), 3.80 (s, 1H), 3.50 (s, 1H), 1.96 (d, *J* = 40.8 Hz, 3H), 1.67 (s, 6H). | MS (ESI, LR) Calc. for $C_{21}H_{22}N_3O$ $[M+H]^+$: 332.2, found: 332.1. |
| **229** | 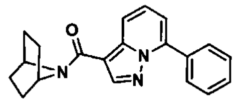 | $^1$H NMR (400 MHz, DMSO-$d_6$) δ(ppm): 8.41 (s, 1H), 8.02 (dd, *J* = 8.9, 1.4 Hz, 1H), 7.94-7.89 (m, 2H), 7.60-7.52 (m, 4H), 7.19 (dd, *J* = 7.1, 1.4 Hz, 1H), 4.53-4.45 (m, 2H), 1.85-1.74 (m, 4H), 1.50 (d, *J* = 7.1 Hz, 4H). | MS (ESI, LR) Calc. for $C_{20}H_{20}N_3O$ $[M+H]^+$: 318.2, found: 318.1. |

**Example 234. 6-(3-(4,4-difluoropiperidine-1-carbonyl)pyrazolo[1,5-*a*]pyridin-7-yl)isoindolin-1-one**

**Example 378. 6-(3-(3-fluoropiperidine-1-carbonyl)pyrazolo[1,5-*a*]pyridin-7-yl)isoindolin-1-one**

**Example 382. 6-(3-(3,3-difluoropiperidine-1-carbonyl)pyrazolo[1,5-*a*]pyridin-7-yl)isoindolin-1-one**

**Example 385. (*R*)-6-(3-(3-methylpiperidine-1-carbonyl)pyrazolo[1,5-*a*]pyridin-7-yl)isoindolin-1-one**

**Example 386. (*S*)-6-(3-(3-methylpiperidine-1-carbonyl)pyrazolo[1,5-*a*]pyridin-7-yl)isoindolin-1-one**

**Example 387. 6-(3-(3-methylpiperidine-1-carbonyl)pyrazolo[1,5-*a*]pyridin-7-yl)isoindolin-1-one**

**Example 390. 6-(3-(2-azaspiro[3.3]heptane-2-carbonyl)pyrazolo[1,5-*a*]pyridin-7-yl)isoindolin-1-one**

**Example 391. 6-(3-(6-azaspiro[3.4]octane-6-carbonyl)pyrazolo[1,5-*a*]pyridin-7-yl)isoindolin-1-one**

**Example 392. 6-(3-(7-azaspiro[3.5]nonane-7-carbonyl)pyrazolo[1,5-*a*]pyridin-7-yl)isoindolin-1-one**

**Example 393. 6-(3-(3-ethylpiperidine-1-carbonyl)pyrazolo[1,5-*a*]pyridin-7-yl)isoindolin-1-one**

**Example 398. 6-(3-(3-(trifluoromethyl)piperidine-1-carbonyl)pyrazolo[1,5-*a*]pyridin-7-yl)isoindolin-1-one**

**Example 400. 6-(3-(3-isopropylpiperidine-1-carbonyl)pyrazolo[1,5-*a*]pyridin-7-yl)isoindolin-1-one**

**Example 401. 6-(3-(1,2,3,6-tetrahydropyridine-1-carbonyl)pyrazolo[1,5-*a*]pyridin-7-yl)isoindolin-1-one**

**Example 405. 6-(3-(6-azaspiro[3.5]nonane-6-carbonyl)pyrazolo[1,5-*a*]pyridin-7-yl)isoindolin-1-one**

**Example 411. 6-(3-(6-fluoro-2-azaspiro[3.3]heptane-2-carbonyl)pyrazolo[1,5-*a*]pyridin-7-yl)isoindolin-1-one**

**Example 412. 6-(3-(6,6-difluoro-2-azaspiro[3.3]heptane-2-carbonyl)pyrazolo[1,5-*a*]pyridin-7-yl)isoindolin-1-one**

For Examples 234, 378, 382, 385, 386, 387, 390, 391, 392, 393, 398, 400, 401, 405, 411, and 412, the intermediates were obtained by the same synthetic method as step 1-2 in Example 1 using compound **1-1** and the corresponding compounds, and then the compounds were obtained by the same synthetic method as step 1-3 in Example 1 using 6-(4,4,5,5-tetramethyl-1,3,2-dioxaborolan-2-yl)isoindolin-1-one. The structural and spectral data are shown in Table 32 below.

[Table 32]

| Example | Structure | $^1$H NMR | MS |
|---|---|---|---|
| **234** | 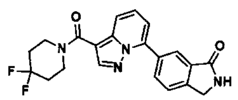 | $^1$H NMR (400 MHz, DMSO-$d_6$) δ(ppm): 8.70 (s, 1H), 8.35 (s, 1H), 8.25 (d, *J* = 1.7 Hz, 1H), 8.12 (dd, *J* = 8.0, 1.7 Hz, 1H), 7.96 (dd, *J* = 8.9, 1.4 Hz, 1H), 7.77 (d, *J* = 8.0 Hz, 1H), 7.56 (dd, *J* = 8.9, 7.0 Hz, 1H), 7.29 (dd, *J* = 7.0, 1.3 Hz, 1H), 4.50 (s, 2H), 3.78 (t, *J* = 5.9 Hz, 4H), 2.15-2.03 (m, 4H). | MS (ESI, LR) Calc. for $C_{21}H_{19}F_2N_4O_2$ $[M+H]^+$: 397.1, found: 397.0. |
| **378** | 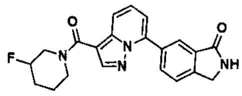 | $^1$H NMR (400 MHz, DMSO-$d_6$) δ(ppm): 8.71 (s, 1H), 8.31-8.19 (m, 2H), 8.12 (dd, *J* = 7.9, 1.7 Hz, 1H), 7.91 (dd, *J* = 8.9, 1.3 Hz, 1H), 7.77 (d, *J* = 7.9 Hz, 1H), 7.55 (dd, *J* = 8.9, 7.1 Hz, 1H), 7.27 (dd, *J* = 7.1, 1.3 Hz, 1H), 4.81 (d, *J* = 47.4 Hz, 1H), 4.14-3.92 (m, 2H), 3.71 (m, 2H), 2.00-1.84 (m, 2H), 1.80 (m, 1H), 1.64-1.49 (m, 1H). | MS (ESI, LR) Calc. for $C_{21}H_{20}FN_4O_2$ $[M+H]^+$: 379.1, found: 379.0. |
| **382** | 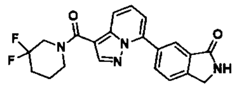 | $^1$H NMR (400 MHz, DMSO-$d_6$) δ(ppm): 8.71 (s, 1H), 8.31 (s, 1H), 8.25 (d, *J* = 1.7 Hz, 1H), 8.13 (dd, *J* = 7.9, 1.7 Hz, 1H), 7.92 (dd, *J* = 8.9, 1.3 Hz, 1H), 7.77 (d, *J* = 8.0 Hz, 1H), 7.58 (dd, *J* = 8.9, 7.1 Hz, 1H), 7.30 (dd, *J* = 7.1, 1.3 Hz, 1H), 4.50 (s, 2H), 4.00 (t, *J* = 12.0 Hz, 2H), 3.71 (t, *J* = 5.5 Hz, 2H), 2.13 (m, 2H), 1.78 (m, 2H). | MS (ESI, LR) Calc. for $C_{21}H_{19}F_2N_4O$; $[M+H]^+$: 397.1, found: 397.1. |
| **385** | 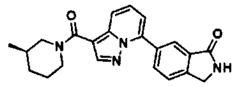 | $^1$H NMR (400 MHz, DMSO-$d_6$) δ(ppm): 8.71 (s, 1H), 8.25 (s, 2H), 8.12 (dd, *J* = 7.9, 1.7 Hz, IH), 7.91 (dd, *J* = 8.9, 1.4 Hz, 1H), 7.77 (d, *J* = 7.9 Hz, 1H), 7.53 (dd, *J* = 8.9, 7.0 Hz, 1H), 7.26 (dd, *J* = 7.0, 1.4 Hz, 1H), 4.50 (s, 2H), 4.19 (m, 2H), 3.00 (m, 1H), 2.69 (m, 1H), 1.82 (m, 1H), 1.67 (m, 2H), 1.48 (m, 1H), 1.20 (m, 1H), 0.89 (d, *J* = 6.6 Hz, 3H). | MS (ESI, LR) Calc. for $C_{22}H_{23}N_4O_2$ $[M+H]^+$: 375.2, found: 375.1. |
| **386** | 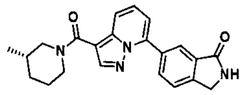 | $^1$H NMR (400 MHz, DMSO-$d_6$) δ(ppm): 8.71 (s, 1H), 8.25 (s, 1H), 8.12 (dd, *J* = 7.9, 1.7 Hz, 1H), 7.90 (dd, *J* = 8.9, 1.3 Hz, 1H), 7.76 (d, *J* = 8.0 Hz, 1H), 7.53 (dd, *J* = 8.9, 7.1 Hz, 1H), 7.26 (dd, *J* = 7.0, 1.4 Hz, 1H), 4.50 (s, 2H), 4.18 (m, 2H), 3.00 (m, 1H), 2.68 (m, 1H), 1.82 (m, 1H), 1.70 (m, 2H), 1.46 (m, 1H), 1.20 (m, 1H), 0.89 (d, *J* = 6.6 Hz, 3H). | MS (ESI, LR) Calc. for $C_{22}H_{23}N_4O_2$ $[M+H]^+$: 375.2, found: 375.1. |
| **387** | 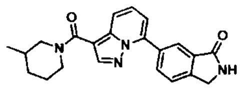 | $^1$H NMR (400 MHz, DMSO-$d_6$) δ(ppm): 8.71 (s, 1H), 8.25 (s, 2H), 8.12 (dd, *J* = 7.9, 1.7 Hz, 1H), 7.91 (dd, *J* = 8.9, 1.3 Hz, 1H), 7.76 (d, *J* = 8.0 Hz, 1H), 7.53 (dd, *J* = 8.9, 7.1 Hz, 1H), 7.26 (dd, *J*= 7.0, 1.4 Hz, 1H), 4.50 (s, 2H), 4.18 (s, 2H), 3.00 (s, 1H), 2.69 (s, 1H), 1.82 (d, *J* = 12.7 Hz, 1H), 1.67 (dd, *J* = 27.1, 10.3 Hz, 2H), 1.48 (q, *J*= 12.1 Hz, 1H), 1.26-1.11 (m, 1H), 0.89 (d, *J* = 6.5 Hz, 3H). | MS (ESI, LR) Calc. for $C_{22}H_{23}N_4O_2$ $[M+H]^+$: 375.2, found: 375.1. |
| **390** | 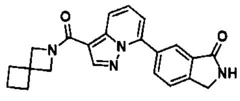 | $^1$H NMR(400 MHz, DMSO-$d_6$) δ(ppm): 8.71 (s, 1H), 8.33 (s, 1H), 8.29 (dd, *J* = 8.9, 1.4 Hz, 1H), 8.23 (d, *J* = 1.6 Hz, 1H), 8.11 (dd, *J* = 8.0, 1.7 Hz, 1H), 7.77 (d, *J* = 7.9 Hz, 1H), 7.59 (dd, *J* = 8.9, 7.1 Hz, 1H), 7.31 (dd, *J* = 7.1, 1.4 Hz, 1H), 4.50 (s, 2H), 4.44 (m, 2H), 4.04 (m, 2H), 2.20 (t, *J*= 7.6 Hz, 4H), 1.82 (m, 2H). | MS (ESI, LR) Calc. for $C_{22}H_{21}N_4O_2$ $[M+H]^+$: 373.2, found: 373.1. |

(continued)

| Example | Structure | $^1H$ NMR | MS |
|---|---|---|---|
| **391** | 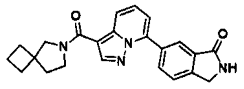 | $^1H$ NMR (400 MHz, DMSO-$d_6$) δ(ppm): 8.71 (s, 1H), 8.47 (s, 1H), 8.26 (d, *J* = 8.8 Hz, 2H), 8.14-8.07 (m, 1H), 7.77 (d, *J* = 8.0 Hz, 1H), 7.57 (dd, *J* = 8.9, 7.0 Hz, 1H), 7.30 (dd, *J* = 7.2, 1.4 Hz, 1H), 4.50 (s, 2H), 3.78 (m, 2H), 3.53 (m, 2H), 1.96 (m, 8H). | MS (ESI, LR) Calc. for $C_{23}H_{23}N_4O_2$ $[M+H]^+$: 387.22, found: 387.1. |
| **392** | 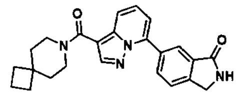 | $^1H$ NMR (400 MHz, DMSO-$d_6$) δ(ppm): 8.71 (s, 1H), 8.31-8.15 (m, 2H), 8.12 (dd, *J* = 8.0, 1.7 Hz, 1H), 7.90 (dd, *J* = 8.9, 1.4 Hz, 1H), 7.76 (d, *J* = 8.0 Hz, 1H), 7.53 (dd, *J* = 8.9, 7.1 Hz, 1H), 7.26 (dd, *J* = 7.1, 1.3 Hz, 1H), 4.50 (s, 2H), 3.63-3.49 (m, 4H), 1.89 (m, 2H), 1.80 (m, 4H), 1.65-1.48 (m, 4H). | MS (ESI, LR) Calc. for $C_{24}H_{25}N_4O_2$ $[M+H]^+$: 401.2, found: 401.1. |
| **393** | 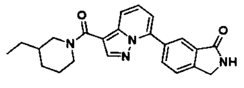 | $^1H$ NMR (400 MHz, DMSO-$d_6$) δ(ppm): 8.71 (s, 1H), 8.25 (s, 1H), 8.12 (dd, *J* = 7.9, 1.7 Hz, 1H), 7.91 (dd, *J* = 8.9, 1.4 Hz, 1H), 7.76 (d, *J* = 8.0 Hz, 1H), 7.53 (dd, *J* = 8.9, 7.0 Hz, 1H), 7.26 (dd, *J* = 7.0, 1.4 Hz, 1H), 4.49 (s, 2H), 4.22 (m, 2H), 3.03 (m, 1H), 2.70 (m, 1H), 1.88 (m, 1H), 1.71 (m, 1H), 1.46 (m, 2H), 1.23 (m, 3H), 0.88 (t, *J* = 7.4 Hz, 3H). | MS (ESI, LR) Calc. for $C_{23}H_{25}N_4O_2$ $[M+H]^+$: 389.2, found: 389.1. |
| **398** | 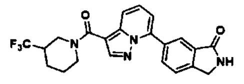 | $^1H$ NMR (400 MHz, DMSO-$d_6$) δ(ppm): 8.71 (s, 1H), 8.30 (s, 1H), 8.24 (d, *J* = 1.6 Hz, 1H), 8.12 (dd, *J* = 7.9, 1.7 Hz, 1H), 7.93 (dd, *J* = 8.9, 1.3 Hz, 1H), 7.77 (d, *J* = 8.0 Hz, 1H), 7.56 (dd, *J* = 8.9, 7.1 Hz, 1H), 7.28 (dd, *J* = 7.1, 1.4 Hz, 1H), 4.50 (m, 3H), 4.18 (m, 1H), 3.11 (d, *J* = 15.6 Hz, 2H), 2.67 (m, 1H), 2.02 (m, 1H), 1.79 (m, 1H), 1.69-1.48 (m, 2H). | MS (ESI, LR) Calc. for $C_{22}H_{20}F_3N_4O_2$ $[M+H]^+$: 429.1, found: 429.1. |
| **400** | 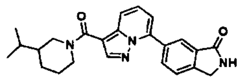 | $^1H$ NMR (400 MHz, DMSO-$d_6$) δ(ppm): 8.71 (s, 1H), 8.25 (d, *J* = 1.9 Hz, 2H), 8.12 (dd, *J* = 7.9, 1.7 Hz, 1H), 7.91 (dd, *J* = 8.9, 1.3 Hz, 1H), 7.77 (d, *J* = 8.0 Hz, 1H), 7.53 (dd, *J* = 8.9, 7.0 Hz, 1H), 7.26 (dd, *J* = 7.0, 1.4 Hz, 1H), 4.50 (s, 2H), 4.28 (m, 2H), 2.96 (m, 1H), 2.72 (m, 1H), 1.86 (m, 1H), 1.73 (m, 1H), 1.45 (m, 2H), 1.37-1.16 (m, 2H), 0.89 (t, *J* = 6.1 Hz, 6H). | MS (ESI, LR) Calc. for $C_{24}H_{27}N_4O_2$ $[M+H]^+$: 403.2, found: 403.1. |
| **401** | 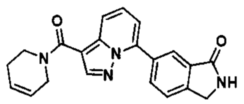 | $^1H$ NMR (400 MHz, DMSO-$d_6$) δ(ppm): 8.71 (s, 1H), 8.33 (s, 1H), 8.25 (d, *J* = 1.7 Hz, 1H), 8.12 (dd, *J* = 7.9, 1.7 Hz, 1H), 7.97 (dd, *J* = 9.0, 1.4 Hz, 1H), 7.77 (d, *J* = 8.0 Hz, 1H), 7.55 (dd, *J* = 8.9, 7.0 Hz, 1H), 7.28 (dd, *J* = 7.1, 1.4 Hz, 1H), 5.89 (d, *J* = 10.1 Hz, 1H), 5.75 (d, *J* = 9.6 Hz, 1H), 4.19 (s, 2H), 3.75 (t, *J* = 5.7 Hz, 2H), 2.24 (s, 2H). | MS (ESI, LR) Calc. for $C_{21}H_{19}N_4O_2$ $[M+H]^+$: 359.1, found: 359.1. |
| **405** | 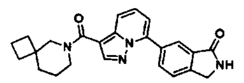 | $^1H$ NMR (400 MHz, DMSO-$d_6$) δ(ppm): 8.71 (s, 1H), 8.26 (m, 2H), 8.13 (dd, *J* = 7.9, 1.7 Hz, 1H), 7.89 (dd, *J* = 8.9, 1.4 Hz, 1H), 7.77 (d, *J* = 8.0 Hz, 1H), 7.53 (dd, *J* = 8.9, 7.0 Hz, 1H), 7.27 (dd, *J* = 7.0, 1.4 Hz, 1H), 4.50 (s, 2H), 3.62 (s, 2H), 3.57 (t, *J* = 5.6 Hz, 2H), 1.92-1.74 (m, 2H), 1.70-1.62 (m, 4H), 1.58-1.45 (m, 2H). | MS (ESI, LR) Calc. for $C_{24}H_{25}N_4O_2$ $[M+H]^+$: 401.2, found: 401.1. |
| **411** | 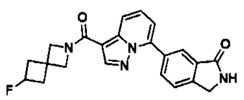 | $^1H$ NMR (400 MHz, DMSO-$d_6$) δ(ppm): 8.71 (s, 1H), 8.34-8.26 (m, 2H), 8.22 (d, *J* = 1.7 Hz, 1H), 8.10 (dd, *J* = 7.9, 1.7 Hz, 1H), 7.77 (d, *J* = 7.9 Hz, 1H), 7.60 (dd, *J* = 8.9, 7.1 Hz, 1H), 7.31 (dd, *J* = 7.1, 1.4 Hz, 1H), 5.13-4.89 (m, 1H), 4.50 (m, 2H), 4.08 (m, 2H), 2.67 (q, *J* = 9.6 Hz, 2H), 2.39 (ddd, *J* = 20.1, 11.4, 7.9 Hz, 2H). | MS (ESI, LR) Calc. for $C_{22}H_{20}FN_4O_2$ $[M+H]^+$: 391.1, found: 391.1. |

(continued)

| Example | Structure | $^1$H NMR | MS |
|---|---|---|---|
| **412** | 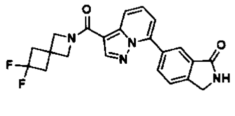 | $^1$H NMR (400 MHz, DMSO-$d_6$) δ(ppm): 8.72 (s, 1H), 8.35-8.25 (m, 2H), 8.23 (d, *J* = 1.7 Hz, 1H), 8.10 (dd, *J* = 8.0, 1.7 Hz, 1H), 7.77 (d, *J* = 7.9 Hz, 1H), 7.61 (dd, *J* = 8.9, 7.1 Hz, 1H), 7.32 (dd, *J* = 7.1, 1.4 Hz, 1H), 4.59 (s, 1H), 4.50 (s, 2H), 4.18 (s, 1H), 2.89 (t, *J* = 12.5 Hz, 4H). | MS (ESI, LR) Calc. for $C_{22}H_{19}F_2N_4O_2$ $[M+H]^+$: 409.1, found: 409.1. |

**Example 336. (7-(6-fluoropyridin-3-yl)pyrazolo[1,5-*a*]pyridin-3-yl)(3-methylpiperidin-1-yl)methanone**

**Example 337. (3-ethylpiperidin-1-yl)(7-(6-fluoropyridin-3-yl)pyrazolo[1,5-*a*]pyridin-3-yl)methanone**

**Example 338. (7-(6-fluoropyridin-3-yl)pyrazolo[1,5-*a*]pyridin-3-yl)(3-(trifluoromethyl)piperidin-1-yl)methanone**

For Examples 336, 337, and 338, the intermediates were obtained by the same synthetic method as step 1-2 in Example 1 using compound **1-1** and the corresponding compound, and then the compounds were obtained by the same synthetic method as step 1-3 in Example 1 using (6-fluoro-3-pyridyl)boronic acid. The structural and spectral data are shown in Table 33 below.

[Table 33]

| Example | Structure | $^1$H NMR | MS |
|---|---|---|---|
| **336** | 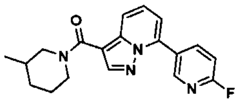 | $^1$H NMR (400 MHz, DMSO-$d_6$) δ(ppm): 8.78 (d, *J* = 2.5 Hz, 1H), 8.61 (td, *J* = 8.2, 2.5 Hz, 1H), 8.25 (s, 1H), 7.93 (dd, *J* = 8.9, 1.4 Hz, 1H), 7.54 (dd, *J* = 8.9, 7.0 Hz, 1H), 7.41 (dd,*J* = 8.6, 2.8 Hz, 1H), 7.30 (dd, *J* = 7.1, 1.4 Hz, 1H), 4.16 (m, 2H), 3.00 (m, 1H), 2.69 (m, 1H), 1.82 (d, *J* = 12.7 Hz, 1H), 1.75-1.55 (m, 2H), 1.47 (q, *J* = 12.2 Hz, 1H), 1.25-1.10 (m, 1H), 0.88 (d, *J* = 6.6 Hz, 3H). | MS (ESI, LR) Calc. for $C_{19}H_{20}FN_4O$ $[M+H]^+$: 339.2, found: 339.1. |
| **337** | 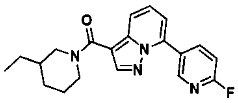 | $^1$H NMR (400 MHz, DMSO-$d_6$) δ(ppm): 8.78 (d, *J* = 2.5 Hz, 1H), 8.61 (td, *J* = 8.2, 2.6 Hz, 1H), 8.25 (s, 1H), 7.93 (dd, *J* = 8.9, 1.3 Hz, 1H), 7.54 (dd, *J* = 8.9, 7.0 Hz, 1H), 7.41 (dd, *J* = 8.6, 2.8 Hz, 1H), 7.30 (dd, *J* = 7.1, 1.3 Hz, 1H), 4.20 (m, 2H), 3.03 (m, 1H), 2.72 (m, 1H), 1.88 (d, *J* = 12.9 Hz, 1H), 1.71 (d, *J* = 13.1 Hz, 1H), 1.57-1.36 (m, 2H), 1.23 (dq, *J* = 19.1, 9.6 Hz, 3H), 0.88 (t, *J* = 7.5 Hz, 3H). | MS (ESI, LR) Calc. for $C_{20}H_{22}FN_4O$ $[M+H]^+$: 353.2, found: 353.1. |
| **338** | 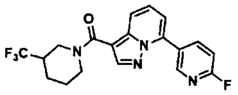 | $^1$H NMR (400 MHz, DMSO-$d_6$) δ(ppm): 8.78 (d, *J* = 2.5 Hz, 1H), 8.61 (td, *J* = 8.2, 2.5 Hz, 1H), 8.30 (s, 1H), 7.96 (dd, *J* = 8.9, 1.4 Hz, 1H), 7.57 (dd, *J* = 8.9, 7.1 Hz, 1H), 7.41 (dd, *J* = 8.6, 2.8 Hz, 1H), 7.32 (dd, *J* = 7.0, 1.3 Hz, 1H), 4.45 (d, *J* = 13.0 Hz, 1H), 4.17 (d, *J* = 13.3 Hz, 1H), 3.13 (dt, *J* = 23.3, 12.0 Hz, 2H), 2.67 (s, 1H), 2.07-1.96 (m, 1H), 1.79 (d, *J* = 12.3 Hz, 1H), 1.69-1.50 (m, 2H). | MS (ESI, LR) Calc. for $C_{19}H_{17}F_4N_4O$ $[M+H]^+$: 393.1, found: 393.1. |

**Example 339. 3-(5-(3-(3-(trifluoromethyl)piperidine-1-carbonyl)pyrazolo[1,5-*a*]pyridin-7-yl)pyridin-2-yl)oxazolidin-2-one**

**Example 353. 3-(5-(3-(3-methylpiperidine-1-carbonyl)pyrazolo[1,5-*a*]pyridin-7-yl)pyridin-2-yl)oxazolidin-2-one**

**Example 354. 3-(5-(3-(3-ethylpiperidine-1-carbonyl)pyrazolo[1,5-*a*]pyridin-7-yl)pyridin-2-yl)oxazolidin-2-one**

**Example 383. 3-(5-(3-(3-fluoropiperidine-1-carbonyl)pyrazolo[1,5-*a*]pyridin-7-yl)pyridin-2-yl)oxazolidin-2-one**

**Example 384. 3-(5-(3-(3,3-difluoropiperidine-1-carbonyl)pyrazolo[1,5-*a*]pyridin-7-yl)pyridin-2-yl)oxazolidin-2-one**

For Examples 339, 353, 354, 383, and 384, the intermediates were obtained by the same synthetic method as step 1-2 in Example 1 using compound **1-1** and the corresponding compounds, and then the compounds were obtained by the same synthetic method as Example 316. The structural and spectral data are shown in Table 34 below.

[Table 34]

| Example | Structure | $^1$H NMR | MS |
|---|---|---|---|
| **339** | 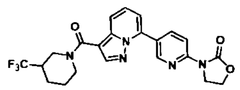 | $^1$H NMR (400 MHz, DMSO-$d_6$) δ(ppm): 8.93 (d, *J* = 2.3 Hz, 1H), 8.46 (dd, *J* = 8.9, 2.4 Hz, 1H), 8.30 (s, 1H), 8.26 (d, *J* = 8.7 Hz, 1H), 7.93 (dd, *J* = 8.9, 1.3 Hz, 1H), 7.56 (dd, *J* = 8.9, 7.1 Hz, 1H), 7.30 (dd, *J* = 7.1, 1.4 Hz, 1H), 4.52 (dd, *J* = 8.7, 7.3 Hz, 2H), 4.45 (d, *J* = 13.1 Hz, 1H), 4.27 (dd, *J* = 8.8, 7.3 Hz, 2H), 4.17 (d, *J* = 12.8 Hz, 1H), 3.11 (dd, *J* = 22.1, 10.8 Hz, 2H), 2.67 (s, 1H), 2.02 (d, *J* = 12.2 Hz, 1H), 1.79 (d, *J* = 12.1 Hz, 1H), 1.68-1.48 (m, 2H). | MS (ESI, LR) Calc. for $C_{20}H_{21}F_3N_5O_3$ $[M+H]^+$: 460.2, found: 460.1. |
| **353** | 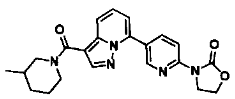 | $^1$H NMR (400 MHz, DMSO-$d_6$) δ(ppm): 8.99-8.87 (m, 1H), 8.46 (dd, *J* = 8.9, 2.4 Hz, 1H), 8.31-8.23 (m, 2H), 7.90 (dd, *J* = 8.9, 1.3 Hz, 1H), 7.53 (dd, *J* = 8.9, 7.1 Hz, 1H), 7.27 (dd, *J* = 7.0, 1.4 Hz, 1H), 4.52 (t, *J* = 8.0 Hz, 2H), 4.27 (dd, *J* = 8.8, 7.3 Hz, 2H), 4.17 (m, 2H), 3.01 (m, 1H), 2.69 (m, 1H), 1.82 (d, *J* = 13.1 Hz, 1H), 1.74-1.56 (m, 2H), 1.48 (q, *J* = 12.4 Hz, 1H), 1.29-1.14 (m, 1H), 0.89 (d, *J* = 6.6 Hz, 3H). | MS (ESI, LR) Calc. for $C_{22}H_{24}N_5O_3$ $[M+H]^+$: 406.2, found: 406.1. |
| **354** | 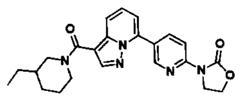 | $^1$H NMR (400 MHz, DMSO-$d_6$) δ(ppm): 9.03-8.78 (m, 1H), 8.46 (dd, *J* = 8.9, 2.4 Hz, 1H), 8.26 (d, *J* = 8.2 Hz, 2H), 7.90 (dd, *J* = 8.9, 1.3 Hz, 1H), 7.53 (dd, *J* = 8.9, 7.0 Hz, 1H), 7.27 (dd, *J* = 7.1, 1.3 Hz, 1H), 4.52 (dd, *J* = 8.7, 7.3 Hz, 2H), 4.27 (dd, *J* = 8.8, 7.3 Hz, 2H), 4.20 (m, 2H), 3.03 (m, 1H), 2.73 (m, 1H), 1.88 *(d, J*= 12.6 Hz, 1H), 1.71 (d, *J* = 13.2 Hz, 1H), 1.52-1.38 (m, 2H), 1.35-1.12 (m, 3H), 0.88 (t, *J* = 7.4 Hz, 3H). | MS (ESI, LR) Calc. for $C_{23}H_{26}N_5O_3$ $[M+H]^+$: 406.2, found: 406.1. |
| **383** | 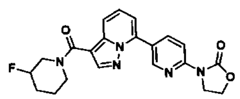 | $^1$H NMR (400 MHz, DMSO-$d_6$) δ(ppm): 9.00-8.86 (m, 1H), 8.46 (dd, *J* = 8.8, 2.5 Hz, 1H), 8.26 (d, *J* = 10.2 Hz, 1H), 7.91 (dd, *J* = 8.9, 1.3 Hz, 1H), 7.55 (dd, *J* = 8.9, 7.1 Hz, 1H), 7.47 (s, 2H), 7.34-7.25 (m, 1H), 4.81 (d, *J* = 47.1 Hz, 2H), 4.52 (t, *J* = 8.0 Hz, 2H), 4.14-3.91 (m, 2H), 3.76-3.61 (m, 1H), 3.57 (dd, *J* = 15.3, 7.1 Hz, 1H), 1.90 (m, 2H), 1.81 (m, 1H), 1.58 (m, 1H). | MS (ESI, LR) Calc. for $C_{21}H_{21}FN_5O_3$ $[M+H]^+$: 410.2, found: 410.1. |
| **384** | 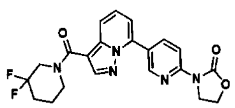 | $^1$H NMR (400 MHz, DMSO-$d_6$) δ(ppm): 8.92 (d, *J* = 2.4 Hz, 1H), 8.46 (dd, *J* = 8.9, 2.4 Hz, 1H), 8.32 (s, 1H), 8.26 (d, *J* = 8.9 Hz, 1H), 7.91 (dd, *J* = 8.9, 1.3 Hz, 1H), 7.57 (dd, *J* = 8.9, 7.1 Hz, 1H), 7.31 (dd, *J* = 7.1, 1.3 Hz, 1H), 4.52 (t, *J* = 8.0 Hz, 2H), 3.99 (t, *J* = 12.0 Hz, 2H), 3.70 (t, *J* = 5.5 Hz, 2H), 2.13 (m, 2H), 1.78 (m, 2H). | MS (ESI, LR) Calc. for $C_{21}H_{20}F_2N_5O_3$ $[M+H]^+$: 428.1, found: 428.0. |

**Example 340. *N*-(2-nitrobenzyl)-*N*-(4-(3-(piperidine-1-carbonyl)pyrazolo|[1,5-*a*]pyridin-7-yl)phenyl)nicotinamide**

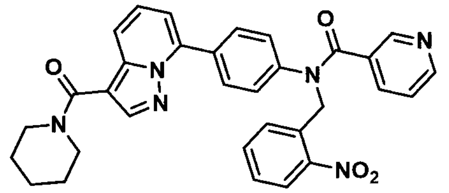

The target compound **340** was obtained by the synthetic method in Example 132 using compound **101** and the corresponding compounds.

$^1$H NMR (400 MHz, DMSO-$d_6$) δ(ppm): 8.61 (d, *J* = 2.2 Hz, 1H), 8.52 (dd, *J* = 4.9, 1.7 Hz, 1H), 8.18 (s, 1H), 8.06 (dd, *J* = 8.1, 1.3 Hz, 1H), 7.89-7.81 (m, 5H), 7.78 (td, *J* = 7.6, 1.3 Hz, 1H), 7.61-7.53 (m, 1H), 7.45 (dd, *J* = 8.7, 6.6 Hz, 3H), 7.35 (dd, *J* = 7.9, 4.9 Hz, 1H), 7.10 (dd, *J* = 7.1, 1.4 Hz, 1H), 5.50 (s, 2H), 3.59 (t, *J* = 5.4 Hz, 4H), 1.63 (m, 2H), 1.55 (m, 4H).

MS (ESI, LR) Calc. for $C_{32}H_{29}N_6O_4$ [M+H]$^+$: 561.2, found: 561.1.

**Example 356. (7-(2-(4-amino-1*H*-pyrazol-1-yl)-6-bromopyridin-4-yl)pyrazolo|[1,5-*a*]pyridin-3-yl)(piperidin-1-yl) methanone**

<Scheme 46>

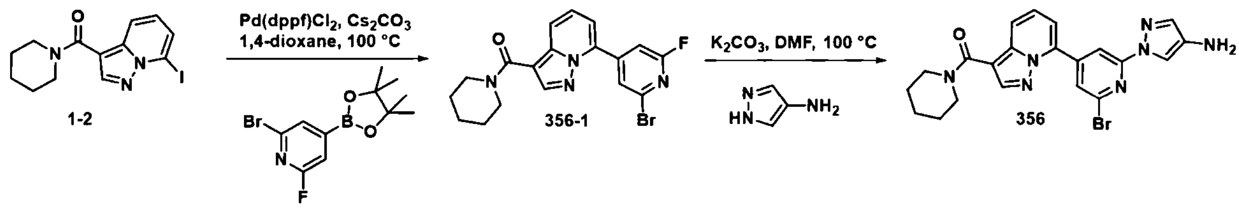

1-2
356-1
356

Step 356-1: Using compound **1-2** and 2-bromo-6-fluoro-4-(4,4,5,5-tetramethyl-1,3,2-dioxaborolan-2-yl)pyridine, the target compound **356-1** was obtained by the same synthetic method as step 159-3 in Example 159.

Step 356-2: The target compound **356** was obtained by the synthetic method in Example 344 using compound **356-1.**

$^1$H NMR (400 MHz, DMSO-$d_6$) δ(ppm): 8.42 (d, *J* = 1.3 Hz, 1H), 8.32 (s, 1H), 8.02-7.97 (m, 2H), 7.82 (d, *J* = 0.9 Hz, 1H), 7.55 (dd, *J* = 8.8, 7.1 Hz, 1H), 7.48 (dd, *J* = 7.1, 1.5 Hz, 1H), 7.46-7.42 (m, 1H), 4.44 (s, 2H), 3.63 (t, *J* = 5.4 Hz, 4H), 1.66 (d, *J* = 5.7 Hz, 2H), 1.58 (d, *J* = 7.0 Hz, 4H).

MS (ESI, LR) Calc. for $C_{21}H_{21}BrN_7O$ [M+H]$^+$: 466.1, found: 466.0.

**Example 363. (7-(5-bromo-2-(3-(hydroxymethyl)azetidin-1-yl)pyridin-3-yl)pyrazolo[1,5-*a*]pyridin-3-yl)(piperidin-1-yl)methanone**

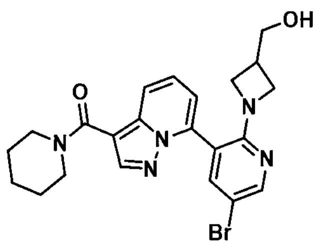

The target compound **363** was obtained by the same synthetic method as Example 221 using compound 356-1.

$^1$H NMR (400 MHz, DMSO-$d_6$) δ(ppm): 8.31 (d, *J* = 2.4 Hz, 1H), 8.24 (s, 1H), 7.92 (dd, *J* = 9.0, 1.4 Hz, 1H), 7.80 (d, *J* = 2.4 Hz, 1H), 7.49 (dd, *J* = 8.9, 6.9 Hz, 1H), 7.11 (dd, *J* = 7.0, 1.4 Hz, 1H), 4.56 (t, *J* = 5.2 Hz, 1H), 3.62 (t, *J* = 5.5 Hz, 4H), 3.18 (m, 2H), 2.48-2.36 (m, 1H), 1.66 (m, 2H), 1.57 (m, 4H).

MS (ESI, LR) Calc. for $C_{22}H_{25}BrN_5O_2$ [M+H]$^+$: 470.1, found: 470.1.

**Example 345. (7-(3-(3-hydroxyazetidin-1-yl)-5-(isoxazol-4-yl)phenyl)pyrazolo[1,5-*a*]pyridin-3-yl)(piperidin-1-yl)methanone**

**Example 357. (7-(2-(3-hydroxyazetidin-1-yl)-6-(1-methyl-1*H*-pyrazol-5-yl)pyridin-4-yl)pyrazolo[1,5-*a*]pyridin-3-yl)(piperidin-1-yl)methanone**

**Example 366. (7-(2-(isoxazol-4-yl)-6-morpholinopyridin-4-yl)pyrazolo[1,5-*a*]pyridin-3-yl)(piperidin-1-yl)methanone**

**Example 369. (7-(2-(1-methyl-1*H*-pyrazol-5-yl)-6-morpholinopyridin-4-yl)pyrazolo[1,5-*a*]pyridin-3-yl)(piperidin-1-yl)methanone**

**Example 371. (7-(2-(3,5-dimethylisoxazol-4-yl)-6-morpholinopyridin-4-yl)pyrazolo[1,5-*a*]pyridin-3-yl)(piperidin-1-yl)methanone**

**Example 376. (7-(5'-fluoro-6-morpholino-[2,3'-bipyridin]-4-yl)pyrazolo[1,5-*a*]pyridin-3-yl)(piperidin-1-yl)methanone**

**Example 377. (7-(2-(2-methylpyrimidin-5-yl)-6-morpholinopyridin-4-yl)pyrazolo[1,5-*a*]pyridin-3-yl)(piperidin-1-yl)methanone**

**Example 367. 3-(6-(1-methyl-1*H*-pyrazol-5-yl)-4-(3-(piperidine-1-carbonyl)pyrazolo[1,5-*a*]pyridin-7-yl)pyridin-2-yl)oxazolidin-2-one**

**Example 368. 3-(6-(3,5-dimethylisoxazol-4-yl)-4-(3-(piperidine-1-carbonyl)pyrazolo[1,5-*a*]pyridin-7-yl)pyridin-2-yl)oxazolidin-2-one**

**Example 370. 3-(5'-fluoro-4-(3-(piperidine-1-carbonyl)pyrazolo[1,5-*a*]pyridin-7-yl)-[2,3'-bipyridin]-6-yl)oxazolidin-2-one**

**Example 372. 3-(6-(2-methylpyrimidin-5-yl)-4-(3-(piperidine-1-carbonyl)pyrazolo[1,5-*a*]pyridin-7-yl)pyridin-2-yl)oxazolidin-2-one**

**Example 375. (7-(6-(3-(dimethylamino)azetidin-1-yl)-5'-fluoro-[2,3'-bipyridin]-4-yl)pyrazolo[1,5-*a*]pyridin-3-yl)(piperidin-1-yl)methanone**

For Examples 345, 357, 366, 369, 371, 376, 377, 367, 368, 370, 372, and 375, the intermediates were obtained by the same synthetic method as in step 356-2 in Example 356 using compound **356-1** and the corresponding compounds, and then the compounds were obtained by using the method for synthesizing Example 344. The structural and spectral data are shown in Table 35 below.

[Table 35]

| Example | Structure | $^1$H NMR | MS |
|---|---|---|---|
| **345** | 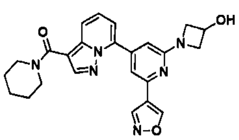 | $^1$H NMR (400 MHz, DMSO-$d_6$) δ(ppm): 9.47 (s, 1H), 9.13 (s, 1H), 8.27 (s, 1H), 7.95 (dd, *J* = 9.0, 1.4 Hz, 1H), 7.57-7.50 (m, 2H), 7.30 (d, *J* = 6.9 Hz, 1H), 6.86 (d, *J* = 1.3 Hz, 1H), 5.67 (d, *J* = 6.5 Hz, 1H), 4.67-4.60 (m, 1H), 4.31-4.22 (m, 2H), 3.81 (dd, *J* = 9.0, 4.7 Hz, 2H), 3.63 (t, *J* = 5.5 Hz, 4H), 1.62 (dd, *J* = 34.0, 6.2 Hz, 6H). | MS (ESI, LR) Calc. for $C_{25}H_{26}N_5O_3$ $[M+H]^+$: 445.2, found: 445.1. |
| **357** | 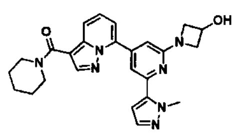 | $^1$H NMR (400 MHz, DMSO-$d_6$) δ(ppm): 8.26 (s, 1H), 7.94 (dd, *J* = 8.9, 1.3 Hz, 1H), 7.56-7.49 (m, 2H), 7.45 (d, *J* = 1.9 Hz, 1H), 7.33 (dd, *J* = 7.2, 1.4 Hz, 1H), 6.88 (d, *J* = 1.2 Hz, 1H), 6.75 (d, *J* = 2.0 Hz, 1H), 5.69 (d, *J* = 6.4 Hz, 1H), 4.70-4.60 (m, 1H), 4.32-4.25 (m, 2H), 4.21 (s, 3H), 3.82 (dd, *J* = 8.9, 4.7 Hz, 2H), 3.63 (t, *J* = 5.4 Hz, 4H), 1.66 (d, *J* = 7.2 Hz, 2H), 1.57 (d, *J*= 7.4 Hz, 4H). | MS (ESI, LR) Calc. for $C_{25}H_{28}N_7O_2$ $[M+H]^+$: 458.2, found: 458.1. |

(continued)

| Example | Structure | $^1$H NMR | MS |
|---|---|---|---|
| **366** | 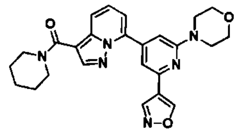 | $^1$H NMR (400 MHz, DMSO-$d_6$) δ(ppm): 8.73 (s, 1H), 8.30 (s, 1H), 7.97 (d, *J* = 8.8 Hz, 1H), 7.57-7.50 (m, 2H), 7.35 (d, *J* = 6.9 Hz, 1H), 7.18 (s, 1H), 7.10 (s, 1H), 3.80 (t, *J* = 5.0 Hz, 4H), 3.61 (d, *J* = 15.5 Hz, 8H), 1.62 (d, *J* = 33.7 Hz, 6H). | MS (ESI, LR) Calc. for $C_{25}H_{27}N_6O_3$ $[M+H]^+$: 459.2, found: 459.1. |
| **369** | 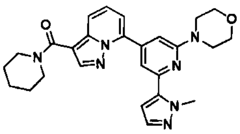 | $^1$H NMR (400 MHz, DMSO-$d_6$) δ(ppm): 8.26 (s, 1H), 7.95 (dd, *J* = 8.9, 1.3 Hz, 1H), 7.58-7.51 (m, 2H), 7.46 (d, *J* = 2.0 Hz, 1H), 7.34 (d, *J* = 8.3 Hz, 2H), 6.77 (d, *J* = 2.0 Hz, 1H), 4.19 (s, 3H), 3.76 (t, *J* = 4.8 Hz, 4H), 3.61 (dt, *J* = 13.8, 5.1 Hz, 8H), 1.65 (d, *J* = 7.6 Hz, 2H), 1.57 (s, 4H). | MS (ESI, LR) Calc. for $C_{26}H_{30}N_7O_2$ $[M+H]^+$: 472.2, found: 472.1. |
| **371** | 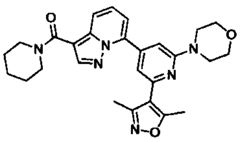 | $^1$H NMR (400 MHz, DMSO-$d_6$) δ(ppm): 8.25 (s, 1H), 7.95 (dd, *J* = 8.9, 1.3 Hz, 1H), 7.54 (dd, *J* = 8.9, 7.0 Hz, 1H), 7.37-7.30 (m, 2H), 7.27 (s, 1H), 3.75 (t, *J* = 5.0 Hz, 4H), 3.63 (t, *J* = 5.4 Hz, 4H), 3.58 (t, *J* = 4.9 Hz, 4H), 2.61 (s, 3H), 2.43 (s, 3H), 1.66 (d, *J* = 5.1 Hz, 2H), 1.57 (s, 4H). | MS (ESI, LR) Calc. for $C_{27}H_{31}N_6O_3$ $[M+H]^+$: 487.2, found: 487.1. |
| **376** | 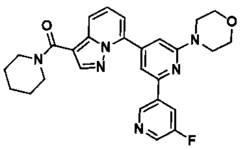 | $^1$H NMR (400 MHz, DMSO-$d_6$) δ(ppm): 9.20 (s, 1H), 8.63 (d, *J* = 2.8 Hz, 1H), 8.40-8.34 (m, 1H), 8.28 (s, 1H), 7.96 (dd, *J* = 8.9, 1.3 Hz, 1H), 7.89 (s, 1H), 7.56 (dd, *J* = 8.9, 7.0 Hz, 1H), 7.46 (s, 1H), 7.39 (d, *J* = 6.9 Hz, 1H), 3.77 (t, *J* = 4.9 Hz, 4H), 3.64 (q, *J* = 5.9 Hz, 8H), 1.66 (s, 2H), 1.58 (s, 4H). | MS (ESI, LR) Calc. for $C_{27}H_{28}FN_6O_2$ $[M+H]^+$: 487.2, found: 487.1. |
| **377** | 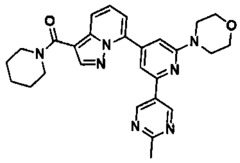 | $^1$H NMR (400 MHz, DMSO-$d_6$) δ(ppm): 9.35 (s, 2H), 8.27 (s, 1H), 7.96 (dd, *J* = 8.9, 1.4 Hz, 1H), 7.84 (s, 1H), 7.55 (dd, *J* = 9.0, 7.0 Hz, 1H), 7.44 (s, 1H), 7.37 (dd, *J* = 7.1, 1.4 Hz, 1H), 3.80-3.74 (m, 4H), 3.64 (q, *J* = 4.8 Hz, 8H), 2.69 (s, 3H), 1.66 (d, *J* = 5.6 Hz, 2H), 1.63-1.54 (m, 4H). | MS (ESI, LR) Calc. for $C_{27}H_{30}N_7O_2$ $[M+H]^+$: 484.2, found: 484.2. |
| **367** | 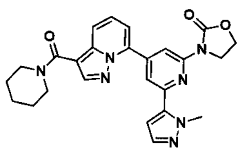 | $^1$H NMR (400 MHz, DMSO-$d_6$) δ(ppm): 8.59 (d, *J* = 1.3 Hz, 1H), 8.28 (s, 1H), 8.06 (d, *J* = 1.3 Hz, 1H), 7.99 (dd, *J* = 9.0, 1.4 Hz, 1H), 7.57 (t, *J* = 1.6 Hz, 1H), 7.52 (d, *J* = 2.1 Hz, 1H), 7.38 (dd, *J* = 7.0, 1.4 Hz, 1H), 6.95 (d, *J* = 2.0 Hz, 1H), 4.55 (t, *J* = 8.0 Hz, 2H), 4.37 (t, *J* = 8.0 Hz, 2H), 4.24 (s, 3H), 3.63 (t, *J* = 5.4 Hz, 4H), 1.66 (s, 2H), 1.58 (s, 4H). | MS (ESI, LR) Calc. for $C_{25}H_{26}N_7O_3$ $[M+H]^+$: 472.2, found: 472.1. |
| **368** | 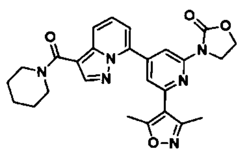 | $^1$H NMR (400 MHz, DMSO-$d_6$) δ(ppm): 8.54 (d, *J* = 1.2 Hz, 1H), 8.27 (s, 1H), 7.98 (dd, *J* = 8.9, 1.3 Hz, 1H), 7.83 (d, *J* = 1.2 Hz, 1H), 7.57 (dd, *J* = 9.0, 7.0 Hz, 1H), 7.35 (dd, *J* = 7.0, 1.4 Hz, 1H), 4.53 (t, *J* = 8.0 Hz, 2H), 4.32 (t, *J* = 8.0 Hz, 2H), 3.63 (t, *J* = 5.4 Hz, 4H), 2.67 (s, 3H), 2.48 (s, 3H), 1.66 (d, *J* = 5.5 Hz, 2H), 1.57 (s, 4H). | MS (ESI, LR) Calc. for $C_{26}H_{27}N_6O_4$ $[M+H]^+$: 487.2, found: 487.1. |
| **370** | 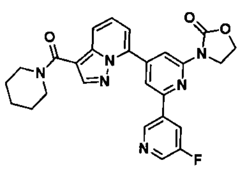 | $^1$H NMR (400 MHz, DMSO-$d_6$) δ(ppm): 9.28 (d, *J* = 1.8 Hz, 1H), 8.74 (d, *J* = 1.2 Hz, 1H), 8.70 (d, *J* = 2.8 Hz, 1H), 8.49-8.43 (m, 1H), 8.37 (d, *J* = 1.3 Hz, 1H), 8.29 (s, 1H), 8.00 (dd, *J* = 8.9, 1.4 Hz, 1H), 7.59 (dd, *J* = 9.0, 7.1 Hz, 1H), 7.44 (dd, *J* = 7.1, 1.4 Hz, 1H), 4.56 (t, *J* = 7.6 Hz, 2H), 4.43 (t, *J* = 7.7 Hz, 2H), 3.64 (t, *J* = 5.4 Hz, 4H), 1.66 (d, *J* = 8.0 Hz, 2H), 1.58 (s, 4H). | MS (ESI, LR) Calc. for $C_{26}H_{24}FN_6O_3$ $[M+H]^+$: 487.2, found: 487.1. |
| **372** | 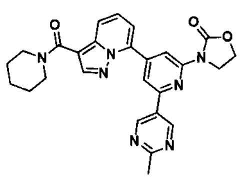 | $^1$H NMR (400 MHz, DMSO-$d_6$) δ(ppm): 9.43 (s, 2H), 8.72 (d, *J* = 1.2 Hz, 1H), 8.33 (d, *J* = 1.3 Hz, 1H), 8.29 (s, 1H), 8.00 (dd, *J* = 8.9, 1.3 Hz, 1H), 7.59 (dd, *J* = 9.0, 7.0 Hz, 1H), 7.42 (dd, *J* = 7.1, 1.4 Hz, 1H), 4.56 (t, *J* = 7.9 Hz, 2H), 4.41 (t, *J* = 7.8 Hz, 2H), 3.64 (t, *J* = 5.4 Hz, 4H), 2.72 (s, 3H), 1.66 (d, *J* = 7.7 Hz, 2H), 1.58 (s, 4H). | MS (ESI, LR) Calc. for $C_{26}H_{26}N_7O_3$ $[M+H]^+$: 484.2, found: 484.1. |

(continued)

| Example | Structure | $^1H$ NMR | MS |
|---|---|---|---|
| **375** | 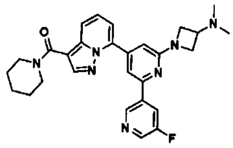 | $^1H$ NMR (400 MHz, DMSO-$d_6$) δ(ppm): 9.18 (t, *J* = 1.8 Hz, 1H), 8.63 (d, *J* = 2.8 Hz, 1H), 8.36-8.30 (m, 1H), 8.28 (s, 1H), 7.96 (dd, *J* = 8.9, 1.4 Hz, 1H), 7.82 (d, *J* = 1.2 Hz, 1H), 7.59 (s, 1H), 7.40 (dd, *J* = 7.1, 1.4 Hz, 1H), 7.04 (d, *J* = 1.2 Hz, 1H), 4.15 (dd, *J* = 8.5, 6.9 Hz, 2H), 3.90 (dd, *J* = 8.6, 5.3 Hz, 2H), 3.63 (t, *J* = 5.4 Hz, 4H), 3.25 (d, *J* = 5.7 Hz, 1H), 2.15 (s, 6H), 1.66 (d, *J* = 6.1 Hz, 2H), 1.58 (d, *J* = 6.7 Hz, 4H). | MS (ESI, LR) Calc. for $C_{28}H_{31}FN_7O$ $[M+H]^+$: 500.2, found: 500.1. |

**Example 325. (7-(2-fluoro-5-(1-methyl-1*H*-pyrazol-5-yl)pyridin-3-yl)pyrazolo[1,5-*a*]pyridin-3-yl)(piperidin-1-yl) methanone**

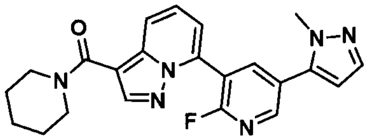

The target compound **325** was obtained by the same synthetic method as step 159-3 in Example 159 using compound **265** and (2-methylpyrazol-3-yl)boronic acid.

$^1H$ NMR (400 MHz, DMSO-$d_6$) δ(ppm): 8.64 (d, *J* = 2.4 Hz, 1H), 8.54 (dd, *J* = 8.7, 2.5 Hz, 1H), 8.24 (s, 1H), 8.00 (dd, *J* = 8.9, 1.3 Hz, 1H), 7.63-7.49 (m, 2H), 7.36 (d, *J* = 7.0 Hz, 1H), 6.64 (d, *J* = 1.9 Hz, 1H), 3.95 (s, 3H), 3.63 (t, *J* = 5.4 Hz, 4H), 1.66 (m, 2H), 1.58 (m, 4H).

MS (ESI, LR) Calc. for $C_{21}H_{23}FN_6O$ $[M+H]^+$: 405.2, found: 405.1.

**Example 381. [7-[6-(3,5-dimethylisoxazol-4-yl)-5-fluoro-3-pyridyl]pyrazolo[1,5-*a*]pyridin-3-yl]-(1-piperidyl) methanone**

**Example 399. [7-[2-(3,5-dimethylisoxazol-4-yl)pyrimidin-5-yl]pyrazolo[1,5-*a*]pyridin-3-yl]-(1-piperidyl)methanone**

For Examples 381 and 399, the compounds were obtained from compounds **281** and **267,** respectively, by the same synthetic method as in step 159-3 of Example 159, using (3,5-dimethylisoxazol-4-yl)boronic acid. The structural and spectral data are shown in Table 36 below.

[Table 36]

| Example | Structure | $^1H$ NMR | MS |
|---|---|---|---|
| **381** | 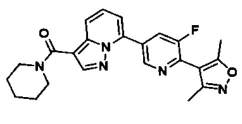 | $^1H$ NMR (400 MHz, DMSO-$d_6$) δ(ppm): 9.12 (t, *J*= 1.7 Hz, 1H), 8.57 (dd, *J* = 11.2, 1.8 Hz, 1H), 8.31 (s, 1H), 7.97 (dd, *J* = 8.9, 1.4 Hz, 1H), 7.57 (dd, *J* = 8.9, 7.1 Hz, 1H), 7.44 (dd, *J* = 7.1, 1.4 Hz, 1H), 3.64 (t, *J*= 5.4 Hz, 4H), 2.50 (s, 3H), 2.33 (s, 3H), 1.66 (d, *J* = 5.1 Hz, 2H), 1.58 (s, 4H). | MS (ESI, LR) Calc. for $C_{23}H_{23}FN_3O_2$ $[M+H]^+$: 420.2, found: 420.1. |
| **399** | 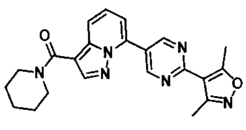 | $^1H$ NMR (400 MHz, DMSO-$d_6$) δ(ppm): 9.47 (s, 2H), 8.30 (s, 1H), 7.95 (dd, *J* = 8.8, 1.0 Hz, 1H), 7.57 (dd, *J* = 8.8, 7.1 H, 1H), 7.46-7.45 (m, 1H), 3.63 (t, *J* = 5.2 Hz, 4H), 2.83 (s, 3H), 2.57 (s, 3H), 1.66 (d, *J* = 4.6 Hz, 2H), 1.58 (d, *J* = 3.7 Hz, 4H). | MS (ESI, LR) Calc. for $C_{22}H_{23}N_6O_2$ $[M+H]^+$: 403.2, found: 403.1. |

**Example 404. [7-[2-[5-(difluoromethyl)-1-oxo-1,3,4-thiadiazol-2-yl]pyrimidin-5-yl]pyrazolo[1,5-*a*]pyridin-3-yl]-(1-piperidyl)methanone**

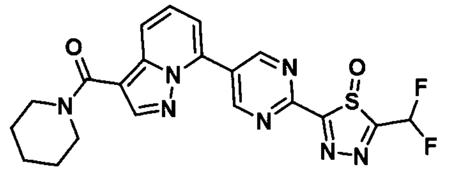

The target compound **404** was obtained by the same synthetic method as in step 159-3 of Example 159, using compound 404-1 and [5-(difluoromethyl)-1,3,4-thiadiazol-2-yl]boronic acid and potassium carbonate.

$^{1}$H NMR (400 MHz, DMSO-$d_6$) δ(ppm): 9.56 (s, 2H), 8.31 (s, 1H), 7.99 (d, *J* = 8.8 Hz, 1H), 7.61-7.57 (m, 1H), 7.50 (d, *J* = 6.4 Hz, 1H), 7.33 (t, *J*= 52.8 Hz, 1H), 3.63 (t, *J* = 5.0 Hz, 4H), 1.66 (d, *J* = 4.4 Hz, 2H), 1.57 (d, *J* = 3.6 Hz, 4H).

MS (ESI, LR) Calc. for $C_{20}H_{18}F_2N_7O_2S$ $[M+H]^+$: 458.1, found: 458.1.

**Example 159. 7-(2-amino-3-(piperidine-1-carbonyl)pyrazolo[1,5-*a*]pyridin-7-yl)-3,4-dihydroisoquinolin-1(2*H*)-one**

<Scheme 47>

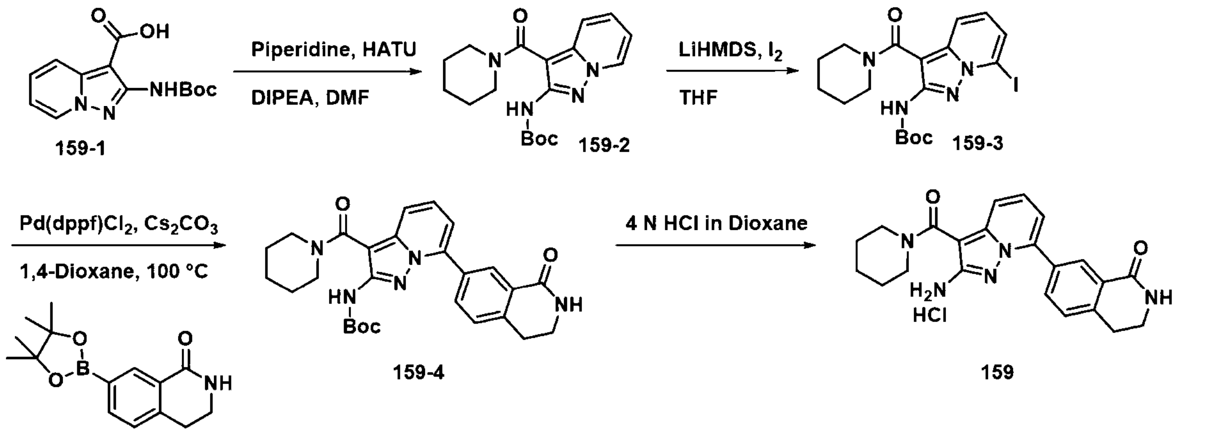


Step 159-1: Using compound **159-1** (Synthesized with reference to literature "Belarousai, R. et al. Synthesis 2013, 45, 2557-2566. Convenient Synthesis of New N-3-Substituted Pyrido[1',2':1,5]pyrazolo[3,4-*d*]pyrimidine-2,4(1*H*,3*H*)dione Derivatives"), target compound **159-2** was obtained by the same synthetic method as steps 1-2 in Example **1.**

Step 159-2: Using compound **159-2,** target compound **159-3** was obtained by the same synthetic method as step 1-1 in Example **1.**

Step 159-3: After compound **159-3** (0.118 g, 0.25 mmol) was dissolved in 1,4-dioxane (5 mL), 7-(4,4,5,5,-tetramethyl- 1,3,2-dioxaborolan-2-yl)-3,4-dihydro-2*H*-isoquinolin-1-one (0.082 g, 0.3 mmol) and [1,1'-bis(diphenylphosphino) ferrocene]dichloropalladium (0.021 g, 0.03 mmol) were added to 2 M cesium carbonate aqueous solution (0.38 mL, 0.75 mmol). The reaction solution was filled with nitrogen and stirred at 100 °C for 12 hours. After the reaction was completed, the temperature was lowered to room temperature, diluted with dichloromethane (10 mL), and palladium was removed using diatomaceous earth (Celite). The organic layer was washed with water and brine, dried over anhydrous magnesium sulfate, filtered, and concentrated. The residue was separated using column chromatography to obtain the target compound **159-4** (117 mg, 95%).

Step 159-4: To compound **159-4** (50 mg, 0.1 mmol), a hydrochloric acid solution (2.56 mL) containing 1,4-dioxane at a concentration of 4 N was added, and the mixture was stirred for 2 hours. After completion of the reaction, the reaction solution was concentrated and separated using column chromatography to obtain the target compound **159** (25.1 mg, 63%).

$^1$H NMR (400 MHz, DMSO-$d_6$) δ(ppm): 8.25 (d, *J* = 2.0 Hz, 1H), 8.05 (d, *J* = 3.0 Hz, 1H), 7.92 (dd, *J* = 7.8, 2.0 Hz, 1H), 7.46 (d, *J* = 7.9 Hz, 1H), 7.40-7.29 (m, 2H), 6.84 (dd, *J* = 6.2, 2.3 Hz, 1H), 5.62 (s, 2H), 3.45 (dq, *J* = 13.3, 4.1 Hz, 6H), 3.00 (t, *J* = 6.5 Hz, 2H), 1.62-1.63 (m, 2H), 1.56-1.54 (m, 4H).

MS (ESI, LR) Calc. for $C_{22}H_{24}N_5O_2$ $[M+H]^+$: 390.2, found: 390.2.

**Example 160. 7-(2-amino-3-(piperidine-1-carbonyl)pyrazolo[1,5-*a*]pyridin-7-yl)-2-methyl-3,4-dihydroisoquinolin-1(2*H*)-one**

<Scheme 48>

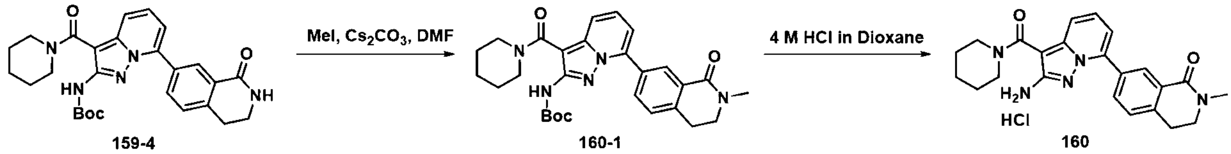


159-4 160-1 160

Step 160-1: Compound **159-4** (0.078 g, 0.16 mmol) was dissolved in *N,N*-dimethylformamide (3 mL), methyl iodide (11.8 μL, 0.19 mmol), and cesium carbonate (0.062 g, 0.19 mmol) were added, and stirred for 12 hours. After completion of the reaction, the mixture was filtered using diatomaceous earth (Celite) and washed with ethyl acetate. The filtrate was concentrated and separated using column chromatography to obtain the target compound **160-1** (75 mg, 94%).

Step 160-2: Using compound **160-1,** the target compound **160** was obtained by the same synthetic method as in step 159-4 in Example **159.**

$^1$H NMR (400 MHz, DMSO-$d_6$) δ(ppm): 8.44 (d, *J* = 2.0 Hz, 1H), 8.08 (dd, *J* = 7.9, 2.0 Hz, 1H), 8.01 (d, *J* = 3.0 Hz, 1H), 7.47 (d, *J* = 8.0 Hz, 1H), 7.37 (dd, *J* = 8.8, 7.0 Hz, 1H), 7.30 (dd, *J* = 8.8, 1.5 Hz, 1H), 6.89 (dd, *J* = 7.0, 1.5 Hz, 1H), 5.75 (q, *J* = 4.8 Hz, 1H), 3.45 (tt, *J* = 6.6, 3.0 Hz, 5H), 3.00 (t, *J* = 6.6 Hz, 2H), 2.79 (d, *J* = 5.0 Hz, 3H), 1.62 (d, *J* = 7.0 Hz, 2H).

MS (ESI, LR) Calc. for $C_{23}H_{26}N_5O_2$ $[M+H]^+$: 404.2, found: 404.1.

**Example 161. (7-(3-chlorophenyl)-1*H*-indazol-3-yl)(piperidin-1-yl)methanone**

<Scheme 49>

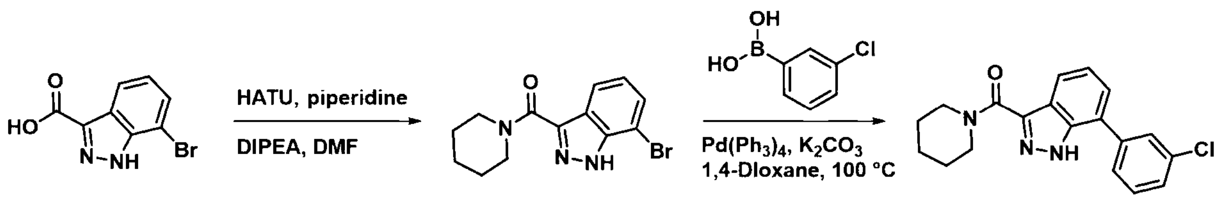


The target compound **161** was obtained by the same synthetic method as Example **154** using 7-bromo-1*H*-indazole-3-carboxylic acid and the corresponding compounds.

$^1$H NMR (400 MHz, DMSO-$d_6$) δ(ppm): 7.96 (d, *J* = 8.0 Hz, 1H), 7.75 (s, 1H), 7.71-7.65 (m, 1H), 7.58 (t, *J* = 7.7 Hz, 1H), 7.55-7.45 (m, 2H), 7.32 (dd, *J* = 8.2, 7.1 Hz, 1H), 3.79 (d, *J* = 63.1 Hz, 4H), 1.63 (d, *J* = 37.2 Hz, 6H).

MS (ESI, LR) Calc. for $C_{19}H_{19}ClN_3O$ $[M+H]^+$: 340.1, found: 340.1.

**Example 162. (8-(3-chlorophenyl)imidazo[1,2-*a*]pyridin-3-yl)(piperidin-1-yl)methanone**

<Scheme 50>

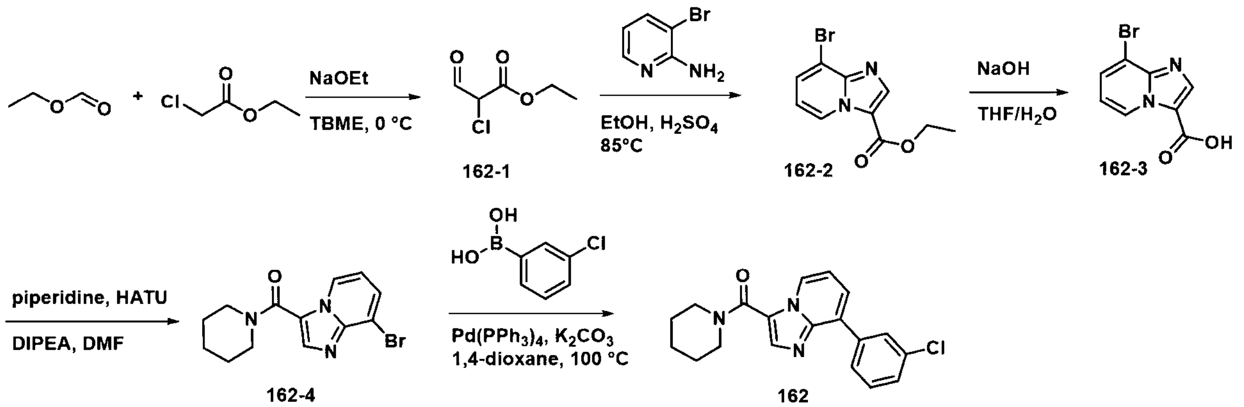


162-1 162-2 162-3 162-4 162

Step 162-1: Methyl *tert*-butyl ether (TBME) (200 mL) was placed in a reaction vessel, and sodium ethylate (20% solution, 27.7 mL, 71.8 mmol) was added under nitrogen atmosphere. After stirring for 15 minutes, the temperature was lowered to 0 °C. Ethyl chloroacetate (6.99 mL, 65.28 mmol) and ethyl formate (6.82 mL, 84.8 mmol) were added over 1 hour. After stirring for 30 minutes, the temperature was increased to room temperature and stirred for 18 hours. The temperature was lowered to 5-10 °C, water (100 mL) was added, and the mixture was stirred for more than 30 minutes. The reaction solution was acidified with hydrochloric acid (pH 1-2), the temperature was increased to room temperature, and the mixture was stirred for 30 minutes. The organic layer was separated, and the aqueous layer was extracted with methyl *tert*-butyl ether. The combined organic layers were washed with brine, dried over anhydrous magnesium sulfate, filtered, and concentrated to obtain the target compound **162-1** (5.2 g, 5%).

Step 162-2: Compound **162-1** (1.3 g, 8.67 mmol) and 3-bromopyridin-2-amine (0.5 g, 2.89 mmol) were dissolved in ethanol (10 mL), and 2 drops of sulfuric acid were added. The reaction solution was stirred at 90 °C for 21 hours. After completion of the reaction, the temperature was lowered to room temperature and filtered using diatomaceous earth (Celite). The filtrate was concentrated, diluted with ethyl acetate (20 mL), washed with 1 N aqueous sodium hydroxide solution and brine, dried over anhydrous magnesium sulfate, filtered, and concentrated. The residue was separated using column chromatography to obtain the target compound **162-2** (0.654 g, 84%).

Step 162-3: Compound **162-2** (3.4 g, 12.8 mmol) was dissolved in tetrahydrofuran (30 mL), and then sodium hydroxide (0.768 g, 19.2 mmol) dissolved in water (30 mL) was added and stirred for 3 hours. After completion of the reaction, the reaction solution was concentrated and acidified using 1 N hydrochloric acid aqueous solution (pH ~2). *n* -butanol was used to extract (30 mL x 4), washed with brine, dried over anhydrous magnesium sulfate, filtered, and concentrated to obtain the target compound **162-3** (3.532 g, 99.5%) in the form of hydrochloride.

Step 162-4: Using compound **162-3,** the target compound **162-4** was obtained by the same synthetic method as step 1-2 in Example **1.**

Step 162-5: Using compound **162-4,** the target compound **162** was obtained by the same synthetic method as step 1-3 in Example **1.**

$^1H$ NMR (400 MHz, DMSO-$d_6$) δ(ppm): 8.87 (dd, $J$ = 7.0, 1.2 Hz, 1H), 8.29 (t, $J$ = 1.9 Hz, 1H), 8.03 (d, $J$ = 7.5 Hz, 2H), 7.73 (dd, $J$ = 7.2, 1.2 Hz, 1H), 7.65-7.43 (m, 2H), 7.18 (t, $J$ = 7.1 Hz, 1H), 3.71 (t, $J$ = 5.4 Hz, 4H), 1.67-1.54 (m, 6H).

MS (ESI, LR) Calc. for $C_{19}H_{19}ClN_3O$ $[M+H]^+$: 340.1, found: 340.1.

**Example 163. (8-(4-nitrophenyl)imidazo[1,2-*a*]pyridin-3-yl)(piperidin-1-yl)methanone**

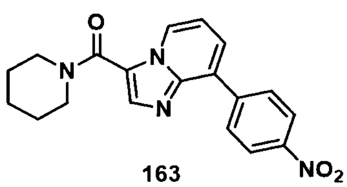

163

The target compound **163** was obtained by the same synthetic method as step 1-3 in Example **1** using compound **162-4** and 4-nitrophenylboronic acid.

$^1H$ NMR (400 MHz, DMSO) δ 8.93 (dd, $J$ = 7.0, 1.2 Hz, 1H), 8.50-8.41 (m, 2H), 8.38 (d, $J$ = 9.0 Hz, 2H), 8.04 (s, 1H),

7.84 (dd, $J$ = 7.2, 1.2 Hz, 1H), 7.24 (t, $J$ = 7.1 Hz, 1H), 3.71 (t, $J$ = 5.4 Hz, 4H), 1.65 (dd, $J$ = 25.7, 5.5 Hz, 6H).

MS (ESI, LR) Calc. for $C_{19}H_{19}N_4O_3$ $[M+H]^+$: 351.1, found: 351.2.

**Example 164. (8-(4-aminophenyl)imidazo[1,2-*a*]pyridin-3-yl)(piperidin-1-yl)methanone**

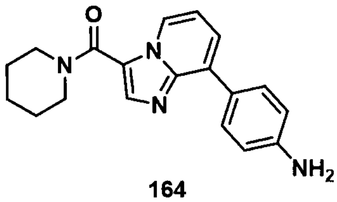

**164**

The target compound **164** was obtained by the same synthetic method as Example **7** using compound **163.**

$^1$H NMR (400 MHz, DMSO-$d_6$) δ(ppm): 8.72 (dd, $J$ = 6.8, 1.2 Hz, 1H), 7.95 (s, 1H), 7.88 (d, $J$ = 8.5 Hz, 2H), 7.49 (dd, $J$ = 7.2, 1.2 Hz, 1H), 7.09 (t, $J$ = 7.0 Hz, 1H), 6.79-6.56 (m, 2H), 5.37 (s, 2H), 3.70 (t, $J$ = 5.5 Hz, 4H), 1.79 - 1.51 (m, 6H).

MS (ESI, LR) Calc. for $C_{19}H_{21}N_4O$ $[M+H]^+$: 321.2, found: 321.2.

**Example 165. (8-(3-chlorophenyl)-6-methylimidazo[1,2-*a*]pyridin-3-yl)(piperidin-1-yl)methanone**

<Scheme 51>

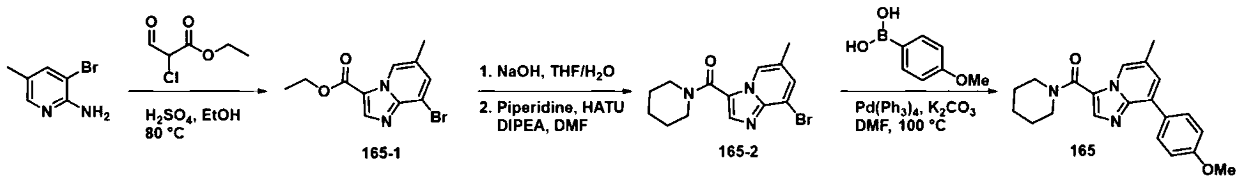


The target compound **165** was obtained by the synthetic method in Example **162** using 3-bromo-5-methylpyridin-2-amine and the corresponding compounds.

$^1$H NMR (400 MHz, DMSO-$d_6$) δ(ppm): 8.68 (t, $J$ = 1.4 Hz, 1H), 8.29 (t, $J$ = 1.9 Hz, 1H), 8.06 (dt, $J$ = 7.6, 1.6 Hz, 1H), 7.95 (s, 1H), 7.64 (d, $J$ = 1.6 Hz, 1H), 7.58-7.45 (m, 2H), 3.70 (t, $J$ = 5.4 Hz, 4H), 2.40 (d, $J$ = 1.1 Hz, 3H), 1.64 (d, $J$ = 29.5 Hz, 6H).

MS (ESI, LR) Calc. for $C_{20}H_{21}ClN_3O$ $[M+H]^+$: 354.1, found: 354.1.

**Example 166. (8-(2-chloropyridin-4-yl)-6-methylimidazo[1,2-*a*]pyridin-3-yl)(piperidin-1-yl)methanone**

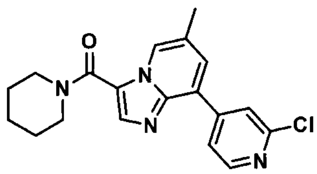

**166**

The target compound **166** was obtained by the same synthetic method as step 1-3 in Example **1** using compound **165-2** and 3-chlorophenylboronic acid.

$^1$H NMR (400 MHz, DMSO-$d_6$) δ(ppm): 8.75 (t, $J$ = 1.4 Hz, 1H), 8.54 (d, $J$ = 5.3 Hz, 1H), 8.44 (d, $J$ = 1.6 Hz, 1H), 8.23 (dd, $J$ = 5.3, 1.6 Hz, 1H), 7.99 (s, 1H), 7.89 (d, $J$ = 1.6 Hz, 1H), 3.70 (t, $J$ = 5.4 Hz, 4H), 2.51 (p, $J$ = 1.9 Hz, 3H), 1.76-1.45 (m, 6H).

MS (ESI, LR) Calc. for $C_{19}H_{20}ClN_4O$ $[M+H]^+$: 355.1, found: 355.1.

**Example 167. (8-(3-chlorophenyl)-6-fluoroimidazo[1,2-*a*]pyridin-3-yl)(piperidin-1-yl)methanone**

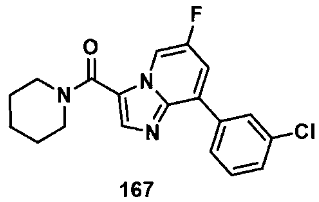

**167**

The target compound **167** was obtained by the synthetic method in Example **162** using 3-bromo-5-fluoropyridin-2-amine and the corresponding compounds.

$^1$H NMR (400 MHz, DMSO-$d_6$) δ(ppm): 8.93 (dd, *J* = 4.7, 2.4 Hz, 1H), 8.34 (d, *J* = 2.1 Hz, 1H), 8.22-7.99 (m, 2H), 7.91 (dd, *J* = 9.4, 2.4 Hz, 1H), 7.56 (dd, *J* = 4.9, 2.3 Hz, 2H), 3.70 (t, *J* = 5.4 Hz, 4H), 1.64 (dq, *J* = 26.3, 6.0 Hz, 6H).

MS (ESI, LR) Calc. for $C_{19}H_{18}ClFN_3O$ [M+H]$^+$: 358.1, found: 358.1.

**Example 168. (7-(4-methoxyphenyl)-[1,2,3]triazolo[1,5-*a*]pyridin-3-yl)(piperidin-1-yl)methanone**

<Scheme 52>

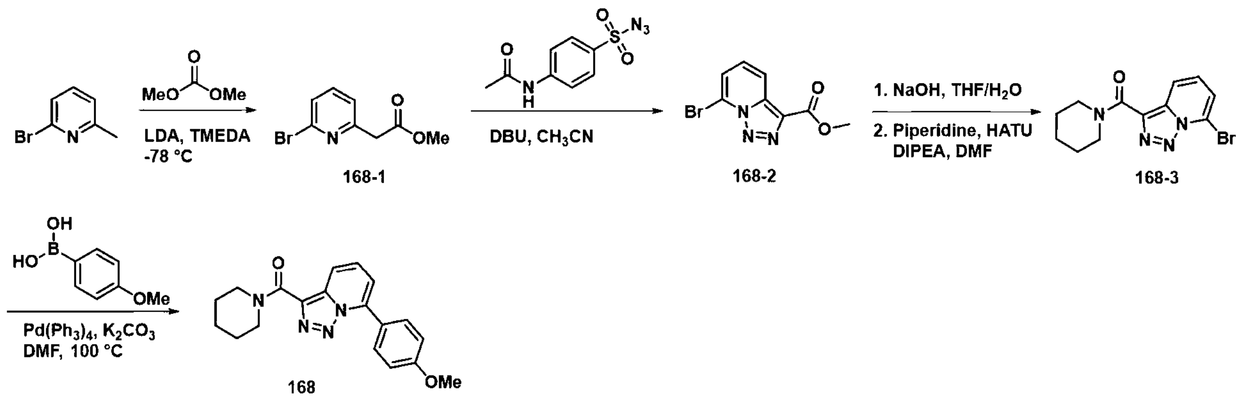


168-1 168-2 168-3 168

Step 168-1: 2-Bromo-6-methyl-pyridine (3 g, 1.98 mmol) was dissolved in tetrahydrofuran (100 mL), the temperature was lowered to -78 °C, lithium diisopropylamide (2 M solution, 17.44 mL, 34.88 mmol) was added, and the mixture was stirred for 10 minutes. *N,N,N',N'*-tetramethylethylenediamine (TMEDA) (5.23 mL, 34.88 mmol) was added, and the mixture was stirred at the same temperature for 1 hour. Methyl chloroformate (2.7 mL, 34.88 mmol) was slowly added, and the temperature was raised to room temperature. After the reaction was completed, water (100 mL) was added, and extracted with ethyl acetate (100 mL x 2). The combined organic layers were washed with brine, dried over anhydrous magnesium sulfate, filtered, and concentrated. The residue was separated using column chromatography to obtain the target compound **168-1** (1.8 g, 45%).

Step 165-2: Compound **168-1** (1.37 g, 5.95 mmol) and 4-acetamidobenzenesulfonyl azide (1.57 g, 6.5 mmol) were dissolved in acetonitrile (20 mL) and stirred. The temperature was lowered to 0 °C, DBU (0.98 mL, 6.5 mmol) was added, the temperature was raised to room temperature, and the mixture was stirred for 12 hours. After the reaction was completed, a saturated ammonium chloride aqueous solution (50 mL) was added and filtered using diatomaceous earth (Celite). The filtrate was extracted with ethyl acetate (50 mL x 3), and the combined organic layers were washed with brine, dried over anhydrous magnesium sulfate, filtered, and concentrated. After solidification with ethyl acetate/diethyl ether (1:10), filtering and air drying, the target compound **168-2** (0.95 g, 62%) was obtained. [DBU(1,8-Diazabicyclo(5,4,0) undec-7-ene)]

Step 168-3,4,5: Using compound **162-2,** the target compound **168** was obtained by the same synthetic method as step 162-3, 4, and 5 in Example **162.**

$^1$H NMR (400 MHz, DMSO-$d_6$) δ(ppm): 8.18 (dd, *J* = 8.8, 1.2 Hz, 1H), 8.10-7.95 (m, 2H), 7.69 (dd, *J* = 8.8, 7.0 Hz, 1H), 7.42 (dd, *J* = 7.1, 1.2 Hz, 1H), 7.27-7.11 (m, 2H), 3.88 (s, 4H), 1.79 - 1.50 (m, 6H).

MS (ESI, LR) Calc. for $C_{19}H_{21}N_4NaO_2$ $[M+Na]^+$: 359.2, found: 359.2.

**Example 169. (7-(3-chlorophenyl)-1*H*-indol-3-yl)(piperidin-1-yl)methanone**

<Scheme 53>

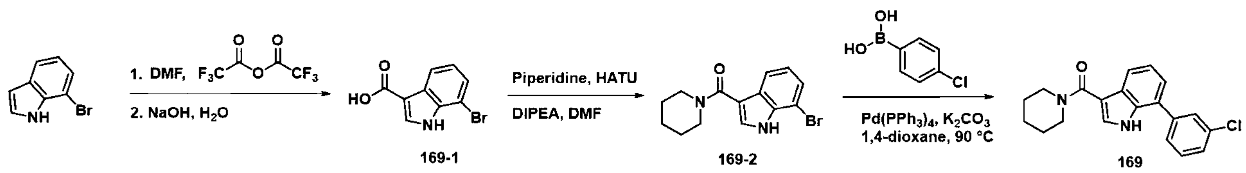


Step 169-1: 7-Bromoindole (0.5 g, 2.55 mmol) was dissolved in *N,N*-dimethylformamide (10 mL), trifluoroacetic anhydride (1.51 mL, 4.46 mmol) was added, and the mixture was stirred for 12 hours. After completion of the reaction, water was added, filtered, and washed with water. The obtained solid product was dried in air. The obtained solid product was dissolved in 4 N aqueous sodium hydroxide solution (10 mL) and stirred at 100 °C for 2 hours. After completion of the reaction, the temperature was lowered to room temperature, acidified with hydrochloric acid (pH ~1), filtered, and washed with water. The obtained solid product was dried in air to obtain the target compound **169-1** (0.548 g, 78%) in the form of hydrochloride.

Step 169-2, 3: Using compound **169-1,** the target compound **169** was obtained by the same synthetic method as step 162-4, 5 in Example **162.**

$^1H$ NMR (400 MHz, DMSO-$d_6$) δ(ppm): 11.50 (s, 1H), 7.71-7.63 (m, 2H), 7.62-7.54 (m, 3H), 7.53-7.46 (m, 1H), 7.28-7.13 (m, 2H), 3.59 (t, *J* = 5.4 Hz, 4H), 1.59 (d, *J* = 40.5 Hz, 6H).

MS (ESI, LR) Calc. for $C_{20}H_{20}ClN_2O$ $[M+H]^+$: 339.1, found: 339.1.

**Example 170. (6-(3-chlorophenyl)pyrazolo[1,5-*a*]pyridin-3-yl)(piperidin-1-yl)methanone**

**Example 171. (6-(4-methoxyphenyl)pyrazolo[1,5-*a*]pyridin-3-yl)(piperidin-1-yl)methanone**

For Examples 170 and 171, the compounds were obtained by the same synthetic method as steps 162-3, 4 and 5 in Example **162** using methyl-6-bromopyrazolo[1,5-*a*]pyridine-3-carboxylate and the corresponding compounds. The structural and spectral data are shown in Table 37 below.

[Table 37]

| Example | Structure | $^1H$ NMR | MS |
|---|---|---|---|
| **170** | 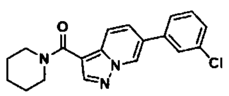 | $^1H$ NMR (400 MHz, DMSO-$d_6$) δ(ppm): 9.22 (t, *J* = 1.3 Hz, 1H), 8.29 (s, 1H), 7.99-7.90 (m, 2H), 7.86-7.77 (m, 2H), 7.59-7.39 (m, 2H), 3.63 (t, *J* = 5.4 Hz, 4H), 1.61 (dt, *J* = 32.5, 5.5 Hz, 6H). | MS (ESI, LR) Calc. for $C_{19}H_{19}ClN_3O$ $[M+H]^+$: 340.1, found: 340.1. |
| **171** | 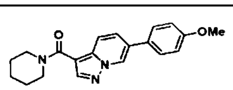 | $^1H$ NMR (400 MHz, DMSO-$d_6$) δ(ppm): 9.05 (t, *J* = 1.3 Hz, 1H), 8.24 (s, 1H), 7.96-7.82 (m, 1H), 7.82-7.70 (m, 3H), 7.10-6.97 (m, 4H), 3.82 (s, 3H), 1.74-1.51 (m, 6H). | MS (ESI, LR) Calc. for $C_{20}H_{22}N_3O_2$ $[M+H]^+$: 336.2, found: 336.2. |

**Example 172. (6-(benzo[*d*][1,3]dioxol-5-yl)-1*H*pyrrolo[3,2-*b*]pyridin-2-yl)(piperidin-1-yl)methanone**

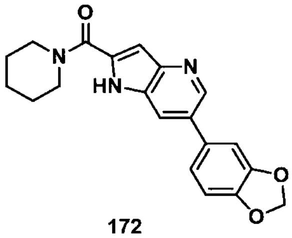

172

The target compound **172** was obtained by the same synthetic method as step 162-4 and 5 in Example **162** using 6-bromo-1*H*-pyrrolo[3,2-*b*]pyridine-2-carboxylic acid and the corresponding compounds.

$^1H$ NMR (400 MHz, DMSO-$d_6$) $\delta$(ppm): 12.96 (d, $J$ = 7.3 Hz, 1H), 8.93 (t, $J$ = 2.6 Hz, 1H), 8.45 (d, $J$ = 7.2 Hz, 1H), 7.46 (d, $J$ = 1.9 Hz, 1H), 7.31 (dd, $J$ = 8.2, 1.9 Hz, 1H), 7.10 (d, $J$ = 8.1 Hz, 1H), 6.99 (s, 1H), 6.12 (s, 2H), 3.65 (t, $J$ = 5.4 Hz, 4H), 1.63 (dt, $J$ = 30.8, 5.8 Hz, 6H).

MS (ESI, LR) Calc. for $C_{20}H_{20}N_3O_3$ $[M+H]^+$: 350.2, found: 350.2.

**Example 173. (5-(benzo[*d*][1,3]dioxol-5-yl)-1*H*-pyrrolo[3,2-*b*]pyridin-2-yl)(piperidin-1-yl)methanone**

<Scheme 54>

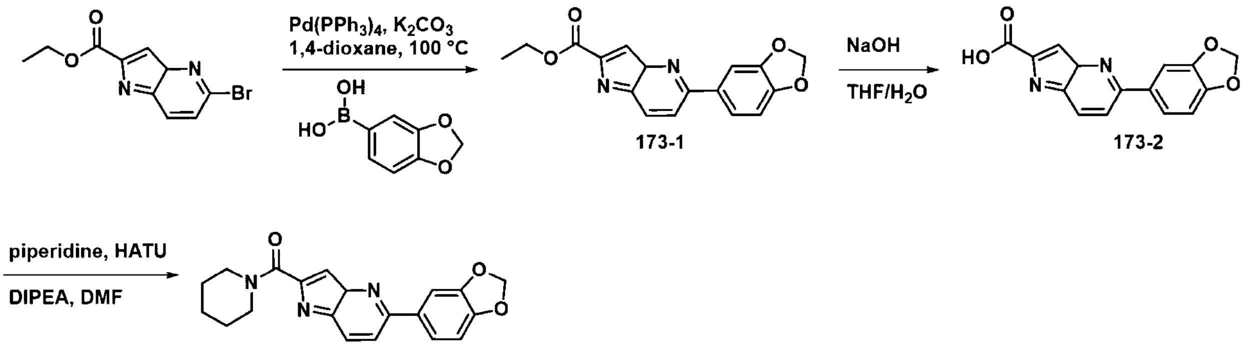


173

The target compound **173** was obtained by the same synthetic method as step 162-5, 3, and 4 in Example **162** using ethyl 5-bromo-1*H*-pyrrolo[3,2-*b*]pyridine-2-carboxylate and the corresponding compounds.

$^1H$ NMR (400 MHz, DMSO-$d_6$) $\delta$(ppm): 12.58 (s, 1H), 8.20 (d, $J$ = 8.6 Hz, 1H), 7.88 (d, $J$ = 8.7 Hz, 1H), 7.65 (d, $J$ = 1.9 Hz, 1H), 7.59 (dd, $J$ = 8.2, 1.9 Hz, 1H), 7.13 (d, $J$ = 8.2 Hz, 1H), 6.92 (d, $J$ = 2.0 Hz, 1H), 6.15 (s, 2H), 3.68 (t, $J$ = 5.3 Hz, 4H), 1.64 (dq, $J$ = 29.9, 5.7 Hz, 6H).

MS (ESI, LR) Calc. for $C_{20}H_{20}N_3O_3$ $[M+H]^+$: 350.2, found: 350.2.

**Example 174. (4-(benzo[*d*][1,3]dioxol-5-yl)-1*H*-pyrrolo[3,2-*c*]pyridin-2-yl)(piperidin-1-yl)methanone**

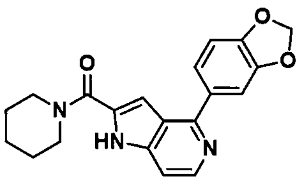

174

The target compound **174** was obtained by the same synthetic method as step 162-5, 3, and 4 in Example **162** using methyl 4-chloro-1*H*-pyrrolo[3,2- *c*]pyridine-2-carboxylate and the corresponding compounds.

$^1H$ NMR (400 MHz, DMSO-$d_6$) $\delta$(ppm): 13.38 (s, 1H), 8.40 (d, $J$ = 6.7 Hz, 1H), 7.84 (d, $J$ = 6.7 Hz, 1H), 7.61-7.51 (m, 2H), 7.28 (d, $J$ = 9.1 Hz, 2H), 6.24 (s, 2H), 3.68 (s, 3H), 1.66 (d, $J$ = 7.0 Hz, 2H), 1.58 (s, 3H), 1.25 (s, 1H).

MS (ESI, LR) Calc. for $C_{20}H_{20}N_3O_3$ [M+H]+: 350.2, found: 350.2.

**Example 175. (4-(benzo[*d*][1,3]dioxol-5-yl)-1*H*-pyrrolo[2,3-*c*]pyridin-2-yl)(piperidin-1-yl)methanone**

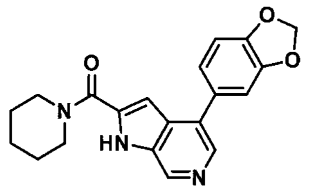

**175**

The target compound **175** was obtained by the same synthetic method as step 162-5, 3, and 4 in Example **162** using ethyl 5-chloro-1*H*-pyrrolo[2,3-*c*]pyridine-2-carboxylate and the corresponding compounds.

$^1H$ NMR (400 MHz, DMSO-$d_6$) δ(ppm): 13.73 (s, 1H), 9.15 (s, 1H), 8.45 (s, 1H), 7.41 (d, *J* = 1.8 Hz, 1H), 7.33 (dd, *J* = 8.0, 1.9 Hz, 1H), 7.14 (d, *J* = 8.1 Hz, 2H), 6.15 (s, 2H), 3.66 (s, 4H), 1.77-1.45 (m, 6H)

MS (ESI, LR) Calc. for $C_{20}H_{20}N_3O_3$ [M+H]+: 350.2, found: 350.2.

**Example 176. (4-(benzo[*d*][1,3]dioxol-5-yl)pyrrolo[2,1-*f*][1,2,4]triazin-6-yl)(piperidin-1-yl)methanone**

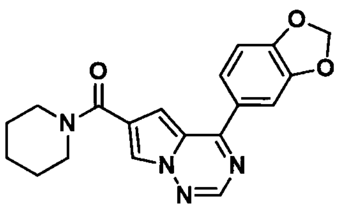

**176**

The target compound **176** was obtained by the same synthetic method as step 162-5, 3, and 4 in Example **162** using ethyl 4-chloropyrrolo[2,1-*f*][1,2,4]triazine-6-carboxylate and the corresponding compounds.

$^1H$ NMR (400 MHz, DMSO-$d_6$) δ(ppm): 8.64 (s, 1H), 8.32 (d, *J* = 1.5 Hz, 1H), 7.80 (dd, *J* = 8.2, 1.8 Hz, 1H), 7.66 (d, *J* = 1.8 Hz, 1H), 7.28 (d, *J* = 1.6 Hz, 1H), 7.14 (d, *J* = 8.2 Hz, 1H), 6.19 (s, 2H), 1.64 (t, *J* = 6.3 Hz, 2H), 1.54 (s, 3H).

MS (ESI, LR) Calc. for $C_{19}H_{19}N_4O_3$ [M+H]+: 351.1, found: 351.1.

**Example 177. (4-(benzo[*d*][1,3]dioxol-5-yl)-7*H*-pyrrolo[2,3-*d*]pyrimidin-6-yl)(piperidin-1-yl)methanone**

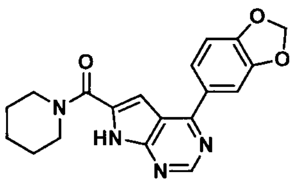

**177**

The target compound **177** was obtained by the same synthetic method as step 162-4 and 5 in Example **162** using 4-chloro-7*H*-pyrrolo[2,3-*d*]pyrimidine-6-carboxylic acid and the corresponding compounds.

$^1H$ NMR (400 MHz, DMSO-$d_6$) δ(ppm): 12.97 (s, 1H), 8.91 (s, 1H), 7.76 (dd, *J* = 8.1, 1.8 Hz, 1H), 7.69 (d, *J* = 1.8 Hz, 1H), 7.16 (d, *J* = 8.2 Hz, 1H), 7.12 (s, 1H), 6.18 (s, 2H), 3.64 (t, *J* = 5.4 Hz, 4H), 1.66 (s, 2H), 1.57 (s, 3H), 1.24 (s, 2H), 0.89-0.82 (m, 1H).

MS (ESI, LR) Calc. for $C_{10}H_{19}N_4O_3$ [M+H]+: 351.1, found: 351.1.

**Example 178. (5-(2,3-dihydrobenzo[*b*][1,4]dioxin-6-yl)-1*H*-pyrrolo[3,2-*b*]pyridin-2-yl)(piperidin-1-yl)methanone**

**Example 179. (5-(4-methoxyphenyl)-1*H*-pyrrolo[3,2-*b*]pyridin-2-yl)(piperidin-1-yl)methanone**

**Example 180. (5-(4-chlorophenyl)-1*H*-pyrrolo[3,2-*b*]pyridin-2-yl)(piperidin-1-yl)methanone**

**Example 181. (5-(3,4-dimethoxyphenyl)-1*H*-pyrrolo[3,2-*b*]pyridin-2-yl)(piperidin-1-yl)methanone**

**Example 182. (5-(4-fluorophenyl)-1*H*-pyrrolo[3,2-*b*]pyridin-2-yl)(piperidin-1-yl)methanone**

**Example 183. (5-(furan-3-yl)-1*H*-pyrrolo[3,2-*b*]pyridin-2-yl)(piperidin-1-yl)methanone**

**Example 184. piperidin-1-yl(5-(pyridin-4-yl)-1*H*-pyrrolo[3,2-*b*]pyridin-2-yl)methanone**

**Example 185. (5-(3-chlorophenyl)-1*H*-pyrrolo[3,2-*b*]pyridin-2-yl)(piperidin-1-yl)methanone**

**Example 186. (5-(3-chloro-4-hydroxyphenyl)-1*H*-pyrrolo[3,2-*b*]pyridin-2-yl)(piperidin-1-yl)methanone**

**Example 187. (2-(benzo[*d*][1,3]dioxol-5-yl)-5*H*-pyrrolo[3,2-*d*]pyrimidin-6-yl)(piperidin-1-yl)methanone**

**Example 188. (5-(benzofuran-3-yl)-1*H*-pyrrolo[3,2-*b*]pyridin-2-yl)(piperidin-1-yl)methanone**

**Example 189. (5-(naphthalen-2-yl)-1*H*-pyrrolo[3,2-*b*]pyridin-2-yl)(piperidin-1-yl)methanone**

**Example 190. (5-(1*H*-indazol-6-yl)-1*H*-pyrrolo[3,2-*b*]pyridin-2-yl)(piperidin-1-yl)methanone**

**Example 191. 2-(2-(piperidine-1-carbonyl)-1*H*-pyrrolo[3,2-*b*]pyridin-5-yl)isoindolin-1-one**

For Examples 178, 179, 180, 181, 182, 183, 184, 185, 186, 187, 188, 189, 190, and 191, the compounds were obtained by the same synthetic method as in step 162-3, 4, 5 of Example **162** using ethyl 5-bromo-1*H*-pyrrolo[3,2-*b*]pyridine-2-carboxylate and the corresponding compounds. The structures and spectral data are shown in Table 38 below.

[Table 38]

| Example | Structure | $^1$H NMR | MS |
|---|---|---|---|
| **178** | 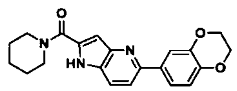 | $^1$H NMR (400 MHz, DMSO-$d_6$) δ(ppm): 8.18 (d, *J* = 8.6 Hz, 1H), 7.88 (d, *J* = 8.7 Hz, 1H), 7.60 (d, *J* = 2.2 Hz, 1H), 7.56 (dd, *J* = 8.5, 2.3 Hz, 1H), 7.06 (d, *J* = 8.4 Hz, 1H), 6.92 (d, *J* = 2.0 Hz, 1H), 4.34 (s, 4H), 3.68 (t, *J* = 5.4 Hz, 4H), 1.67 (t, *J* = 5.8 Hz, 2H), 1.60 (p, *J* = 5.5 Hz, 4H), 1.25 (s, 1H). | MS (ESI, LR) Calc. for $C_{21}H_{22}N_3O_3$ $[M+H]^+$: 364.2, found: 364.1. |
| **179** | 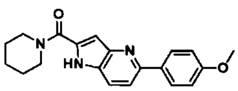 | $^1$H NMR (400 MHz, DMSO-$d_6$) δ(ppm): 8.19 (s, 1H), 8.06-7.98 (m, 2H), 7.89 (d, *J* = 8.7 Hz, 1H), 7.15 (d, *J* = 8.7 Hz, 2H), 6.92 (d, *J* = 2.0 Hz, 1H), 6.78-6.70 (m, 1H), 6.70-6.64 (m, 1H), 3.86 (s, 3H), 3.68 (dd, *J* = 11.2, 6.0 Hz, 4H), 1.69 (t, *J* = 6.4 Hz, 1H), 1.67-1.58 (m, 4H), 1.24 (s, 1H). | MS (ESI, LR) Calc. for $C_{20}H_{22}N_3O_2$ $[M+H]^+$: 336.2, found: 336.2. |
| **180** | 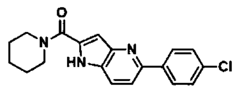 | $^1$H NMR (400 MHz, DMSO-$d_6$) δ(ppm): 12.03 (s, 1H), 8.17-8.09 (m, 2H), 7.95 (d, *J* = 8.6 Hz, 1H), 7.85 (d, *J* = 8.7 Hz, 1H), 7.60-7.53 (m, 2H), 6.92 (d, *J* = 2.1 Hz, 1H), 3.71 (s, 5H), 1.71-1.64 (m, 2H), 1.61 (q, J = 5.4 Hz, 4H), 1.24 (s, 1H). | MS (ESI, LR) Calc. for $C_{19}H_{19}ClN_3O$ $[M+H]^+$: 340.1, found: 340.1. |
| **181** | 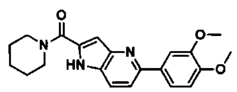 | $^1$H NMR (400 MHz, DMSO-$d_6$) δ(ppm): 8.24 (d, J = 8.5 Hz, 1H), 7.96 (d, *J* = 8.7 Hz, 1H), 7.69-7.59 (m, 2H), 7.17 (d, *J* = 8.5 Hz, 1H), 6.94 (d, *J* = 2.0 Hz, 1H), 3.88 (d, *J* = 17.8 Hz, 6H), 3.68 (t, *J* = 5.4 Hz, 4H), 1.69 (q, *J* = 5.6 Hz, 2H), 1.63-1.55 (m, 4H), 1.24 (s, 1H). | MS (ESI, LR) Calc. for $C_{21}H_{24}N_3O_3$ $[M+H]^+$: 366.2, found: 366.2. |

(continued)

| Example | Structure | $^1$H NMR | MS |
|---|---|---|---|
| **182** | 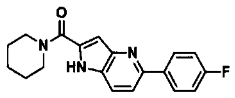 | $^1$H NMR (400 MHz, DMSO-$d_6$) δ(ppm): 8.18-8.09 (m, 2H), 7.99 (d, *J* = 8.7 Hz, 1H), 7.84 (d, *J* = 8.6 Hz, 1H), 7.35 (t, *J* = 8.9 Hz, 2H), 6.92 (d, *J* = 2.0 Hz, 1H), 3.71 (s, 3H), 1.71-1.64 (m, 2H), 1.61 (dt, *J* = 9.9, 4.1 Hz, 4H). | MS (ESI, LR) Calc. for $C_{19}H_{19}FN_3O$ $[M+H]^+$: 324.2, found: 324.1. |
| **183** | 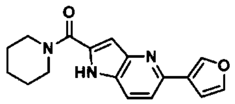 | $^1$H NMR (400 MHz, DMSO-$d_6$) δ(ppm): 12.48 (s, 1H), 8.51 (s, 1H), 8.14 (d, *J* = 8.6 Hz, 1H), 7.87 (t, *J* = 1.8 Hz, 1H), 7.79 (d, *J* = 8.6 Hz, 1H), 7.20 (d, *J* = 1.9 Hz, 1H), 6.90 (d, *J* = 2.0 Hz, 1H), 3.68 (t, *J* = 5.4 Hz, 4H), 1.68 (q, *J* = 5.8 Hz, 2H), 1.60 (h, *J* = 4.6 Hz, 4H). | MS (ESI, LR) Calc. for $C_{17}H_{18}N_3O_2$ $[M+H]^+$: 296.1, found: 296.1. |
| **184** | 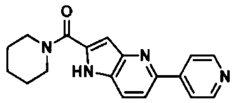 | $^1$H NMR (400 MHz, DMSO-$d_6$) δ(ppm): 12.19 (s, 1H), 8.92-8.86 (m, 2H), 8.54 (d, *J* = 6.0 Hz, 2H), 8.17 (d, *J* = 8.6 Hz, 1H), 8.01 (d, *J* = 8.6 Hz, 1H), 7.01 (d, *J* = 2.1 Hz, 1H), 3.71 (s, 2H), 1.69 (q, *J* = 5.9 Hz, 2H), 1.61 (dt, *J* = 10.8, 5.4 Hz, 4H). | MS (ESI, LR) Calc. for $C_{18}H_{19}N_4O$ $[M+H]^+$: 307.2, found: 307.2. |
| **185** | 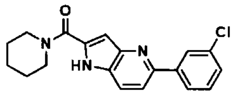 | $^1$H NMR (400 MHz, DMSO-$d_6$) δ(ppm): 12.10 (s, 1H), 8.17 (t, *J* = 1.9 Hz, 1H), 8.06 (dt, *J* = 7.7, 1.5 Hz, 1H), 7.98 (d, *J* = 8.7 Hz, 1H), 7.90 (d, *J* = 8.6 Hz, 1H), 7.59-7.46 (m, 2H), 6.94 (d, *J* = 2.1 Hz, 1H), 3.71 (s, 2H), 1.71-1.61 (m, 2H), 1.60 (d, *J* = 6.4 Hz, 4H). | MS (ESI, LR) Calc. for $C_{19}H_{19}ClN_3O$ $[M+H]^+$: 340.1, found: 340.1. |
| **186** | 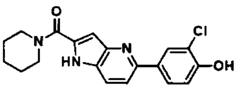 | $^1$H NMR (400 MHz, DMSO-$d_6$) δ(ppm): 12.47(s, 1H), 10.76(s, 1H), 8.15 (d, *J* = 8.7 Hz, 1H), 8.10 (d, *J* = 2.3 Hz, 1H), 7.87 (dd, *J* = 8.6, 2.3 Hz, 2H), 7.14 (d, *J* = 8.5 Hz, 1H), 6.92 (d, *J* = 2.0 Hz, 1H), 3.68 (t, *J* = 5.4 Hz, 4H), 1.68 (q, *J* = 5.6 Hz, 2H), 1.63 - 1.55 (m, 4H). | MS (ESI, LR) Calc. for $C_{19}H_{19}ClN_3O_2$ $[M+H]^+$: 356.1, found: 356.1. |
| **187** | 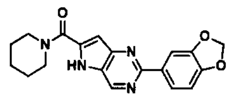 | $^1$H NMR (400 MHz, DMSO-$d_6$) δ(ppm): 12.30 (s, 1H), 8.98 (s, 1H), 8.04 (dd, *J* = 8.3, 1.8 Hz, 1H), 7.90 (d, *J* = 1.7 Hz, 1H), 7.03 (d, *J* = 8.2 Hz, 1H), 6.87 (d, *J* = 1.9 Hz, 1H), 6.10 (s, 2H), 3.67 (t, *J* = 5.4 Hz, 4H), 1.67 (d, *J* = 9.8 Hz, 3H), 1.60 (d, *J* = 7.6 Hz, 4H). | MS (ESI, LR) Calc. for $C_{19}H_{19}N_4O_3$ $[M+H]^+$: 351.1, found: 351.1. |
| **188** | 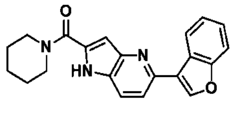 | $^1$H NMR (400 MHz, DMSO-$d_6$) δ(ppm): 12.07 (s, 1H), 8.70 (s, 1H), 8.58-8.51 (m, 1H), 7.96 (d, *J* = 8.6 Hz, 1H), 7.83 (d, *J* = 8.6 Hz, 1H), 7.72-7.65 (m, 1H), 7.42 (qd, *J* = 7.3, 3.7 Hz, 2H), 6.96 (d, *J* = 2.1 Hz, 1H), 3.73 (s, 4H), 1.72-1.65 (m, 2H), 1.65-1.58 (m, 4H). | MS (ESI, LR) Calc. for $C_{21}H_{20}N_3O_2$ $[M+H]^+$: 346.2, found: 346.1. |
| **189** | 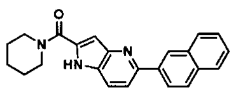 | $^1$H NMR (400 MHz, DMSO-$d_6$) δ(ppm): 12.06 (s, 1H), 8.65 (s, 1H), 8.31 (dd, *J* = 8.8, 1.8 Hz, 1H), 8.15-7.85 (m, 5H), 7.57 (dq, *J* = 5.9, 3.7 Hz, 2H), 6.97 (d, *J* = 2.1 Hz, 1H), 3.73 (s, 4H), 1.69 (s, 2H), 1.62 (s, 4H). | MS (ESI, LR) Calc. for $C_{23}H_{22}N_3O$ $[M+H]^+$: 356.2, found: 356.1. |
| 190 | 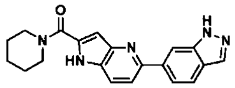 | $^1$H NMR (400 MHz, DMSO-$d_6$) δ(ppm): 13.36 (s, 1H), 12.48 (s, 1H), 8.20 (d, *J*= 16.8 Hz, 2H), 8.01 (d, *J*= 8.6 Hz, 1H), 7.94 (d, *J* = 8.5 Hz, 1H), 7.82 (d, *J*= 8.5 Hz, 1H), 6.97 (d, *J* = 2.0 Hz, 1H), 1.98-1.67 (m, 2H), 1.62 (q, *J*= 5.7 Hz, 4H). | MS (ESI, LR) Calc. for $C_{20}H_{20}N_5O$ $[M+H]^+$: 346.2, found: 346.1. |
| 191 | 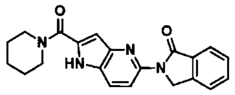 | $^1$H NMR (400 MHz, DMSO-$d_6$) δ(ppm): 11.88 (s, 1H), 8.50 (d, *J* = 9.0 Hz, 1H), 7.91 (d, *J* = 9.0 Hz, 1H), 7.83 (d, *J* = 7.5 Hz, 1H), 7.79-7.68 (m, 2H), 7.56 (t, *J* = 7.7 Hz, 1H), 6.82 (dd, J = 11.2, 2.1 Hz, 1H), 5.19 (s, 2H), 3.70 (s, 6H), 1.76-1.63 (m, 2H), 1.59 (dd, *J* = 11.2, 5.5 Hz, 4H). | MS (ESI, LR) Calc. for $C_{21}H_{21}N_4O_2$ $[M+H]^+$: 361.2, found: 361.1. |

**Example 413. 3-hydroxy-5-[2-(piperidine-1-carbonyl)pyrazolo[1,5-*a*]pyridin-6-yl]pyridine-2-carbonitrile**

<Scheme 55>

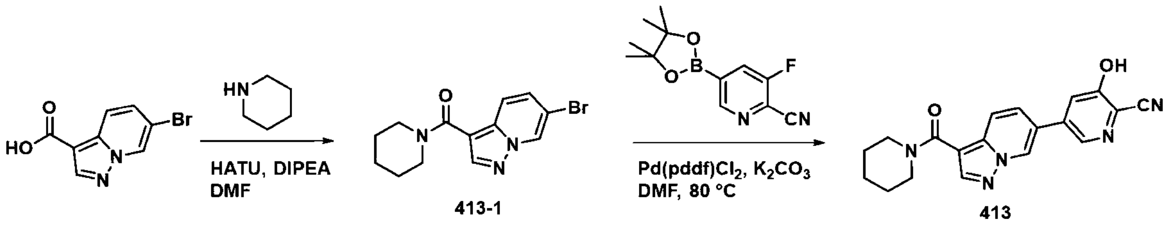


Step 413-1: The target compound **413-1** was obtained by the same synthetic method as step 1-2 in Example 1 using 6-bromopyrazolo[1,5-*a*]pyridine-3-carboxylic acid.

Step 413-2: Using compound **413-1,** the target compound 413 was obtained by the same synthetic method as step 159-3 in Example 159.

$^1$H NMR (400 MHz, DMSO-$d_6$) δ(ppm): 9.23 (s, 1H), 8.62 (d, *J* = 1.9 Hz, 1H), 7.88 (d, *J* = 9.3 Hz, 1H), 7.68 (d, *J* = 1.9 Hz, 1H), 7.62 (dd, *J* = 9.3, 1.7 Hz, 1H), 6.89 (s, 1H), 3.67 (dt, *J* = 16.9, 5.1 Hz, 4H), 1.65 (q, *J* = 6.1 Hz, 2H), 1.55 (d, *J* = 22.3 Hz, 4H).

MS (ESI, LR) Calc. for $C_{19}H_{18}N_5O_2$ $[M+H]^+$: 348.1, found: 348.1.

**Experimental Example 1. Evaluation of 15-PGDH inhibitory activity**

15-PGDH inhibition activity of Example compounds was measured as follows. 6.7 ng of human 15-PGDH (R&D System# 5660-DH) enzyme (10 μL) was added to a black 96 well plate, 60 μL of buffer (50 mM Tris-HCl, pH 7.5, 0.01% Tween 20) was added, and 10 μL of 200 μM β-Nicotinamide adenine dinucleotide sodium salt (Sigma, Cat# N0632-5G) was added. 10 μL of (10x) compound solution in DMSO was added and reacted for 10 minutes at room temperature. Then, 10 μL of 200 μM prostaglandin $E_2$ was added, and the values were read at 30-second intervals for 30 minutes at ex (λ 360 nm)/em (λ 450 nm) to obtain the result value $V_{max}$ (milliunit per min). 0% inhibition for enzyme alone and 100% inhibition for substrate alone were defined from the $V_{max}$ slope of the result value, i.e., the linear range, and the % inhibition of the compound at each concentration was converted accordingly using Equation 1 below. The result value was subjected to nonlinear regression analysis using Prism, and the concentration that exhibited 50% inhibition ($IC_{50}$) was obtained from the concentration-response curve of the test compound.

<Equation 1>

$$\%\ \text{inhibition} = 100 - \left( \frac{\text{Vmax at compound concentration} - \text{Substrate alone Vmax}}{\text{Enzyme alone Vmax} - \text{substrate alone Vmax}}\ \text{X}\ 100 \right)$$

The results are shown in Table 39 below. In Table 39, $IC_{50}$ value ≤ 100 nM: A, 100 nM < $IC_{50}$ value ≤ 1000 nM: B, $IC_{50}$ value > 1000 nM: C. Through these values, it was confirmed that each compound inhibited 15-PGDH activity. Therefore, it was confirmed that the compound of the present invention is a direct 15-PGDH inhibitor.

**Experimental example 2. Measurement of PGE2-increasing activity in A549 cells**

A549 cells were prepared at a cell number of 1 x $10^5$/mL in F12K medium containing 10% FBS (Fetal Bovine Serum), and 2 x $10^4$/200 μL was added to each well of a 96-well cell culture plate, and then cultured in an incubator with 5% $CO_2$ at 37 °C for 24 hours. After the culture was complete and all F12K medium was removed, all wells were washed once with 100 μL of PBS, and treated with 160 μL of serum-free F12K medium. Then, 20 μL of IL-1β diluted 10-fold to a concentration of 1 ng/mL in serum-free F12K medium was treated to wells to be treated with IL-1β, and 20 μL of serum-free F12K medium was treated to wells not treated with IL-1β. For the wells to be treated with the compound, 20 μL of the compound dissolved in 100% DMSO was diluted to a concentration 10 times of the desired compound concentration in serum-free F12K medium so that the final DMSO concentration treated to the cells was 0.1%, and for the wells not to be treated with the compound, 20 μL of DMSO diluted in serum-free F12K medium so that the final DMSO concentration

treated to the cells was 0.1% was treated. In order to confirm the increase or decrease in the secretion amount of $PGE_2$ in the supernatant collected after culturing for 24 hours in an incubator with 5% $CO_2$ at 37 °C, $PGE_2$ was quantified using the Prostaglandin $E_2$ Parameter (SKGE004B).

As a result, as shown in Table 39 below, it was confirmed that the amount of $PGE_2$ increased due to inhibition of 15-PGDH activity at a concentration of 1 μM of each compound (0 < $PGE_2$ increasing ability < 1.5 : +, $PGE_2$ increasing ability ≥ 1.5 : ++).

[Table 39]

| Example | $IC_{50}$ value | Increased $PGE_2$ production in A549 cells | Example | $IC_{50}$ value | Increased $PGE_2$ production in A549 cells |
|---|---|---|---|---|---|
| 1 | A | | 209 | A | ++ |
| 2 | A | | 210 | A | ++ |
| 3 | A | + | 211 | A | ++ |
| 4 | A | | 212 | A | ++ |
| 5 | A | | 213 | A | ++ |
| 6 | A | ++ | 214 | A | ++ |
| 7 | A | ++ | 215 | A | ++ |
| 8 | A | ++ | 216 | A | ++ |
| 9 | A | ++ | 217 | A | ++ |
| 10 | A | + | 218 | A | ++ |
| 11 | A | | 219 | A | ++ |
| 12 | A | ++ | 220 | A | ++ |
| 13 | A | | 221 | A | ++ |
| 14 | A | | 222 | A | ++ |
| 15 | A | ++ | 223 | A | ++ |
| 16 | A | ++ | 224 | A | ++ |
| 17 | A | + | 225 | A | ++ |
| 18 | A | ++ | 226 | A | ++ |
| 19 | A | ++ | 227 | A | ++ |
| 20 | A | ++ | 228 | A | + |
| 21 | A | ++ | 229 | A | ++ |
| 22 | A | ++ | 230 | A | ++ |
| 23 | A | ++ | 231 | A | ++ |
| 24 | A | | 232 | A | ++ |
| 25 | A | | 233 | A | ++ |
| 26 | A | ++ | 234 | A | ++ |
| 27 | A | + | 235 | A | ++ |
| 28 | A | ++ | 236 | A | ++ |
| 29 | A | ++ | 237 | A | ++ |
| 30 | A | ++ | 238 | A | ++ |
| 31 | A | + | 239 | A | ++ |
| 32 | A | + | 240 | C | |
| 33 | A | ++ | 241 | A | ++ |
| 34 | A | ++ | 242 | A | + |

(continued)

| Example | $IC_{50}$ value | Increased $PGE_2$ production in A549 cells | Example | $IC_{50}$ value | Increased $PGE_2$ production in A549 cells |
|---|---|---|---|---|---|
| 35 | A | | 243 | A | ++ |
| 36 | A | ++ | 244 | A | + |
| 37 | A | ++ | 245 | A | ++ |
| 38 | A | ++ | 246 | A | ++ |
| 39 | A | + | 247 | A | ++ |
| 40 | A | ++ | 248 | A | ++ |
| 41 | A | ++ | 249 | A | + |
| 42 | A | ++ | 250 | A | ++ |
| 43 | A | | 251 | A | ++ |
| 44 | A | | 252 | A | ++ |
| 45 | A | ++ | 253 | A | ++ |
| 46 | A | ++ | 254 | A | ++ |
| 47 | A | ++ | 255 | A | ++ |
| 48 | A | + | 256 | A | ++ |
| 49 | A | ++ | 257 | A | + |
| 50 | A | ++ | 258 | A | + |
| 51 | A | ++ | 259 | A | ++ |
| 52 | A | ++ | 260 | A | ++ |
| 53 | A | ++ | 261 | A | ++ |
| 54 | A | ++ | 262 | A | ++ |
| 55 | A | ++ | 263 | A | ++ |
| 56 | A | ++ | 264 | A | ++ |
| 57 | A | ++ | 265 | A | ++ |
| 58 | A | ++ | 266 | A | ++ |
| 59 | A | ++ | 267 | A | ++ |
| 60 | A | ++ | 268 | A | ++ |
| 61 | A | ++ | 269 | A | ++ |
| 62 | A | ++ | 270 | A | ++ |
| 63 | A | ++ | 271 | A | ++ |
| 64 | A | ++ | 272 | A | |
| 65 | A | + | 273 | A | ++ |
| 66 | A | ++ | 274 | A | ++ |
| 67 | A | ++ | 275 | A | ++ |
| 68 | A | ++ | 276 | A | ++ |
| 69 | A | ++ | 277 | A | ++ |
| 70 | A | ++ | 278 | A | ++ |
| 71 | A | | 279 | A | + |
| 72 | A | ++ | 280 | A | ++ |
| 73 | A | ++ | 281 | A | ++ |

(continued)

| Example | $IC_{50}$ value | Increased $PGE_2$ production in A549 cells | Example | $IC_{50}$ value | Increased $PGE_2$ production in A549 cells |
|---|---|---|---|---|---|
| 74 | A | ++ | 282 | A | + |
| 75 | A | ++ | 283 | A | + |
| 76 | A | ++ | 284 | A | ++ |
| 77 | A | ++ | 285 | A | ++ |
| 78 | A | ++ | 286 | A | ++ |
| 79 | A | ++ | 287 | A | ++ |
| 80 | A | ++ | 288 | A | ++ |
| 81 | A | ++ | 289 | A | ++ |
| 82 | A | + | 290 | A | ++ |
| 83 | A | ++ | 291 | A | ++ |
| 84 | A | ++ | 292 | A | ++ |
| 85 | A | ++ | 293 | A | ++ |
| 86 | A | ++ | 294 | A | + |
| 87 | A | + | 295 | A | ++ |
| 88 | A | ++ | 296 | A | ++ |
| 89 | A | ++ | 297 | A | + |
| 90 | A | ++ | 298 | A | ++ |
| 91 | A | ++ | 299 | A | ++ |
| 92 | A | ++ | 300 | A | + |
| 93 | A | ++ | 301 | A | ++ |
| 94 | A | ++ | 302 | A | + |
| 95 | A | ++ | 303 | A | ++ |
| 96 | A | + | 304 | A | ++ |
| 97 | A |  | 305 | A | ++ |
| 98 | A | ++ | 306 | A | ++ |
| 99 | A | + | 307 | A | ++ |
| 100 | A | ++ | 308 | A | ++ |
| 101 | A | ++ | 309 | A | ++ |
| 102 | A | + | 310 | A | ++ |
| 103 | A | + | 311 | A | ++ |
| 104 | A | ++ | 312 | A | ++ |
| 105 | A | ++ | 313 | A | ++ |
| 106 | A |  | 314 | A | ++ |
| 107 | A | ++ | 315 | A | ++ |
| 108 | A | ++ | 316 | A | ++ |
| 109 | A | ++ | 317 | A | ++ |
| 110 | A | + | 318 | A | ++ |
| 111 | A | ++ | 319 | A | ++ |
| 112 | A | ++ | 320 | A | ++ |

(continued)

| Example | $IC_{50}$ value | Increased $PGE_2$ production in A549 cells | Example | $IC_{50}$ value | Increased $PGE_2$ production in A549 cells |
|---|---|---|---|---|---|
| 113 | A | ++ | 321 | A | ++ |
| 114 | A | ++ | 322 | A | ++ |
| 115 | A | ++ | 323 | A | ++ |
| 116 | A | ++ | 324 | A | ++ |
| 117 | A | ++ | 325 | A | ++ |
| 118 | A | ++ | 326 | A | ++ |
| 119 | A | ++ | 327 | A | ++ |
| 120 | A | ++ | 328 | A | ++ |
| 121 | A | | 329 | A | ++ |
| 122 | A | | 330 | A | ++ |
| 123 | A | | 331 | A | ++ |
| 124 | A | ++ | 332 | A | ++ |
| 125 | A | | 333 | A | ++ |
| 126 | A | | 334 | A | ++ |
| 127 | A | ++ | 335 | A | ++ |
| 128 | A | | 336 | A | ++ |
| 129 | A | | 337 | A | ++ |
| 130 | A | ++ | 338 | A | ++ |
| 131 | A | ++ | 339 | A | ++ |
| 132 | A | ++ | 340 | A | ++ |
| 133 | A | ++ | 341 | A | ++ |
| 134 | A | ++ | 342 | A | ++ |
| 135 | A | | 343 | A | ++ |
| 136 | A | ++ | 344 | A | ++ |
| 137 | A | | 345 | A | ++ |
| 138 | A | ++ | 346 | A | ++ |
| 139 | A | | 347 | A | + |
| 140 | A | ++ | 348 | A | ++ |
| 141 | A | ++ | 349 | A | ++ |
| 142 | A | ++ | 350 | A | ++ |
| 143 | A | ++ | 351 | A | ++ |
| 144 | A | | 352 | A | ++ |
| 145 | A | | 353 | A | ++ |
| 146 | A | ++ | 354 | A | ++ |
| 147 | A | ++ | 355 | A | ++ |
| 148 | A | ++ | 356 | A | ++ |
| 149 | A | ++ | 357 | A | ++ |
| 150 | B | + | 358 | A | ++ |
| 151 | A | ++ | 359 | A | ++ |

(continued)

| Example | $IC_{50}$ value | Increased $PGE_2$ production in A549 cells | Example | $IC_{50}$ value | Increased $PGE_2$ production in A549 cells |
|---|---|---|---|---|---|
| 152 | B | | 360 | A | ++ |
| 153 | C | + | 361 | A | ++ |
| 154 | A | ++ | 362 | A | ++ |
| 155 | A | ++ | 363 | A | + |
| 156 | A | ++ | 364 | A | ++ |
| 157 | A | ++ | 365 | A | ++ |
| 158 | B | | 366 | A | ++ |
| 159 | B | ++ | 367 | A | ++ |
| 160 | C | + | 368 | A | ++ |
| 161 | C | | 369 | A | ++ |
| 162 | A | | 370 | A | ++ |
| 163 | A | | 371 | A | ++ |
| 164 | C | | 372 | A | ++ |
| 165 | B | | 373 | A | ++ |
| 166 | B | | 374 | A | ++ |
| 167 | A | | 375 | A | ++ |
| 168 | B | | 376 | A | ++ |
| 169 | A | | 377 | A | ++ |
| 170 | A | | 378 | A | ++ |
| 171 | A | | 379 | A | ++ |
| 172 | C | | 380 | A | ++ |
| 173 | A | | 381 | A | ++ |
| 174 | C | | 382 | A | ++ |
| 175 | C | | 383 | A | ++ |
| 176 | C | | 384 | A | ++ |
| 177 | C | | 385 | A | ++ |
| 178 | B | | 386 | A | ++ |
| 179 | B | | 387 | A | ++ |
| 180 | B | | 388 | A | ++ |
| 181 | B | | 389 | A | ++ |
| 182 | B | | 390 | A | ++ |
| 183 | C | | 391 | A | ++ |
| 184 | B | | 392 | A | ++ |
| 185 | B | | 393 | A | ++ |
| 186 | B | | 394 | A | ++ |
| 187 | B | | 395 | A | ++ |
| 188 | B | | 396 | A | ++ |
| 189 | C | | 397 | A | ++ |
| 190 | A | | 398 | A | ++ |

(continued)

| Example | $IC_{50}$ value | Increased $PGE_2$ production in A549 cells | Example | $IC_{50}$ value | Increased $PGE_2$ production in A549 cells |
|---|---|---|---|---|---|
| 191 | B | | 399 | A | ++ |
| 192 | A | ++ | 400 | A | ++ |
| 193 | A | ++ | 401 | A | ++ |
| 194 | A | + | 402 | A | ++ |
| 195 | A | ++ | 403 | A | ++ |
| 196 | A | ++ | 404 | A | ++ |
| 197 | A | ++ | 405 | A | ++ |
| 198 | A | ++ | 406 | A | ++ |
| 199 | A | ++ | 407 | A | ++ |
| 200 | A | ++ | 408 | A | ++ |
| 201 | A | ++ | 409 | A | ++ |
| 202 | A | ++ | 410 | A | ++ |
| 203 | A | ++ | 411 | A | ++ |
| 204 | A | + | 412 | A | ++ |
| 205 | A | ++ | 413 | B | |
| 206 | A | ++ | | | |
| 207 | A | + | | | |
| 208 | A | ++ | | | |

The foregoing description of the invention is for illustrative purposes only, and it will be readily apparent to those skilled in the art to which the invention belongs that varying substitutions and modifications may be made to the invention disclosed herein without departing from the spirit of the invention or essential features of the invention. It should therefore be understood that the embodiments described above are for the purpose of illustration of the invention only and are not intended in any way to limit the scope of the present invention. For example, each of the components described in a single form may also be implemented in a distributed manner, and similarly, components described as distributed may also be implemented in a combined form.

The scope of the invention is indicated by the following patent claims. The meaning and scope of the patent claims and all modifications or variations derived from their equivalents are considered to be falling within the scope of the invention.

## Claims

**1.** A heterocyclic compound represented by the following Formula 1, a hydrate thereof, a solvate thereof or a pharmaceutically acceptable salt thereof, wherein:

<Formula 1>

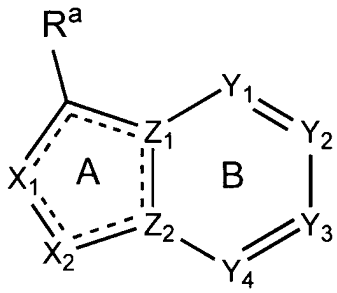

a fused bicyclic ring of A ring and B ring is formed,
$X_1$, $Y_1$, $Y_2$, $Y_3$ and $Y_4$ are independently CH or N,
$X_2$ is CH, N or NH,
$Z_1$ and $Z_2$ are C or N,
at least one of $X_1$, $X_2$, $Y_1$, $Y_2$, $Y_3$, $Y_4$, $Z_1$ and $Z_2$ is N or NH,
$R^a$ is H, $C_{1-6}$ straight or branched chain alkyl substituted with $R^{a-1}$, or C(O)$R^{a-1}$,
$R^{a-1}$ is $C_{1-6}$ straight or branched chain alkyl, $C_{3-8}$ cycloalkyl, hydroxy, $R^s$, or $R^u$, and $R^{a-1}$ is optionally substituted with one or more independent $R^{a-2}$,
$R^{a-2}$ is $C_{1-6}$ straight or branched chain alkyl, halogen, $C_{1-4}$ haloalkyl,
$X_1$ is optionally substituted with $R^b$, wherein $R^b$ is amino, or C(O)$R^s$,
$Y_1$ is optionally substituted with $R^1$, $R^1$ is $R^f$,
$Y_2$ is optionally substituted with $R^2$,
$R^2$ is $C_{1-6}$ straight or branched chain alkyl, halogen, $C_{5-12}$ aryl with 1 to 2 ring(s), $R^u$, or $R^f$, and $R^2$ is optionally substituted with one or more independent $R^{2-1}$,
$R^{2-1}$ is $C_{1-4}$ alkoxy, halogen or hydroxy,
$Y_3$ is optionally substituted with $R^3$,
$R^3$ is $C_{5-7}$ aryl, $R^u$, or $R^f$, and $R^3$ is optionally substituted with one or more independent $R^{3}$-1
$R^{3-1}$ is $C_{1-4}$ alkoxy, halogen, hydroxy or cyano,
$Y_4$ is optionally substituted with $R^4$ or -L-$R^4$,
L is $-NH(CH_2)_m-$, -NHC(O)-, $-NHSO_2-$ or -S-, m is an integer from 0 to 3,
$R^4$ is $C_{5-12}$ aryl with 1 to 2 ring(s), $R^u$, $R^s$, or $R^f$, and $R^4$ is optionally substituted with one or more independent Q,
Q is $C_{1-6}$ straight or branched chain alkyl, halogen, hydroxy, $C_{1-4}$ haloalkyl, $C_{1-4}$ alkoxy, nitro, cyano, amino, carboxylate, carboxylic acid, $CHR^5$, $CH_2R^5$, $NR^5R^6$, C(O)$R^5$, C(O)O$R^5$, C(O)$NR^5R^6$, NHC(O)$(CH_2)_pR^5$, NHC(O)$CH_2OR^5$, NHC(O)$CH_2NR^5R^6$, $NHCH_2CH_2R^5$, $NR^5C(O)R^6$, $R^u$, or $R^s$, p is an integer from 0 to 3, and Q is optionally substituted with one or more independent $R^7$,
$R^5$ and $R^6$ are independently H, $C_{1-6}$ straight or branched chain alkyl, $C_{3-8}$ cycloalkyl, $C_{1-4}$ alkoxy, amino, sulfonyl, $C_{5-7}$ aryl, arylalkyl, $R^u$, or $R^s$,
$R^7$ is $C_{1-6}$ straight or branched chain alkyl, $C_{3-8}$ cycloalkyl, halogen, $C_{1-4}$ haloalkyl, hydroxy, $C_{1-4}$ alkoxy, hydroxyalkyl, cyano, amino, alkylamino, $C_{1-6}$ alkylcarboxylate, nitro, $R^u$, $C_{1-4}$ alkyl substituted with $R^u$, or $R^s$, and the bond between Q and $R^7$ is a single bond or a double bond,
$R^s$ is a 4- to 10-membered saturated heterocycloalkyl ring with 1 to 2 ring(s) having 1 to 3 heteroatoms selected from N, O and S,
$R^u$ is a 5- to 7-membered unsaturated heterocycloalkyl ring having 1 to 4 heteroatoms selected from N, O and S,
$R^f$ is a fused bicyclic ring in which 5- to 7-membered saturated or unsaturated heterocycloalkyl ring having 1 to 2 heteroatoms selected from N, O and S is fused with an aryl ring or a 5- to 7-membered heteroaryl ring having 1 to 2 N,
$R^s$, $R^u$ and $R^f$ are independently optionally substituted with one to two oxo (O) or thioxo (S).

**2.** The compound, a hydrate thereof, a solvate thereof or a pharmaceutically acceptable salt thereof of Claim 1, wherein the A ring is pyrrole, pyrazole, imidazole, or triazole.

**3.** The compound, a hydrate thereof, a solvate thereof or a pharmaceutically acceptable salt thereof of Claim 1, wherein the B ring is benzene, pyridine, pyrimidine or triazine.

**4.** The compound, a hydrate thereof, a solvate thereof or a pharmaceutically acceptable salt thereof of Claim 1, wherein

the fused bicyclic ring of A ring and B ring is indole, indazole, pyrrolopyridine, pyrrolopyrimidine, pyrrolotriazine, pyrazolopyridine, imidazopyridine, or triazolopyridine.

**5.** The compound, a hydrate thereof, a solvate thereof or a pharmaceutically acceptable salt thereof of Claim 1, wherein the fused bicyclic ring of A ring and B ring is one selected from the group consisting of the following:

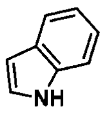, 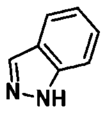, 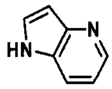, 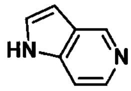, 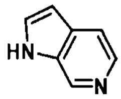, 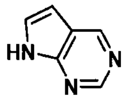, 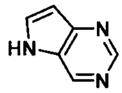, 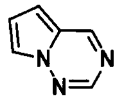,

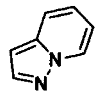, 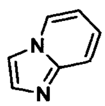, and 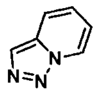.

**6.** The compound, a hydrate thereof, a solvate thereof or a pharmaceutically acceptable salt thereof of Claim 1, wherein

$R^a$ is H, propyl, C(O)$R^{a-1}$,
$R^{a-1}$ is methyl, butyl, cyclohexyl, hydroxy, piperidinyl, azaspiroheptanyl, azaspirooctanyl, azaspirononanyl, azabicycloheptanyl, azabicyclooctanyl, azabicyclononanyl or tetrahydropyridinyl, and
$R^{a-2}$ is methyl, ethyl, propyl, isopropyl, fluoro, or trifluoromethyl.

**7.** The compound, a hydrate thereof, a solvate thereof or a pharmaceutically acceptable salt thereof of Claim 1, wherein $R^b$ is amino or piperidinyl-carbonyl.

**8.** The compound, a hydrate thereof, a solvate thereof or a pharmaceutically acceptable salt thereof of Claim 1, wherein $R^1$ is benzodioxolyl.

**9.** The compound, a hydrate thereof, a solvate thereof or a pharmaceutically acceptable salt thereof of Claim 1, wherein

$R^2$ is methyl, fluoro, phenyl, naphthyl (naphthalenyl), furanyl, pyridinyl, isoindolinone, benzodioxolyl, dihydrobenzodioxinyl, benzofuranyl or indazolyl, and
$R^{2-1}$ is methoxy, chloro, fluoro or hydroxy.

**10.** The compound, a hydrate thereof, a solvate thereof or a pharmaceutically acceptable salt thereof of Claim 1, wherein

$R^3$ is phenyl, pyridinyl or benzodioxolyl, and
$R^{3-1}$ is methoxy, chloro, hydroxy or cyano.

**11.** The compound, a hydrate thereof, a solvate thereof or a pharmaceutically acceptable salt thereof of Claim 1, wherein L is -NH-, $-NHCH_2-$, $-NH(CH_2)_2-$, -NHC(O)-, $-NHSO_2-$ or -S-.

**12.** The compound, a hydrate thereof, a solvate thereof or a pharmaceutically acceptable salt thereof of Claim 1, wherein $R^4$ is phenyl, naphthyl (naphthalenyl), furanyl, pyrazolyl, dihydropyridinyl, pyridinyl, pyrimidinyl, pyridinone, pyrimidinone, isoxazolyl, piperidinyl, benzodioxolyl, isoindolinone, dihydroisoquinolinone, benzofuranyl, benzothiophenyl, indolyl, indazolyl, quinolinyl, quinolinone, isoquinolinone, dihydropyrrolopyridinone, benzoisoxazolone, dihydronaphthyridinone, benzoxazolyl, benzoisoxazolyl, triazolopyridinone, phthalazinone, quinazolinone, or pyridopyrimidinone.

**13.** The compound, a hydrate thereof, a solvate thereof or a pharmaceutically acceptable salt thereof of Claim 1, wherein Q is methyl, propyl, isopropyl, fluoro, chloro, bromo, hydroxy, trifluoromethyl, methoxy, nitro, cyano, amino, carboxylate, carboxylic acid, $CHR^5$, $CH_2R^5$, $NR^5R^6$, C(O)$R^5$, C(O)O$R^5$, C(O)N$R^5R^6$, NHC(O)$R^5$, NHC(O)$CH_2R^5$, NHC(O)$(CH_2)_2R^5$, NHC(O)$CH_2OR^5$, NHC(O)$CH_2NR^5R^6$, $NHCH_2CH_2R^5$, $NR^5$C(O)$R^6$, tetrazolyl, oxadiazolyl, oxadiazolone, furyl, thienyl, imidazolyl, pyrazolyl, isoxazolyl, thiazolyl, oxo-thiadiazolyl, thioxo-oxadiazolyl, pyridinyl, pyrimidinyl, azetidinyl, oxazolidinone, piperidinyl, piperazinyl, morpholino, azaspiroheptanyl, azaspirooctanyl, azaspirononanyl, azabicycloheptanyl, or azabicyclooctanyl,

$R^5$ is H, methyl, ethyl, butyl, cyclopropyl, cyclohexane, methoxy, amino, phenyl, benzyl, imidazolyl, oxadiazolyl, oxadiazolone, pyridinyl, pyrimidinyl, azetidinyl, piperidinyl, piperidinone, piperazinyl, morpholino, thiazolidinedione, piperazinone, piperazine-dione, azaspiroheptanyl, or azaspirononanyl, and
$R^6$ is H, methyl, ethyl, propyl, isopropyl, sulfonyl, pyrazolyl, pyridinyl, pyridazinyl, azetidinyl or piperidinyl.

**14.** The compound, a hydrate thereof, a solvate thereof or a pharmaceutically acceptable salt thereof of Claim 1, wherein $R^7$ is methyl, propyl, isopropyl, cyclopropyl, fluoro, chloro, difluoromethyl, trifluoromethyl, hydroxy, methoxy, hydroxymethyl, cyano, amino, dimethylamino, methylcarboxylate, tert-butylcarboxylate, nitro, isoxazolyl, thiazolyl, pyridinyl, or oxadiazolylmethyl.

**15.** The compound, a hydrate thereof, a solvate thereof or a pharmaceutically acceptable salt thereof of Claim 1, wherein the compound represented by the Formula 1 is selected from the group consisting of:

[1] 7-phenylpyrazolo[1,5-*a*]pyridin-3-yl)(piperidin-1-yl)methanone,
[2] (7-(3-chlorophenyl)pyrazolo[1,5-a]pyridin-3-yl)(piperidin-1-yl)methanone,
[3] methyl 3-(3-(piperidine- 1-carbonyl)pyrazolo[1,5-a]pyridin-7-yl)benzoate,
[4] (7-(3-methoxyphenyl)pyrazolo[1,5-a]pyridin-3-yl)(piperidin-1-yl)methanone,
[5] (7-(3-nitrophenyl)pyrazolo[1,5-a]pyridin-3-yl)(piperidin-1-yl)methanone,
[6] 3-(3-(piperidine-1-carbonyl)pyrazolo[1,5-a]pyridin-7-yl)benzonitrile,
[7] (7-(3-aminophenyl)pyrazolo[1,5-a]pyridin-3-yl)(piperidin-1-yl)methanone,
[8] (7-(2-chloropyridin-4-yl)pyrazolo[1,5-*a*]pyridin-3-yl)(piperidin-1-yl)methanone,
[9] (7-(5-aminopyridin-3-yl)pyrazolo[1,5-a]pyridin-3-yl)(piperidin-1-yl)methanone,
[10] 4-(3-(piperidine-1-carbonyl)pyrazolo[1,5-a]pyridin-7-yl)benzoic acid,
[11] methyl 4-(3-(piperidine-1-carbonyl)pyrazolo[1,5-a]pyridin-7-yl)benzoate,
[12] (7-(4-chlorophenyl)pyrazolo[1,5-a]pyridin-3-yl)(piperidin-1-yl)methanone,
[13] (7-(4-nitrophenyl)pyrazolo[1,5-a]pyridin-3-yl)(piperidin-1-yl)methanone,
[14] (7-(4-aminophenyl)pyrazolo[1,5-a]pyridin-3-yl)(piperidin-1-yl)methanone,
[15] 4-(3-(piperidine-1-carbonyl)pyrazolo[1,5-a]pyridin-7-yl)benzonitrile,
[16] (7-(6-nitropyridin-3-yl)pyrazolo[1,5-a]pyridin-3-yl)(piperidin-1-yl)methanone,
[17] (7-(6-aminopyridin-3-yl)pyrazolo[1,5-a]pyridin-3-yl)(piperidin-1-yl)methanone,
[18] 5-(3-(piperidine-1-carbonyl)pyrazolo[1,5-a]pyridin-7-yl)picolinonitrile,
[19] (7-(6-fluoropyridin-3-yl)pyrazolo[1,5-a]pyridin-3-yl)(piperidin-1-yl)methanone,
[20] (7-(4-methoxy-3-(trifluoromethyl)phenyl)pyrazolo[1,5-a]pyridin-3-yl)(piperidin-1-yl)methanone,
[21] (7-(3-chloro-4-hydroxyphenyl)pyrazolo[1,5-*a*]pyridin-3-yl)(piperidin-1-yl)methanone],
[22] 2-fluoro-4-(3-(piperidine-1-carbonyl)pyrazolo[1,5-*a*]pyridin-7-yl)benzonitrile,
[23] 2-(dimethylamino)-4-(3-(piperidine-1-carbonyl)pyrazolo[1,5-*a*]pyridin-7-yl)benzonitrile,
[24] (7-(3,4-dimethoxyphenyl)pyrazolo[1,5-a]pyridin-3-yl)(piperidin-1-yl)methanone,
[25] (7-(1-methyl-1*H*-pyrazol-5-yl)pyrazolo[1,5-*a*]pyridin-3-yl)(piperidin-1-yl)methanone,
[26] (7-(3-(morpholine-4-carbonyl)phenyl)pyrazolo[1,5-a]pyridin-3-yl)(piperidin-1-yl)methanone,
[27] *N*-methyl-3-(3-(piperidine-1-carbonyl)pyrazolo[1,5-*a*]pyridin-7-yl)benzamide,
[28] *N*-isopropyl-3-(3-(piperidine-1-carbonyl)pyrazolo[1,5-*a*]pyridin-7-yl)benzamide,
[29] azetidin-1-yl(3-(3-(piperidine-1-carbonyl)pyrazolo[1,5-*a*]pyridin-7-yl)phenyl)methanone,
[30] *N*-(2-methoxyethyl)-3-(3-(piperidine-1-carbonyl)pyrazolo[1,5-a]pyridin-7-yl)benzamide,
[31] *N*-(2-(dimethylamino)ethyl)-3-(3-(piperidine-1-carbonyl)pyrazolo[1,5-*a*]pyridin-7-yl)benzamide,
[32] *N*-(cyclopropylmethyl)-3-(3-(piperidine-1-carbonyl)pyrazolo[1,5-*a*]pyridin-7-yl)benzamide,
[33] *N*-(1-methylpiperidin-4-yl)-3-(3-(piperidine-1-carbonyl)pyrazolo[1,5-*a*]pyridin-7-yl)benzamide,
[34] piperazin-1-yl(3-(3-(piperidine-1-carbonyl)pyrazolo[1,5-*a*]pyridin-7-yl)phenyl)methanone,
[35] *tert-butyl* 4-(3-(3-(piperidine-1-carbonyl)pyrazolo[1,5-*a*]pyridin-7-yl)benzoyl)piperazine-1-carboxylate,
[36] *N*-(2-cyanoethyl)-3-(3-(piperidine-1-carbonyl)pyrazolo[1,5-*a*]pyridin-7-yl)benzamide,
[37] 3-(3-(piperidine-1-carbonyl)pyrazolo[1,5-*a*]pyridin-7-yl)-*N*-(pyridin-3-yl)benzamide,
[38] 3-(3-(piperidine-1-carbonyl)pyrazolo[1,5-*a*]pyridin-7-yl)-*N*-(pyridin-4-yl)benzamide,
[39] (7-(5-(morpholine-4-carbonyl)pyridin-3-yl)pyrazolo[1,5-*a*]pyridin-3-yl)(piperidin-1-yl)methanone,
[40] *N*-methyl-5-(3-(piperidine-1-carbonyl)pyrazolo[1,5-*a*]pyridin-7-yl)nicotinamide,
[41] azetidin-1-yl(5-(3-(piperidine-1-carbonyl)pyrazolo[1,5-*a*]pyridin-7-yl)pyridin-3-yl)methanone,
[42] (3-hydroxyazetidin-1-yl)(5-(3-(piperidine-1-carbonyl)pyrazolo[1,5-*a*]pyridin-7-yl)pyridin-3-yl)methanone,
[43] (7-(5-(2-azaspiro[3.3]heptane-2-carbonyl)pyridin-3-yl)pyrazolo[1,5-*a*]pyridin-3-yl)(piperidin-1-yl)methanone,
[44] (3,3-difluoroazetidin-1-yl)(5-(3-(piperidine-1-carbonyl)pyrazolo[1,5-*a*]pyridin-7-yl)pyridin-3-yl)methanone,

[45] *N*-(2-methoxyethyl)-5-(3-(piperidine-1-carbonyl)pyrazolo[1,5-*a*]pyridin-7-yl)nicotinamide,
[46] *N*-(cyclopropylmethyl)-5-(3-(piperidine-1-carbonyl)pyrazolo[1,5-*a*]pyridin-7-yl)nicotinamide,
[47] *N*-(1-methylpiperidin-4-yl)-5-(3-(piperidine-1-carbonyl)pyrazolo[1,5-*a*]pyridin-7-yl)nicotinamide,
[48] piperazin-1-yl(5-(3-(piperidine-1-carbonyl)pyrazolo[1,5-*a*]pyridin-7-yl)pyridin-3-yl)methanone,
[49] (4-methylpiperazin-1-yl)(5-(3-(piperidine-1-carbonyl)pyrazolo[1,5-*a*]pyridin-7-yl)pyridin-3-yl)methanone,
[50] (4-isopropylpiperazin-1-yl)(5-(3-(piperidine-1-carbonyl)pyrazolo[1,5-*a*]pyridin-7-yl)pyridin-3-yl)methanone,
[51] *N*-(2-cyanoethyl)-5-(3-(piperidine-1-carbonyl)pyrazolo[1,5-*a*]pyridin-7-yl)nicotinamide,
[52] *N*-(cyanomethyl)-5-(3-(piperidine-1-carbonyl)pyrazolo[1,5-*a*]pyridin-7-yl)nicotinamide,
[53] 5-(3-(piperidine-1-carbonyl)pyrazolo[1,5-*a*]pyridin-7-yl)-*N*-(pyridin-3-yl)nicotinamide,
[54] 5-(3-(piperidine-1-carbonyl)pyrazolo[1,5-a]pyridin-7-yl)-N-(pyridin-4-yl)nicotinamide,
[55] *N*-(isoxazol-3-ylmethyl)-5-(3-(piperidine-1-carbonyl)pyrazolo[1,5-*a*]pyridin-7-yl)nicotinamide,
[56] 5-(3-(piperidine-1-carbonyl)pyrazolo[1,5-*a*]pyridin-7-yl)-*N*-(pyridazin-4-yl)nicotinamide,
[57] 5-(3-(piperidine-1-carbonyl)pyrazolo[1,5-*a*]pyridin-7-yl)-*N*-(thiazol-5-ylmethyl)nicotinamide,
[58] *N*-methyl-4-(3-(piperidine-1-carbonyl)pyrazolo[1,5-*a*]pyridin-7-yl)benzamide,
[59] *N,N*-dimethyl-4-(3-(piperidine-1-carbonyl)pyrazolo[1,5-*a*]pyridin-7-yl)benzamide,
[60] *N*-isopropyl-4-(3-(piperidine-1-carbonyl)pyrazolo[1,5-*a*]pyridin-7-yl)benzamide,
[61] azetidin-1-yl(4-(3-(piperidine-1-carbonyl)pyrazolo[1,5-*a*]pyridin-7-yl)phenyl)methanone,
[62] *N*-(2-methoxyethyl)-4-(3-(piperidine-1-carbonyl)pyrazolo[1,5-*a*]pyridin-7-yl)benzamide,
[63] *N*-(2-(dimethylamino)ethyl)-4-(3-(piperidine-1-carbonyl)pyrazolo[1,5-*a*]pyridin-7-yl)benzamide,
[64] *N*-(cyclopropylmethyl)-4-(3-(piperidine-1-carbonyl)pyrazolo[1,5-*a*]pyridin-7-yl)benzamide,
[65] N-(1-methylpiperidin-4-yl)-4-(3-(piperidine-1-carbonyl)pyrazolo[1,5-*a*]pyridin-7-yl)benzamide,
[66] piperazin-1-yl(4-(3-(piperidine-1-carbonyl)pyrazolo[1,5-*a*]pyridin-7-yl)phenyl)methanone,
[67] (4-methylpiperazin-1-yl)(4-(3-(piperidine-1-carbonyl)pyrazolo[1,5-*a*]pyridin-7-yl)phenyl)methanone,
[68] (4-isopropylpiperazin-1-yl)(4-(3-(piperidine-1-carbonyl)pyrazolo[1,5-*a*]pyridin-7-yl)phenyl)methanone,
[69] *N*-(2-cyanoethyl)-4-(3-(piperidine-1-carbonyl)pyrazolo[1,5-*a*]pyridin-7-yl)benzamide,
[70] *N*-(cyanomethyl)-4-(3-(piperidine-1-carbonyl)pyrazolo[1,5-*a*]pyridin-7-yl)benzamide,
[71] 4-(3-(piperidine-1-carbonyl)pyrazolo[1,5-*a*]pyridin-7-yl)-*N*-(pyridin-3-yl)benzamide,
[72] 4-(3-(piperidine-1-carbonyl)pyrazolo[1,5-*a*]pyridin-7-yl)-*N*-(pyridin-4-yl)benzamide,
[73] *N*-(isoxazol-3-ylmethyl)-4-(3-(piperidine-1-carbonyl)pyrazolo[1,5-*a*]pyridin-7-yl)benzamide,
[74] (7-(6-(morpholine-4-carbonyl)pyridin-3-yl)pyrazolo[1,5-*a*]pyridin-3-yl)(piperidin-1-yl)methanone,
[75] *N*-methyl-5-(3-(piperidine-1-carbonyl)pyrazolo[1,5-*a*]pyridin-7-yl)picolinamide,
[76] *N*-isopropyl-5-(3-(piperidine-1-carbonyl)pyrazolo[1,5-*a*]pyridin-7-yl)picolinamide,
[77] azetidin-1-yl(5-(3-(piperidine-1-carbonyl)pyrazolo[1,5-*a*]pyridin-7-yl)pyridin-2-yl)methanone,
[78] *N*-(2-methoxyethyl)-5-(3-(piperidine-1-carbonyl)pyrazolo[1,5-*a*]pyridin-7-yl)picolinamide,
[79] *N*-(2-(dimethylamino)ethyl)-5-(3-(piperidine-1-carbonyl)pyrazolo[1,5-*a*]pyridin-7-yl)picolinamide,
[80] *N*-(cyclopropylmethyl)-5-(3-(piperidine-1-carbonyl)pyrazolo[1,5-*a*]pyridin-7-yl)picolinamide,
[81] *N*-(1-methylpiperidin-4-yl)-5-(3-(piperidine-1-carbonyl)pyrazolo[1,5-*a*]pyridin-7-yl)picolinamide,
[82] piperazin-1-yl(5-(3-(piperidine-1-carbonyl)pyrazolo[1,5-*a*]pyridin-7-yl)pyridin-2-yl)methanone,
[83] (4-methylpiperazin-1-yl)(5-(3-(piperidine-1-carbonyl)pyrazolo[1,5-*a*]pyridin-7-yl)pyridin-2-yl)methanone,
[84] (4-isopropylpiperazin-1-yl)(5-(3-(piperidine-1-carbonyl)pyrazolo[1,5-*a*]pyridin-7-yl)pyridin-2-yl)methanone,
[85] *N*-(2-cyanoethyl)-5-(3-(piperidine-1-carbonyl)pyrazolo[1,5-*a*]pyridin-7-yl)picolinamide,
[86] *N*-(cyanomethyl)-5-(3-(piperidine-1-carbonyl)pyrazolo[1,5-*a*]pyridin-7-yl)picolinamide,
[87] 5-(3-(piperidine-1-carbonyl)pyrazolo[1,5-*a*]pyridin-7-yl)-*N*-(pyridin-3-yl)picolinamide,
[88] 5-(3-(piperidine-1-carbonyl)pyrazolo[1,5-*a*]pyridin-7-yl)-*N*-(pyridin-4-yl)picolinamide,
[89] *N*-(isoxazol-3-ylmethyl)-5-(3-(piperidine-1-carbonyl)pyrazolo[1,5-*a*]pyridin-7-yl)picolinamide,
[90] *N*-(2-cyanoethyl)-5-(3-(piperidine-1-carbonyl)pyrazolo[1,5-*a*]pyridin-7-yl)furan-2-carboxamide,
[91] 3-methoxy-*N*-(3-(3-(piperidine-1-carbonyl)pyrazolo[1,5-*a*]pyridin-7-yl)phenyl)propanamide,
[92] 1-(3-(3-(piperidine-1-carbonyl)pyrazolo[1,5-*a*]pyridin-7-yl)phenyl)urea,
[93] 1-methyl-*N*-(3-(3-(piperidine-1-carbonyl)pyrazolo[1,5-*a*]pyridin-7-yl)phenyl)piperidine-4-carboxamide,
[94] *N*-(3-(3-(piperidine-1-carbonyl)pyrazolo[1,5-*a*]pyridin-7-yl)phenyl)cyclopropanecarboxamide,
[95] *N*-(3-(3-(piperidine-1-carbonyl)pyrazolo[1,5-*a*]pyridin-7-yl)phenyl)nicotinamide,
[96] *N*-(3-(3-(piperidine-1-carbonyl)pyrazolo[1,5-*a*]pyridin-7-yl)phenyl)cyclohexanecarboxamide,
[97] *N*-(4-(3-(piperidine-1-carbonyl)pyrazolo[1,5-*a*]pyridin-7-yl)phenyl)acetamide,
[98] 3-methoxy-*N*-(4-(3-(piperidine-1-carbonyl)pyrazolo[1,5-*a*]pyridin-7-yl)phenyl)propanamide,
[99] 1-methyl-*N*-(4-(3-(piperidine-1-carbonyl)pyrazolo[1,5-*a*]pyridin-7-yl)phenyl)piperidine-4-carboxamide,
[100] *N*-(4-(3-(piperidine-1-carbonyl)pyrazolo[1,5-*a*]pyridin-7-yl)phenyl)cyclopropanecarboxamide,
[101] *N*-(4-(3-(piperidine-1-carbonyl)pyrazolo[1,5-*a*]pyridin-7-yl)phenyl)nicotinamide,
[102] *N*-(4-(3-(piperidine-1-carbonyl)pyrazolo[1,5-*a*]pyridin-7-yl)phenyl)cyclohexanecarboxamide,

[103] 3-methoxy-*N*-(5-(3-(piperidine-1-carbonyl)pyrazolo[1,5-*a*]pyridin-7-yl)pyridin-2-yl)propanamide,
[104] 2-(dimethylamino)-*N*-(5-(3-(piperidine-1-carbonyl)pyrazolo[1,5-*a*]pyridin-7-yl)pyridin-2-yl)acetamide,
*[105]* 2-oxo-*N*-(5-(3-(piperidine-1-carbonyl)pyrazolo[1,5-*a*]pyridin-7-yl)pyridin-2-yl)piperidine-4-carboxamide,
[106] *N*-(5-(3-(piperidine-1-carbonyl)pyrazolo[1,5-*a*]pyridin-7-yl)pyridin-2-yl)-2-(pyridin-3-yl)acetamide,
[107] (7-(6-((3-methoxypropyl)amino)pyridin-3-yl)pyrazolo[1,5-*a*]pyridin-3-yl)(piperidin-1-yl)methanone,
[108] (7-(6-morpholinopyridin-3-yl)pyrazolo[1,5-*a*]pyridin-3-yl)(piperidin-1-yl)methanone,
[109] (7-(6-(isopropylamino)pyridin-3-yl)pyrazolo[1,5-*a*]pyridin-3-yl)(piperidin-1-yl)methanone,
[110] piperidin-1-yl(7-(6-(piperidin-1-yl)pyridin-3-yl)pyrazolo[1,5-*a*]pyridin-3-yl)methanone,
[111] (7-(6-(4-methylpiperazin-1-yl)pyridin-3-yl)pyrazolo[1,5-*a*]pyridin-3-yl)(piperidin-1-yl)methanone,
[112] *N*-(5-(3-(piperidine-1-carbonyl)pyrazolo[1,5-*a*]pyridin-7-yl)pyridin-2-yl)methanesulfonamide,
[113] (7-(3-(1*H*-tetrazol-5-yl)phenyl)pyrazolo[1,5-*a*]pyridin-3-yl)(piperidin-1-yl)methanone,
[114] (7-(4-(1*H*-tetrazol-5-yl)phenyl)pyrazolo[1,5-*a*]pyridin-3-yl)(piperidin-1-yl)methanone,
[115] 3-(3-(3-(piperidine-1-carbonyl)pyrazolo[1,5-*a*]pyridin-7-yl)phenyl)-1,2,4-oxadiazol-5(4H)-one,
[116] 3-(4-(3-(piperidine-1-carbonyl)pyrazolo[1,5-*a*]pyridin-7-yl)phenyl)-1,2,4-oxadiazol-5(4H)-one,
[117] 3-(5-(3-(piperidine-1-carbonyl)pyrazolo[1,5-*a*]pyridin-7-yl)pyridin-2-yl)-1,2,4-oxadiazol-5(4H)-one,
[118] (7-(4-(5-methyl-1,2,4-oxadiazol-3-yl)phenyl)pyrazolo[1,5-*a*]pyridin-3-yl)(piperidin-1-yl)methanone,
[119] (*Z*)-5-(3-(3-(piperidine-1-carbonyl)pyrazolo[1,5-*a*]pyridin-7-yl)benzylidene)thiazolidine-2,4-dione,
[120] *tert*-butyl 4-(3-(piperidine-1-carbonyl)pyrazolo[1,5-*a*]pyridin-7-yl)-3,6-dihydropyridine-1(2*H*)-carboxylate,
[121] (7-(benzo[*d*][1,3]dioxol-5-yl)pyrazolo[1,5-*a*]pyridin-3-yl)(piperidin-1-yl)methanone,
[122] (7-(benzofuran-3-yl)pyrazolo[1,5-*a*]pyridin-3-yl)(piperidin-1-yl)methanone,
[123] (7-(benzofuran-2-yl)pyrazolo[1,5-*a*]pyridin-3-yl)(piperidin-1-yl)methanone,
[124] (7-(benzo[b]thiophen-3-yl)pyrazolo[1,5-*a*]pyridin-3-yl)(piperidin-1-yl)methanone,
[125] (7-(1*H*-indazol-6-yl)pyrazolo[1,5-*]*pyridin-3-yl)(piperidin-1-yl)methanone,
[126] (7-(1*H*-indol-5-yl)pyrazolo[1,5-*a*]pyridin-3-yl)(piperidin-1-yl)methanone,
[127] (7-(benzofuran-5-yl)pyrazolo[1,5-*a*]pyridin-3-yl)(piperidin-1-yl)methanone,
[128] (7-(naphthalen-1-yl)pyrazolo[1,5-*a*]pyridin-3-yl)(piperidin-1-yl)methanone,
[129] (7-(naphthalen-2-yl)pyrazolo[1,5-*a*]pyridin-3-yl)(piperidin-1-yl)methanone,
[130] piperidin-1-yl(7-(quinolin-3-yl)pyrazolo[1,5-*a*]pyridin-3-yl)methanone,
[131] 7-(3-(piperidine-1-carbonyl)pyrazolo[1,5-*a*]pyridin-7-yl)-3,4-dihydroisoquinolin-1(2H)-one,
[132] 2-methyl-7-(3-(piperidine-1-carbonyl)pyrazolo[1,5-*a*]pyridin-7-yl)-3,4-dihydroisoquinolin-1(2*H*)-one,
[133] 2-isopropyl-7-(3-(piperidine-1-carbonyl)pyrazolo[1,5-*a*]pyridin-7-yl)-3,4-dihydroisoquinolin-1(2H)-one,
[134] 7-(3-(piperidine-1-carbonyl)pyrazolo[1,5-*a*]pyridin-7-yl)isoquinolin-1(2*H*)-one,
[135] 2-methyl-7-(3-(piperidine-1-carbonyl)pyrazolo[1,5-*a*]pyridin-7-yl)isoquinolin-1(2*H*)-one,
[136] 6-(3-(piperidine-1-carbonyl)pyrazolo[1,5-*a*]pyridin-7-yl)-3,4-dihydroisoquinolin-1(2*H*)-one,
[137] 2-methyl-6-(3-(piperidine-1-carbonyl)pyrazolo[1,5-*a*]pyridin-7-yl)-3,4-dihydroisoquinolin-1(2*H*)-one,
[138] 6-(3-(piperidine-1-carbonyl)pyrazolo[1,5-*a*]pyridin-7-yl)isoquinolin-1(2*H*)-one,
[139] 2-methyl-6-(3-(piperidine-1-carbonyl)pyrazolo[1,5-*a*]pyridin-7-yl)isoquinolin-1(2H)-one,
[140] 6-(3-(piperidine-1-carbonyl)pyrazolo[1,5-*a*]pyridin-7-yl)isoindolin-1-one,
[141] 2-methyl-6-(3-(piperidine-1-carbonyl)pyrazolo[1,5-*a*]pyridin-7-yl)isoindolin-1-one,
[142] 2-isopropyl-6-(3-(piperidine-1-carbonyl)pyrazolo[1,5-*a*]pyridin-7-yl)isoindolin-1-one,
[143] 5-(3-(piperidine-1-carbonyl)pyrazolo[1,5-*a*]pyridin-7-yl)isoindolin-1-one,
[144] 2-methyl-5-(3-(piperidine-1-carbonyl)pyrazolo[1,5-*a*]pyridin-7-yl)isoindolin-1-one,
[145] 6-(3-(piperidine-1-carbonyl)pyrazolo[1,5-*a*]pyridin-7-yl)quinolin-2(1*H*)-one,
[146] 4-((3-(piperidine-1-carbonyl)pyrazolo[1,5-*a*]pyridin-7-yl)amino)benzonitrile,
[147] 3-((3-(piperidine-1-carbonyl)pyrazolo[1,5-*a*]pyridin-7-yl)amino)pyridin-2(1*H*)-one,
[148] piperidin-1-yl(7-((pyrimidin-2-ylmethyl)amino)pyrazolo[1,5-*a*]pyridin-3-yl)methanone,
[149] piperidin-1-yl(7-((2-(pyridin-4-yl)ethyl)amino)pyrazolo[1,5-*a*]pyridin-3-yl)methanone,
[150] (7-((1-methylpiperidin-4-yl)amino)pyrazolo[1,5-*a*]pyridin-3-yl)(piperidin-1-yl)methanone,
[151] 1-(7-(quinolin-3-yl)pyrazolo[1,5-*a*]pyridin-3-yl)pentan-1-one,
[152] 4-(3-acetylpyrazolo[1,5-*a*]pyridin-7-yl)benzonitrile,
[153] 4-(3-(2-hydroxypropan-2-yl)pyrazolo[1,5-*a*]pyridin-7-yl)benzonitrile,
[154] 7-(3-(4-fluoropiperidine-1-carbonyl)pyrazolo[1,5-*a*]pyridin-7-yl)-3,4-dihydroisoquinolin-1(2*H*)-one,
[155] 6-(3-(4-fluoropiperidine-1-carbonyl)pyrazolo[1,5-*a*]pyridin-7-yl)isoindolin-1-one,
[156] 4-(3-(4-fluoropiperidine-1-carbonyl)pyrazolo[1,5-*a*]pyridin-7-yl)benzonitrile,
[157] (4-fluoropiperidin-1-yl)(7-(4-nitrophenyl)pyrazolo[1,5-*a*]pyridin-3-yl)methanone,
[158] cyclohexyl(7-phenylpyrazolo[1,5-*a*]pyridin-3-yl)methanone,
[159] 7-(2-amino-3-(piperidine-1-carbonyl)pyrazolo[1,5-*a*]pyridin-7-yl)-3,4-dihydroisoquinolin-1 (2*H*)-one,
[160] 7-(2-amino-3-(piperidine-1-carbonyl)pyrazolo[1,5-*a*]pyridin-7-yl)-2-methyl-3,4-dihydroisoquinolin-1

(2*H*)-one,
[161] (7-(3-chlorophenyl)-1H-indazol-3-yl)(piperidin-1-yl)methanone,
[162] (8-(3-chlorophenyl)imidazo[1,2-*a*]pyridin-3-yl)(piperidin-1-yl)methanone,
[163] (8-(4-nitrophenyl)imidazo[1,2-*a*]pyridin-3-yl)(piperidin-1-yl)methanone,
[164] (8-(4-aminophenyl)imidazo[1,2-*a*]pyridin-3-yl)(piperidin-1-yl)methanone,
[165] (8-(3-chlorophenyl)-6-methylimidazo[1,2-*a*]pyridin-3-yl)(piperidin-1-yl)methanone,
[166] (8-(2-chloropyridin-4-yl)-6-methylimidazo[1,2-*a*]pyridin-3-yl)(piperidin-1-yl)methanone,
[167] (8-(3-chlorophenyl)-6-fluoroimidazo[1,2-*a*]pyridin-3-yl)(piperidin-1-yl)methanone,
[168] (7-(4-methoxyphenyl)-[1,2,3]triazolo[1,5-*a*]pyridin-3-yl)(piperidin-1-yl)methanone,
[169] (7-(3-chlorophenyl)-1*H*-indol-3-yl)(piperidin-1-yl)methanone,
[170] (6-(3-chlorophenyl)pyrazolo[1,5-*a*]pyridin-3-yl)(piperidin-1-yl)methanone,
[171] (6-(4-methoxyphenyl)pyrazolo[1,5-*a*]pyridin-3-yl)(piperidin-1-yl)methanone,
[172] (6-(benzo[d][1,3]dioxol-5-yl)-1H-pyrrolo[3,2-b]pyridin-2-yl)(piperidin-1-yl)methanone,
[173] (5-(benzo[d][1,3]dioxol-5-yl)-1H-pyrrolo[3,2-b]pyridin-2-yl)(piperidin-1-yl)methanone,
[174] (4-(benzo[d][1,3]dioxol-5-yl)-1H-pyrrolo[3,2-c]pyridin-2-yl)(piperidin-1-yl)methanone,
[175] (4-(benzo[d][1,3]dioxol-5-yl)-1H-pyrrolo[2,3-c]pyridin-2-yl)(piperidin-1-yl)methanone,
[176] (4-(benzo[d][1,3]dioxol-5-yl)pyrrolo[2,1-f][1,2,4]triazin-6-yl)(piperidin-1-yl)methanone,
[177] (4-(benzo[*d*][1,3]dioxol-5-yl)-7*H*-pyrrolo[2,3-*d*]pyrimidin-6-yl)(piperidin-1-yl)methanone,
[178] (5-(2,3-dihydrobenzo[b][1,4]dioxin-6-yl)-1H-pyrrolo[3,2-b]pyridin-2-yl)(piperidin-1-yl)methanone,
[179] (5-(4-methoxyphenyl)-1H-pyrrolo[3,2-b]pyridin-2-yl)(piperidin-1-yl)methanone,
[180] (5-(4-chlorophenyl)-1H-pyrrolo[3,2-b]pyridin-2-yl)(piperidin-1-yl)methanone,
[181] (5-(3,4-dimethoxyphenyl)-1H-pyrrolo[3,2-b]pyridin-2-yl)(piperidin-1-yl)methanone,
[182] (5-(4-fluorophenyl)-1H-pyrrolo[3,2-b]pyridin-2-yl)(piperidin-1-yl)methanone,
[183] (5-(furan-3-yl)-1H-pyrrolo[3,2-b]pyridin-2-yl)(piperidin-1-yl)methanone,
[184] piperidin-1-yl(5-(pyridin-4-yl)-1H-pyrrolo[3,2-b]pyridin-2-yl)methanone,
[185] (5-(3-chlorophenyl)-1H-pyrrolo[3,2-b]pyridin-2-yl)(piperidin-1-yl)methanone,
[186] (5-(3-chloro-4-hydroxyphenyl)-1H-pyrrolo[3,2-b]pyridin-2-yl)(piperidin-1-yl)methanone,
[187] (2-(benzo[d][1,3]dioxol-5-yl)-5H-pyrrolo[3,2-d]pyrimidin-6-yl)(piperidin-1-yl)methanone,
[188] (5-(benzofuran-3-yl)-1H-pyrrolo[3,2-b]pyridin-2-yl)(piperidin-1-yl)methanone,
[189] (5-(naphthalen-2-yl)-1H-pyrrolo[3,2-b]pyridin-2-yl)(piperidin-1-yl)methanone,
[190] (5-(1H-indazol-6-yl)-1H-pyrrolo[3,2-b]pyridin-2-yl)(piperidin-1-yl)methanone,
[191] 2-(2-(piperidine-1-carbonyl)-1H-pyrrolo[3,2-b]pyridin-5-yl)isoindolin-1-one,
[192] (3-fluoroazetidin-1-yl)(5-(3-(piperidine-1-carbonyl)pyrazolo[1,5-*a*]pyridin-7-yl)pyridin-3-yl)methanone,
[193] *N*-(1-methyl-1*H*-pyrazol-3-yl)-5-(3-(piperidine-1-carbonyl)pyrazolo[1,5-*a*]pyridin-7-yl)nicotinamide,
[194] (7-((3-methoxyphenyl)thio)pyrazolo[1,5-*a*]pyridin-3-yl)(piperidin-1-yl)methanone,
[195] 3-(3-(piperidine-1-carbonyl)pyrazolo[1,5-*a*]pyridin-7-yl)-7,8-dihydro-1,6-naphthyridin-5(6*H*)-one,
[196] 2-(dimethylamino)-*N*-(5-(3-(piperidine-1-carbonyl)pyrazolo[1,5-*a*]pyridin-7-yl)pyridin-3-yl)acetamide,
[197] 3-methoxy-*N*-(5-(3-(piperidine-1-carbonyl)pyrazolo[1,5-*a*]pyridin-7-yl)pyridin-3-yl)propenamide,
[198] 2-oxo-*N*-(5-(3-(piperidine-1-carbonyl)pyrazolo[1,5-*a*]pyridin-7-yl)pyridin-3-yl)piperidine-4-carboxamide,
[199] 6-methyl-3-(3-(piperidine-1-carbonyl)pyrazolo[1,5-*a*]pyridin-7-yl)-7,8-dihydro-1,6-naphthyridin-5(6*H*)-one,
[200] *N*-(azetidin-3-yl)-5-(3-(piperidine-1-carbonyl)pyrazolo[1,5-*a*]pyridin-7-yl)nicotinamide,
[201] *N*-methylsulfonyl-5-[3-(piperidine-1-carbonyl)pyrazolo[1,5-*a*]pyridin-7-yl]pyridine-3-carboxamide,
[202] *N*-(1-methylazetidin-3-yl)-5-[3-(piperidine-1-carbonyl)pyrazolo[1,5-*a*]pyridin-7-yl]pyridine-3-carboxamide,
[203] 4-[5-[3-(piperidine-1-carbonyl)pyrazolo[1,5-*a*]pyridin-7-yl]pyridine-3-carbonyl]piperazin-2-one,
[204] 4-[5-[3-(piperidine-1-carbonyl)pyrazolo[1,5-*a*]pyridin-7-yl]pyridine-3-carbonyl]piperazine-2,6-dione,
[205] 3-fluoro-5-[3-(piperidine-1-carbonyl)pyrazolo[1,5-*a*]pyridin-7-yl]pyridine-2-carbonitrile,
[206] (7-(6-(azetidin-1-yl)pyridin-3-yl)pyrazolo[1,5-*a*]pyridin-3-yl)(piperidin-1-yl)methanone,
[207] 3-methyl-6-(3-(piperidine-1-carbonyl)pyrazolo[1,5-*a*]pyridin-7-yl)pyrimidin-4(3*H*)-one,
[208] (7-(6-(3-fluoroazetidin-1-yl)pyridin-3-yl)pyrazolo[1,5-*a*]pyridin-3-yl)(piperidin-1-yl)methanone,
[209] (7-(6-(3,3-difluoroazetidin-1-yl)pyridin-3-yl)pyrazolo[1,5-*a*]pyridin-3-yl)(piperidin-1-yl)methanone,
[210] (7-(6-(3-hydroxyazetidin-1-yl)pyridin-3-yl)pyrazolo[1,5-*a*]pyridin-3-yl)(piperidin-1-yl)methanone,
[211] 7-[[3-(piperidine-1-carbonyl)pyrazolo[1,5-*a*]pyridin-7-yl]amino]-3,4-dihydro-2*H*-isoquinolin-1-one,
[212] 6-[[3-(piperidine-1-carbonyl)pyrazolo[1,5-*a*]pyridin-7-yl]amino]isoindolin-1-one,
[213] (3-fluoroazetidin-1-yl)-[5-[3-(piperidine-1-carbonyl)pyrazolo[1,5-a]pyridin-7-yl]-2-pyridyl]methanone,
[214] 2-azaspiro[3.3]heptan-2-yl-[5-[3-(piperidine-1-carbonyl)pyrazolo[1,5-*a*]pyridin-7-yl]-2-pyridyl]methanone,
[215] (7-(6-(2-azabicyclo[2.2.1]heptan-2-yl)pyridin-3-yl)pyrazolo[1,5-*a*]pyridin-3-yl)(piperidin-1-yl)methanone,

[216] (7-(6-(2-azabicyclo[2.2.2]octan-2-yl)pyridin-3-yl)pyrazolo[1,5-*a*]pyridin-3-yl)(piperidin-1-yl)methanone,
[217] -(6-(7-azabicyclo[2.2.1]heptan-7-yl)pyridin-3-yl)pyrazolo[1,5-*a*]pyridin-3-yl)(piperidin-1-yl)methanone,
[218] (7-(6-(3-(dimethylamino)azetidin-1-yl)pyridin-3-yl)pyrazolo[1,5-*a*]pyridin-3-yl)(piperidin-1-yl)methanone,
[219] methyl 1-(5-(3-(piperidine-1-carbonyl)pyrazolo[1,5-*a*]pyridin-7-yl)pyridin-2-yl)azetidine-3-carboxylate,
[220] (7-(6-(2-azaspiro[3.3]heptan-2-yl)pyridin-3-yl)pyrazolo[1,5-*a*]pyridin-3-yl)(piperidin-1-yl)methanone,
[221] (7-(6-(3-(hydroxymethyl)azetidin-1-yl)pyridin-3-yl)pyrazolo[1,5-*a*]pyridin-3-yl)(piperidin-1-yl)methanone,
[222] (7-(6-(7-azaspiro[3.5]nonan-7-yl)pyridin-3-yl)pyrazolo[1,5-*a*]pyridin-3-yl)(piperidin-1-yl)methanone,
[223] (7-(6-(6-azaspiro[3.4]octan-6-yl)pyridin-3-yl)pyrazolo[1,5-*a*]pyridin-3 - yl)(piperidin-1-yl)methanone,
[224] 7-(3-(piperidine-1-carbonyl)pyrazolo[1,5-*a*]pyridin-7-yl)phthalazin-1(2*H*)-one,
[225] (6-hydroxy-2-azaspiro[3.3]heptan-2-yl)-[5-[3-(piperidine-1-carbonyl)pyrazolo[1,5-*a*]pyridin-7-yl]-3-pyridyl]methanone,
[226] 2-oxa-7-azaspiro[3.5]nonan-7-yl-[5-[3-(piperidine-1-carbonyl)pyrazolo[1,5-*a*]pyridin-7-yl]-3-pyridyl] methanone,
[227] (2-azabicyclo[2.2.1]heptan-2-yl)(7-phenylpyrazolo[1,5-*a*]pyridin-3-yl)methanone,
[228] (2-azabicyclo[2.2.2]octan-2-yl)(7-phenylpyrazolo[1,5-*a*]pyridin-3-yl)methanone,
[229] (7-azabicyclo[2.2.1]heptan-7-yl)(7-phenylpyrazolo[1,5-*a*]pyridin-3-yl)methanone,
[230] (3-hydroxyiminoazetidin-1-yl)-[5-[3-(piperidine-1-carbonyl)pyrazolo[1,5-*a*]pyridin-7-yl]-3-pyridyl]methanone,
[231] 6-(3-(4-fluoropiperidine-1-carbonyl)pyrazolo[1,5-*a*]pyridin-7-yl)-2-methylisoindolin-1-one,
[232] 3-(3-(piperidine-1-carbonyl)pyrazolo[1,5-*a*]pyridin-7-yl)-6,7-dihydro-5*H*-pyrrolo[3,4-*b*]pyridin-5-one,
[233] 6-(3-(piperidine-1-carbonyl)pyrazolo[1,5-*a*]pyridin-7-yl)phthalazin-1(2*H*)-one,
[234] 6-(3-(4,4-difluoropiperidine-1-carbonyl)pyrazolo[1,5-*a*]pyridin-7-yl)isoindolin-1-one,
[235] 4-[[3-(piperidine-1-carbonyl)pyrazolo[1,5-*a*]pyridin-7-yl]amino]-1*H*-pyrimidin-6-one,
[236] 3-methyl-6-[[3-(piperidine-1-carbonyl)pyrazolo[1,5-*a*]pyridin-7-yl]amino]pyrimidin-4-one,
[237] (3,3-difluoroazetidin-1-yl)-[5-[3-(piperidine-1-carbonyl)pyrazolo[1,5-*a*]pyridin-7-yl]-2-pyridyl]methanone,
[238] [7-[(4-chlorophenyl)methylamino]pyrazolo[1,5-*a*]pyridin-3-yl]-(1-piperidyl)methanone,
[239] 4-chloro-*N*-[3-(piperidine-1-carbonyl)pyrazolo[1,5-*a*]pyridin-7-yl]benzamide,
[240] 1,3,5-trimethyl-*N*-[3-(piperidine-1-carbonyl)pyrazolo[1,5-*a*]pyridin-7-yl]pyrazole-4-sulfonamide,
[241] 5-fluoro-*N*-[3-(piperidine-1-carbonyl)pyrazolo[1,5-*a*]pyridin-7-yl]pyridine-3-carboxamide,
[242] [7-[2-(4-chlorophenyl)ethylamino]pyrazolo[1,5-*a*]pyridin-3-yl]-(1-piperidyl)methanone,
[243] 3-fluoro-5-[3-(piperidine-1-carbonyl)pyrazolo[1,5-*a*]pyridin-7-yl]pyridine-2-carboxylic acid,
[244] methyl 3-fluoro-5-[3-(piperidine-1-carbonyl)pyrazolo[1,5-*a*]pyridin-7-yl]pyridine-2-carboxylate,
[245] 3-methyl-6-(3-(piperidine-1-carbonyl)pyrazolo[1,5-*a*]pyridin-7-yl)quinazolin-4(3H)-one,
[246] 2-methyl-6-(3-(piperidine-1-carbonyl)pyrazolo[1,5-*a*]pyridin-7-yl)-[1,2,4]triazolo[4,3-*a*]pyridin-3(2*H*)-one,
[247] 3-[3-fluoro-5-[3-(piperidine-1-carbonyl)pyrazolo[1,5-*a*]pyridin-7-yl]-2-pyridyl]-4*H*-1,2,4-oxadiazol-5-one,
[248] [7-[6-(azetidine-1-carbonyl)-5-fluoro-3-pyridyl]pyrazolo[1,5-*a*]pyridin-3-yl]-(1-piperidyl)methanone,
[249] [7-[5-fluoro-6-(morpholine-4-carbonyl)-3-pyridyl]pyrazolo[1,5-*a*]pyridin-3-yl]-(1-piperidyl)methanone,
[250] 3-fluoro-*N*-methyl-5-[3-(piperidine-1-carbonyl)pyrazolo[1,5-*a*]pyridin-7-yl]pyridine-2-carboxamide,
[251] 3-methyl-7-(3-(piperidine-1-carbonyl)pyrazolo[1,5-*a*]pyridin-7-yl)quinazolin-4(3H)-one,
[252] 2-methyl-7-(3-(piperidine-1-carbonyl)pyrazolo[1,5-*a*]pyridin-7-yl)-[1,2,4]triazolo[4,3-*a*]pyridin-3(2*H*)-one,
[253] 3-(3-(piperidine-1-carbonyl)pyrazolo[1,5-*a*]pyridin-7-yl)benzaldehyde oxime,
[254] 2-fluoro-5-[3-(piperidine-1-carbonyl)pyrazolo[1,5-*a*]pyridin-7-yl]benzonitrile,
[255] 3-methyl-6-(3-(piperidine-1-carbonyl)pyrazolo[1,5-*a*]pyridin-7-yl)pyrido[2,3-d]pyrimidin-4(3H)-one,
[256] 3-methyl-7-(3-(piperidine-1-carbonyl)pyrazolo[1,5-*a*]pyridin-7-yl)pyrido[3,2-d]pyrimidin-4(3H)-one,
[257] (7-(2-(3-fluoroazetidin-1-yl)pyridin-4-yl)pyrazolo[1,5-*a*]pyridin-3-yl)(piperidin-1-yl)methanone,
[258] 3-chloro-5-[3-(piperidine-1-carbonyl)pyrazolo[1,5-*a*]pyridin-7-yl]pyridine-2-carboxylic acid,
[259] [7-(3-amino-1,2-benzoxazol-5-yl)pyrazolo[1,5-*a*]pyridin-3-yl]-(1-piperidyl)methanone,
[260] [7-(3-amino-1*H*-indazol-5-yl)pyrazolo[1,5-*a*]pyridin-3-yl]-(1-piperidyl)methanone,
[261] [7-(3-amino-1-methyl-indazol-5-yl)pyrazolo[1,5-*a*]pyridin-3-yl]-(1-piperidyl)methanone,
[262] 3-chloro-*N*-methyl-5-[3-(piperidine-1-carbonyl)pyrazolo[1,5-*a*]pyridin-7-yl]pyridine-2-carboxamide,
[263] [7-[5-fluoro-6-(5-methyl-1,2,4-oxadiazol-3-yl)-3-pyridyl]pyrazolo[1,5-*a*]pyridin-3-yl]-(1-piperidyl)methanone,
[264] (7-(2-chlorophenyl)pyrazolo[1,5-*a*]pyridin-3-yl)(piperidin-1-yl)methanone,
[265] (7-(5-bromo-2-fluoropyridin-3-yl)pyrazolo[1,5-*a*]pyridin-3-yl)(piperidin-1-yl)methanone,
[266] (7-(2,6-difluoropyridin-3-yl)pyrazolo[1,5-*a*]pyridin-3-yl)(piperidin-1-yl)methanone,
[267] (7-(2-chloropyrimidin-5-yl)pyrazolo[1,5-*a*]pyridin-3-yl)(piperidin-1-yl)methanone,
[268] 2-methyl-5-(3-(piperidine-1-carbonyl)pyrazolo[1,5-*a*]pyridin-7-yl)benzo[*d*]isoxazol-3(2*H*)-one,
[269] (7-(3-methoxybenzo[*d*]isoxazol-5-yl)pyrazolo[1,5-*a*]pyridin-3-yl)(piperidin-1-yl)methanone,

[270] 2-(dimethylamino)-5-(3-(piperidine-1-carbonyl)pyrazolo[1,5-*a*]pyridin-7-yl)nicotinonitrile,
[271] (7-(6-fluoropyridin-2-yl)pyrazolo[1,5-*a*]pyridin-3-yl)(piperidin-1-yl)methanone,
[272] (7-(2-chloro-6-(trifluoromethyl)pyridin-3-yl)pyrazolo[1,5-*a*]pyridin-3-yl)(piperidin-1-yl)methanone,
[273] (7-(2,6-difluoropyridin-4-yl)pyrazolo[1,5-*a*]pyridin-3-yl)(piperidin-1-yl)methanone,
*[274]* *N*-(4-(3-(piperidine-1-carbonyl)pyrazolo[1,5-*a*]pyridin-7-yl)phenyl)-2-(pyridin-3-yl)acetamide,
[275] 3-[2-fluoro-5-[3-(piperidine-1-carbonyl)pyrazolo[1,5-*a*]pyridin-7-yl]phenyl]-4*H*-1,2,4-oxadiazol-5-one,
[276] [7-[4-fluoro-3-(5-methyl-1,2,4-oxadiazol-3-yl)phenyl]pyrazolo[1,5-*a*]pyridin-3-yl]-(1-piperidyl)methanone,
[277] [7-[2-(azetidine-1-carbonyl)pyrimidin-5-yl]pyrazolo[1,5-*a*]pyridin-3-yl]-(1-piperidyl)methanone,
[278] *N*-(2,6-difluoro-4-(3-(piperidine-1-carbonyl)pyrazolo[1,5-*a*]pyridin-7-yl)phenyl)-2-(pyridin-3-yl)acetamide,
[279] (7-(3-chloro-5-fluorophenyl)pyrazolo[1,5-*a*]pyridin-3-yl)(piperidin-1-yl)methanone,
[280] 5,6-dimethyl-3-(3-(piperidine-1-carbonyl)pyrazolo[1,5-*a*]pyridin-7-yl)-5,6-dihydro-7*H*-pyrrolo[3,4-*b*]pyridin-7-one,
[281] (7-(6-chloro-5-fluoropyridin-3-yl)pyrazolo[1,5-*a*]pyridin-3-yl)(piperidin-1-yl)methanone,
[282] (7-(2,6-dichloropyridin-4-yl)pyrazolo[1,5-*a*]pyridin-3-yl)(piperidin-1-yl)methanone,
[283] (7-(5-chloro-2-fluoropyridin-3-yl)pyrazolo[1,5-*a*]pyridin-3-yl)(piperidin-1-yl)methanone,
[284] (7-(5-fluoropyridin-3-yl)pyrazolo[1,5-*a*]pyridin-3-yl)(piperidin-1-yl)methanone,
[285] 3-fluoro-4-[3-(piperidine-1-carbonyl)pyrazolo[1,5-*a*]pyridin-7-yl]benzonitrile,
[286] [7-[6-(azetidine-1-carbonyl)-5-chloro-3-pyridyl]pyrazolo[1,5-*a*]pyridin-3-yl]-(1-piperidyl)methanone,
*[287]* *N*-(5-(3-(piperidine-1-carbonyl)pyrazolo[1,5-*a*]pyridin-7-yl)pyridin-2-yl)nicotinamide,
[288] (7-(5,6-difluoropyridin-3-yl)pyrazolo[1,5-*a*]pyridin-3-yl)(piperidin-1-yl)methanone,
[289] (7-(5-chloro-6-fluoropyridin-3-yl)pyrazolo[1,5-*a*]pyridin-3-yl)(piperidin-1-yl)methanone,
[290] 2,6-difluoro-4-(3-(piperidine-1-carbonyl)pyrazolo[1,5-*a*]pyridin-7-yl)benzonitrile,
[291] 3-fluoro-5-(3-(piperidine-1-carbonyl)pyrazolo[1,5-*a*]pyridin-7-yl)benzonitrile,
[292] (7-(4-bromophenyl)pyrazolo[1,5-*a*]pyridin-3-yl)(piperidin-1-yl)methanone,
[293] 5-[3-(piperidine-1-carbonyl)pyrazolo[1,5-*a*]pyridin-7-yl]pyrimidine-2-carbonitrile,
[294] [7-(2-hydroxypyrimidin-5-yl)pyrazolo[1,5-*a*]pyridin-3-yl]-(1-piperidyl)methanone,
[295] 3-[3-fluoro-4-[3-(piperidine-1-carbonyl)pyrazolo[1,5-*a*]pyridin-7-yl]phenyl]-4H-1,2,4-oxadiazol-5-one,
[296] [7-[2-fluoro-4-(5-methyl-1,2,4-oxadiazol-3-yl)phenyl]pyrazolo[1,5-*a*]pyridin-3-yl]-(1-piperidyl)methanone,
[297] 3-(5-(3-(piperidine-1-carbonyl)pyrazolo[1,5-*a*]pyridin-7-yl)pyridin-3-yl)-1,2,4-oxadiazol-5(4H)-one,
[298] (7-(5-chloro-2-fluoropyridin-4-yl)pyrazolo[1,5-*a*]pyridin-3-yl)(piperidin-1-yl)methanone,
[299] 3-(3-fluoro-5-(3-(piperidine-1-carbonyl)pyrazolo[1,5-*a*]pyridin-7-yl)phenyl)-1,2,4-oxadiazol-5(4H)-one,
[300] [7-[4-(2-methylpyrazol-3-yl)phenyl]pyrazolo[1,5-*a*]pyridin-3-yl]-(1-piperidyl)methanone,
[301] *N*-(3-chloro-5-(3-(piperidine-1-carbonyl)pyrazolo[1,5-*a*]pyridin-7-yl)pyridin-2-yl)nicotinamide,
[302] [7-[4-(3-furyl)phenyl]pyrazolo[1,5-*a*]pyridin-3-yl]-(1-piperidyl)methanone,
[303] [7-[3-fluoro-4-(5-methyl-1,2,4-oxadiazol-3-yl)phenyl]pyrazolo[1,5-*a*]pyridin-3-yl]-(1-piperidyl)methanone,
[304] 3-[2-fluoro-4-[3-(piperidine-1-carbonyl)pyrazolo[1,5-*a*]pyridin-7-yl]phenyl]-4*H*-1,2,4-oxadiazol-5-one,
[305] 5-[4-[3-(piperidine-1-carbonyl)pyrazolo[1,5-*a*]pyridin-7-yl]phenyl]pyrimidine-2-carbonitrile,
[306] [7-[4-(2-hydroxypyrimidin-5-yl)phenyl]pyrazolo[1,5-*a*]pyridin-3-yl]-(1-piperidyl)methanone,
[307] 5-[4-[3-(piperidine-1-carbonyl)pyrazolo[1,5-*a*]pyridin-7-yl]phenyl]pyrimidine-2-carboxamide,
[308] 3-(5-(3-(piperidine-1-carbonyl)pyrazolo[1,5-*a*]pyridin-7-yl)pyrimidin-2-yl)oxazolidin-2-one,
[309] [7-(3-amino-1*H*-indazol-6-yl)pyrazolo[1,5-*a*]pyridin-3-yl]-(1-piperidyl)methanone,
[310] [7-(3-amino-1-methyl-indazol-6-yl)pyrazolo[1,5-*a*]pyridin-3-yl]-(1-piperidyl)methanone,
[311] *N*-(3-chloro-5-(3-(piperidine-1-carbonyl)pyrazolo[1,5-*a*]pyridin-7-yl)pyridin-2-yl)-2-(pyridin-3-yl)acetamide,
[312] 3-nitro-5-(3-(piperidine-1-carbonyl)pyrazolo[1,5-*a*]pyridin-7-yl)benzonitrile,
[313] (7-(6-bromopyridin-3-yl)pyrazolo[1,5-*a*]pyridin-3-yl)(piperidin-1-yl)methanone,
[314] (7-(6-chloropyridin-3-yl)pyrazolo[1,5-*a*]pyridin-3-yl)(piperidin-1-yl)methanone,
[315] (7-(6-chloro-2-fluoropyridin-3-yl)pyrazolo[1,5-*a*]pyridin-3-yl)(piperidin-1-yl)methanone,
[316] 3-(5-(3-(piperidine-1-carbonyl)pyrazolo[1,5-*a*]pyridin-7-yl)pyridin-2-yl)oxazolidin-2-one,
[317] [7-[2-(5-methyl-1,2,4-oxadiazol-3-yl)pyrimidin-5-yl]pyrazolo[1,5-*a*]pyridin-3-yl]-(1-piperidyl)methanone,
[318] (7-(5-nitropyridin-3-yl)pyrazolo[1,5-*a*]pyridin-3-yl)(piperidin-1-yl)methanone,
[319] (7-(5-(5-methyl-1,2,4-oxadiazol-3-yl)pyridin-3-yl)pyrazolo[1,5-*a*]pyridin-3-yl)(piperidin-1-yl)methanone,
[320] [7-(3-amino-1,2-benzoxazol-6-yl)pyrazolo[1,5-*a*]pyridin-3-yl]-(1-piperidyl)methanone,
[321] 3-[5-[3-(piperidine-1-carbonyl)pyrazolo[1,5-*a*]pyridin-7-yl]pyrimidin-2-yl]-4H-1,2,4-oxadiazol-5-one,
[322] (7-(3-fluoro-5-(5-methyl-1,2,4-oxadiazol-3-yl)phenyl)pyrazolo[1,5-*a*]pyridin-3-yl)(piperidin-1-yl)methanone,
[323] 3-(3-fluoro-5-(3-(piperidine-1-carbonyl)pyrazolo[1,5-*a*]pyridin-7-yl)pyridin-2-yl)oxazolidin-2-one,
[324] [7-[4-(3,5-dimethylisoxazol-4-yl)phenyl]pyrazolo[1,5-*a*]pyridin-3-yl]-(1-piperidyl)methanone,

[325] (7-(2-fluoro-5-(1-methyl-1*H*-pyrazol-5-yl)pyridin-3-yl)pyrazolo[1,5-a]pyridin-3-yl)(piperidin-1-yl)methanone,
[326] *N*-(5-(3-(piperidine-1-carbonyl)pyrazolo[1,5-*a*]pyridin-7-yl)pyridin-3-yl)nicotinamide,
*[327]* N-(5-(3-(piperidine-1-carbonyl)pyrazolo[1,5-*a*]pyridin-7-yl)pyridin-3-yl)-2-(pyridin-3-yl)acetamide,
[328] (7-(3,5-difluoro-4-(5-methyl-1,2,4-oxadiazol-3-yl)phenyl)pyrazolo[1,5-a]pyridin-3-yl)(piperidin-1-yl)methanone,
[329] 3-(3-nitro-5-(3-(piperidine-1-carbonyl)pyrazolo[1,5-*a*]pyridin-7-yl)phenyl)-1,2,4-oxadiazol-5(4H)-one,
[330] (7-(3-(5-methyl-1,2,4-oxadiazol-3-yl)-5-nitrophenyl)pyrazolo[1,5-*a*]pyridin-3-yl)(piperidin-1-yl)methanone,
[331] 3-(2,6-difluoro-4-(3-(piperidine-1-carbonyl)pyrazolo[1,5-*a*]pyridin-7-yl)phenyl)-1,2,4-oxadiazol-5(4H)-one,
[332] piperidin-1-yl(7-(6-((2-(pyridin-3-yl)ethyl)amino)pyridin-3-yl)pyrazolo[1,5-*a*]pyridin-3-yl)methanone,
[333] 1,2-dimethyl-*N*-(5-(3-(piperidine-1-carbonyl)pyrazolo[1,5-*a*]pyridin-7-yl)pyridin-3-yl)-1H-imidazole-5-carboxamide,
[334] *N*-(5-(3-(piperidine-1-carbonyl)pyrazolo[1,5-*a*]pyridin-7-yl)pyridin-3-yl)pyrimidine-5-carboxamide,
[335] 5-fluoro-*N*-(5-(3-(piperidine-1-carbonyl)pyrazolo[1,5-*a*]pyridin-7-yl)pyridin-3-yl)nicotinamide,
[336] (7-(6-fluoropyridin-3-yl)pyrazolo[1,5-*a*]pyridin-3-yl)(3-methylpiperidin-1-yl)methanone,
[337] (3-ethylpiperidin-1-yl)(7-(6-fluoropyridin-3-yl)pyrazolo[1,5-*a*]pyridin-3-yl)methanone,
[338] (7-(6-fluoropyridin-3-yl)pyrazolo[1,5-*a*]pyridin-3-yl)(3-(trifluoromethyl)piperidin-1-yl)methanone,
[339] 3-(5-(3-(3-(trifluoromethyl)piperidine-1-carbonyl)pyrazolo[1,5-*a*]pyridin-7-yl)pyridin-2-yl)oxazolidin-2-one,
[340] *N*-(2-nitrobenzyl)-N-(4-(3-(piperidine-1-carbonyl)pyrazolo[1,5-*a*]pyridin-7-yl)phenyl)nicotinamide,
[341] (7-(3-fluoro-5-(1,2,4-oxadiazol-3-yl)phenyl)pyrazolo[1,5-*a*]pyridin-3-yl)(piperidin-1-yl)methanone,
[342] (7-(3-nitro-5-(1,2,4-oxadiazol-3-yl)phenyl)pyrazolo[1,5-*a*]pyridin-3-yl)(piperidin-1-yl)methanone,
[343] (7-(3-amino-5-(5-methyl-1,2,4-oxadiazol-3-yl)phenyl)pyrazolo[1,5-*a*]pyridin-3-yl)(piperidin-1-yl)methanone,
[344] (7-(6-(4-amino-]1*H*-pyrazol-1-yl)pyridin-3-yl)pyrazolo[1,5-*a*]pyridin-3-yl)(piperidin-1-yl)methanone,
[345] (7-(3-(3-hydroxyazetidin-1-yl)-5-(isoxazol-4-yl)phenyl)pyrazolo[1,5-*a*]pyridin-3-yl)(piperidin-1-yl)methanone,
[346] 1-piperidyl-[7-[4-(1*H*-pyrazol-4-yl)phenyl]pyrazolo[1,5-*a*]pyridin-3-yl]methanone,
[347] 1-piperidyl-[7-[4-(3-thienyl)phenyl]pyrazolo[1,5-*a*]pyridin-3-yl]methanone,
[348] [7-[4-(5-methyl-1,3,4-oxadiazol-2-yl)phenyl]pyrazolo[1,5-*a*]pyridin-3-yl]-(1-piperidyl)methanone,
[349] 5-[4-[3-(piperidine-1-carbonyl)pyrazolo[1,5-*a*]pyridin-7-yl]phenyl]-3*H*-1,3,4-oxadiazol-2-one,
[350] [7-[4-(1,3,4-oxadiazol-2-yl)phenyl]pyrazolo[1,5-*a*]pyridin-3-yl]-(1-piperidyl)methanone,
[351] [7-[4-(5-methylthiazol-2-yl)phenyl]pyrazolo[1,5-*a*]pyridin-3-yl]-(1-piperidyl)methanone,
[352] [7-[4-(1,2,4-oxadiazol-3-yl)phenyl]pyrazolo[1,5-*a*]pyridin-3-yl]-(1-piperidyl)methanone,
[353] 3-(5-(3-(3-methylpiperidine-1-carbonyl)pyrazolo[1,5-*a*]pyridin-7-yl)pyridin-2-yl)oxazolidin-2-one,
[354] 3-(5-(3-(3-ethylpiperidine-1-carbonyl)pyrazolo[1,5-*a*]pyridin-7-yl)pyridin-2-yl)oxazolidin-2-one,
[355] (7-(6-(4-nitro-1*H*-pyrazol-1-yl)pyridin-3-yl)pyrazolo[1,5-*a*]pyridin-3-yl)(piperidin-1-yl)methanone,
[356] (7-(2-(4-amino-1*H*-pyrazol-1-yl)-6-bromopyridin-4-yl)pyrazolo[1,5-*a*]pyridin-3-yl)(piperidin-1-yl)methanone,
[357] (7-(2-(3-hydroxyazetidin-1-yl)-6-(1-methyl-1H-pyrazol-5-yl)pyridin-4-yl)pyrazolo[1,5-*a*]pyridin-3-yl)(piperidin-1-yl)methanone,
[358] 7-[4-(1,2-dimethylimidazol-4-yl)phenyl]pyrazolo[1,5-*a*]pyridin-3-yl]-(1-piperidyl)methanone,
[359] [7-[4-(5-amino-1,2,4-oxadiazol-3-yl)phenyl]pyrazolo[1,5-*a*]pyridin-3-yl]-(1-piperidyl)methanone,
[360] 5-[5-[3-(piperidine-1-carbonyl)pyrazolo[1,5-*a*]pyridin-7-yl]-2-pyridyl]-3*H*-1,3,4-oxadiazol-2-one,
[361] 1-piperidyl-[7-[4-[5-(trifluoromethyl)-1,2,4-oxadiazol-3-yl]phenyl]pyrazolo[1,5-*a*]pyridin-3-yl]methanone,
[362] *N*-(3-fluoro-5-(3-(piperidine-1-carbonyl)pyrazolo[1,5-*a*]pyridin-7-yl)pyridin-2-yl)-2-(pyridin-3-yl)acetamide,
[363] (7-(5-bromo-2-(3-(hydroxymethyl)azetidin-1-yl)pyridin-3-yl)pyrazolo[1,5-*a*]pyridin-3-yl)(piperidin-1-yl)methanone,
[364] [7-[6-(5-methyl-1,3,4-oxadiazol-2-yl)-3-pyridyl]pyrazolo[1,5-*a*]pyridin-3-yl]-(1-piperidyl)methanone,
[365] 1-piperidyl-[7-[6-(2-thioxo-3*H*-1,3,4-oxadiazol-5-yl)-3-pyridyl]pyrazolo[1,5-*a*]pyridin-3-yl]methanone,
[366] (7-(2-(isoxazol-4-yl)-6-morpholinopyridin-4-yl)pyrazolo[1,5-*a*]pyridin-3-yl)(piperidin-1-yl)methanone,
[367] 3-(6-(1-methyl-1H-pyrazol-5-yl)-4-(3-(piperidine-1-carbonyl)pyrazolo[1,5-*a*]pyridin-7-yl)pyridin-2-yl)oxazolidin-2-one,
[368] 3-(6-(3,5-dimethylisoxazol-4-yl)-4-(3-(piperidine-1-carbonyl)pyrazolo[1,5-*a*]pyridin-7-yl)pyridin-2-yl)oxazolidin-2-one,

[369] (7-(2-(1-methyl-1*H*-pyrazol-5-yl)-6-morpholinopyridin-4-yl)pyrazolo[1,5-*a*]pyridin-3-yl)(piperidin-1-yl) methanone,
[370] 3-(5'-fluoro-4-(3-(piperidine-1-carbonyl)pyrazolo[1,5-*a*]pyridin-7-yl)-[2,3'-bipyridin]-6-yl)oxazolidin-2-one,
[371] (7-(2-(3,5-dimethylisoxazol-4-yl)-6-morpholinopyridin-4-yl)pyrazolo[1,5-*a*]pyridin-3-yl)(piperidin-1-yl) methanone,
[372] 3-(6-(2-methylpyrimidin-5-yl)-4-(3-(piperidine-1-carbonyl)pyrazolo[1,5-*a*]pyridin-7-yl)pyridin-2-yl)oxazolidin-2-one,
[373] 5-[3-fluoro-5-[3-(piperidine-1-carbonyl)pyrazolo[1,5-*a*]pyridin-7-yl]-2-pyridyl]-3*H*-1,3,4-oxadiazol-2-one,
[374] (7-(5-(1,2,4-oxadiazol-3-yl)pyridin-3-yl)pyrazolo[1,5-*a*]pyridin-3-yl)(piperidin-1-yl)methanone,
[375] (7-(6-(3-(dimethylamino)azetidin-1-yl)-5'-fluoro-[2,3'-bipyridin]-4-yl)pyrazolo[1,5-*a*]pyridin-3-yl)(piperidin-1-yl)methanone,
[376] (7-(5'-fluoro-6-morpholino-[2,3'-bipyridin]-4-yl)pyrazolo[1,5-*a*]pyridin-3-yl)(piperidin-1-yl)methanone,
[377] (7-(2-(2-methylpyrimidin-5-yl)-6-morpholinopyridin-4-yl)pyrazolo[1,5-*a*]pyridin-3-yl)(piperidin-1-yl)methanone,
[378] 6-(3-(3-fluoropiperidine-1-carbonyl)pyrazolo[1,5-*a*]pyridin-7-yl)isoindolin-1-one,
[379] [7-[5-fluoro-6-(5-methyl-1,3,4-oxadiazol-2-yl)-3-pyridyl]pyrazolo[1,5-*a*]pyridin-3-yl]-(1-piperidyl)methanone,
[380] [7-[5-fluoro-6-(1,3,4-oxadiazol-2-yl)-3-pyridyl]pyrazolo[1,5-*a*]pyridin-3-yl]-(1-piperidyl)methanone,
[381] [7-[6-(3,5-dimethylisoxazol-4-yl)-5-fluoro-3-pyridyl]pyrazolo[1,5-*a*]pyridin-3-yl]-(1-piperidyl)methanone,
[382] 6-(3-(3,3-difluoropiperidine-1-carbonyl)pyrazolo[1,5-*a*]pyridin-7-yl)isoindolin-1-one,
[383] 3-(5-(3-(3-fluoropiperidine-1-carbonyl)pyrazolo[1,5-*a*]pyridin-7-yl)pyridin-2-yl)oxazolidin-2-one,
[384] 3-(5-(3-(3,3-difluoropiperidine-1-carbonyl)pyrazolo[1,5-*a*]pyridin-7-yl)pyridin-2-yl)oxazolidin-2-one,
[385] (*R*)-6-(3-(3-methylpiperidine-1-carbonyl)pyrazolo[1,5-*a*]pyridin-7-yl)isoindolin-1-one,
[386] (*S*)-6-(3-(3-methylpiperidine-1-carbonyl)pyrazolo[1,5-*a*]pyridin-7-yl)isoindolin-1-one,
[387] 6-(3-(3-methylpiperidine-1-carbonyl)pyrazolo[1,5-*a*]pyridin-7-yl)isoindolin-1-one,
[388] *N*-(5-(3-(piperidine-1-carbonyl)pyrazolo[1,5-*a*]pyridin-7-yl)pyridin-3-yl)-2-(6-(trifluoromethyl)pyridin-3-yl) acetamide,
[389] 2-phenyl-N-(5-(3-(piperidine-1-carbonyl)pyrazolo[1,5-*a*]pyridin-7-yl)pyridin-3-yl)acetamide,
[390] 6-(3-(2-azaspiro[3.3]heptane-2-carbonyl)pyrazolo[1,5-*a*]pyridin-7-yl)isoindolin-1-one,
[391] 6-(3-(6-azaspiro[3.4]octane-6-carbonyl)pyrazolo[1,5-*a*]pyridin-7-yl)isoindolin-1-one,
[392] 6-(3-(7-azaspiro[3.5]nonane-7-carbonyl)pyrazolo[1,5-*a*]pyridin-7-yl)isoindolin-1-one,
[393] 6-(3-(3-ethylpiperidine-1-carbonyl)pyrazolo[1,5-*a*]pyridin-7-yl)isoindolin-1-one,
[394] [7-[5-fluoro-6-[5-(trifluoromethyl)-1,3,4-oxadiazol-2-yl]-3-pyridyl]pyrazolo[1,5-*a*]pyridin-3-yl]-(1-piperidyl) methanone,
[395] (7-(1-((1,2,4-oxadiazol-3-yl)methyl)-1H-pyrazol-4-yl)pyrazolo[1,5-*a*]pyridin-3-yl)(piperidin-1-yl)methanone,
[396] 3-((4-(3-(piperidine-1-carbonyl)pyrazolo[1,5-*a*]pyridin-7-yl)-1*H*-pyrazol-1-yl)methyl)-1,2,4-oxadiazol-5(4H)-one,
[397] (7-(2-(1-((1,2,4-oxadiazol-3-yl)methyl)-1H-pyrazol-4-yl)pyridin-4-yl)pyrazolo[1,5-*a*]pyridin-3-yl)(piperidin-1-yl)methanone,
[398] 6-(3-(3-(trifluoromethyl)piperidine-1-carbonyl)pyrazolo[1,5-*a*]pyridin-7-yl)isoindolin-1-one,
[399] [7-[2-(3,5-dimethylisoxazol-4-yl)pyrimidin-5-yl]pyrazolo[1,5-*a*]pyridin-3-yl]-(1-piperidyl)methanone,
[400] 6-(3-(3-isopropylpiperidine-1-carbonyl)pyrazolo[1,5-*a*]pyridin-7-yl)isoindolin-1-one,
[401] 6-(3-(1,2,3,6-tetrahydropyridine-1-carbonyl)pyrazolo[1,5-*a*]pyridin-7-yl)isoindolin-1-one,
[402] 2-(5-fluoropyridin-3-yl)-*N*-(5-(3-(piperidine-1-carbonyl)pyrazolo[1,5-*a*]pyridin-7-yl)pyridin-3-yl)acetamide,
[403] *N*-(5-(3-(piperidine-1-carbonyl)pyrazolo[1,5-*a*]pyridin-7-yl)pyridin-3-yl)-2-(pyrimidin-5-yl)acetamide,
[404] [7-[2-[5-(difluoromethyl)-1-oxo-1,3,4-thiadiazol-2-yl]pyrimidin-5-yl]pyrazolo[1,5-*a*]pyridin-3-yl]-(1-piperidyl)methanone,
[405] 6-(3-(6-azaspiro[3.5]nonane-6-carbonyl)pyrazolo[1,5-*a*]pyridin-7-yl)isoindolin-1-one,
[406] 2-(4-chlorophenoxy)-*N*-(5-(3-(piperidine-1-carbonyl)pyrazolo[1,5-*a*]pyridin-7-yl)pyridin-3-yl)acetamide,
*[407]* *N*-[5-[3-(piperidine-1-carbonyl)pyrazolo[1,5-*a*]pyridin-7-yl]-2-pyridyl]-2-pyrimidin-5-yl-acetamide,
[408] *N*-[5-[3-(piperidine-1-carbonyl)pyrazolo[1,5-*a*]pyridin-7-yl]-2-pyridyl]-2-[6-(trifluoromethyl)-3-pyridyl] acetamide,
[409] 2-(5-fluoropyridin-3-yl)-*N*-(5-(3-(piperidine-1-carbonyl)pyrazolo[1,5-*a*]pyridin-7-yl)pyridin-2-yl)acetamide,
[410] 2-(4-chlorophenoxy)-*N*-[5-[3-(piperidine-1-carbonyl)pyrazolo[1,5-*a*]pyridin-7-yl]-2-pyridyl]acetamide,
[411] 6-(3-(6-fluoro-2-azaspiro[3.3]heptane-2-carbonyl)pyrazolo[1,5-*a*]pyridin-7-yl)isoindolin-1-one,
[412] 6-(3-(6,6-difluoro-2-azaspiro[3.3]heptane-2-carbonyl)pyrazolo[1,5-*a*]pyridin-7-yl)isoindolin-1-one, and
[413] 3-hydroxy-5-[2-(piperidine-1-carbonyl)pyrazolo[1,5-*a*]pyridin-6-yl]pyridine-2-carbonitrile.

**16.** A pharmaceutical composition for preventing or treating a 15-PGDH-related disease, comprising a heterocyclic compound, a hydrate thereof, a solvate thereof, or a pharmaceutically acceptable salt thereof of any one of claims 1 to 15, as an active ingredient.

**17.** The pharmaceutical composition of Claim 16, wherein the 15-PGDH-related disease is one or more selected from the group consisting of an inflammatory disease (inflammatory bowel disease (ulcerative colitis, Crohn's disease), chronic obstructive pulmonary disease (COPD), Behcet's disease, acute liver injury, acute kidney injury, acute lung injury, sepsis, exacerbation of asthma and lung disease, peptic ulcer (ulcer caused by NSAIDs), vasculitis syndrome, non-alcoholic fatty liver disease (NASH), atopic dermatitis, psoriasis, interstitial cystitis, prostatitis syndrome), fibrosis (pulmonary fibrosis (idiopathic pulmonary fibrosis), renal fibrosis (glomerulosclerosis), cardiac fibrosis (myocardial fibrosis), oral fibrosis, deltoid fibrosis, liver fibrosis, myelofibrosis, scleroderma), autoimmune disease (systemic lupus erythematosus, rheumatoid arthritis, autoimmune hepatitis, severe combined immunodeficiency syndrome (SCID)), ophthalmic disease (dry eye, conjunctivitis, keratitis), thrombocytopenia (drug-induced thrombocytopenia, autoimmune thrombocytopenia, idiopathic thrombocytopenia, thrombocytopenia due to viral infection), myopathy (muscular dystrophy, muscle damage), anemia (aplastic anemia, anemia of chronic disease, Fanconi anemia, beta-thalassemia or anemia of unknown cause), circulatory disease (cardiac disease (angina pectoris, heart failure, myocardial infarction), kidney disease (chronic kidney disease, renal failure), stroke, pulmonary hypertension, peripheral circulatory disorder), nerve cell death (psychiatric disorders, neurodegenerative diseases, nerve injuries), cytopenia (immune cytopenia, neutropenia, lymphopenia), osteoporosis, fracture, leukemia (acute myeloid leukemia (AML), acute lymphoblastic leukemia (ALL), chronic myeloid leukemia (CML)), lymphoma (Hodgkin's lymphoma, non-Hodgkin's lymphoma), radiation exposure toxicity, 15-PGDH expressing cancer, multiple myeloma, paroxysmal nocturnal hemoglobinuria (PNH), pure red cell aplasia, megakaryocytosis, Wiskott-Aldrich syndrome, sickle cell disease, Hurler syndrome, adrenoleukodystrophy, metachromatic leukodystrophy, myelodysplasia, Duchenne muscular dystrophy, solid tumors, chronic granulomatous disease, systemic sclerosis, scars, wounds, otologic diseases (vertigo, tinnitus, balance disorders), hair loss, skin aging, periodontal disease, diabetes, stem cell and bone marrow transplantation or organ transplantation, cervical ripening, Alzheimer's disease, and Parkinson's disease.

**18.** A pharmaceutical composition comprising a heterocyclic compound, a hydrate thereof, a solvate thereof, or a pharmaceutically acceptable salt thereof of any one of claims 1 to 15, and a pharmaceutically acceptable additive.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020230009009


### Non-patent literature cited in the description


- **BELAROUSAI, R. et al.** *Synthesis*, 2013, vol. 45, 2557-2566